# EUROPEAN PATENT SPECIFICATION

(11) **EP 1 869 032 B1**
(45) Date of publication and mention of the grant of the patent: **13.08.2008**
(21) Application number: 06726601.5
(22) Date of filing: 31.03.2006
(51) Int. Cl.: C07D 413/14, A61K 31/495, A61P 35/00

(54) **PYRIMIDINE DERIVATIVES FOR USE AS ANTICANCER AGENTS**
PYRIMIDINDERIVATE ZUR VERWENDUNG ALS ANTIKREBSMITTEL
DERIVES DE LA PYRIMIDINE DESTINES A UNE UTILISATION EN TANT QU AGENTS ANTICANCEREUX

(30) Priority: 05.04.2005 GB 0506883; 21.12.2005 GB 0525953
(43) Date of publication of application: 26.12.2007
(73) Proprietor: AstraZeneca AB, 151 85 Södertälje (SE)
(72) Inventor: THOMAS, Andrew Peter, Alderley Park, Macclesfield, Cheshire SK10 4TG (GB); NOWAK, Thorsten, Alderley Park, Macclesfield, Cheshire SK10 4TG (GB)
(86) International application number: PCT/GB2006/001195
(87) International publication number: WO 2006/106307

(56) References cited:
- EP-A- 1 317 447
- EP-A- 1 345 925
- WO-A-20/04048365
- WO-A-20/05040159

## Description

The invention concerns certain novel pyrimidine derivatives, or pharmaceutically-acceptable salts thereof, which possess anti-tumour activity and are accordingly useful in the manufacture of a medicament for use in the treatment of the human or animal body. The invention also concerns processes for the manufacture of the pyrimidine derivatives, pharmaceutical compositions containing them and their use in therapeutic methods, for example in the manufacture of medicaments for use in the prevention or treatment of solid tumour disease in a warm-blooded animal such as man.

The insulin-like growth factor (IGF) axis consists of ligands, receptors, binding proteins and proteases. The two ligands, IGF-I and IGF-II, are mitogenic peptides that signal through interaction with the type 1 insulin-like growth factor receptor (IGF-1R), a hetero-tetrameric cell surface receptor. Binding of either ligand stimulates activation of a tyrosine kinase domain in the intracellular region of the β-chain and results in phosphorylation of several tyrosine residues resulting in the recruitment and activation of various signalling molecules. The intracellular domain has been shown to transmit signals for mitogenesis, survival, transformation, and differentiation in cells. The structure and function of the IGF-1R has been reviewed by Adams et al (Cellular and Molecular Life Science, 57, 1050-1093, 2000). The IGF-IIR (also known as mannose 6-phosphate receptor) has no such kinase domain and does not signal mitogenesis but may act to regulate ligand availability at the cell surface, counteracting the effect of the IGF-1R. The IGF binding proteins (IGFBP) control availability of circulating IGF and release of IGF from these can be mediated by proteolytic cleavage. These other components of the IGF axis have been reviewed by Collett-Solberg and Cohen (Endocrine, 12, 121-136, 2000).

There is considerable evidence linking IGF signalling with cellular transformation and the onset and progression of tumours. IGF has been identified as the major survival factor that protects from oncogene induced cell death (Harrington et al, EMBO J, 13, 3286-3295, 1994). Cells lacking IGF-1R have been shown to be refractory to transformation by several different oncogenes (including SV40T antigen and ras) that efficiently transform corresponding wild-type cells (Sell et al., Mol. Cell Biol., 14, 3604-12, 1994). Upregulation of components of the IGF axis has been described in various tumour cell lines and tissues, particularly tumours of the breast (Surmacz, Journal of Mammary Gland Biology & Neoplasia, 5, 95-105, 2000), prostate (Djavan et al, World J. Urol., 19, 225-233, 2001, and O'Brien et al, Urology, 58, 1-7, 2001) and colon (Guo et al, Gastroenterology, 102, 1101-1108, 1992). Conversely, IGF-IIR has been implicated as a tumour suppressor and is deleted in some cancers (DaCosta et al, Journal of Mammary Gland Biology & Neoplasia, 5, 85-94, 2000). There are a growing number of epidemiological studies linking increased circulating IGF (or increased ratio of IGF-1 to IGFBP3) with cancer risk (Yu and Rohan, J. Natl. Cancer Inst., 92, 1472-1489, 2000). Transgenic mouse models also implicate IGF signalling in the onset of tumour cell proliferation (Lamm and Christofori, Cancer Res. 58, 801-807, 1998, Foster et al, Cancer Metas. Rev., 17, 317-324, 1998, and DiGiovanni et al, Proc. Natl. Acad. Sci., 97, 3455-3460, 2000).

Several in *vitro* and *in vivo* strategies have provided the proof of principal that inhibition of IGF-1R signalling reverses the transformed phenotype and inhibits tumour cell growth. These include neutralizing antibodies (Kalebic et al Cancer Res., 54, 5531-5534, 1994), antisense oligonucleotides (Resnicoff et al, Cancer Res., 54, 2218-2222, 1994), triple-helix forming oligonucleotides (Rinninsland et al, Proc. Natl. Acad. Sci., 94, 5854-585.9, 1997), antisense mRNA (Nakamura et al, Cancer Res., 60, 760-765, 2000) and dominant negative receptors (D'Ambrosio et al., Cancer Res., 56, 4013-4020, 1996). Antisense oligonucleotides have shown that inhibition of IGF-1R expression results in induction of apoptosis in cells *in vivo* (Resnicoff et al, Cancer Res., 55, 2463-2469, 1995) and have been taken into man (Resnicoff et al, Proc. Amer. Assoc. Cancer Res., 40 Abs 4816, 1999). However, none of these approaches is particularly attractive for the treatment of major solid tumour disease.

Since increased IGF signalling is implicated in the growth and survival of tumour cells, and blocking IGF-1R function can reverse this, inhibition of the IGF-1R tyrosine kinase domain is an appropriate therapy by which to treat cancer. *In vitro* and *in vivo* studies with the use of dominant-negative IGF-1R variants support this. In particular, a point mutation in the ATP binding site which blocks receptor tyrosine kinase activity has proved effective in preventing tumour cell growth (Kulik et al, Mol. Cell. Biol., 17, 1595-1606, 1997). Several pieces of evidence imply that normal cells are less susceptible to apoptosis caused by inhibition of IGF signalling, indicating that a therapeutic margin is possible with such treatment (Baserga, Trends Biotechnol., 14, 150-2, 1996).

There are few reports of selective IGF-1R tyrosine kinase inhibitors. Parrizas *et al.* described tyrphostins that had some efficacy *in vitro and in vivo* (Parrizas et al., Endocrinology, 138:1427-33 (1997)). These compounds were of modest potency and selectivity over the insulin receptor. Telik Inc. have described heteroaryl-aryl ureas which have selectivity over insulin receptors but potency against tumour cells *in vitro* is still modest (Published PCT Patent Application No. WO 00/35455). Novartis have disclosed a pyrazolopyrimidine compound (known as NVP-AEW541), which is reported to inhibit IGF-1R tyrosine kinase (Garcia-Echeverria et al., Cancer Cell, 5:231-39 (2004)). Axelar have described podophyllotoxin derivatives as specific IGFR tyrosine kinase inhibitors (Vasilcanu et al., Oncogene, 23: 7854-62 (2004)) and Aventis have described cyclic urea derivatives and their use as IGF-1R tyrosine kinase inhibitors (WO 2004/070050).

Additionally, several anti-IGFR antibodies are reported to block receptor signalling and show inhibition of tumour growth in animal models (Cohen et al., Clin. Canc. Res., 11: 2063-73 (2005); Burtrum et al., Canc. Res., 63: 8912-21 (2003); Goetsch et al., Int. J. Cancer, 113: 316-28 (2005) and Maloney et al., Canc. Res., 63: 5073-83 (2003)).

Pyrimidine derivatives substituted at the 2- and 4- positions by a substituted amino group having IGF-1R tyrosine kinase inhibitory activity are described in WO 03/048133. Compounds in which the nitrogen atom of the amino substituent forms part of a heterocyclic ring are not disclosed.

EP1345925 discloses that certain pyrazolyl-amino substituted pyrimidine derivatives have protein kinase inhibitory activity, especially as inhibitors of Aurora-2 and glycogen synthase kinase-3 (GSK-3), and are useful for treating diseases such as cancer, diabetes and Alzheimer's disease. The compounds disclosed have a substituted amino substituent at the 2-position of the pyrimidine ring but again there is no disclosure of compounds in which the nitrogen atom of the amino substituent forms part of a heterocyclic ring.

Pyrazolyl-amino substituted pyrimidine derivatives having Aurora-2 and glycogen synthase kinase-3 (GSK-3) inhibitory activity in which the 2-position of the pyrimidine ring is substituted by an N-linked heterocyclic ring are disclosed generically in WO 02/22601, WO 02/22602, WO 02/22603, WO 02/22604, WO 02/22605, WO 02/22606, WO 02/22607 and WO 02/22608. There is no disclosure of a compound in which the N-linked heterocyclic ring is itself substituted by an isoxazolyl group, which isoxazolyl group is substituted by a substituted 5- or 6-membered heteroaromatic ring.

WO 01/60816 discloses that certain substituted pyrimidine derivatives have protein kinase inhibitory activity. There is no disclosure in WO 01/60816 of pyrimidine derivatives having a pyrazolyl-amino substituent at the 4-position on the pyrimidine ring and a N-linked pyrrolidine ring at the 2-position on the pyrimidine ring.

Substituted pyrimidine derivatives are also described in WO 00/39101, WO 2004/056786, WO 2004/080980 and WO 2004/048365, but none of these documents describe pyrimidine derivatives having a N-linked pyrrolidine ring at the 2-position on the pyrimidine ring, which pyrrolidine ring is itself substituted by an isoxazolyl group, which isoxazolyl group is substituted by a substituted 5- or 6-membered heteroaromatic ring.

WO 2005/040159 (International patent application number PCT/GB2004/004307) discloses certain pyrimidine derivatives and their use in modulating insulin-like growth factor 1 receptor activity.

We have now found that a certain select group of pyrimidine compounds that contain a 2-(isoxazol-5-yl)pyrrolidin-1-yl group at the 2-position on the pyrimidine ring, wherein isoxazole ring is substituted by a substituted 5- or 6-membered heteroaromatic ring, possess potent anti-tumour activity. Without wishing to imply that the compounds disclosed in the present invention possess pharmacological activity only by virtue of an effect on a single biological process, it is believed that the compounds provide an anti-tumour effect by way of inhibition of IGF-1R tyrosine kinase activity.

According to a first aspect of the invention, there is provided a compound of formula (I): 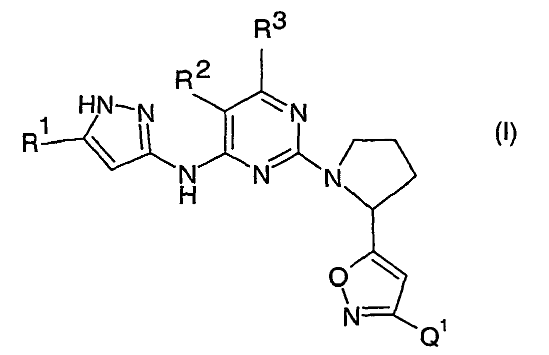 wherein:
**R¹** is selected from methyl, ethyl, isopropyl and cyclopropyl;
**R²** is selected from hydrogen and halogeno;
**R³** is selected from hydrogen, hydroxy and halogeno, or from a (C1-C6)alkyl, (C2-C6)alkenyl, (C2-C6)alkynyl, (C1-C6)alkoxy, (C1-C6)alkylcarbonyl, (C1-C6)alkoxycarbonyl, amino, (C1-C6)alkylamino, di-[(C1-C6)alkyl]amino, (C3-C8)cycloalkylamino, carbamoyl, (C1-C6)alkylcarbamoyl, di-[(C1-C6)alkyl]carbamoyl, -C(O)R^{3b}, -NHR^{3b}, -SR^{3a} or -N(R^{3c})C(O)R^{3a} group, wherein R^{3a} is selected from a (C1-C6)alkyl or (C1-C6)alkoxy group, R^{3b} is a saturated monocyclic 4-, 5- or 6-membered heterocyclic ring comprising at least one ring heteroatom selected from nitrogen, oxygen and sulfur and R^{3c} is selected from hydrogen and (C1-C6)alkyl,
or R³ is a saturated monocyclic 5- or 6-membered heterocyclic ring comprising at least one ring heteroatom selected from nitrogen, oxygen and sulfur,
each of which groups or rings within R³ may be optionally substituted by one or more substituents independently selected from (C1-C6)alkyl, (C1-C6)alkoxy, (C1-C6)alkoxy(C1-C6)alkyl, (C1-C6)alkoxy(C1-C6)alkoxy, halogeno, hydroxy, trifluoromethyl, tri-[(C1-C4)alkyl]silyl, cyano, amino, (C1-C6)alkylamino, di-[(C1-C6)alkyl]amino, amino(C1-C6)alkyl, (C1-C6)alkylamino(C1-C6)alkyl, di-[(C1-C6)alkyl]amino(C1-C6)alkyl, (C1-C6)alkoxycarbonyl, carbamoyl, (C1-C6)alkylcarbamoyl, di-[(C1-C6)alkyl]carbamoyl, (C1-C6)alkylthio, (C1-C6)alkylsulfonyl, (C1-C6)alkylsulfinyl, (C1-C6)alkanoyl, an alkanoylamino group -N(R^{3d})C(O)R^{3e} wherein R^{3d} is selected from hydrogen and (C1-C6)alkyl and R^{3e} is selected from a (C1-C6)alkyl or (C1-C6)alkoxy group, or a saturated monocyclic 3-, 4-, 5-, 6- or 7-membered ring, which ring may optionally comprise one or more heteroatoms selected from nitrogen, oxygen and sulfur, any of which substituents may be optionally substituted by one or more (C1-C4)alkyl, hydroxy or cyano groups;
**Q¹** is a 5- or 6-membered heteroaromatic ring comprising at least one ring heteroatom selected from nitrogen, oxygen and sulfur,
and wherein Q¹ is substituted by one or more substituents independently selected from (C1-C6)alkyl and (C1-C6)alkoxy (either of which (C1-C6)alkyl and (C1-C6)alkoxy groups may be optionally substituted by one or more substituents independently selected from (C1-C4)alkoxy, halogeno, amino, hydroxy and trifluoromethyl), oxo, halogeno, nitro, cyano, -NR⁴R⁵, carboxy, hydroxy, (C2-C6)alkenyl, (C3-C8)cycloalkyl, (C1-C6)alkoxycarbonyl, (C1-C6)alkylcarbonyl, (C2-C6)alkanoylamino, phenylcarbonyl, -S(O)ₙ(C1-C6)alkyl, -C(O)NR⁶R⁷ and -SO₂NR⁸R⁹, wherein R⁴, R⁵, R⁶, R⁷, R⁸ and R⁹ are each independently selected from hydrogen and (C1-C6)alkyl, or R⁴ and R⁵, or R⁶ and R⁷, or R⁸ and R⁹, when taken together with the nitrogen atom to which they are attached, may each independently form a saturated heterocyclic ring and n is 0, 1 or 2,
and wherein any saturated monocyclic ring optionally bears 1 or 2 oxo or thioxo substituents;
or a pharmaceutically-acceptable salt thereof, provided that the compound of formula (I) is not:
   5-chloro-2-{2-[3-(2-methoxypyrid-3-yl)isoxazol-5-yl]pyrrolidin-1-yl}-4-(5-methyl-1*H-*pyrazol-3-ylamino)pyrimidine;
   S-5-chloro-2-{2-[3-(2-methoxypyrid-3-yl)isoxazol-5-yl]pyrrolidin-1-yl}-4-(5-methyl-1*H-*pyrazol-3-ylamino)pyrimidine;
   S-4-(5-ethyl-1*H*-pyrazol-3-ylamino)-2-[2-[3-(2-methoxypyrid-3-yl)isoxazol-5-yl]pyrrolidin-1-yl]pyrimidine;
   S-4-(5-methyl-1H-pyrazol-3-ylamino)-2-[2-[3-(2-methoxypyrid-3-yl)isoxazol-5-yl]pyrrolidin-1-yl]pyrimidine;
   S-4-(5-cyclopropyl-1H-pyrazol-3-ylamino)-2-[2-[3-(2-methoxypyrid-3-yl)isoxazol-5-yl]pyrrolidin-1-yl]pyrimidine;
   S-6-chloro-4-(5-methyl-1*H*-pyrazol-3-ylamino)-2-[2-{3-(3-methoxypyrazin-2-yl)isoxazol-5-yl}pyrrolidin-1-yl]pyrimidine;
   S-6-morpholino-4-(5-methyl-1*H*-pyrazol-3-ylamino)-2-[2-{3-(3-methoxypyrazin-2-yl)isoxazol-5-yl}pyrrolidin-1-yl]pyrimidine;
   S-6-morpholino-4-(5-methyl-1*H*-pyrazol-3-ylamino)-2-[2-{3-(3-hydroxypyrazin-2-yl)isoxazol-5-yl }pyrrolidin-1-yl]pyrimidine;
   S-6-methyl-4-(5-methyl-1*H-*pyrazol-3-ylamino)-2-[2-{3-(3-methoxypyrazin-2-yl)isoxazol-5-yl }pyrrolidin-1-yl]pyrimidine;
   S-6-methyl-4-(5-ethyl-1*H*-pyrazol-3-ylamino)-2-[2-{3-(3-methoxypyrazin-2-yl)isoxazol-5-yl}pyrrolidin-1-yl]pyrimidine;
   S-6-chloro-4-(5-ethyl-1*H*-pyrazol-3-ylamino)-2-[2-{3-(3-methoxypyrazin-2-yl)isoxazol-5-yl}pyrrolidin-1-yl]pyrimidine;
   S-6-methyl-4-(5-methyl-1*H*-pyrazol-3-ylamino)-2-[2-[3-(2-methoxypyrid-3-yl)isoxazol-5-yl]pyrrolidin-1-yl]pyrimidine;
   S-5-fluoro-4-(5-cyclopropyl-1*H*-pyrazol-3-ylamino)-2-[2-[3-(2-methoxypyrid-3-yl)isoxazol-5-yl]pyrrolidin-1-yl]pyrimidine;
   S-5-fluoro-4-(5-ethyl-1*H*-pyrazol-3-ylamino)-2-[2-[3-(2-methoxypyrid-3-yl)isoxazol-5-yl]pyrrolidin-1-yl]pyrimidine;
   S-6-(2-hydroxyethoxy)-4-(5-methyl-1*H*-pyrazol-3-ylamino)-2-[2-[3-(2-methoxypyrid-3-yl)isoxazol-5-yl]pyrrolidin-1-yl]pyrimidine;
   S-6-chloro-4-(5-cyclopropyl-1*H*-pyrazol-3-ylamino)-2-[2-[3-(2-methoxypyrid-3-yl)isoxazol-5-yl]pyrrolidin-1-yl]pyrimidine;
   S-6-chloro-4-(5-ethyl-1*H*-pyrazol-3-ylamino)-2-[2-[3-(2-methoxypyrid-3-yl)isoxazol-5-yl]pyrrolidin-1-yl]pyrimidine;
   S-6-(2-hydroxyethoxy)-4-(5-cyclopropyl-1*H*-pyrazol-3-ylamino)-2-[2-[3-(2-methoxypyrid-3-yl)isoxazol-5-yl]pyrrolidin-1-yl]pyrimidine;
   S-5-fluoro-4-(5-methyl-1*H*-pyrazol-3-ylamino)-2-[2-[3-(2-methoxypyrid-3-yl)isoxazol-5-yl]pyrrolidin-1-yl]pyrimidine;
   S-6-(2-hydroxyethoxy)-4-(5-ethyl-1*H*-pyrazol-3-ylamino)-2-[2-[3-(2-methoxypyrid-3-yl)isoxazol-5-yl]pyrrolidin-1-yl]pyrimidine;
   S-6-methyl-4-(5-cyclopropyl-1H-pyrazol-3-ylamino)-2-[2-{3 -(3 -methoxypyrazin-2-yl)isoxazol-5-yl }pyrrolidin-1-yl]pyrimidine;
   S-6-morpholino-4-(5-ethyl-1H-pyrazol-3-ylamino)-2-[2-{3-(3-methoxypyrazin-2-yl)isoxazol-5-yl}pyrrolidin-1-yl]pyrimidine;
   S-6-chloro-4-(5-methyl-1H-pyrazol-3-ylamino)-2-[2-[3-(2-methoxypyrid-3-yl)isoxazol-5-yl]pyrrolidin-1-yl]pyrimidine; or
   S-6-morpholino-4-(5-ethyl-1H-pyrazol-3-ylamino)-2-[2-{3-(3-hydroxypyrazin-2-yl)isoxazol-5-yl}pyrrolidin-1-yl]pyrimidine.
   According to a second aspect of the invention, there is provided a compound of formula (I) as defined above wherein:

R¹ is selected from methyl, ethyl, isopropyl and cyclopropyl;
R² is selected from hydrogen and halogeno;
R³ is selected from hydrogen, hydroxy and halogeno, or from a (C1-C6)alkyl, (C2-C6)alkenyl, (C2-C6)alkynyl, (C1-C6)alkoxy, (C1-C6)alkylcarbonyl, (C1-C6)alkoxycarbonyl, amino, (C1-C6)alkylamino, di-[(C1-C6)alkyl]amino, (C3-C8)cycloalkylamino, carbamoyl, (C1-C6)alkylcarbamoyl, di-[(C1-C6)alkyl]carbamoyl, -C(O)R^{3b}, -NHR^{3b}, -SR^{3a} or -N(R^{3c})C(O)R^{3a} group, wherein R^{3a} is selected from a (C1-C6)alkyl or (C1-C6)alkoxy group, R^{3b} is a saturated monocyclic 4-, 5- or 6-membered heterocyclic ring comprising at least one ring heteroatom selected from nitrogen, oxygen and sulfur and R^{3c} is selected from hydrogen and (C1-C6)alkyl,
   or R³ is a saturated monocyclic 5- or 6-membered heterocyclic ring comprising at least one ring heteroatom selected from nitrogen, oxygen and sulfur,
   each of which groups or rings within R³ may be optionally substituted by one or more substituents independently selected from (C1-C6)alkyl, (C1-C6)alkoxy, (C1-C6)alkoxy(C1-C6)alkyl, (C1-C6)alkoxy(C1-C6)alkoxy, halogeno, hydroxy, trifluoromethyl, tri-[(C1-C4)alkyl]silyl, cyano, amino, (C1-C6)alkylamino, di-[(C1-C6)alkyl)amino, amino(C1-C6)alkyl, (C1-C6)alkylamino(C1-C6)alkyl, di-[(C1-C6)alkyl]amino(C1-C6)alkyl, (C1-C6)alkoxycarbonyl, carbamoyl, (C1-C6)alkylcarbamoyl, di-[(C1-C6)alkyl]carbamoyl, (C1-C6)alkylthio, (C1-C6)alkylsulfonyl, (C1-C6)alkylsulfinyl, (C1-C6)alkanoyl, an alkanoylamino group -N(R^{3d})C(O)R^{3e} wherein R^{3d} is selected from hydrogen and (C1-C6)alkyl and R^{3e} is selected from a (C1-C6)alkyl or (C1-C6)alkoxy group, or a saturated monocyclic 3-, 4-, 5-, 6- or 7-membered ring, which ring may optionally comprise one or more heteroatoms selected from nitrogen, oxygen and sulfur, any of which substituents may be optionally substituted by one or more (C1-C4)alkyl, hydroxy or cyano groups;
   Q¹ is a 5- or 6-membered heteroaromatic ring comprising at least one ring heteroatom selected from nitrogen, oxygen and sulfur,
   and wherein Q¹ is substituted by one or more substituents independently selected from (C1-C6)alkyl and (C1-C6)alkoxy (either of which (C1-C6)alkyl and (C1-C6)alkoxy groups may be optionally substituted by one or more substituents independently selected from (halogeno, amino, hydroxy and trifluoromethyl), halogeno, nitro, cyano, -NR⁴R⁵, carboxy, hydroxy, (C2-C6)alkenyl, (C3-C8)cycloalkyl, (C1-C6)alkoxycarbonyl, (C1-C6)alkylcarbonyl, (C2-C6)alkanoylamino, phenylcarbonyl, -S(O)ₙ(C1-C6)alkyl, -C(O)NR⁶R⁷ and -SO₂NR⁸R⁹, wherein R⁴, R⁵, R⁶, R⁷, R⁸ and R⁹ are each independently selected from hydrogen and (C1-C6)alkyl, or R⁴ and R⁵, or R⁶ and R⁷, or R⁸ and R⁹, when taken together with the nitrogen atom to which they are attached, may each independently form a saturated heterocyclic ring and n is 0, 1 or 2,
   and wherein any saturated monocyclic ring optionally bears 1 or 2 oxo or thioxo substituents;
   or a pharmaceutically-acceptable salt thereof.

According to a third aspect of the invention, there is provided a compound of formula (I) (or a pharmaceutically-acceptable salt thereof) as defined above, provided that when Q¹ carries a substituent at an ortho-position relative to the point of attachment of the ring Q¹ to the isoxazolyl group, the substituent is not hydroxy or methoxy.

According to a fourth aspect of the invention, there is provided a compound of formula (I) (or a pharmaceutically-acceptable salt thereof) as defined above, provided that the substituent on Q¹ is not hydroxy or methoxy.

In this specification, unless otherwise indicated, the term "alkyl" when used alone or in combination, includes both straight chain and branched chain alkyl groups, such as propyl, isopropyl and tert-butyl. However, references to individual alkyl groups such as "propyl" are specific for the straight-chain version only and references to individual branched-chain alkyl groups such as "isopropyl" are specific for the branched-chain version only. A (C1-C6)alkyl group has from one to six carbon atoms including methyl, ethyl, n-propyl, isopropyl, tert-butyl, n-pentyl, n-hexyl and the like. References to "(C1-C4)alkyl" will be understood accordingly to mean a straight or branched chain alkyl moiety having from one to four carbon atoms.

An analogous convention applies to other generic terms, for example, the terms "(C1-C6)alkoxy" and "(C1-C4)alkoxy", when used alone or in combination, will be understood to refer to straight or branched chain groups having from one to six, or from one to four, carbon atoms respectively and include such groups as methoxy, ethoxy, propoxy, isopropoxy and butoxy.

A "(C2-C6)alkenyl" group includes both straight chain and branched chain alkenyl groups having from two to six carbon atoms, such as vinyl, isopropenyl, allyl and but-2-enyl. Similarly, a "(C2-C6)alkynyl" group includes both straight chain and branched chain alkynyl groups having from two to six carbon atoms, such as ethynyl, 2-propynyl and but-2-ynyl.

The term "(C3-C8)cycloalkyl", when used alone or in combination, refers to a saturated alicyclic moiety having from three to eight carbon atoms and includes, for example, cyclopropyl, cyclobutyl, cyclopentyl, cyclohexyl and cycloheptyl. References to "(C3-C6)cycloalkyl" will be understood accordingly to mean a saturated alicyclic moiety having from three to six carbon atoms, representative examples of which are listed above.

As used herein, the term "halogeno" includes fluoro, chloro, bromo and iodo.

The term "optionally substituted" is used herein to indicate optional substitution by the group or groups specified at any suitable available position.

A "heteroatom" is a nitrogen, sulfur or oxygen atom. Where rings include nitrogen atoms, these may be substituted as necessary to fulfil the bonding requirements of nitrogen or they may be linked to the rest of the structure by way of the nitrogen atom. Nitrogen atoms may also be in the form of N-oxides. Sulfur atoms may be in the form of S, S(O) or SO₂.

Suitable values for the generic radicals referred to above include those set out below.

A suitable value for a substituent on R³ when it is a "saturated monocyclic 3-, 4-, 5-, 6- or 7-membered ring, which ring may optionally comprise one or more heteroatoms selected from nitrogen, oxygen and sulfur" is a carbocyclic ring containing 3, 4, 5, 6 or 7 atoms (that is an alicyclic ring having ring carbon atoms only) or a heterocyclic ring containing 3, 4, 5, 6 or 7 atoms of which at least one is a heteroatom selected from nitrogen, oxygen and sulfur. When the "saturated monocyclic 3-, 4-, 5-, 6- or 7-membered ring, which ring may optionally comprise one or more heteroatoms selected from nitrogen, oxygen and sulfur" is a heterocyclic ring, the heterocyclic ring suitably contains from one to four (for example, from one to three, or one or two) heteroatoms independently selected from nitrogen, oxygen and sulfur. Unless specified otherwise, the heterocyclic ring may be carbon or nitrogen linked. Examples of suitable saturated monocyclic 3-, 4-, 5-, 6- or 7-membered carbocyclic rings include cyclopropyl, cyclobutyl, cyclopentyl, cyclohexyl and cycloheptyl. Examples of suitable saturated monocyclic 3-, 4-, 5-, 6- or 7-membered heterocyclic rings include oxiranyl, azetidinyl, dioxanyl, trioxanyl, oxepanyl, dithianyl, trithianyl, oxathianyl, thiomorpholinyl, pyrrolidinyl, piperidinyl, imidazolidinyl, morpholinyl, tetrahydrofuranyl, tetrahydropyranyl and piperazinyl (particularly azetidinyl, pyrrolidinyl, piperidinyl, morpholinyl, tetrahydrofuranyl, tetrahydropyranyl and piperazinyl). A saturated heterocyclic ring that bears 1 or 2 oxo or thioxo substituents may, for example, be 2-oxopyrrolidinyl, 2-thioxopyrrolidinyl, 2-oxoimidazolidinyl, 2-thioxoimidazolidinyl, 2-oxopiperidinyl, 2,5-dioxopyrrolidinyl, 2,5-dioxoimidazolidinyl or 2,6-dioxopiperidinyl.

A suitable value for R^{3b} when it is a "saturated monocyclic 4-, 5- or 6-membered heterocyclic ring comprising at least one ring heteroatom selected from nitrogen, oxygen and sulfur" is a heterocyclic ring containing four, five or six ring atoms, representative examples of which are listed above.

A suitable value for R³ when it is a "saturated monocyclic 5- or 6-membered heterocyclic ring comprising at least one ring heteroatom selected from nitrogen, oxygen and sulfur" is a heterocyclic ring containing five or six ring atoms, representative examples of which are listed above.

A suitable value for Q¹, the "5- or 6-membered heteroaromatic ring comprising at least one ring heteroatom selected from nitrogen, oxygen and sulfur", is a fully unsaturated, aromatic monocyclic ring containing five or six atoms of which at least one is a heteroatom selected from nitrogen, oxygen and sulfur, which ring may, unless otherwise specified, be carbon or nitrogen linked. Particularly, the 5- or 6-membered heteroaromatic ring may contain from one to four (for example, from one to three, or one or two) heteroatoms independently selected from nitrogen, oxygen and sulfur. Examples of such heteroaromatic rings include pyridyl, imidazolyl, isoxazolyl, pyrazolyl, furyl, pyrazinyl, pyridazinyl, pyrimidinyl, pyrrolyl, thiazolyl, oxazolyl, oxadiazolyl, isothiazolyl, triazolyl, tetrazolyl and thienyl.

Where R⁴ and R⁵, or R⁶ and R⁷, or R⁸ and R⁹ form a saturated heterocyclic ring, the only heteroatom present is the nitrogen atom to which R⁴ and R⁵, or R⁶ and R⁷, or R⁸ and R⁹ are attached. The saturated heterocyclic ring is preferably a 4-, 5-, 6- or 7-membered ring, including the nitrogen atom to which R⁴ and R⁵, or R⁶ and R⁷, or R⁸ and R⁹ are attached.

For the avoidance of any doubt the nitrogen atom in the pyrrolidine ring to which the pyrimidine group is attached is not quaternised; namely the pyrimidine group is attached to the nitrogen atom in the pyrrolidine ring via. substitution of an NH group in the pyrrolidine ring.

Suitable values for any of the substituents herein, for example the 'R' groups (R¹ to R¹², R^{3a}, R^{3b}, R^{3c}, R^{3d} or R^{3e}) or for various groups within a Q¹ group include:-
- for halogeno:: fluoro, chloro, bromo and iodo;
- for (C1-C6)alkyl:: methyl, ethyl, propyl, isopropyl, tert-butyl, n-pentyl and n-hexyl;
- for (C2-C6)alkenyl:: vinyl, isopropenyl, allyl and but-2-enyl;
- for (C2-C6)alkynyl:: ethynyl, 2-propynyl and but-2-ynyl;
- for (C1-C6)alkoxy:: methoxy, ethoxy, propoxy, isopropoxy and butoxy;
- for (C1-C6)alkoxy(C1-C6)alkoxy:: methoxymethoxy, methoxyethoxy, ethoxymethoxy, propoxymethoxy and butoxymethoxy;
- for (C1-C6)alkoxy(C1-C6)alkyl:: methoxymethyl, methoxyethyl, ethoxymethyl, propoxymethyl and butoxymethyl;
- for tri-[(C1-C4)alkyl]silyl: trimethylsilyl, triethylsilyl, dimethyl-ethylsilyl and methyl-diethylsilyl;
- for (C1-C6)alkylthio:: methylthio, ethylthio and propylthio;
- for (C1-C6)alkylamino:: methylamino, ethylamino, propylamino, isopropylamino and butylamino;
- for di-[(C1-C6)alkyl]amino:: dimethylamino, diethylamino, N-methyl-N-methylamino and diisopropylamio;
- for amino(C1-C6)alkyl:: aminomethyl, aminoethyl, aminopropyl and aminobutyl;
- for (C1-C6)alkylamino(C1-C6)alkyl:: methylaminomethyl, methylaminoethyl, methylaminopropyl, ethylaminomethyl, ethylaminoethyl, propylaminomethyl, isopropylaminoethyl and butylaminomethyl;
- for di-[(C1-C6)alkyl]amino(C1-C6)alkyl:: dimethylaminomethyl, dimethylaminoethyl, dimethylaminobutyl, diethylaminomethyl, diethylaminoethyl, diethylaminopropyl, N-methyl-N-methylaminomethyl,-N-ethyl-N-methylaminomethyl and diisopropylaminoethyl;
- for (C1-C6)alkylcarbonyl:: methylcarbonyl, ethylcarbonyl, propylcarbonyl and tert-butylcarbonyl;
- for (C1-C6)alkoxycarbonyl:: methoxycarbonyl, ethoxycarbonyl, propoxycarbonyl and tert-butoxycarbonyl;
- for (C1-C6)alkylcarbamoyl:: N-methylcarbamoyl, N-ethylcarbamoyl and N-propylcarbamoyl;
- for di-[(C1-C6)alkyl]carbamoyl:: N-N-dimethylcarbamoyl, N-ethyl-N-methylcarbamoyl and N,N-diethylcarbamoyl;
- for (C3-C8)cycloalkyl:: cyclopropyl, cyclobutyl, cyclopentyl, cyclohexyl and cycloheptyl;
- for (C3-C8)cycloalkylamino:: cyclopropylamino, cyclobutylamino, cyclopentylamino, cyclohexylamino and cycloheptylamino;
- for (C1-C6)alkanoyl:: formyl, acetyl, propionyl, butyryl and isobuyryl;
- for (C2-C6)alkanoylamino:: acetamido and propionamido;
- for (C1-C6)alkylsulfonyl:: methylsulfonyl and ethylsulfonyl; and
- for (C1-C6)alkylsulfinyl:: methylsulfinyl and ethylsulfinyl.

Where the compounds according to the invention contain one or more asymmetrically substituted carbon atoms, the invention includes all stereoisomers, including enantiomers and diastereomers, and mixtures including racemic mixtures thereof.

Thus, it is to be understood that, insofar as certain of the compounds of formula (I) defined above may exist in optically active or racemic forms by virtue of one or more asymmetric carbon atoms, the invention includes in its definition any such optically active or racemic form which possesses the above-mentioned activity. In particular, the compound of formula (I) has a chiral centre on the pyrrolidine ring at the pyrrolidine ring carbon atom attached to the isoxazolyl group. The present invention encompasses all such stereoisomers having activity as herein defined, for example the (2R) and (2S) isomers (in particular the (2S) isomers). It is further to be understood that in the names of chiral compounds (R,S) denotes any scalemic or racemic mixture while (R) and (S) denote the enantiomers. In the absence of (R,S), (R) or (S) in the name it is to be understood that the name refers to any scalemic or racemic mixture, wherein a scalemic mixture contains R and S enantiomers in any relative proportions and a racemic mixture contains R and S enantiomers in the ratio 50:50. The synthesis of optically active forms may be carried out by standard techniques of organic chemistry well known in the art, for example by synthesis from optically active starting materials or by resolution of a racemic form. Racemates may be separated into individual enantiomers using known procedures (cf. Advanced Organic Chemistry: 3rd Edition: author J March, pages 104 to 107). A suitable procedure involves formation of diastereomeric derivatives by reaction of the racemic material with a chiral auxiliary, followed by separation, for example by chromatography, of the diastereomers and then cleavage of the auxiliary species. Similarly, the above-mentioned activity may be evaluated using the standard laboratory techniques referred to hereinafter.

It is also to be understood that, insofar as certain of the compounds of formula (I) defined above may exist in tautomeric forms, the invention includes in its definition any such tautomeric form which possesses the above-mentioned activity. Thus, the invention relates to all tautomeric forms of the compounds of formula (I) which inhibit IGF-1R tyrosine kinase activity in a human or animal. For example, the compounds of the invention may exist in the following alternative tautomeric forms (I') and (I"):

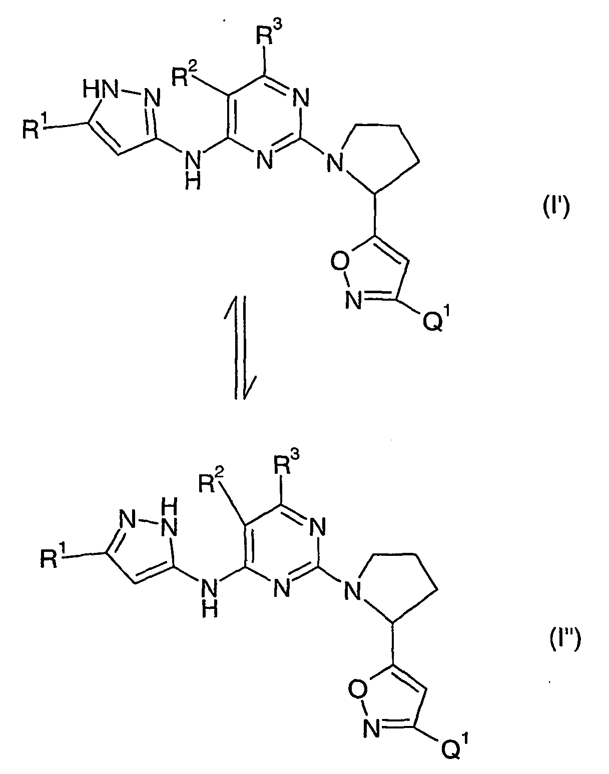

It is to be understood that certain compounds of formula (I) may exist in solvated as well as unsolvated forms such as, for example, hydrated forms. It is to be understood that the invention encompasses all such solvated forms which inhibit IGF-1R tyrosine kinase activity in a human or animal.

It is also to be understood that certain compounds of formula (I) may exhibit polymorphism, and that the invention encompasses all such forms which inhibit IGF-1R tyrosine kinase activity in a human or animal.

The compounds according to the invention may be provided as pharmaceutically-acceptable salts. Suitable pharmaceutically-acceptable salts include base salts such as an alkali metal salt for example sodium, an alkaline earth metal salt for example calcium or magnesium, an organic amine salt for example triethylamine, morpholine, *N*-methylpiperidine, *N*-ethylpiperidine, procaine, dibenzylamine, *N,N-*dibenzylethylamine or amino acids for example lysine. In another aspect, where the compound is sufficiently basic, suitable salts include acid addition salts such as methanesulfonate, fumarate, hydrochloride, hydrobromide, citrate, maleate and salts formed with phosphoric and sulfuric acid.

In one aspect of the invention, a suitable value for R¹ is methyl, ethyl or isopropyl, particularly methyl or ethyl, more particularly methyl.

In another aspect of the invention, a suitable value for R¹ is methyl or cyclopropyl.

In another aspect of the invention, a suitable value for R¹is cyclopropyl.

In one aspect of the invention, a suitable value for R² is hydrogen, chloro or fluoro, particularly hydrogen or chloro.

In another aspect of the invention, a suitable value for R² is hydrogen.

In one aspect of the invention, R³ is selected from hydrogen, hydroxy and halogeno, or from a (C1-C6)alkyl, (C2-C6)alkenyl, (C2-C6)alkynyl, (C1-C6)alkoxy, (C1-C6)alkoxycarbonyl, amino, (C1-C6)alkylamino, di-[(C1-C6)alkyl]amino, (C3-C8)cycloalkylamino, carbamoyl, (C1-C6)alkylcarbamoyl, di-[(C1-C6)alkyl]carbamoyl, - C(O)R^{3b}, -NHR^{3b} or -SR^{3a} group, wherein R^{3a} is a (C1-C6)alkyl group and R^{3b} is a saturated monocyclic 4-, 5- or 6-membered heterocyclic ring comprising at least one ring heteroatom selected from nitrogen, oxygen and sulfur, or R³ is a saturated monocyclic 5- or 6-membered heterocyclic ring comprising at least one ring heteroatom selected from nitrogen and oxygen. Each of these groups or rings within R³ may be optionally substituted by one or more (for example one or two, particularly one) substituents independently selected from (C1-C6)alkyl, (C1-C6)alkoxy, (C1-C6)alkoxy(C1-C6)alkyl, (C1-C6)alkoxy(C1-C6)alkoxy, halogeno, hydroxy, trifluoromethyl, tri-[(C1-C4)alkyl]silyl, amino, (C1-C6)alkylamino, di-[(C1-C6)alkyl]amino, amino(C1-C6)alkyl, (C1-C6)alkoxycarbonyl, carbamoyl, (C1-C6)alkylcarbamoyl, (C1-C6)alkylthio, (C1-C6)alkylsulfonyl, (C1-C6)alkanoyl, an alkanoylamino group -N(R^{3d})C(O)R^{3e} wherein R^{3d} is selected from hydrogen and (C1-C6)alkyl and R^{3e} is selected from a (C1-C6)alkyl or (C1-C6)alkoxy group, or a saturated monocyclic 3-, 4-, 5- or 6-membered ring, which ring may optionally comprise one or more heteroatoms selected from nitrogen, oxygen and sulfur, any of which substituents may be optionally substituted by one or more (for example one or two, particularly one) (C1-C4)alkyl, hydroxy or cyano groups. Any saturated monocyclic ring within R³ optionally bears 1 or 2 oxo substituents.

In another aspect of the invention, R³ is selected from hydrogen, hydroxy or halogeno, or from a (C1-C4)alkyl, (C2-C4)alkenyl, (C2-C4)alkynyl, (C1-C3)alkoxy, amino, (C1-C3)alkylamino, di-[(C1-C3)alkyl]amino, (C3-C6)cycloalkylamino, carbamoyl, (C1-C3)alkylcarbamoyl, di-[(C1-C3)alkyl]carbamoyl, -C(O)R^{3b}, -NHR^{3b} or -SR^{3a} group, wherein R^{3a} is a (C1-C3)alkyl group and R^{3b} is a saturated monocyclic 4-, 5- or 6-membered heterocyclic ring comprising at least one ring heteroatom selected from nitrogen, oxygen and sulfur, or R³ is a saturated monocyclic 5- or 6-membered heterocyclic ring comprising at least one ring heteroatom selected from nitrogen and oxygen. Each of these groups or rings within R³ may be optionally substituted by one or more substituents as defined above, in particular by one or more (for example one or two, particularly one) substituents independently selected from (C1-C3)alkyl, (C1-C3)alkoxy, (C1-C3)alkoxy(C1-C3)alkyl, (C1-C3)alkoxy(C1-C3)alkoxy, halogeno, hydroxy, trifluoromethyl, amino, (C1-C3)alkylamino, di-[(C1-C3)alkyl]amino, amino(C1-C3)alkyl, carbamoyl, (C1-C3)alkylcarbamoyl, (C1-C3)alkylthio, (C1-C3)alkylsulfonyl, (C1-C3)alkanoyl, an alkanoylamino group -N(R^{3d})C(O)R^{3e} wherein R^{3d} is selected from hydrogen and (C1-C3)alkyl and R^{3e} is selected from a (C1-C3)alkyl or (C1-C3)alkoxy group, or a saturated monocyclic 3-, 4-, 5- or 6-membered ring, which ring may optionally comprise one or more heteroatoms selected from nitrogen, oxygen and sulfur, any of which substituents may be optionally substituted by one or more (for example one or two, particularly one) (C1-C2)alkyl, hydroxy or cyano groups. Any saturated monocyclic ring within R³ optionally bears 1 oxo substituent.

In another aspect of the invention, R³ is selected from hydrogen, hydroxy or halogeno, or from a (C1-C4)alkyl, (C2-C4)allcenyl, (C2-C4)alkynyl, (C1-C3)alkoxy, amino, (C1-C3)alkylamino, di-[(C2-C3)alkyl]amino, (C3-C6)cycloalkylamino, carbamoyl, (C1-C3)alkylcarbamoyl, di-[(C1-C3)alkyl]carbamoyl, -C(O)R^{3b}, -NHR^{3b} or -SR^{3a} group, wherein R^{3a} is a (C1-C3)alkyl group and R^{3b} is a saturated monocyclic 4-, 5- or 6-membered heterocyclic ring comprising at least one ring heteroatom selected from nitrogen, oxygen and sulfur, or R³ is a saturated monocyclic 5- or 6-membered heterocyclic ring comprising at least one ring heteroatom selected from nitrogen and oxygen. Each of these groups or rings within R³ may be optionally substituted by one or more substituents as defined above, in particular by one or more (for example one or two) substituents independently selected from cyano, (C1-C3)alkyl, (C1-C3)alkoxy, (C1-C3)alkoxy(C1-C3)alkyl, (C1-C3)alkoxy(C1-C3)alkoxy, halogeno, hydroxy, trifluoromethyl, amino, (C1-C3)alkylamino, di-[(C1-C3)alkyl]amino, amino(C1-C3)alkyl, carbamoyl, (C1-C3)alkylcarbamoyl, (C1-C3)alkylthio, (C1-C3)alkylsulfonyl, (C1-C3)alkanoyl, an alkanoylamino group -N(R^{3d})C(O)R^{3e} wherein R^{3d} is selected from hydrogen and (C1-C3)alkyl and R^{3e} is selected from a (C1-C3)alkyl or (C1-C3)alkoxy group, or a saturated monocyclic 3-, 4-, 5- or 6-membered ring, which ring may optionally comprise one or more heteroatoms selected from nitrogen, oxygen and sulfur, any of which substituents may be optionally substituted by one or more (for example one or two, particularly one) (C1-C2)alkyl, hydroxy or cyano groups. Any saturated monocyclic ring within R³ optionally bears 1 oxo substituent.

In one aspect of the invention, R³, when it is substituted, may be substituted by one or more (for example, one, two or three, particularly one or two, more particularly one) substituents independently selected from (C1-C6)alkoxy (such as methoxy or ethoxy), (C1-C6)alkoxy(C1-C6)alkoxy (such as methoxyethoxy) or a saturated monocyclic 3-, 4-, 5-, 6- or 7-membered (for example 4-, 5-, 6- or 7-membered) ring, which ring may optionally comprise one or more heteroatoms selected from nitrogen, oxygen and sulfur (such as cyclopentyl, cyclohexyl, pyrrolidinyl, piperidinyl, dioxanyl, morpholinyl, tetrahydrofuranyl or piperazinyl).

In another aspect of the invention, R³, when it is substituted, may be substituted by one or more (for example, one or two, particularly one) substituents independently selected from (C1-C6)alkyl, (C1-C6)alkoxy, halogeno, hydroxy, trifluoromethyl, amino, (C1-C6)alkylamino and di-[(C1-C6)alkyl]amino, or a saturated monocyclic 3-, 4-, 5-, 6- or 7-membered (for example 4-, 5-, 6- or 7-membered) ring, which ring may optionally comprise one or more heteroatoms selected from nitrogen, oxygen and sulfur.

In another aspect of the invention, R³, when it is substituted, may be substituted by one or more (for example, one or two) substituents independently selected from (C1-C6)alkoxy, hydroxy and cyano, or a saturated monocyclic 3-, 4-, 5-, 6- or 7-membered (for example a 3-membered) ring, which ring may optionally comprise one or more heteroatoms selected from nitrogen, oxygen and sulfur, any of which substituents may be optionally substituted by one or more cyano groups.

In another aspect of the invention, when R³ carries a substituent that is a saturated monocyclic 3-, 4-, 5-, 6- or 7-membered (for example 4-, 5-, 6- or 7-membered) ring, which ring may optionally comprise one or more heteroatoms selected from nitrogen, oxygen and sulfur, that ring preferably comprises nitrogen and, optionally, one or two additional heteroatoms selected from nitrogen, oxygen and sulfur. For example, the saturated monocyclic 3-, 4-, 5-, 6- or 7-membered ring substituent on R³ may be pyrrolidine.

In another aspect of the invention, when R³ carries a substituent that is a saturated monocyclic 3-, 4-, 5-, 6- or 7-membered ring, which ring may optionally comprise one or more heteroatoms selected from nitrogen, oxygen and sulfur, that ring preferably comprises no heteroatoms. For example, the saturated monocyclic 3-, 4-, 5-, 6- or 7-membered ring substituent on R³ may be cyclopropyl. The saturated monocyclic 3-, 4-, 5-, 6- or 7-membered ring substituent on R³ (such as cyclopropyl) may be optionally substituted by one or more cyano groups.

In another aspect of the invention, R³ is selected from hydrogen or from a (C1-C4)alkyl or (C1-C3)alkoxy group, or R³ is a saturated monocyclic 5- or 6-membered heterocyclic ring comprising at least one ring heteroatom selected from nitrogen and oxygen. Each of these groups or rings within R³ may be optionally substituted by one or more (for example one or two, particularly one) substituents as defined above, in particular by one or more substituents independently selected from hydroxy and (C1-C3)alkoxy.

In another aspect of the invention, R³ is selected from hydrogen and halogeno, or from a (C1-C4)alkyl or (C1-C3)alkoxy group, or R³ is a saturated monocyclic 5- or 6-membered heterocyclic ring comprising at least one ring heteroatom selected from nitrogen and oxygen. Each of these groups or rings within R³ may be optionally substituted by one or more (for example one or two, particularly one) substituents as defined above, in particular by one or more substituents independently selected from hydroxy and (C1-C3)alkoxy.

In another aspect of the invention, R³ is selected from hydrogen and halogeno (such as chloro), or from a (C1-C3)alkyl or (C1-C3)alkoxy group, or R³ is a saturated monocyclic 6-membered heterocyclic ring comprising at least one ring heteroatom selected from nitrogen and oxygen. Each of these groups or rings within R³ may be optionally substituted by one or more (for example one or two) substituents as defined above, in particular by one or more substituents independently selected from cyano, hydroxy, cyclopropyl and (C1-C3)allcoxy (such as methoxy or ethoxy), any of which substituents may be optionally substituted by one or more cyano groups.

In yet another aspect of the invention, R³ is selected from halogeno, or from a (C1-C4)alkyl or (C1-C3)alkoxy group, or R³ is a saturated monocyclic 5- or 6-membered heterocyclic ring comprising at least one ring heteroatom selected from nitrogen and oxygen. Each of these groups or rings within R³ may be optionally substituted by one or more (for example one or two, particularly one) substituents as defined above, in particular by one or more substituents independently selected from hydroxy and (C1-C3)alkoxy.

In another aspect of the invention, R³ is selected from hydrogen or halogeno, or from a (C1-C6)alkyl, (C2-C6)alkenyl, (C2-C6)alkynyl, (C1-C6)alkoxy, (C1-C6)alkylcarbonyl, (C1-C6)alkoxycarbonyl, amino, (C1-C6)alkylamino, di-[(C1-C6)alkyl]amino, carbamoyl, -C(O)R^{3b}, -NHR^{3b} or -SR^{3a} group (wherein R^{3a} and R^{3b} are as defined above), or R³ is a saturated monocyclic 5- or 6-membered heterocyclic ring comprising at least one ring heteroatom selected from nitrogen, oxygen and sulfur, each of which groups or rings may be optionally substituted by one or more (for example one or two, particularly one) substituents as defined hereinbefore.

In another aspect of the invention, R³ is selected from hydrogen or from a substituted or unsubstituted group selected from (C1-C6)alkyl (for example (C1-C4)alkyl, such as methyl, ethyl, propyl, isopropyl or tert-butyl), (C1-C6)alkoxy (for example (C1-C4)alkoxy, such as methoxy, ethoxy, propoxy, isopropoxy or butoxy), (C1-C6)alkylcarbonyl (for example (C1-C4alkylcarbonyl, such as methylcarbonyl), (C1-C6)alkoxycarbonyl (for example (C1-C4)alkoxycarbonyl, such as methoxycarbonyl), (C1-C6)alkylamino (for example (C1-C4)alkylamino, such as methylamino or ethylamino), (C3-C8)cycloalkylamino or -SR^{3a} (wherein R^{3a} is as defined above).

In another aspect of the invention, suitable values for R³ include, for example, hydrogen, hydroxy, chloro, fluoro or iodo, or a methyl, ethyl, n-propyl, iso-propyl, n-butyl, tert-butyl, ethenyl, propenyl, butenyl, pentenyl, ethynyl, propynyl, butynyl, methoxy, ethoxy, propoxy, tert-butoxy, methoxycarbonyl, ethoxycarbonyl, propoxycarbonyl, tert-butoxycarbonyl, methylamino, ethylamino, propylamino, dimethylamino, diethylamino, cyclobutylamino, cyclohexylamino, carbamoyl, N-methylcarbamoyl, N-ethylcarbamoyl, N-propylcarbamoyl, N-butylcarbamoyl, N,N-dimethylcarbamoyl, N-ethyl-N-methylcarbamoyl, pyrrolidinylcarbonyl, morpholinylcarbonyl, azetidinylcarbonyl, methylthio, ethylthio, piperidinylamino, tetrahydropyranylamino, pyrrolidinyl, morpholinyl or piperazinyl group, each of which groups or rings may be optionally substituted by one or more (for example one or two, particularly one) substituents as defined above.

In yet another aspect of the invention, suitable values for R³ include, for example, hydrogen, hydroxy, chloro, fluoro, bromo, iodo, methyl, ethyl, propyl, iso-propyl, butyl, tert-butyl, trifluoromethyl, hydroxymethyl, methoxymethyl, ethoxymethyl, (2-methoxyethoxy)methyl, aminomethyl, methylaminomethyl, ethylaminomethyl, morpholinomethyl, piperazin-1-ylmethyl, 4-methylpiperazin-1-ylmethyl, pyrrolidin-1-ylmethyl, 2-hydroxyethyl, 2-methoxyethyl, 2-ethoxyethyl, 2-(ethoxycarbonyl)ethyl, 2-(N-methylcarbamoyl)ethyl, 3-hydroxypropyl, 3-methoxypropyl, 3-ethoxypropyl, 3-aminoprop-1-yl, 3-N,N-dimethylaminopropyl, 3-(tert-butoxycarbonylamino)prop-1-yl, 3-pyrrolidin-1-ylpropyl, ethenyl, propenyl, butenyl, pentenyl, 3-hydroxyprop-1-en-1-yl, 3-aminoprop-1-en-1-yl, 2-(methoxycarbonyl)ethen-1-yl, 3-(tert-butoxycarbonylamino)prop-1-en-1-yl, ethynyl, propynyl, butynyl, pentynyl, 3-hydroxyprop-1-yn-1-yl, 3-methoxyprop-1-yn-1-yl, 2-(trimethylsilyl)ethynyl, 3-aminoprop-1-yn-1-yl, 3-methylaminoprop-1-yn-1-yl, 3-(dimethylamino)prop-1 -yn- 1-yl, 3-(N-methylacetamido)prop-1-yn-1-yl, 3-acetamidoprop-1-yn-1-yl, methoxy, ethoxy, propoxy, butoxy, pentoxy, (5-oxopyrrolidin-2-yl)methoxy, tetrahydrofuran-3-ylmethoxy, 2-hydroxyethoxy, 2-ethoxyethoxy, 2-(2-hydroxyethoxy)ethoxy, 2-methoxyethoxy, (2-methoxyethoxy)ethoxy, 2-{N-[2-hydroxyethyl]-N-methylamino}ethoxy, 2-morpholinoethoxy, 2-(2-oxopyrrolidin-1-yl)ethoxy, 2-(imidazolid-2-on-1-yl)ethoxy, 3-hydroxypropyloxy, 2-hydroxyprop-1-yloxy, 3-methoxyprop-1-yloxy, 2-methoxyprop-1-yloxy, 3-morpholinoprop-1-yloxy, 3-(methylthio)prop-1-yloxy, 3-(methylsulfonyl)propyl-1-oxy, methoxycarbonyl, tert-butoxycarbonyl, N-(tert-butoxycarbonyl)amino, methylamino, 2-methoxyethylamino, 2-aminoethylamino, 2-(dimethylamino)ethylamino, (N-2-methoxyethyl)-N-methylamino, 3-isopropoxyprop-1-ylamino, 2-(2-hydroxyethoxy)ethylamino, 2-(acetoamido)ethylamino, 2-(morpholin-4-yl)ethylamino, 2-methylprop-1-ylamino, 2-hydroxyprop-1-ylamino; 3-methoxypropylamino, , 3-ethoxypropylamino, 2-isopropoxyethylamino, tetrahydrofuran-2-ylmethylamino, dimethylamino, N-(2-hydroxyethyl)-N-ethylamino, cyclopropylamino, cyclobutylamino, cyclopentylamino, 4-methylcyclohexylamino, 4-hydroxycyclohexylamino, carbamoyl, N-hydroxycarbamoyl, N-cyclopropylcarbamoyl, N-cyclopentylcarbamoyl, N-aminocarbamoyl, N-(acetylamino)carbamoyl, N-methylcarbamoyl, 2-hydroxyethylcarbamoyl, N-(2-hydroxypropyl)carbamoyl, N-(2,3-dihydroxypropyl)carbamoyl, N-(4-hydroxybutyl)carbamoyl, N-(2-methoxyethyl)carbamoyl, N-(2-(acetylamino)ethyl)carbamoyl, N-[2-(2-hydroxyethoxy)ethyl]carbamoyl, N-(carbamoylmethyl)carbamoyl, N-[2-(methylthio)ethyl]carbamoyl, N-(2-methoxyethyl)-N-methylcarbamoyl, pyrrolidin-1-ylcarbonyl, morpholinocarbonyl, azetidin-1-ylcarbonyl, (3-hydroxypyrrolidin-1-yl)carbonyl, methylthio, ethylthio, propylthio, 2,2,6,6-tetramethylpipendin-4-ylamino, 4-tetrahydropyranylamino, pyrrolidin-1-yl, morpholino, piperazin-1-yl, 4-methylpiperazin-1-yl, 4-ethylpiperazin-1-yl, 4-isopropylpiperazin-1-yl, 4-(2-hydroxyethyl)piperazin-1-yl, 4-(3-hydroxypropyl)piperazin-1-yl, 4-(2-methoxyethyl)piperazin-1-yl, 4-(2-aminoethyl)piperazin-1-yl, 4-[2-(2-hydroxyethoxy)ethyl]piperazin-1-yl, 4-(2-cyanoethyl)piperazin-1-yl, 4-(tert-butoxycarbonyl)piperazin-1-yl, 1-formyl-piperazin-4-yl, 4-acetylpiperazin-1-yl, 4-(ethylsulfonyl)piperazin-1-yl, 4-aminopiperidin-1-yl, 4-(N-(tert-butoxycarbonylamino)piperidin-1-yl, 3-hydroxypyrrolidin-1-yl, 3-dimethylamino-pyrrolidin-1-yl and cis-3,4-dihydroxypyrrolidin-1-yl.

Further suitable values for R³ include, for example, hydrogen, hydroxy, chloro, iodo, methyl, ethyl, propyl, trifluoromethyl, hydroxymethyl, methoxymethyl, ethoxymethyl, (2-methoxyethoxy)methyl, aminomethyl, methylaminomethyl, morpholinomethyl, 4-methylpiperazin-1-ylmethyl, pyrrolidin-1-ylmethyl, 2-methoxyethyl, 2-(ethoxycarbonyl)ethyl, 2-(N-methylcarbamoyl)ethyl 3-hydroxypropyl, 3-methoxypropyl, 3-aminoprop-1-yl, 3-N,N-dimethylaminopropyl, 3-(tert-butoxycarbonylamino)prop-1-yl, 3-pyrrolidin-1-ylpropyl, ethenyl, pent-3-en-1-yl, 3-hydroxyprop-1-en-1-yl, 3-aminoprop-1-en-1-yl, 2-(methoxycarbonyl)ethen-1-yl, 3-(tert-butoxycarbonylamino)prop-1-en-1-yl, ethynyl, 3-hydroxyprop-1-yn-1-yl, 3-methoxyprop-1-yn-1-yl, 2-(trimethylsilyl)ethynyl, 3-aminoprop-1-yn-1-yl, 3-methylaminoprop-1-yn-1-yl, 3-(dimethylamino)prop-1-yn-1-yl, 3-(N-methylacetamido)prop-1-yn-1-yl, 3-acetamidoprop-1-yn-1-yl, methoxy, ethoxy, (5-oxopyrrolidin-2-yl)methoxy (for example (2S)-(5-oxopyrrolidin-2-yl)methoxy or (2R)-(5-oxopyrrolidin-2-yl)methoxy), tetrahydrofuran-3-ylmethoxy, 2-hydroxyethoxy, 2-ethoxyethoxy, 2-(2-hydroxyethoxy)ethoxy, 2-methoxyethoxy, (2-methoxyethoxy)ethoxy, 2-{N-[2-hydroxyethyl]-N-methyl-amino} ethoxy, 2-morpholinoethoxy, 2-(2-oxopyrrolidin-1-yl)ethoxy, 2-(imidazolid-2-on-1-yl)ethoxy, 3-hydroxypropyloxy, 2-hydroxyprop-1-yloxy (for example (2R)-2-hydroxyprop-1-yloxy), 3-methoxyprop-1-yloxy, 2-methoxyprop-1-yloxy (for example (2S)-2-methoxyprop-1-yloxy), 3-morpholinoprop-1-ylox , 3-(methylthio)prop-1-yloxy, 3-(methylsulfonyl)propyl-1-oxy, methoxycarbonyl, N-(tert-butoxycarbonyl)amino, methylamino, 2-methoxyethylamino, 2-aminoethylamino, 2-(dimethylamino)ethylamino, (N-2-methoxyethyl)-N-methylamino, 3-isopropoxyprop-1-ylamino, 2-(2-hydroxyethoxy)ethylamino, 2-(acetoamido)ethylamino, 2-(morpholin-4-yl)ethylamino, 2-methylprop-1-ylamino, 2-hydroxyprop-1-ylamino (for example (2R)-2-hydroxyprop-1-ylamino or (2S)-2-hydroxyprop-1-ylamino), 3-methoxypropylamino, 3-ethoxypropylamino, 2-isopropoxyethylamino, tetrahydrofuran-2-ylmethylamino (for example (2R)-tetrahydrofuran-2-ylmethylamino), dimethylamino, N-(2-hydroxyethyl)-N-ethylamino, cyclobutylamino, 4-methylcyclohexylamino, 4-hydroxycyclohexylamino, carbamoyl, N-hydroxycarbamoyl, N-cyclopropylcarbamoyl, N-cyclopentylcarbamoyl, N-aminocarbamoyl, N-(acetylamino)carbamoyl, N-methylcarbamoyl, 2-hydroxyethylcarbamoyl, N-(2-hydroxypropyl)carbamoyl (for example N-((R)-2-hydroxypropyl)carbamoyl), N-(2,3-dihydroxypropyl)carbamoyl (for example N-((2R)-2,3-dihydroxypropyl)carbamoyl), N-(4-hydroxybutyl)carbamoyl, N-(2-methoxyethyl)carbamoyl, N-(2-(acetylamino)ethyl)carbamoyl, N-[2-(2-hydroxyethoxy)ethyl]carbamoyl, N-(carbamoylmethyl)carbamoyl, N-[2-(methylthio)ethyl]carbamoyl, N-(2-methoxyethyl)-N-methylcarbamoyl, pyrrolidin-1-ylcarbonyl, morpholinocarbonyl, azetidin-1-ylcarbonyl, (3-hydroxypyrrolidin-1-yl)carbonyl (for example (3R)-3-hydroxypyrrolidin-1-ylcarbonyl), methylthio, 2,2,6,6-tetramethylpiperidin-4-ylamino, 4-tetrahydropyranylamino, pyrrolidin-1-yl, morpholino, piperazin-1-yl, 4-methylpiperazin-1-yl, 4-ethylpiperazin-1-yl, 4-isopropylpiperazin-1-yl, 4-(2-hydroxyethyl)piperazin-1-yl, 4-(3-hydroxypropyl)piperazin-1-yl, 4-(2-methoxyethyl)piperazin-1-yl, 4-(2-aminoethyl)piperazin-1-yl, 4-[2-(2-hydroxyethoxy)ethyl]piperazin-1-yl, 4-(2-cyanoethyl)piperazin-1-yl, 4-(tert-butoxycarbonyl)piperazin-1-yl, 1-formyl-piperazin-4-yl, 4-acetylpiperazin-1-yl, 4-(ethylsulfonyl)piperazin-1-yl, 4-aminopiperidin-1-yl, 4-(N-tert-butoxycarbonylamino)piperidin-1-yl, 3-hydroxypyrrolidin-1-yl (for example (3R)-3-hydroxypyrrolidin-1-yl), 3-dimethylamino-pyrrolidin-1-yl (for example (3R)-3-dimethylamino-pyrrolidin-1-yl) and cis-3,4-dihydroxypyrrolidin-1-yl.

Yet further suitable values for R³ include, for example, hydrogen, chloro, iodo, methyl, ethyl, trifluoromethyl, hydroxymethyl, methoxymethyl, ethoxymethyl, (2-methoxyethoxy)methyl, morpholinomethyl, 3-hydroxypropyl, 3-methoxypropyl, 3-N,N-dimethylaminopropyl, ethenyl, 3-hydroxyprop-1-en-1-yl, ethynyl, 3-hydroxyprop-1-yn-1-yl, 3-methoxyprop-1-yn-1-yl, 3-aminoprop-1-yn-1-yl, 3-methylaminoprop-1-yn-1-yl, 3-(dimethylamino)prop-1-yn-1-yl, 3-(N-methylacetamido)prop-1-yn-1-yl, 3-acetamidoprop-1-yn-1-yl, methoxy, ethoxy, (5-oxopyrrolidin-2-yl)methoxy (for example (2S)-(5-oxopyrrolidin-2-yl)methoxy or (2R)-(5-oxopyrrolidin-2-yl)methoxy), tetrahydrofuran-3-ylmethoxy, 2-hydroxyethoxy, 2-ethoxyethoxy, 2-(2-hydroxyethoxy)ethoxy, 2-methoxyethoxy, (2-methoxyethoxy)ethoxy, 2-{N-[2-hydroxyethyl]-N-methyl-amino}ethoxy, 2-morpholinoethoxy, 2-(2-oxopyrrolidin-1-yl)ethoxy, 2-(imidazolid-2-on-1-yl)ethoxy, 3-hydroxypropyloxy, 2-hydroxyprop-1-yloxy (for example (2R)-2-hydroxyprop-1-yloxy), 3-methoxyprop-1-yloxy, 2-methoxyprop-1-yloxy (for example (2S)-2-methoxyprop-1-yloxy), 3-morpholinoprop-1-yloxy, 3-(methylthio)prop-1-yloxy, 3-(methylsulfonyl)propyl-1-oxy, methylamino, 2-methoxyethylamino, 2-(methoxyethyl)amino, 2-(2-hydroxyethoxy)ethylamino, 2-(morpholin-4-yl)ethylamino, 2-methylprop-1-ylamino, 2-hydroxyprop-1-ylamino (for example (2R)-2-hydroxyprop-1-ylamino or (2S)-2-hydroxyprop-1-ylamino), 3-methoxypropylamino, 3-ethoxypropylamino, 2-isopropoxyethylamino, tetrahydrofuran-2-ylmethylamino (for example (2R)-tetrahydrofuran-2-ylmethylamino), dimethylamino, N-(2-hydroxyethyl)-N-ethylamino, cyclobutylamino, carbamoyl, N-cyclopropylcarbamoyl, N-methylcarbamoyl, 2-hydroxyethylcarbamoyl, N-(2-hydroxypropyl)carbamoyl (for example N-((R)-2-hydroxypropyl)carbamoyl), N-(2-methoxyethyl)carbamoyl, N-[2-(methylthio)ethyl]carbamoyl, pyrrolidin-1-ylcarbonyl, azetidin-1-ylcarbonyl, methylthio, 4-tetrahydropyranylamino, pyrrolidin-1-yl, morpholino, piperazin-1-yl, 4-methylpiperazin-1-yl, 4-ethylpiperazin-1-yl, 4-isopropylpiperazin-1-yl, 4-(2-hydroxyethyl)piperazin-1-yl, 4-(3-hydroxypropyl)piperazin-1-yl, 4-(2-methoxyethyl)piperazin-1-yl, 4-(2-cyanoethyl)piperazin-1-yl, , 4-acetylpiperazin-1-yl, 4-(ethylsulfonyl)piperazin-1-yl, 3-hydroxypyrrolidin-1-yl (for example (3R)-3-hydroxypyrrolidin-1-yl), 3-dimethylamino-pyrrolidin-1-yl (for example (3R)-3-dimethylamino-pyrrolidin-1-yl) and 1-formyl-piperazin-4-yl.

Yet further suitable values for R³ include, for example, hydrogen, chloro, iodo, methyl, ethyl, trifluoromethyl, hydroxymethyl, methoxymethyl, ethoxymethyl, (2-methoxyethoxy)methyl, morpholinomethyl, 3-hydroxypropyl, 3-methoxypropyl, 3-cyanopropyl, (1-cyanocyclopropyl)methoxy, 3-N,N-dimethylaminopropyl, ethenyl, 3-hydroxyprop-1-en-1-yl, ethynyl, 3-hydroxyprop-1-yn-1-yl, 3-methoxyprop-1-yn-1-yl, 3-aminoprop-1-yn-1-yl, 3-methylaminoprop-1-yn-1-yl, 3-(dimethylamino)prop-1-yn-1-yl, 3-(N-methylacetamido)prop-1-yn-1-yl, 3-acetamidoprop-1-yn-1-yl, methoxy, ethoxy, (5-oxopyrrolidin-2-yl)methoxy, tetrahydrofuran-3-ylmethoxy, 2-hydroxyethoxy, 2-ethoxyethoxy, 2-(2-hydroxyethoxy)ethoxy, 2-methoxyethoxy, (2-methoxyethoxy)ethoxy, 2-{N-[2-hydroxyethyl]-N-methyl-amino}ethoxy, 2-morpholinoethoxy, 2-(2-oxopyrrolidin-1-yl)ethoxy, 2-(imidazolid-2-on-1-yl)ethoxy, 3-hydroxypropyloxy, 2-hydroxyprop-1-yloxy, 3-methoxyprop-1-yloxy, 2-methoxyprop-1-yloxy, 3-morpholinoprop-1-yloxy, 3-(methylthio)prop-1-yloxy, 3-(methylsulfonyl)propyl-1-oxy, methylamino, 2-methoxyethylamino, 2-(methoxyethyl)amino, 2-(2-hydroxyethoxy)ethylamino, 2-(morpholin-4-yl)ethylamino, 2-methylprop-1-ylamino, 2-hydroxyprop-1-ylamino, 3-methoxypropylamino, 3-ethoxypropylamino, 2-isopropoxyethylamino, tetrahydrofuran-2-ylmethylamino, dimethylamino, N-(2-hydroxyethyl)-N-ethylamino, cyclobutylamino, carbamoyl, N-cyclopropylcarbamoyl, N-methylcarbamoyl, 2-hydroxyethylcarbamoyl, N-(2-hydroxypropyl)carbamoyl, N-(2-methoxyethyl)carbamoyl, N-[2-(methylthio)ethyl]carbamoyl, pyrrolidin-1-ylcarbonyl, azetidin-1-ylcarbonyl, methylthio, 4-tetrahydropyranylamino, pyrrolidin-1-yl, morpholino, piperazin-1-yl, 4-methylpiperazin-1-yl, 4-ethylpiperazin-1-yl, 4-isopropylpiperazin-1-yl, 4-(2-hydroxyethyl)piperazin-1-yl, 4-(3-hydroxypropyl)piperazin-1-yl, 4-(2-methoxyethyl)piperazin-1-yl, 4-(2-cyanoethyl)piperazin-1-yl, , 4-acetylpiperazin-1-yl, 4-(ethylsulfonyl)piperazin-1-yl, 3-hydroxypyrrolidin-1-yl, 3-dimethylamino-pyrrolidin-1-yl and 1-formyl-piperazin-4-yl.

In another aspect of the invention, R³ is selected from hydrogen, methyl, ethyl and methoxy.

In yet another aspect of the invention, R³ is selected from hydrogen, chloro, methyl, ethyl, methoxy, ethoxy, 2-hydroxyethoxy, 2-methoxyethoxy, 2-hydroxypropoxy, (1-cyanocyclopropyl)methoxy, 3-cyanopropoxy, 3-hydroxypropoxy, 3-methoxypropoxy, 2-ethoxyethoxy and morpholino (especially hydrogen, methyl, ethyl, methoxy, ethoxy, 2-hydroxyethoxy, 2-methoxyethoxy, 2-hydroxypropoxy, (1-cyanocyclopropyl)methoxy, 3-cyanopropoxy, 3-hydroxypropoxy, 3-methoxypropoxy, 2-ethoxyethoxy and morpholino).

In yet another aspect of the invention, R³ is methyl.

In one aspect of the invention, a suitable value for Q¹ is a substituted 5- or 6-membered heteroaromatic ring comprising one, two, three or four ring heteroatoms (for example, one, two or three, especially one or two, ring heteroatoms) selected from nitrogen, oxygen and sulfur (such as pyridyl, imidazolyl, isoxazolyl, pyrazolyl, furyl, pyrazinyl, pyridazinyl, pyrimidinyl, pyrrolyl, thiazolyl, oxazolyl, isothiazolyl, triazolyl, tetrahydrofuranyl or thienyl, particularly pyridyl, pyrazinyl, thiazolyl, tetrahydrofuranyl or pyrimidinyl, especially pyridyl or pyrazinyl).

In another aspect of the invention, a suitable value for Q¹ is a substituted 5- or 6-membered heteroaromatic ring selected from pyridyl, imidazolyl, isoxazolyl, pyrazolyl, furyl, pyrazinyl (such as pyrazin-2-yl), pyridazinyl, pyrimidinyl (such as pyrimidin-2-yl), pyrrolyl, oxazolyl, isothiazolyl, triazolyl, tetrahydrofuranyl or thienyl, especially pyridyl (such as pyrid-2-yl or pyrid-3-yl) or pyrazinyl (such as pyrazin-2-yl).

In yet another aspect of the invention, a suitable value for Q¹ is a substituted 5- or 6-membered heteroaromatic ring comprising one or two ring nitrogen atoms, such as pyridyl (for example pyrid-2-yl or pyrid-3-yl, especially pyrid-3-yl), pyrazinyl (for example pyrazin-2-yl) or pyrimidinyl (for example pyrimidin-2-yl). A particular value for Q¹ in this aspect of the invention is pyridyl (for example pyrid-2-yl or pyrid-3-yl, especially pyrid-2-yl).

In yet another aspect of the invention, a suitable value for Q¹ is pyrid-3-yl or pyrazin-2-yl.

In one aspect of the invention, suitable substituents for Q¹ include one, two, three or four (for example one, two or three, especially one or two) substituents independently selected from (C1-C6)alkyl and (C1-C6)alkoxy (either of which (C1-C6)alkyl and (C1-C6)alkoxy groups may be optionally substituted by at least one substituent (for example, one, two, three or four substituents) independently selected from (C1-C3)alkoxy, halogeno, amino, hydroxy and trifluoromethyl), oxo, halogeno, nitro, cyano, -NR⁴R⁵, carboxy, hydroxy, (C2-C6)alkenyl, (C3-C8)cycloalkyl, (C1-C6)alkoxycarbonyl, (C1-C6)alkylcarbonyl, (C2-C6)alkanoylamino, phenylcarbonyl, -S(O)ₙ(C1-C6)alkyl, -C(O)NR⁶R⁷ and -SO₂NR⁸R⁹ (where n, R⁴, R⁵, R⁶, R⁷, R⁸ and R⁹ are as defined above).

In another aspect of the invention, suitable substituents for Q¹ include one, two, three or four (for example one, two or three, especially one or two) substituents independently selected from (C1-C6)alkyl and (C1-C6)alkoxy (either of which (C1-C6)alkyl and (C1-C6)alkoxy groups may be optionally substituted by at least one substituent (for example, one, two, three or four substituents) independently selected from halogeno, amino, hydroxy and trifluoromethyl), halogeno, nitro, cyano, -NR⁴R⁵, carboxy, hydroxy, (C2-C6)alkenyl, (C3-C8)cycloalkyl, (C1-C6)alkoxycarbonyl, (C1-C6)alkylcarbonyl, (C2-C6)alkanoylamino, phenylcarbonyl, -S(O)ₙ(C1-C6)alkyl, -C(O)NR⁶R⁷ and -SO₂NR⁸R⁹ (where n, R⁴, R⁵, R⁶, R⁷, R⁸ and R⁹ are as defined above).

In another aspect of the invention, suitable substituents for Q¹ include one or more (for example, one or two, particularly one) substituents independently selected from oxo, halogeno (such as chloro), hydroxy, cyano, (C1-C4)alkyl (such as methyl or ethyl), (C1-C4)alkoxy, which (C1-C4)alkoxy may be optionally substituted by (C1-C3)alkoxy (such as methoxy, ethoxy or methoxyethoxy) and -NR⁴R⁵ (where R⁴ and R⁵ are as defined above). For example, suitable values for the substituent on Q¹ include oxo, chloro, hydroxy, cyano, methyl, ethyl, methoxy, ethoxy, methoxyethoxy, methylamino and ethylamino.

In another aspect of the invention, suitable substituents for Q¹ include one or more (for example, one or two, particularly one) substituents independently selected from halogeno (such as chloro), hydroxy, cyano, (C1-C4)alkyl (such as methyl or ethyl), (C1-C4)alkoxy, which (C1-C4)alkoxy may be optionally substituted by (C1-C3)alkoxy (such as methoxy, ethoxy or methoxyethoxy) and -NR⁴R⁵ (where R⁴ and R⁵ are as defined above). For example, suitable values for the substituent on Q¹ include chloro, hydroxy, cyano, methyl, ethyl, methoxy, ethoxy, methoxyethoxy, methylamino and ethylamino.

In another aspect of the invention, suitable substituents for Q¹ include one or more (for example, one or two, particularly one) substituents independently selected from hydroxy, cyano, (C1-C4)alkyl (such as methyl or ethyl), (C1-C4)alkoxy, which (C1-C4)alkoxy may be optionally substituted by (C1-C3)alkoxy (such as methoxy, ethoxy or methoxyethoxy) and - NR⁴R⁵ (where R⁴ and R⁵ are as defined above). For example, suitable values for the substituent on Q¹ include hydroxy, cyano, methyl, ethyl, methoxy, ethoxy, methoxyethoxy, methylamino and ethylamino.

In yet another aspect of the invention, suitable substituents for Q¹ include one or more (for example, one or two, particularly one) substituents independently selected from halogeno, (C1-C4)alkyl, (C1-C4)alkoxy, cyano and -NR⁴R⁵ (where R⁴ and R⁵ are as defined above). For example, suitable values for the substituent on Q¹ include chloro, methyl, methoxy, ethoxy, cyano, methylamino and ethylamino.

Suitably, R⁴, R⁵, R⁶, R⁷, R⁸ and R⁹ may each independently represent hydrogen or (C1-C4)alkyl (such as methyl), or R⁴ and R⁵, or R⁶ and R⁷, or R⁸ and R⁹, when taken together with the nitrogen atom to which they are attached, may each suitably form a saturated heterocyclic ring, such as pyrrolidinyl or piperidinyl.

It will be appreciated that the number and nature of substituents on rings in the compounds of the invention will be selected so as to avoid sterically undesirable combinations.

In one group of compounds of formula (I) according to the invention, R¹ is methyl; R² is hydrogen; R³ is selected from hydrogen, (C1-C4)alkyl and (C1-C4)alkoxy; Q¹ is a 5- or 6-membered heteroaromatic ring comprising at least one ring heteroatom selected from nitrogen, oxygen and sulfur, which ring Q¹ is substituted by one or more substituents selected from (C1-C4)alkyl, (C1-C4)alkoxy, halogeno, cyano and -NR⁴R⁵ (wherein R⁴ and R⁵ are as defined above). For example, within this group, suitable values for Q¹ are pyrazinyl and pyridyl. Suitable values for the substituent on Q¹ within this group include chloro, methyl, methoxy, ethoxy, cyano, methylamino and ethylamino.

In another group of compounds of formula (I) according to the invention, R¹ is selected from methyl and cyclopropyl; R² is hydrogen; R³ is selected from hydrogen, halogeno, (C1-C4)alkyl, (C1-C4)alkoxy and a saturated monocyclic 6-membered ring comprising at least one ring heteroatom selected from nitrogen and oxygen (each of which groups or rings within R³ may carry one or more substituents as hereinbefore defined); Q¹ is a 6-membered heteroaromatic ring comprising at least one ring heteroatom selected from nitrogen, oxygen and sulfur, which ring Q¹ is substituted by one or more substituents selected from hydroxy, (C1-C4)alkyl, (C1-C4)alkoxy (optionally substituted by (C1-C2)alkoxy), oxo, halogeno, cyano and -NR⁴R⁵ (wherein R⁴ and R⁵ are as defined above) (especially hydroxy, (C1-C4)alkyl, (C1-C4)alkoxy (optionally substituted by (C1-C2)alkoxy), cyano and -NR⁴R⁵ (wherein R⁴ and R⁵ are as defined above)). For example, within this group, suitable values for Q¹ are pyrazinyl and pyridyl. Suitable values for the substituent on Q¹ within this group include hydroxy, oxo, chloro, methyl, ethyl, methoxy, ethoxy, methoxyethoxy, cyano, methylamino and ethylamino (especially hydroxy, methyl, ethyl, methoxy, ethoxy, methoxyethoxy, cyano, methylamino and ethylamino).

In one aspect of the invention, suitable values for the group of sub-formula (i) (which is attached to the 2-position of the pyrimidine ring of formula (I)): 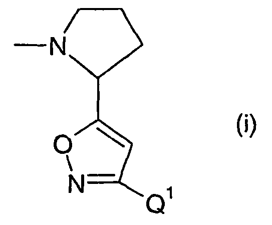 include, for example, 2-[3-(3-ethoxypyrazin-2-yl)isoxazol-5-yl]pyrrolidin-1-yl, 2-[3-(3-ethylaminopyrazin-2-yl)isoxazol-5-yl]pyrrolidin-1-yl, 2-[3-(2-methylpyrid-3-yl)isoxazol-5-yl]pyrrolidin-1-yl, 2-[3-(2-chloropyrid-3-yl)isoxazol-5-yl]pyrrolidin-1-yl, 2-[3-(2-cyanopyrid-3-yl)isoxazol-5-yl]pyrrolidin-1-yl, 2-[3-(3-methylpyrazin-2-yl)isoxazol-5-yl]pyrrolidin-1-yl, 2-[3-(2-methylaminopyrid-3-yl)isoxazol-5-yl]pyrrolidin-1-yl, 2-[3-(2-methoxypyrid-3-yl)isoxazol-5-yl]pyrrolidin-1-yl, 2-[3-(3-ethylpyrazin-2-yl)isoxazol-5-yl]pyrrolidin-1-yl, 2-[3-(3-(methoxyethoxy)pyrazin-2-yl)isoxazol-5-yl]pyrrolidin-1-yl, 2-[3-(3-hydroxypyrazin-2-yl)isoxazol-5-yl]pyrrolidin-1-yl, 2-[3-(3-methoxypyrazin-2-yl)isoxazol-5-yl]pyrrolidin-1-yl and 2-{3-(1-oxopyrid-3-yl)isoxazol-5-yl}pyrrolidin-1-yl (where, for the avoidance of any doubt, it is the pyrrolidin-1-yl group that is attached to the 2-position of the pyrimidine ring in formula (I)).

In another aspect of the invention, suitable values for the group of sub-formula (i) (which is attached to the 2-position of the pyrimidine ring of formula (I)) include, for example, 2-[3-(3-ethoxypyrazin-2-yl)isoxazol-5-yl]pyrrolidin-1-yl, 2-[3-(3-ethylaminopyrazin-2-yl)isoxazol-5-yl]pyrrolidin-1-yl, 2-[3-(2-methylpyrid-3-yl)isoxazol-5-yllpyrrolidin-1-yl, 2-[3-(2-chloropyrid-3-yl)isoxazol-5-yl]pyrrolidin-1-yl, 2-[3-(2-cyanopyrid-3-yl)isoxazol-5-yl]pyrrolidin-1-yl, 2-[3-(3-methylpyrazin-2-yl)isoxazol-5-yl]pyrrolidin-1-yl, 2-[3-(2-methylaminopyrid-3-yl)isoxazol-5-yl]pyrrolidin-1-yl, 2-[3-(2-methoxypyrid-3-yl)isoxazol-5-yl]pyrrolidin-1-yl, 2-[3-(3-ethylpyrazin-2-yl)isoxazol-5-yl]pyrrolidin-1-yl, 2-[3-(3-(methoxyethoxy)pyrazin-2-yl)isoxazol-5-yl]pyrrolidin-1-yl, 2-[3-(3-hydroxypyrazin-2-yl)isoxazol-5-yl]pyrrolidin-1-yl and 2-[3-(3-methoxypyrazin-2-yl)isoxazol-5-yl]pyrrolidin-1-yl (where, for the avoidance of any doubt, it is the pyrrolidin-1-yl group that is attached to the 2-position of the pyrimidine ring in formula (I)).

In another aspect of the invention, suitable values for the group of sub-formula (i) (which is attached to the 2-position of the pyrimidine ring of formula (I)) include, for example, 2-[3-(3-ethoxypyrazin-2-yl)isoxazol-5-yl]pyrrolidin-1-yl, 2-[3-(3-ethylaminopyrazin-2-yl)isoxazol-5-yl]pyrrolidin-1-yl, 2-[3-(2-methylpyrid-3-yl)isoxazol-5-yl]pyrrolidin-1-yl, 2-[3-(2-chloropyrid-3-yl)isoxazol-5-yl]pyrrolidin-1-yl, 2-[3-(2-cyanopyrid-3-yl)isoxazol-5-yl]pyrrolidin-1-yl, 2-[3-(3-methylpyrazin-2-yl)isoxazol-5-yl]pyrrolidin-1-yl, 2-[3-(2-methylaminopyrid-3-yl)isoxazol-5-yl]pyrrolidin-1-yl, 2-[3-(2-methoxypyrid-3-yl)isoxazol-5-yl]pyrrolidin-1-yl, 2-[3-(3-ethylpyrazin-2-yl)isoxazol-5-yl]pyrrolidin-1-yl, 2-[3-(3-(methoxyethoxy)pyrazin-2-yl)isoxazol-5-yl]pyrrolidin-1-yl and 2-[3-(3-methoxypyrazin-2-yl)isoxazol-5-yl]pyrrolidin-1-yl (where, for the avoidance of any doubt, it is the pyrrolidin-1-yl group that is attached to the 2-position of the pyrimidine ring in formula (I)).

In another aspect of the invention, suitable values for the group of sub-formula (i) (which is attached to the 2-position of the pyrimidine ring of formula (I)) include, for example, 2-[3-(3-ethoxypyrazin-2-yl)isoxazol-5-yl]pyrrolidin-1-yl, 2-[3-(3-ethylaminopyrazin-2-yl)isoxazol-5-yl]pyrrolidin-1-yl, 2-[3-(2-methylpyrid-3-yl)isoxazol-5-yl]pyrrolidin-1-yl, 2-[3-(2-chloropyrid-3-yl)isoxazol-5-yl]pyrrolidin-1-yl, 2-[3-(2-cyanopyrid-3-yl)isoxazol-5-yl]pyrrolidin-1-yl, 2-[3-(3-methylpyrazin-2-yl)isoxazol-5-yl]pyrrolidin-1-yl, 2-[3-(2-methylaminopyrid-3-yl)isoxazol-5-yl]pyrrolidin-1-yl and 2-[3-(3-methoxypyrazin-2-yl)isoxazol-5-yl]pyrrolidin-1-yl (where, for the avoidance of any doubt, it is the pyrrolidin-1-yl group that is attached to the 2-position of the pyrimidine ring in formula (I)).

In yet another aspect of the invention, suitable values for the group of sub-formula (i) (which is attached to the 2-position of the pyrimidine ring of formula (I)) include, for example, 2-[3-(3-ethoxypyrazin-2-yl)isoxazol-5-yl]pyrrolidin-1-yl, 2-[3-(3-ethylaminopyrazin-2-yl)isoxazol-5-yl]pyrrolidin-1-yl, 2-[3-(2-methylpyrid-3-yl)isoxazol-5-yl]pyrrolidin-1-yl, 2-[3-(2-chloropyrid-3-yl)isoxazol-5-yl]pyrrolidin-1-yl and 2-[3-(2-cyanopyrid-3-yl)isoxazol-5-yl]pyrrolidin-1-yl (where, for the avoidance of any doubt, it is the pyrrolidin-1-yl group that is attached to the 2-position of the pyrimidine ring in formula (I)).

A particular embodiment of the present invention is a compound of formula (Ia): 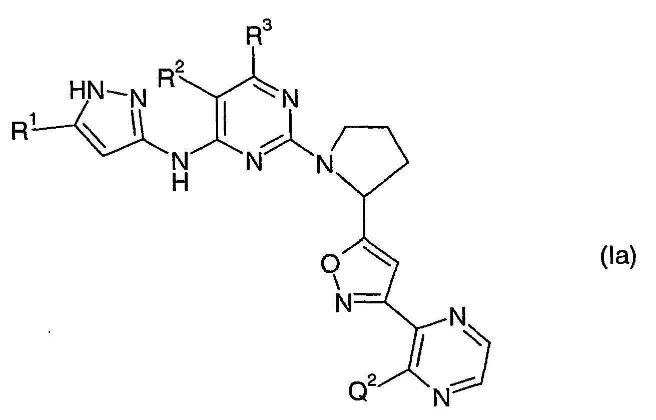 wherein:
R¹ is selected from methyl, ethyl, isopropyl and cyclopropyl;
R² is selected from hydrogen and halogeno;
R³ is selected from hydrogen, hydroxy and halogeno, or from a (C1-C6)alkyl, (C2-C6)alkenyl, (C2-C6)alkynyl, (C1-C6)alkoxy, (C1-C6)alkylcarbonyl, (C1-C6)alkoxycarbonyl, amino, (C1-C6)alkylamino, di-[(C1-C6)alkyl]amino, (C3-C8)cycloalkylamino, carbamoyl, (C1-C6)alkylcarbamoyl, di-[(C1-C6)alkyl]carbamoyl, -C(O)R^{3b}, -NHR^{3b}, -SR^{3a} or - N(R^{3c})C(O)R^{3a} group, wherein R^{3a} is selected from a (C1-C6)alkyl or (C1-C6)alkoxy group, R^{3b} is a saturated monocyclic 4-, 5- or 6-membered heterocyclic ring comprising at least one ring heteroatom selected from nitrogen, oxygen and sulfur and R^{3c} is selected from hydrogen and (C1-C6)alkyl,
   or R³ is a saturated monocyclic 5- or 6-membered heterocyclic ring comprising at least one ring heteroatom selected from nitrogen, oxygen and sulfur,
   each of which groups or rings within R³ may be optionally substituted by one or more substituents independently selected from (C1-C6)alkyl, (C1-C6)alkoxy, (C1-C6)alkoxy(C1-C6)alkyl, (C1-C6)alkoxy(C1-C6)alkoxy, halogeno, hydroxy, trifluoromethyl, tri-[(C1-C4)alkyl]silyl, cyano, amino, (C1-C6)alkylamino, di-[(C1-C6)alkyl]amino, amino(C1-C6)alkyl, (C1-C6)alkylamino(C1-C6)alkyl, di-[(C1-C6)alkyl]amino(C1-C6)alkyl, (C1-C6)alkoxycarbonyl, carbamoyl, (C1-C6)alkylcarbamoyl, di-[(C1-C6)alkyl]carbamoyl, (C1-C6)alkylthio, (C1-C6)alkylsulfonyl, (C1-C6)alkylsulfinyl, (C1-C6)alkanoyl, an alkanoylamino group -N(R^{3d})C(O)R^{3e} wherein R^{3d} is selected from hydrogen and (C1-C6)alkyl and R^{3e} is selected from a (C1-C6)alkyl or (C1-C6)alkoxy group, or a saturated monocyclic 3-, 4-, 5-, 6- or 7-membered ring, which ring may optionally comprise one or more heteroatoms selected from nitrogen, oxygen and sulfur, any of which substituents may be optionally substituted by one or more (C1-C4)alkyl, hydroxy or cyano groups;
Q² is selected from (C1-C6)alkyl and (C1-C6)alkoxy (either of which (C1-C6)alkyl and (C1-C6)alkoxy groups may be optionally substituted by one or more substituents independently selected from (C1-C4)alkoxy, halogeno, amino, hydroxy and trifluoromethyl), halogeno, nitro, cyano, -NR⁴R⁵, carboxy, hydroxy, (C2-C6)alkenyl, (C3-C8)cycloalkyl, (C1-C6)alkoxycarbonyl, (C1-C6)alkylcarbonyl, (C2-C6)alkanoylamino, phenylcarbonyl, -S(O)ₙ(C1-C6)alkyl, -C(O)NR⁶R⁷ and -SO₂NR⁸R⁹, wherein R⁴, R⁵, R⁶, R⁷, R⁸ and R⁹ are each independently selected from hydrogen and (C1-C6)alkyl, or R⁴ and R⁵, or R⁶ and R⁷, or R⁸ and R⁹, when taken together with the nitrogen atom to which they are attached, may each independently form a saturated heterocyclic ring and n is 0, 1 or 2,
   and wherein any saturated monocyclic ring optionally bears 1 or 2 oxo or thioxo substituents;
   or a pharmaceutically-acceptable salt thereof.

Another particular embodiment of the present invention is a compound of formula (Ia) wherein:
R¹ is selected from methyl, ethyl, isopropyl and cyclopropyl;
R² is selected from hydrogen and halogeno;
R³ is selected from hydrogen, hydroxy and halogeno, or from a (C1-C6)alkyl, (C2-C6)alkenyl, (C2-C6)alkynyl, (C1-C6)alkoxy, (C1-C6)alkylcarbonyl, (C1-C6)alkoxycarbonyl, amino, (C1-C6)alkylamino, di-[(C1-C6)alkyl]amino, (C3-C8)cycloalkylamino, carbamoyl, (C1-C6)alkylcarbamoyl, di-[(C1-C6)alkyl]carbamoyl, -C(O)R^{3b}, -NHR^{3b}, -SR^{3a} or - N(R^{3c})C(O)R^{3a} group, wherein R^{3a} is selected from a (C1-C6)alkyl or (C1-C6)alkoxy group, R^{3b} is a saturated monocyclic 4-, 5- or 6-membered heterocyclic ring comprising at least one ring heteroatom selected from nitrogen, oxygen and sulfur and R^{3c} is selected from hydrogen and (C1-C6)alkyl,
   or R³ is a saturated monocyclic 5- or 6-membered heterocyclic ring comprising at least one ring heteroatom selected from nitrogen, oxygen and sulfur,
   each of which groups or rings within R³ may be optionally substituted by one or more substituents independently selected from (C1-C6)alkyl, (C1-C6)alkoxy, (C1-C6)alkoxy(C1-C6)alkyl, (C1-C6)alkoxy(C1-C6)alkoxy, halogeno, hydroxy, trifluoromethyl, tri-[(C1-C4)alkyl]silyl, cyano, amino, (C1-C6)alkylamino, di-[(C1-C6)alkyl]amino, amino(C1-C6)alkyl, (C1-C6)alkylamino(C1-C6)alkyl, di-[(C1-C6)alkyl]amino(C1-C6)alkyl, (C1-C6)alkoxycarbonyl, carbamoyl, (C1-C6)alkylcarbamoyl, di-[(C1-C6)alkyl]carbamoyl, (C1-C6)alkylthio, (C1-C6)alkylsulfonyl, (C1-C6)alkylsulfinyl, (C1-C6)alkanoyl, an alkanoylamino group -N(R^{3d})C(O)R^{3e} wherein R^{3d} is selected from hydrogen and (C1-C6)alkyl and R^{3e} is selected from a (C1-C6)alkyl or (C1-C6)alkoxy group, or a saturated monocyclic 3-, 4-, 5-, 6- or 7-membered ring, which ring may optionally comprise one or more heteroatoms selected from nitrogen, oxygen and sulfur, any of which substituents may be optionally substituted by one or more (C1-C4)alkyl, hydroxy or cyano groups;
Q² is selected from (C1-C6)alkyl and (C1-C6)alkoxy (either of which (C1-C6)alkyl and (C1-C6)alkoxy groups may be optionally substituted by one or more substituents independently selected from halogeno, amino, hydroxy and trifluoromethyl), halogeno, nitro, cyano, -NR⁴R⁵, carboxy, hydroxy, (C2-C6)alkenyl, (C3-C8)cycloalkyl, (C1-C6)alkoxycarbonyl, (C1-C6)alkylcarbonyl, (C2-C6)alkanoylamino, phenylcarbonyl, -S(O)ₙ(C1-C6)alkyl, -C(O)NR⁶R⁷ and -SO₂NR⁸R⁹, wherein R⁴, R⁵, R⁶, R⁷, R⁸ and R⁹ are each independently selected from hydrogen and (C1-C6)alkyl, or R⁴ and R⁵, or R⁶ and R⁷, or R⁸ and R⁹, when taken together with the nitrogen atom to which they are attached, may each independently form a saturated heterocyclic ring and n is 0, 1 or 2,
   and wherein any saturated monocyclic ring optionally bears 1 or 2 oxo or thioxo substituents;
   or a pharmaceutically-acceptable salt thereof.
   In the compounds of formula (Ia), suitable values for Q² are (C1-C4)alkyl (such as methyl), (C1-C4)alkoxy (such as methoxy or ethoxy), halogeno (such as chloro), cyano and -NR⁴R⁵ (wherein R⁴ and R⁵ are as defined above, for example R⁴ may be hydrogen and R⁵ may be (C1-C4)alkyl, such as methyl or ethyl).
   In another aspect of the invention, in the compounds of formula (Ia), suitable values for Q² are hydroxy, (C1-C4)alkyl (such as methyl or ethyl), (C1-C4)alkoxy, optionally substituted by (C1-C2)alkoxy (such as methoxy, ethoxy or methoxyethoxy) and -NR⁴R⁵ (wherein R⁴ and R⁵ are as defined above, for example R⁴ may be hydrogen and R⁵ may be (C1-C4)alkyl, such as ethyl).
   In yet another aspect of the invention, in the compounds of formula (Ia), suitable values for Q² are (C1-C4)alkyl (such as methyl or ethyl) and -NR⁴R⁵ (wherein R⁴ and R⁵ are as defined above, for example R⁴ may be hydrogen and R⁵ may be (C1-C4)alkyl, such as ethyl).
   As stated above, the compounds of the present invention, for example of formula (Ia) (or a pharmaceutically-acceptable salt thereof) wherein R¹, R², R³ and Q¹ are as defined above do not include:
   S-6-chloro-4-(5-methyl-1*H*-pyrazol-3-ylamino)-2-[2-{3-(3-methoxypyrazin-2-yl)isoxazol-5-yl}pyrrolidin-1-yl]pyrimidine;
   S-6-morpholino-4-(5-methyl-1*H*-pyrazol-3-ylamino)-2-[2-{3-(3-methoxypyrazin-2-yl)isoxazol-5-yl}pyrrolidin-1-yl]pyrimidine;
   S-6-morpholino-4-(5-methyl-1*H*-pyrazol-3-ylamino)-2-[2-{3-(3-hydroxypyrazin-2-yl)isoxazol-5-yl}pyrrolidin-1-yl]pyrimidine;
   S-6-methyl-4-(5-methyl-1*H*-pyrazol-3-ylamino)-2-[2-{3-(3-methoxypyrazin-2-yl)isoxazol-5-yl}pyrrolidin-1-yl]pyrimidine;
   S-6-methyl-4-(5-ethyl-1*H*-pyrazol-3-ylamino)-2-[2-{3-(3-methoxypyrazin-2-yl)isoxazol-5-yl}pyrrolidin-1-yl]pyrimidine;
   S-6-chloro-4-(5-ethyl-1*H*-pyrazol-3-ylamino)-2-[2-{3-(3-methoxypyrazin-2-yl)isoxazol-5-yl}pyrrolidin-1-yl]pyrimidine;
   S-6-methyl-4-(5-cyclopropyl-1H-pyrazol-3-ylamino)-2-[2-{3-(3-methoxypyrazin-2-yl)isoxazol-5-yl}pyrrolidin-1-yl]pyrimidine;
   S-6-morpholino-4-(5-ethyl-1H-pyrazol-3-ylamino)-2-[2-{3-(3-methoxypyrazin-2-yl)isoxazol-5-yl}pyrrolidin-1-yl]pyrimidine; or
   S-6-morpholino-4-(5-ethyl-1H-pyrazol-3-ylamino)-2-[2-{3-(3-hydroxypyrazin-2-yl)isoxazol-5-yl }pyrrolidin-1-yl]pyrimidine.

A particular embodiment of the present invention is a compound of formula (Ib): 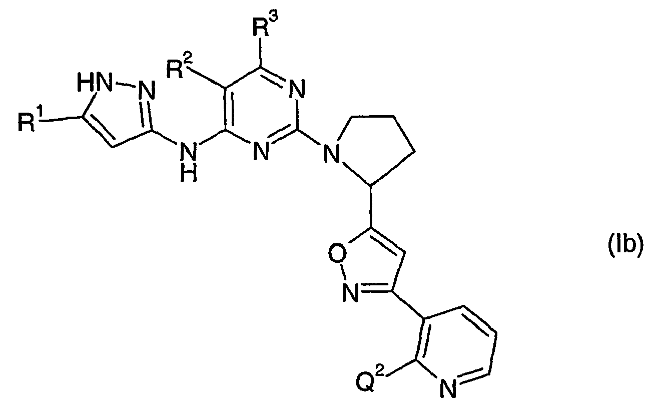 wherein:
R¹ is selected from methyl, ethyl, isopropyl and cyclopropyl;
R² is selected from hydrogen and halogeno;
R³ is selected from hydrogen, hydroxy and halogeno, or from a (C1-C6)alkyl, (C2-C6)alkenyl, (C2-C6)alkynyl, (C1-C6)alkoxy, (C1-C6)alkylcarbonyl, (C1-C6)alkoxycarbonyl, amino, (C1-C6)alkylamino, di-[(C1-C6)alkyl]amino, (C3-C8)cycloalkylamino, carbamoyl, (C1-C6)alkylcarbamoyl, di-[(C1-C6)alkyl]carbamoyl, -C(O)R^{3b}, -NHR^{3b}, -SR^{3a} or - N(R^{3c})C(O)R^{3a} group, wherein R^{3a} is selected from a (C1-C6)alkyl or (C1-C6)alkoxy group, R^{3b} is a saturated monocyclic 4-, 5- or 6-membered heterocyclic ring comprising at least one ring heteroatom selected from nitrogen, oxygen and sulfur and R^{3c} is selected from hydrogen and (C1-C6)alkyl,
   or R³ is a saturated monocyclic 5- or 6-membered heterocyclic ring comprising at least one ring heteroatom selected from nitrogen, oxygen and sulfur,
   each of which groups or rings within R³ may be optionally substituted by one or more substituents independently selected from (C1-C6)alkyl, (C1-C6)alkoxy, (C1-C6)alkoxy(C1-C6)alkyl, (C1-C6)alkoxy(C1-C6)alkoxy, halogeno, hydroxy, trifluoromethyl, tri-[(C1-C4)alkyl]silyl, cyano, amino, (C1-C6)alkylamino, di-[(C1-C6)alkyl]amino, amino(C1-C6)alkyl, (C1-C6)alkylamino(C1-C6)alkyl, di-[(C1-C6)alkyl]amino(C1-C6)alkyl, (C1-C6)alkoxycarbonyl, carbamoyl, (C1-C6)alkylcarbamoyl, di-[(C1-C6)alkyl]carbamoyl, (C1-C6)alkylthio, (C1-C6)alkylsulfonyl, (C1-C6)alkylsulfinyl, (C1-C6)alkanoyl, an alkanoylamino group -N(R^{3d})C(O)R^{3e} wherein R^{3d} is selected from hydrogen and (C1-C6)alkyl and R^{3e} is selected from a (C1-C6)alkyl or (C1-C6)alkoxy group, or a saturated monocyclic 3-, 4-, 5-, 6- or 7-membered ring, which ring may optionally comprise one or more heteroatoms selected from nitrogen, oxygen and sulfur, any of which substituents may be optionally substituted by one or more (C1-C4)alkyl, hydroxy or cyano groups;
Q² is selected from (C1-C6)alkyl and (C1-C6)alkoxy (either of which (C1-C6)alkyl and (C1-C6)alkoxy groups may be optionally substituted by one or more substituents independently selected from halogeno, amino, hydroxy and trifluoromethyl), halogeno, nitro, cyano, -NR⁴R⁵, carboxy, hydroxy, (C2-C6)alkenyl, (C3-C8)cycloalkyl, (C1-C6)alkoxycarbonyl, (C1-C6)alkylcarbonyl, (C2-C6)alkanoylamino, phenylcarbonyl, -S(O)ₙ(C1-C6)alkyl, -C(O)NR⁶R⁷ and -SO₂NR⁸R⁹, wherein R⁴, R⁵, R⁶, R⁷, R⁸ and R⁹ are each independently selected from hydrogen and (C1-C6)alkyl, or R⁴ and R⁵, or R⁶ and R⁷, or R⁸ and R⁹, when taken together with the nitrogen atom to which they are attached, may each independently form a saturated heterocyclic ring and n is 0, 1 or 2,
   and wherein any saturated monocyclic ring optionally bears 1 or 2 oxo or thioxo substituents;
   or a pharmaceutically-acceptable salt thereof.
   In the compounds of formula (Ib), suitable values for Q² are (C1-C4)alkyl (such as methyl), (C1-C4)alkoxy (such as methoxy or ethoxy), halogeno (such as chloro), cyano and -NR⁴R⁵ (wherein R⁴ and R⁵ are as defined above, for example R⁴ may be hydrogen and R⁵ may be (C1-C4)alkyl, such as methyl or ethyl).

In another aspect of the invention, in the compounds of formula (Ib), suitable values for Q² are (C1-C4)alkyl (such as methyl), (C1-C4)alkoxy (such as methoxy), halogeno (such as chloro), cyano and -NR⁴R⁵ (wherein R⁴ and R⁵ are as defined above, for example R⁴ may be hydrogen and R⁵ may be (C1-C4)alkyl, such as methyl).

In yet another aspect of the invention, in the compounds of formula (Ib), suitable values for Q² are (C1-C4)alkyl (such as methyl), halogeno (such as chloro), cyano and - NR⁴R⁵ (wherein R⁴ and R⁵ are as defined above, for example R⁴ may be hydrogen and R⁵ may be (C1-C4)alkyl, such as methyl).

As stated above, the compounds of the present invention, for example of formula (Ib) (or a pharmaceutically-acceptable salt thereof) wherein R¹, R², R³ and Q¹ are as defined above do not include:
5-chloro-2-{2-[3-(2-methoxypyrid-3-yl)isoxazol-5-yl]pyrrolidin-1-yl}-4-(5-methyl-1*H-*pyrazol-3-ylamino)pyrimidine;
S-5-chloro-2-{2-[3-(2-methoxypyrid-3-yl)isoxazol-5-yl]pyrrolidin-1-yl}-4-(5-methyl-1*H-*pyrazol-3-ylamino)pyrimidine;
S-4-(5-ethyl-1*H*-pyrazol-3-ylamino)-2-[2-[3-(2-methoxypyrid-3-yl)isoxazol-5-yl]pyrrolidin-1-yl]pyrimidine;
S-4-(5-methyl-1H-pyrazol-3-ylamino)-2-[2-[3-(2-methoxypyrid-3-yl)isoxazol-5-yl]pyrrolidin-1-yl]pyrimidine;
S-4-(5-cyclopropyl-1H-pyrazol-3-ylamino)-2-[2-[3-(2-methoxypyrid-3-yl)isoxazol-5-yl]pyrrolidin-1-yl]pyrimidine;
S-6-methyl-4-(5-methyl-1*H*-pyrazol-3-ylamino)-2-[2-[3-(2-methoxypyrid-3-yl)isoxazol-5-yl]pyrrolidin-1-yl]pyrimidine;
S-5-fluoro-4-(5-cyclopropyl-1*H*-pyrazol-3-ylamino)-2-[2-[3-(2-methoxypyrid-3-yl)isoxazol-5-yl]pyrrolidin-1-yl]pyrimidine;
S-5-fluoro-4-(5-ethyl-1*H*-pyrazol-3-ylamino)-2-[2-[3-(2-methoxypyrid-3-yl)isoxazol-5-yl]pyrrolidin-1-yl]pyrimidine;
S-6-(2-hydroxyethoxy)-4-(5-methyl-1*H*-pyrazol-3-ylamino)-2-[2-[3-(2-methoxypyrid-3-yl)isoxazol-5-yl]pyrrolidin-1-yl]pyrimidine;
S-6-chloro-4-(5-cyclopropyl-1*H*-pyrazol-3-ylamino)-2-[2-[3-(2-methoxypyrid-3-yl)isoxazol-5-yl]pyrrolidin-1-yl]pyrimidine;
S-6-chloro-4-(5-ethyl-1*H*-pyrazol-3-ylamino)-2-[2-[3-(2-methoxypyrid-3-yl)isoxazol-5-yl]pyrrolidin-1-yl]pyrimidine;
S-6-(2-hydroxyethoxy)-4-(5-cyclopropyl-1*H*-pyrazol-3-ylamino)-2-[2-[3-(2-methoxypyrid-3-yl)isoxazol-5-yl]pyrrolidin-1-yl]pyrimidine;
S-5-fluoro-4-(5-methyl-1*H*-pyrazol-3-ylamino)-2-[2-[3-(2-methoxypyrid-3-yl)isoxazol-5-yl]pyrrolidin-1-yl]pyrimidine;
S-6-(2-hydroxyethoxy)-4-(5-ethyl-1*H*-pyrazol-3-ylamino)-2-[2-[3-(2-methoxypyrid-3-yl)isoxazol-5-yl]pyrrolidin-1-yl]pyrimidine; or
S-6-chloro-4-(5-methyl-1H-pyrazol-3-ylamino)-2-[2-[3-(2-methoxypyrid-3-yl)isoxazol-5-yl]pyrrolidin-1-yl]pyrimidine.

Particular compounds of the invention include, for example, any one or more compounds of formula (I) selected from:-
S-6-methyl-2-{2-[3-(3-ethoxypyrazin-2-yl)isoxazol-5-yl]pyrrolidin-1-yl}-4-(5-methyl-1H-pyrazol-3-ylamino)pyrimidine;
S-6-methyl-2-{2-[3-(3-ethylaminopyrazin-2-yl)isoxazol-5-yl]pyrrolidin-1-yl}-4-(5-methyl-1H-pyrazol-3-ylamino)pyrimidine;
S-6-methyl-4-(5-methyl-1H-pyrazol-3-ylamino)-2-[2-[3-(2-methylpyrid-3-yl)isoxazol-5-yl]pyrrolidin-1-yl]pyrimidine;
S-6-methyl-4-(5-methyl-1H-pyrazol-3-ylamino)-2-[2-[3-(2-chloropyrid-3-yl)isoxazol-5-yl]pyrrolidin-1-yl]pyrimidine;
S-6-methoxy-4-(5-methyl-1H-pyrazol-3-ylamino)-2-[2-[3-(2-methylpyrid-3-yl)isoxazol-5-yl]pyrrolidin-1-yl]pyrimidine;
S-6-methyl-4-(5-methyl-1H-pyrazol-3-ylamino)-2-[2-[3-(2-cyanopyrid-3-yl)isoxazol-5-yl]pyrrolidin-1-yl]pyrimidine;
S-6-ethyl-2-{2-[3-(3-methylpyrazin-2-yl)isoxazol-5-yl]pyrrolidin-1-yl}-4-(5-methyl-1H-pyrazol-3-ylamino)pyrimidine;
S-6-methoxy-2-{2-[3-(3-methylpyrazin-2-yl)isoxazol-5-yl]pyrrolidin-1-yl}-4-(5-methyl-1H-pyrazol-3-ylamino)pyrimidine;
S-4-(5-methyl-1H-pyrazol-3-ylamino)-2-[2-[3-(2-methylpyrid-3-yl)isoxazol-5-yl]pyrrolidin-1-yl]pyrimidine;
S-4-(5-methyl-1H-pyrazol-3-ylamino)-2-[2-[3-(2-methylaminopyrid-3-yl)isoxazol-5-yl]pyrrolidin-1-yl]pyrimidine;
S-6-methyl-4-(5-methyl-1H-pyrazol-3-ylamino)-2-[2-[3-(2-methylaminopyrid-3-yl)isoxazol-5-yl]pyrrolidin-1-yl]pyrimidine;
S-6-ethyl-4-(5-methyl-1H-pyrazol-3-ylamino)-2-[2-[3-(2-cyanopyrid-3-yl)isoxazol-5-yl]pyrrolidin-1-yl]pyrimidine; and
S-6-methoxy-2-{2-[3-(3-methoxypyrazin-2-yl)isoxazol-5-yl]pyrrolidin-1-yl}-4-(5-methyl-1H-pyrazol-3-ylamino)pyrimidine;
and pharmaceutically-acceptable salts thereof.

In another aspect, particular compounds of the invention include, for example, any one or more compounds of formula (I) selected from:-
S-6-methyl-2-{2-[3-(3-ethoxypyrazin-2-yl)isoxazol-5-yl]pyrrolidin-1-yl}-4-(5-methyl-1H-pyrazol-3-ylamino)pyrimidine;
S-6-methyl-2-{2-[3-(3-ethylaminopyrazin-2-yl)isoxazol-5-yl]pyrrolidin-1-yl}-4-(5-methyl-1H-pyrazol-3-ylamino)pyrimidine;
S-6-methyl-4-(5-methyl-1H-pyrazol-3-ylamino)-2-[2-[3-(2-methylpyrid-3-yl)isoxazol-5-yl]pyrrolidin-1-yl]pyrimidine;
S-6-methyl-4-(5-methyl-1H-pyrazol-3-ylamino)-2-[2-[3-(2-chloropyrid-3-yl)isoxazol-5-yl]pyrrolidin-1-yl]pyrimidine;
S-6-methoxy-4-(5-methyl-1H-pyrazol-3-ylamino)-2-[2-[3-(2-methylpyrid-3-yl)isoxazol-5-yl]pyrrolidin-1-yl]pyrimidine;
S-6-methyl-4-(5-methyl-1H-pyrazol-3-ylamino)-2-[2-[3-(2-cyanopyrid-3-yl)isoxazol-5-yl]pyrrolidin-1-yl]pyrimidine;
S-6-ethyl-2-{2-[3-(3-methylpyrazin-2-yl)isoxazol-5-yl]pyrrolidin-1-yl}-4-(5-methyl-1H-pyrazol-3-ylamino)pyrimidine;
S-6-methoxy-2-{2-[3-(3-methylpyrazin-2-yl)isoxazol-5-yl]pyrrolidin-1-yl}-4-(5-methyl-1H-pyrazol-3-ylamino)pyrimidine;
S-4-(5-methyl-1H-pyrazol-3-ylamino)-2-[2-[3-(2-methylpyrid-3-yl)isoxazol-5-yl]pyrrolidin-1-yl]pyrimidine;
S-4-(5-methyl-1H-pyrazol-3-ylamino)-2-[2-[3-(2-methylaminopyrid-3-yl)isoxazol-5-yl]pyrrolidin-1-yl]pyrimidine;
S-6-methyl-4-(5-methyl-1H-pyrazol-3-ylamino)-2-[2-[3-(2-methylaminopyrid-3-yl)isoxazol-5-yl]pyrrolidin-1-yl]pyrimidine; and
S-6-methoxy-2-{2-[3-(3-methoxypyrazin-2-yl)isoxazol-5-yl]pyrrolidin-1-yl}-4-(5-methyl-1H-pyrazol-3-ylamino)pyrimidine;
and pharmaceutically-acceptable salts thereof.

In another aspect, particular compounds of the invention include, for example, any one or more compounds of formula (I) selected from:-
S-6-methyl-2-{2-[3-(3-ethoxypyrazin-2-yl)isoxazol-5-yl]pyrrolidin-1-yl}-4-(5-methyl-1H-pyrazol-3-ylamino)pyrimidine;
S-6-methyl-2-{2-[3-(3-ethylaminopyrazin-2-yl)isoxazol-5-yl]pyrrolidin-1-yl}-4-(5-methyl-1H-pyrazol-3-ylamino)pyrimidine;
S-6-methyl-4-(5-methyl-1H-pyrazol-3-ylamino)-2-[2-[3-(2-methylpyrid-3-yl)isoxazol-5-yl]pyrrolidin-1-yl]pyrimidine;
S-6-methyl-4-(5-methyl-1H-pyrazol-3-ylamino)-2-[2-[3-(2-chloropyrid-3-yl)isoxazol-5-yl]pyrrolidin-1-yl]pyrimidine;
S-6-methoxy-4-(5-methyl-1H-pyrazol-3-ylamino)-2-[2-[3-(2-methylpyrid-3-yl)isoxazol-5-yl]pyrrolidin-1-yl]pyrimidine;
S-6-methyl-4-(5-methyl-1H-pyrazol-3-ylamino)-2-[2-[3-(2-cyanopyrid-3-yl)isoxazol-5-yl]pyrrolidin-1-yl]pyrimidine;
S-2-[2-{3-(2-methylpyrid-3-yl)isoxazol-5-yl}pyrrolidin-1-yl]-4-(5-methyl-1*H*-pyrazol-3-ylamino)-6-morpholinopyrimidine;
S-4-(5-cyclopropyl-1*H*-pyrazol-3-ylamino)-2-[2-{3-(2-methoxypyrid-3-yl)isoxazol-5-yl}pyrrolidin-1-yl]-6-morpholinopyrimidine;
S-2-[2-{3-(2-methoxypyrid-3-yl)isoxazol-5-yl}pyrrolidin-1-yl]-4-(5-methyl-1*H*-pyrazol-3-ylamino-6-morpholinopyrimidine;
S-2-[2-{3-(2-cyanopyrid-3-yl)isoxazol-5-yl}pyrrolidin-1-yl]-4-(5-methyl-1*H*-pyrazol-3-ylamino)-6-morpholinopyrimidine;
S-2-[2-{3-(2-chloropyrid-3-yl)isoxazol-5-yl}pyrrolidin-1-yl]-4-(5-methyl-1*H*-pyrazol-3-ylamino)pyrimidine;
S-2-[2-{3-(2-methylaminopyrid-3-yl)isoxazol-5-yl}pyrrolidin-1-yl]-4-(5-methyl-1*H*-pyrazol-3-ylamino)pyrimidine;
S-6-methyl-2-[2-{3-(2-methylaminopyrid-3-yl)isoxazol-5-yl}pyrrolidin-1-yl]-4-(5-methyl-1*H*-pyrazol-3-ylamino)pyrimidine;
S-2-[2-{3-(2-methylpyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(2-methylpyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(3-ethylpyrazin-2-yl)isoxazol-5-yl}pyrrolidin-1-yl]-4-(5-methyl-1*H*-pyrazol-3-ylamino)pyrimidine;
S-6-methoxy-2-[2-{3-(3-ethylpyrazin-2-yl)isoxazol-5-yl}pyrrolidin-1-yl]-4-(5-methyl-1*H-*pyrazol-3-ylamino)pyrimidine;
S-6-(2-hydroxyethoxy)-2-[2-{3-(2-methylpyrid-3-yl)isoxazol-5-yl}pyrrolidin-1-yl]-4-(5-methyl-1*H*-pyrazol-3-ylamino)pyrimidine;
S-6-(3-hydroxypropoxy)-2-[2-{3-(2-methylpyrid-3-yl)isoxazol-5-yl}pyrrolidin-1-yl]-4-(5-methyl-1*H*-pyrazol-3-ylamino)pyrimidine;
6-((2R)-2-hydroxypropoxy)-2-[(2S)-2-{3-(2-methylpyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(2-cyanopyrid-3-yl)isoxazol-5-yl}pyrrolidin-1-yl]-4-(5-methyl-1*H*-pyrazol-3-ylamino)pyrimidine;
S-6-(1-cyanocyclopropyl)methoxy-2-[2-{3-(2-methylpyrid-3-yl)isoxazol-5-yl}pyrrolidin-1-yl]-4-(5-methyl-1*H*-pyrazol-3-ylamino)pyrimidine;
S-6-(3-cyanopropoxy)-2-[2-{3-(2-methylpyrid-3-yl)isoxazol-5-yl}pyrrolidin-1-yl]-4-(5-methyl-1*H*-pyrazol-3-ylamino)pyrimidine;
S-6-methoxy-2-[2-{3-(2-cyanopyrid-3-yl)isoxazol-5-yl}pyrrolidin-1-yl]-4-(5-methyl-1*H-*pyrazol-3-ylamino)pyrimidine;
S-6-methoxy-2-[2-{3-(3-methylpyrazin-2-yl)isoxazol-5-yl}pyrrolidin-1-yl]-4-(5-methyl-1*H-*pyrazol-3-ylamino)pyrimidine;
S-6-ethyl-2-[2-{3-(3-methylpyrazin-2-yl)isoxazol-5-yl}pyrrolidin-1-yl]-4-(5-methyl-1*H-*pyrazol-3-ylamino)pyrimidine;
S-6-ethyl-2-[2-{3-(2-cyanopyrid-3-yl)isoxazol-5-yl}pyrrolidin-1-yl]-4-(5-methyl-1*H-*pyrazol-3-ylamino)pyrimidine;
S-6-(2-methoxyethoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(2-ethoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(3-methoxypropoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(2-ethoxyethoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-[(1-cyanocyclopropyl)methoxy]-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(3-cyanopropoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-methoxy-2-[2-{3-(3-methoxypyrazin-2-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(3-(2-methoxyethoxy)pyrazin-2-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(3-hydroxypyrazin-2-yl)isoxazol-5-yl}pyrrolidin-1-yl]-4-(5-methyl-1*H*-pyrazol-3-ylamino)pyrimidine;
S-6-methoxy-2-[2-{3-(1-oxopyrid-3-yl)isoxazol-5-yl}pyrrolidin-1-yl]-4-(5-methyl-1*H-*pyrazol-3-ylamino)pyrimidine;
S-6-chloro-2-[2-{3-(1-oxopyrid-3-yl)isoxazol-5-yl}pyrrolidin-1-yl]-4-(5-methyl-1*H*-pyrazol-3-ylamino)pyrimidine; and
S-6-(2-methoxyethoxy)-2-[2-{3-(1-oxopyrid-3-yl)isoxazol-5-yl}pyrrolidin-1-yl]-4-(5-methyl-1*H*-pyrazol-3-ylamino)pyrimidine;
and pharmaceutically-acceptable salts thereof.

In yet another aspect, particular compounds of the invention include, for example, any one or more compounds of formula (I) selected from:-
S-6-methyl-2-{2-[3-(3-ethoxypyrazin-2-yl)isoxazol-5-yl]pyrrolidin-1-yl}-4-(5-methyl-1H-pyrazol-3-yl amino)pyrimidine;
S-6-methyl-2-{2-[3-(3-ethylaminopyrazin-2-yl)isoxazol-5-yl]pyrrolidin-1-yl}-4-(5-methyl-1H-pyrazol-3-ylamino)pyrimidine;
S-6-methyl-4-(5-methyl-1H-pyrazol-3-ylamino)-2-[2-[3-(2-methylpyrid-3-yl)isoxazol-5-yl]pyrrolidin-1-yl]pyrimidine;
S-6-methyl-4-(5-methyl-1H-pyrazol-3-ylamino)-2-[2-[3-(2-chloropyrid-3-yl)isoxazol-5-yl]pyrrolidin-1-yl]pyrimidine;
S-6-methoxy-4-(5-methyl-1H-pyrazol-3-ylamino)-2-[2-[3-(2-methylpyrid-3-yl)isoxazol-5-yl]pyrrolidin-1-yl]pyrimidine;
S-6-methyl-4-(5-methyl-1H-pyrazol-3-ylamino)-2-[2-[3-(2-cyanopyrid-3-yl)isoxazol-5-yl]pyrrolidin-1-yl]pyrimidine;
S-2-[2-{3-(2-methylpyrid-3-yl)isoxazol-5-yl}pyrrolidin-1-yl]-4-(5-methyl-1*H*-pyrazol-3-ylamino)-6-morpholinopyrimidine;
S-4-(5-cyclopropyl-1*H*-pyrazol-3-ylamino)-2-[2-{3-(2-methoxypyrid-3-yl)isoxazol-5-yl }pyrrolidin-1-yl]-6-morpholinopyrimidine;
S-2-[2-{3-(2-methoxypyrid-3-yl)isoxazol-5-yl}pyrrolidin-1-yl]-4-(5-methyl-1*H*-pyrazol-3-ylamino-6-morpholinopyrimidine;
S-2-[2-{3-(2-cyanopyrid-3-yl)isoxazol-5-yl}pyrrolidin-1-yl]-4-(5-methyl-1*H*-pyrazol-3-ylamino)-6-morpholinopyrimidine;
S-2-[2-{3-(2-chloropyrid-3-yl)isoxazol-5-yl}pyrrolidin-1-yl]-4-(5-methyl-1*H* pyrazol-3-ylamino)pyrimidine;
S-2-[2-{3-(2-methylaminopyrid-3-yl)isoxazol-5-yl}pyrrolidin-1-yl]-4-(5-methyl-1*H*-pyrazol-3-ylamino)pyrimidine;
S-6-methyl-2-[2-{3-(2-methylaminopyrid-3-yl)isoxazol-5-yl}pyrrolidin-1-yl]-4-(5-methyl-1*H*-pyrazol-3-ylamino)pyrimidine;
S-2-[2-{3-(2-methylpyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(2-methylpyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(3-ethylpyrazin-2-yl)isoxazol-5-yl}pyrrolidin-1-yl]-4-(5-methyl-1*H*-pyrazol-3-ylamino)pyrimidine;
S-6-methoxy-2-[2-{3-(3-ethylpyrazin-2-yl)isoxazol-5-yl}pyrrolidin-1-yl]-4-(5-methyl-1*H-*pyrazol-3-ylamino)pyrimidine;
S-6-(2-hydroxyethoxy)-2-[2-{3-(2-methylpyrid-3-yl)isoxazol-5-yl}pyrrolidin-1-yl]-4-(5-methyl-1*H*-pyrazol-3-ylamino)pyrimidine;
S-6-(3-hydroxypropoxy)-2-[2-{3-(2-methylpyrid-3-yl)isoxazol-5-yl}pyrrolidin-1-yl]-4-(5-methyl-1*H*-pyrazol-3-ylamino)pyrimidine;
6-((2R)-2-hydroxypropoxy)-2-[(2S)-2-{3-(2-methylpyrid-3-yl)isoxazol-5-yl}pyrrolidin-1-yl]-4-(5-methyl-1*H*-pyrazol-3-ylamino)pyrimidine; S-6-(2-methoxyethoxy)-2-[2-{3-(2-cyanopyrid-3-yl)isoxazol-5-yl}pyrrolidin-1-yl]-4-(5-methyl-1*H*-pyrazol-3-ylamino)pyrimidine;
S-6-(1-cyanocyclopropyl)methoxy-2-[2-{3-(2-methylpyrid-3-yl)isoxazol-5-yl}pyrrolidin-1-yl]-4-(5-methyl-1*H*-pyrazol-3-ylamino)pyrimidine;
S-6-(3-cyanopropoxy)-2-[2-{3-(2-methylpyrid-3-yl)isoxazol-5-yl}pyrrolidin-1-yl]-4-(5-methyl-1*H*-pyrazol-3-ylamino)pyrimidine;
S-6-methoxy-2-[2-{3-(2-cyanopyrid-3-yl)isoxazol-5-yl}pyrrolidin-1-yl]-4-(5-methyl-1*H-*pyrazol-3-ylamino)pyrimidine;
S-6-methoxy-2-[2-{3-(3-methylpyrazin-2-yl)isoxazol-5-yl}pyrrolidin-1-yl]-4-(5-methyl-1*H-*pyrazol-3-ylamino)pyrimidine;
S-6-ethyl-2-[2-{3-(3-methylpyrazin-2-yl)isoxazol-5-yl}pyrrolidin-1-yl]-4-(5-methyl-1*H-*pyrazol-3-ylamino)pyrimidine;
S-6-ethyl-2-[2-{3-(2-cyanopyrid-3-yl)isoxazol-5-yl}pyrrolidin-1-yl]-4-(5-methyl-1*H-*pyrazol-3-ylamino)pyrimidine;
S-6-(2-methoxyethoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(2-ethoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(3-methoxypropoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(2-ethoxyethoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-[(1-cyanocyclopropyl)methoxy]-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(3-cyanopropoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-methoxy-2-[2-{3-(3-methoxypyrazin-2-yl)isoxazol-5-yl}pyrrolidin-1-yl]-4-(5-methyl-1*H*-pyrazol-3-ylamino)pyrimidine; and
S-6-(2-methoxyethoxy)-2-[2-{3-(3-(2-methoxyethoxy)pyrazin-2-yl)isoxazol-5-yl}pyrrolidin-1-yl]-4-(5-methyl-1*H*-pyrazol-3-ylamino)pyrimidine;
and pharmaceutically-acceptable salts thereof.

In yet another aspect, particular compounds of the invention include, for example, any one or more compounds of formula (I) selected from:-
S-2-[2-{3-(3-ethoxypyrazin-2-yl)isoxazol-5-yl}pyrrolidin-1-yl]-6-methyl-4-(5-methyl-1*H-*pyrazol-3-ylamino)pyrimidine;
S-2-[2-{3-(3-ethylaminopyrazin-2-yl)isoxazol-5-yl}pyrrolidin-1-yl]-6-methyl-4-(5-methyl-1*H*-pyrazol-3-ylamino)pyrimidine;
S-2-[2-{3-(2-methylpyrid-3-yl)isoxazol-5-yl}pyrrolidin-1-yl]-6-methyl-4-(5-methyl-1*H-*pyrazol-3-ylamino)pyrimidine;
S-2-[2-{3-(2-chloropyrid-3-yl)isoxazol-5-yl}pyrrolidin-1-yl]-6-methyl-4-(5-methyl-1*H-*pyrazol-3-ylamino)pyrimidine;
S-6-methoxy-2-[2-{3-(2-methylpyrid-3-yl)isoxazol-5-yl}pyrrolidin-1-yl]-4-(5-methyl-1*H-*pyrazol-3-ylamino)pyrimidine;
S-2-[2-{3-(2-cyanopyrid-3-yl)isoxazol-5-yl}pyrrolidin-1-yl]-6-methyl-4-(5-methyl-1*H-*pyrazol-3-ylamino)pyrimidine;
S-2-[2-{3-(2-methylpyrid-3-yl)isoxazol-5-yl}pyrrolidin-1-yl]-4-(5-methyl-1*H*-pyrazol-3-ylamino)-6-morpholinopyrimidine;
S-4-(5-cyclopropyl-1*H-*pyrazol-3-ylamino)-2-[2-{3-(2-methoxypyrid-3-yl)isoxazol-5-yl }pyrrolidin-1-yl]-6-morpholinopyrimidine;
S-2-[2-{3-(2-methoxypyrid-3-yl)isoxazol-5-yl}pyrrolidin-1-yl]-4-(5-methyl-1*H*-pyrazol-3-ylamino-6-morpholinopyrimidine;
S-2-[2-{3-(2-cyanopyrid-3-yl)isoxazol-5-yl}pyrrolidin-1-yl]-4-(5-methyl-1*H*-pyrazol-3-ylamino)-6-morpholinopyrimidine;
S-2-[2-{3-(2-chloropyrid-3-yl)isoxazol-5-yl}pyrrolidin-1-yl]-4-(5-methyl-1*H*-pyrazol-3-ylamino)pyrimidine;
S-2-[2-{3-(2-methylaminopyrid-3-yl)isoxazol-5-yl}pyrrolidin-1-yl]-4-(5-methyl-1*H*-pyrazol-3-ylamino)pyrimidine;
S-6-methyl-2-[2-{3-(2-methylaminopyrid-3-yl)isoxazol-5-yl}pyrrolidin-1-yl]-4-(5-methyl-1*H*-pyrazol-3-ylamino)pyrimidine;
S-2-[2-{3-(2-methylpyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(2-methylpyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(3-ethylpyrazin-2-yl)isoxazol-5-yl}pyrrolidin-1-yl]-4-(5-methyl-1*H*-pyrazol-3-ylamino)pyrimidine;
S-6-methoxy-2-[2-{3-(3-ethylpyrazin-2-yl)isoxazol-5-yl}pyrrolidin-1-yl]-4-(5-methyl-1*H-*pyrazol-3-ylamino)pyrimidine;
S-6-(2-hydroxyethoxy)-2-[2-{3-(2-methylpyrid-3-yl)isoxazol-5-yl}pyrrolidin-1-yl]-4-(5-methyl-1*H*-pyrazol-3-ylamino)pyrimidine;
S-6-(3-hydroxypropoxy)-2-[2-{3-(2-methylpyrid-3-yl)isoxazol-5-yl}pyrrolidin-1-yl]-4-(5-methyl-1*H*-pyrazol-3-ylamino)pyrimidine;
6-((2R)-2-hydroxypropoxy)-2-[(2S)-2-{3-(2-methylpyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(2-cyanopyrid-3-yl)isoxazol-5-yl}pyrrolidin-1-yl]-4-(5-methyl-1*H*-pyrazol-3-ylamino)pyrimidine;
S-6-(1-cyanocyclopropyl)methoxy-2-[2-{3-(2-methylpyrid-3-yl)isoxazol-5-yl}pyrrolidin-1-yl]-4-(5-methyl-1*H*-pyrazol-3-ylamino)pyrimidine;
S-6-(3-cyanopropoxy)-2-[2-{3-(2-methylpyrid-3-yl)isoxazol-5-yl}pyrrolidin-1-yl]-4-(5-methyl-1*H*-pyrazol-3-ylamino)pyrimidine;
S-6-methoxy-2-[2-{3-(2-cyanopyrid-3-yl)isoxazol-5-yl}pyrrolidin-1-yl]-4-(5-methyl-1H-pyrazol-3-ylamino)pyrimidine;
S-6-methoxy-2-[2-{3-(3-methylpyrazin-2-yl)isoxazol-5-yl}pyrrolidin-1-yl]-4-(5-methyl-1*H-*pyrazol-3-ylamino)pyrimidine;
S-6-ethyl-2-[2-{3-(3-methylpyrazin-2-yl)isoxazol-5-yl}pyrrolidin-1-yl]-4-(5-methyl-1*H-*pyrazol-3-ylamino)pyrimidine;
S-6-ethyl-2-[2-{3-(2-cyanopyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(2-ethoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(3-methoxypropoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(2-ethoxyethoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-[(1-cyanocyclopropyl)methoxy]-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(3-cyanopropoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-methoxy-2-[2-{3-(3-methoxypyrazin-2-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(3-(2-methoxyethoxy)pyrazin-2-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(4-cyanopyrid-3-yl)isoxazol-5-yl}pyrrolidin-1-yl]-4-(5-methyl-1*H*-pyrazol-3-ylamino)pyrimidine; and
S-6-methoxy-2-[2-{3-(2-cyanopyrid-3-yl)isoxazol-5-yl}pyrrolidin-1-yl]-4-(5-methyl-1H-pyrazol-3-ylamino)pyrimidine;
and pharmaceutically-acceptable salts thereof.

In yet another aspect, particular compounds of the invention include, for example, any one or more compounds of formula (I) selected from:-
S-2-[2-{3-(3-ethoxypyrazin-2-yl)isoxazol-5-yl}pyrrolidin-1-yl]-6-methyl-4-(5-methyl-1*H-*pyrazol-3-ylamino)pyrimidine;
S-2-[2-{3-(3-ethylaminopyrazin-2-yl)isoxazol-5-yl}pyrrolidin-1-yl]-6-methyl-4-(5-methyl-1*H*-pyrazol-3-ylamino)pyrimidine;
S-2-[2-{3-(2-methylpyrid-3-yl)isoxazol-5-yl}pyrrolidin-1-yl]-6-methyl-4-(5-methyl-1*H-*pyrazol-3-ylamino)pyrimidine;
S-6-chloro-2-[2-{3-(2-methylpyrid-3-yl)isoxazol-5-yl}pyrrolidin-1-yl]-4-(5-methyl-1*H-*pyrazol-3-ylamino)pyrimidine;
S-2-[2-{3-(2-chloropyrid-3-yl)isoxazol-5-yl}pyrrolidin-1-yl]-6-methyl-4-(5-methyl-1*H-*pyrazol-3-ylamino)pyrimidine;
S-6-methoxy-2-[2-{3-(2-methylpyrid-3-yl)isoxazol-5-yl}pyrrolidin-1-yl]-4-(5-methyl-1*H-*pyrazol-3-ylamino)pyrimidine;
S-2-[2-{3-(2-cyanopyrid-3-yl)isoxazol-5-yl}pyrrolidin-1-yl]-6-methyl-4-(5-methyl-1*H-*pyrazol-3-ylamino)pyrimidine;
S-2-[2-{3-(2-methylpyrid-3-yl)isoxazol-5-yl}pyrrolidin-1-yl]-4-(5-methyl-1*H*-pyrazol-3-ylamino)-6-morpholinopyrimidine;
S-4-(5-cyclopropyl-1*H*-pyrazol-3-ylamino)-2-[2-{3-(2-methoxypyrid-3-yl)isoxazol-5-yl }pyrrolidin-1-yl]-6-morpholinopyrimidine;
S-2-[2-{3-(2-methoxypyrid-3-yl)isoxazol-5-yl}pyrrolidin-1-yl]-4-(5-methyl-1*H*-pyrazol-3-ylamino-6-morpholinopyrimidine;
S-6-chloro-2-[2-{3-(2-cyanopyrid-3-yl)isoxazol-5-yl}pyrrolidin-1-yl]-4-(5-methyl-1*H-*pyrazol-3-ylamino)pyrimidine;
S-2-[2-{3-(2-cyanopyrid-3-yl)isoxazol-5-yl}pyrrolidin-1-yl]-4-(5-methyl-1*H*-pyrazol-3-ylamino)-6-morpholinopyrimidine;
S-2-[2-{3-(2-chloropyrid-3-yl)isoxazol-5-yl}pyrrolidin-1-yl]-4-(5-methyl-1*H*-pyrazol-3-ylamino)pyrimidine;
S-2-[2-{3-(2-methylaminopyrid-3-yl)isoxazol-5-yl}pyrrolidin-1-yl]-4-(5-methyl-1*H*-pyrazol-3-ylamino)pyrimidine;
S-6-methyl-2-[2-{3-(2-methylaminopyrid-3-yl)isoxazol-5-yl}pyrrolidin-1-yl]-4-(5-methyl-1*H*-pyrazol-3-ylamino)pyrimidine;
S-2-[2-{3-(2-methylpyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(2-methylpyrid-3-yl)isoxazol-5-yl}pyrrolidin-1-yl]-4-(5-methyl-1*H-*pyrazol-3-ylamino)pyrimidine;
S-6-chloro-2-[2-{3-(3-ethylpyrazin-2-yl)isoxazol-5-yl}pyrrolidin-1-yl]-4-(5-methyl-1*H-*pyrazol-3-ylamino)pyrimidine;
S-6-(2-methoxyethoxy)-2-[2-{3-(3-ethylpyrazin-2-yl)isoxazol-5-yl}pyrrolidin-1-yl]-4-(5-methyl-1*H*-pyrazol-3-ylamino)pyrimidine;
S-6-methoxy-2-[2-{3-(3-ethylpyrazin-2-yl)isoxazol-5-yl}pyrrolidin-1-yl]-4-(5-methyl-1*H-*pyrazol-3-ylamino)pyrimidine;
S-6-(2-hydroxyethoxy)-2-[2-{3-(2-methylpyrid-3-yl)isoxazol-5-yl}pyrrolidin-1-yl]-4-(5-methyl-1*H*-pyrazol-3-ylamino)pyrimidine;
S-6-(3-hydroxypropoxy)-2-[2-{3-(2-methylpyrid-3-yl)isoxazol-5-yl}pyrrolidin-1-yl]-4-(5-methyl-1*H*-pyrazol-3-ylamino)pyrimidine;
6-((2R)-2-hydroxypropoxy)-2-[(2S)-2-{3-(2-methylpyrid-3-yl)isoxazol-5-yl}pyrrolidin-1-yl]-4-(5-methyl-1*H*-pyrazol-3-ylamino)pyrimidine;
S-6-chloro-2-[2-{3-(1-oxopyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(1-oxopyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(2-cyanopyrid-3-yl)isoxazol-5-yl}pyrrolidin-1-yl]-4-(5-methyl-1*H*-pyrazol-3-ylamino)pyrimidine;
S-6-(1-cyanocyclopropyl)methoxy-2-[2-{3-(2-methylpyrid-3-yl)isoxazol-5-yl}pyrrolidin-1-yl]-4-(5-methyl-1*H*-pyrazol-3-ylamino)pyrimidine;
S-6-(3-cyanopropoxy)-2-[2-{3-(2-methylpyrid-3-yl)isoxazol-5-yl}pyrrolidin-1-yl]-4-(5-methyl-1*H*-pyrazol-3-ylamino)pyrimidine;
S-6-methoxy-2-[2-{3-(1-oxopyrid-3-yl)isoxazol-5-yl}pyrrolidin-1-yl]-4-(5-methyl-1*H-*pyrazol-3-ylamino)pyrimidine;
S-6-methoxy-2-[2-{3-(2-cyanopyrid-3-yl)isoxazol-5-yl}pyrrolidin-1-yl]-4-(5-methyl-1H-pyrazol-3-ylamino)pyrimidine;
S-6-chloro-2-[2-{3-(3-methylpyrazin-2-yl)isoxazol-5-yl}pyrrolidin-1-yl]-4-(5-methyl-1*H-*pyrazol-3-ylamino)pyrimidine;
S-6-methoxy-2-[2-{3-(3-methylpyrazin-2-yl)isoxazol-5-yl}pyrrolidin-1-yl]-4-(5-methyl-1*H-*pyrazol-3-ylamino)pyrimidine;
S-6-ethyl-2-[2-{3-(3-methylpyrazin-2-yl)isoxazol-5-yl}pyrrolidin-1-yl]-4-(5-methyl-1*H-*pyrazol-3-ylamino)pyrimidine;
S-6-ethyl-2-[2-{3-(2-cyanopyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(2-ethoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(3-methoxypropoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(2-ethoxyethoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-[(1-cyanocyclopropyl)methoxy]-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(3-cyanopropoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-methoxy-2-[2-{3-(3-methoxypyrazin-2-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(3-(2-methoxyethoxy)pyrazin-2-yl)isoxazol-5-yl}pyrrolidin-1-yl]-4-(5-methyl-1*H*-pyrazol-3-ylamino)pyrimidine; S-6-(2-methoxyethoxy)-2-[2-{3-(3-hydroxypyrazin-2-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(4-cyanopyrid-3-yl)isoxazol-5-yl}pyrrolidin-1-yl]-4-(5-methyl-1*H*-pyrazol-3-ylamino)pyrimidine; and
S-6-methoxy-2-[2-{3-(2-cyanopyrid-3-yl)isoxazol-5-yl}pyrrolidin-1-yl]-4-(5-methyl-1H-pyrazol-3-ylamino)pyrimidine;
and pharmaceutically-acceptable salts thereof.

A compound of formula (I), or a pharmaceutically-acceptable salt thereof, may be prepared by any process known to be applicable to the preparation of chemically-related compounds. Such processes, when used to prepare a compound of formula (I) are provided as a further feature of the invention and are illustrated by the following representative process variants in which, unless otherwise stated, Q¹, R¹, R² and R³ have any of the meanings defined hereinbefore. Necessary starting materials may be obtained by standard procedures of organic chemistry. The preparation of such starting materials is described in conjunction with the following representative process variants and within the accompanying Examples. Alternatively necessary starting materials are obtainable by analogous procedures to those illustrated which are within the ordinary skill of an organic chemist.
Process (a) the reaction, conveniently in the presence of a suitable base, of a compound of formula (II): 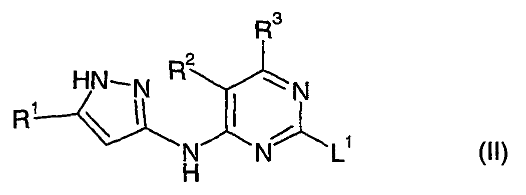 wherein L¹ represents a suitable displaceable group and R¹, R² and R³ are as defined in formula (I) except that any functional group is protected if necessary, with a compound of formula (III): 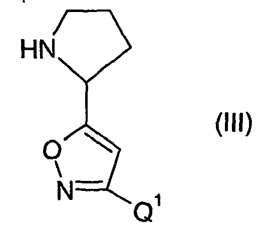 wherein Q¹ is as defined in formula (I) except that any functional group is protected if necessary;
or
Process (b) the reaction, conveniently in the presence of a suitable acid, of a compound of formula (IV): 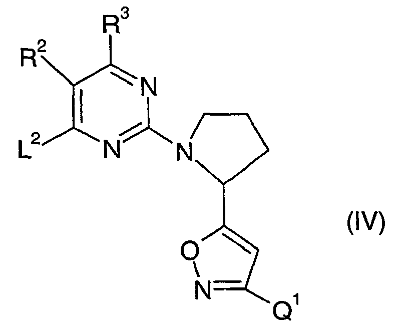 wherein L² is a suitable displaceable group and R², R³ and Q¹ are as defined in formula (I) except that any functional group is protected if necessary, with a pyrazole of formula (V): 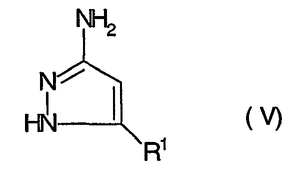 wherein R¹ is as defined in formula (I) except that any functional group is protected if necessary;
or
Process (c) the reaction, conveniently in the presence of a suitable base, of a compound of formula (VI): 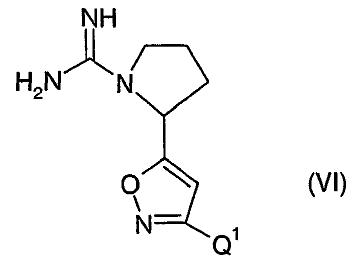 wherein Q¹ is as defined in formula (I) except that any functional group is protected if necessary, with a compound of formula (VII): 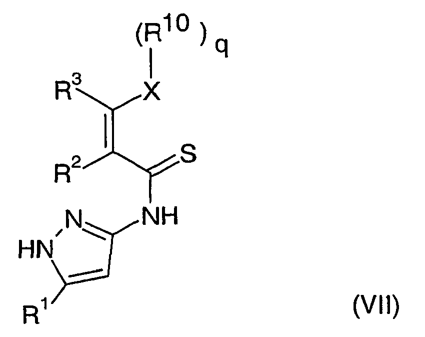 wherein X represents an oxygen atom and q is 1 or X represents a nitrogen atom and q is 2, R¹⁰ is a (C1-C6)alkyl group and R¹, R² and R³ are as defined in formula (I) except that any functional group is protected if necessary;
or
Process (d) the reaction of a compound of formula (VIII): 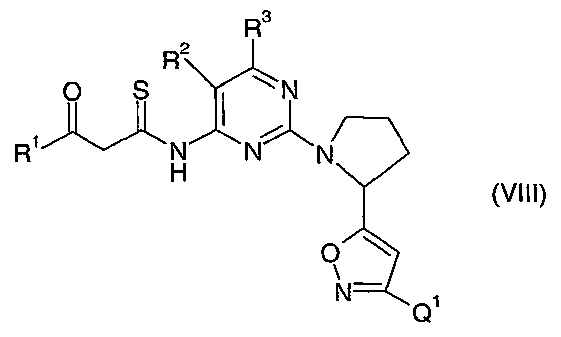 wherein R¹, R², R³ and Q¹ are as defined in formula (I) except that any functional group is protected if necessary, with hydrazine;
or
Process (e) for compounds of formula (I) wherein R³ is a (C1-C6)alkoxy, amino, (C1-C6)alkylamino, di-[(C1-C6)alkyl]amino, -NHR^{3b} or -SR^{3a} group wherein R^{3a} and R^{3b} are as defined above (and the group R³ is optionally substituted by at least one group as defined above), the reaction, conveniently in the presence of a suitable base, of a compound of formula (IX): 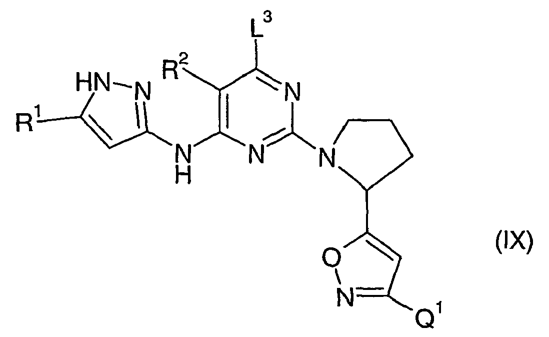 wherein L³ is a suitable displaceable group and R¹, R² and Q¹ are as defined in formula (I) except that any functional group is protected if necessary, with a compound of formula:
H-Xa
wherein Xa represents OR¹¹, NH₂, NHR¹¹, N(R¹¹)₂, NHR^{3b} or SR^{3a}, wherein R¹¹ is an, optionally substituted, (C1-C6)alkyl group and R^{3a} and R^{3b} are each as defined above except that any functional group is protected if necessary;
or
Process (f) for compounds of formula (I) wherein R³ is an, optionally substituted, saturated monocyclic 5- or 6-membered heterocyclic ring comprising at least one ring nitrogen and, optionally, one or more additional heteroatoms selected from nitrogen, oxygen and sulfur, the reaction, conveniently in the presence of a suitable base, of a compound of formula (IX) as defined above, with a compound of formula (Xb): 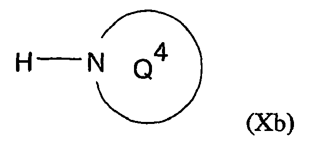 wherein Q⁴ is a saturated monocyclic 5- or 6-membered heterocyclic ring optionally comprising one or more heteroatoms selected from nitrogen, oxygen and sulfur in addition to the nitrogen atom shown above, which ring is optionally substituted by at least one group as defined above;
or
Process (g) for compounds of formula (I) wherein R³ is a (C2-C6)alkenyl or (C2-C6)alkynyl group (and the group R³ is optionally substituted by at least one group as defined above), the reaction, conveniently in the presence of a suitable base and a suitable catalyst, of a compound of formula (IX) as defined above, with a compound of formula (Xc) or of formula (Xc'):

H-C≡C-R¹² (Xc)

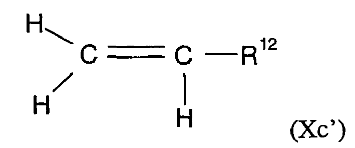

wherein R¹² is selected from hydrogen and an optionally substituted (1-4C)alkyl or (C1-C4)alkoxycarbonyl group;
or
Process (h) for compounds of formula (I) wherein R³ is attached to the pyrimidine ring through a carbon atom, the reaction, conveniently in the presence of a suitable catalyst, of a compound of formula (IX) as defined above, with a compound of the formula:

M-R³

wherein R³ is appropriately selected from the R³ groups as defined above and M is a metallic group, such as ZnBr, B(OH)₂, CuCN or SnBu₃;
or
Process (i) for compounds of formula (I) wherein R³ is a (C1-C6)alkoxycarbonyl group (and the group R³ is optionally substituted by at least one group as defined above), the reaction, conveniently in the presence of a suitable acid, of a compound of formula (X): 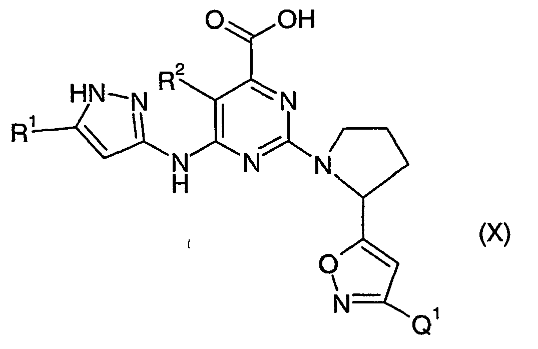 wherein R¹, R² and Q¹ are as defined in formula (I) except that any functional group is protected if necessary, with a compound of formula:

H-O-(C1-C6)alkyl

wherein the (C1-C6)alkyl group is optionally substituted by at least one group as defined above as a substituent for R³ and any functional group is protected if necessary;
or
Process (j) for compounds of formula (I) wherein R³ is a (C1-C6)alkyl, (C3-C6)alkenyl, (C3-C6)alkynyl or (C1-C6)alkoxy group substituted by at least one group as defined above, reacting a compound of formula (XI): 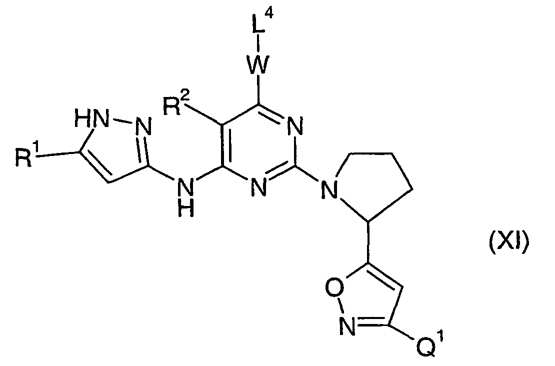 wherein L⁴ is a suitable displaceable group, W is an optionally substituted (C1-C6)alkyl, (C3-C6)alkenyl, (C3-C6)alkynyl or (C1-C6)alkoxy group and R¹, R² and Q¹ are as defined in formula (I) except that any functional group is protected if necessary, with a compound of formula H-Xa, (Xb), (Xc), (Xc') or M-R³ as defined above;
or
Process (k) for compounds of formula (I) wherein Q¹ is a pyridyl group containing a substituent in the ortho or the para position in the ring relative to the pyridine nitrogen (and optionally containing one or more additional substituents as hereinbefore defined), reacting a compound of formula (XII): 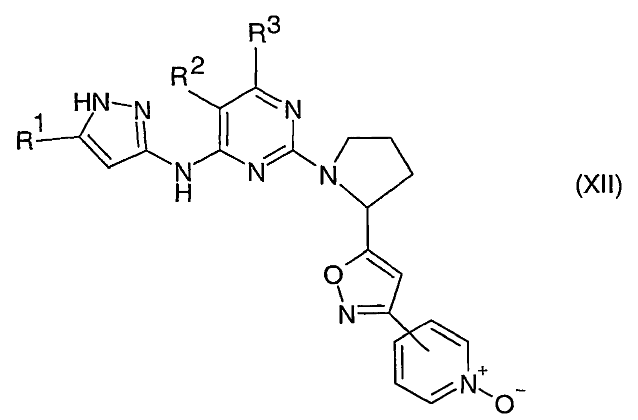 wherein R¹, R² and R³ are as defined in formula (I) except that any functional group is protected if necessary, with a reagent or reagents that combine an acylating, phosphorylating, sulfonating, sulfenylating or silylating agent with a nucleophile. Examples of such reagents include acetyl chloride, trimethylsilyl cyanide and thionyl chloride;
and optionally after process (a), (b), (c), (d) (e), (f), (g), (h), (i), (j) or (k) carrying out one or more of the following:
- converting the compound obtained to a further compound of the invention
- forming a pharmaceutically-acceptable salt of the compound.

### Process (a)

### Reaction Conditions for Process (a)

A suitable displaceable group L¹ in the compound of formula (II) is for example a halogeno or a sulfonyloxy group, for example a fluoro, chloro, methylsulfonyloxy or toluene-4-sulfonyloxy group. A particular group L¹ is fluoro, chloro or methylsulfonyloxy.

Process (a) conveniently may be carried out in the presence of a suitable base and/or in the presence of a suitable Lewis acid. A suitable base is, for example, an organic amine base such as pyridine, 2,6-lutidine, collidine, 4-dimethylaminopyridine, triethylamine, di-isopropylethylamine, N-methylmorpholine or diazabicyclo[5.4.0]undec-7-ene, or, for example, an alkali or alkaline earth metal carbonate, such as sodium carbonate, potassium carbonate, cesium carbonate or calcium carbonate, or, for example, an alkali metal hydride, such as sodium hydride. A particular base is an organic amine base, for example di-isopropylethylamine. A suitable Lewis acid is zinc acetate.

Process (a) may conveniently be carried out in the presence of a suitable inert solvent or diluent for example a ketone such as acetone, an alcohol such as ethanol, butanol or n-hexanol, an ether such as dioxane or an aromatic hydrocarbon such as xylene, toluene or N-methyl pyrrolid-2-one and at a temperature in the range from 0°C to reflux, particularly reflux.

Process (a) may alternatively conveniently be carried out under standard Buchwald conditions (see, for example, J. Am. Chem. Soc.,118, 7215; J. Am. Chem. Soc.,119, 8451; J. Org. Chem., 62, 1568 and 6066). For example, process (a) may conveniently be carried out in the presence of palladium acetate, in a suitable inert solvent or diluent for example an aromatic solvent such as toluene, benzene or xylene, in the presence of a suitable base, for example an inorganic base such as caesium carbonate or an organic base such as potassium-*t*-butoxide and in the presence of a suitable ligand such as 2,2'-bis(diphenylphosphino)-1,1'-binaphthyl and at a temperature in the range from 25 to 80°C.

### Starting Materials for Process (a)

A compound of formula (II) may be obtained by conventional procedures. For example, a compound of formula (II) may be obtained by the reaction, conveniently in the presence of a suitable base, of a pyrimidine of formula (IIa): 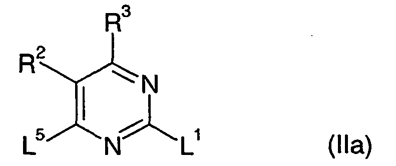 wherein L⁵ is a suitable displaceable group and L¹, R² and R³ have any of the meanings defined hereinbefore except that any functional group is protected if necessary, with a pyrazole of formula (V): 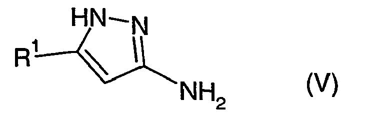 wherein R¹ has any of the meanings defined hereinbefore except that any functional group is protected if necessary.

A suitable displaceable group L⁵ in the compound of formula (IIa) is, for example, a halogeno or a sulfonyloxy group, for example a fluoro, chloro, methylsulfonyloxy or toluene-4-sulfonyloxy group. A particular group L⁵ is chloro.

A suitable base for the reaction of a pyrimidine of formula (IIa) and a pyrazole of formula (V) includes, for example, an alkali or alkaline earth metal carbonate, such as sodium carbonate, potassium carbonate, cesium carbonate or calcium carbonate or an organic amine base such as di-isopropylethylamine.

The reaction may conveniently be carried out in the presence of a suitable inert solvent or diluent for example a ketone such as acetone or an alcohol such as ethanol, butanol or n-hexanol or an aromatic hydrocarbon such as toluene or *N-*methyl pyrrolid-2-one. The reaction is conveniently carried out at a temperature in the range of, for example, 10 to 150°C, particularly at room temperature.

Pyrimidines of formula (IIa) and pyrazoles of formula (V) are commercially available compounds or they are known in the literature, or they can be prepared by standard processes known in the art.

A compound of formula (III) may be obtained by conventional procedures. For example, a compound of formula (III) may be obtained as illustrated in *Reaction Scheme 1:*

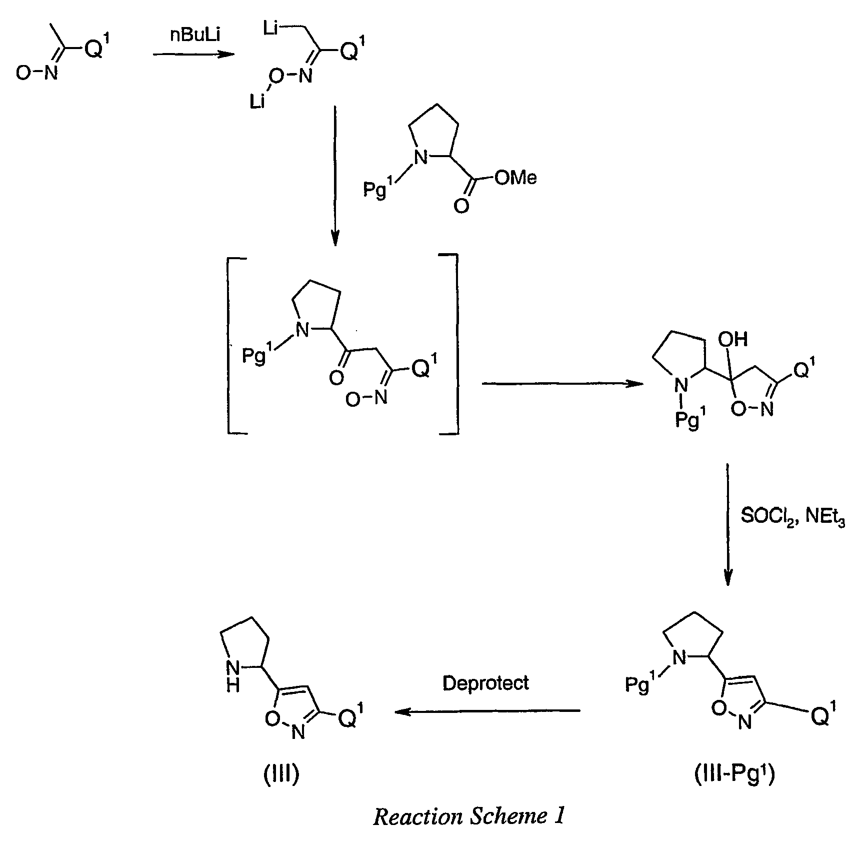

In *Reaction Scheme 1*, Pg¹ is a suitable protecting group, such as, for example, tert-butoxycarbonyl. The group Q¹ is as previously defined. For example, Q¹ may be pyridyl (such as pyrid-2-yl).

Alternatively, for example, a compound of formula (III) may be obtained as illustrated in *Reaction Scheme 2:*

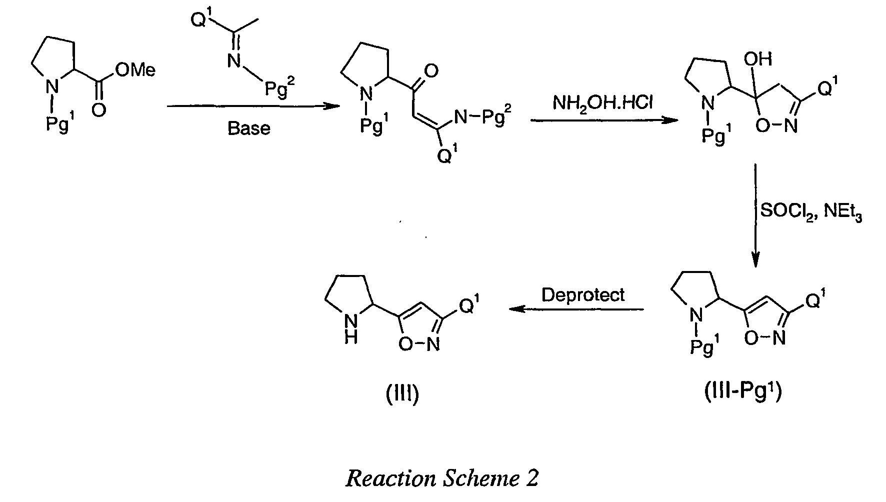

In *Reaction Scheme 2*, Pg¹ is a suitable protecting group as described above. Similarly, Pg² is a suitable protecting group such as, for example, cyclohexyl. The group Q¹ is as previously defined.

Alternatively, for example, a compound of formula (III) may be obtained as illustrated in *Reaction Scheme 3*:

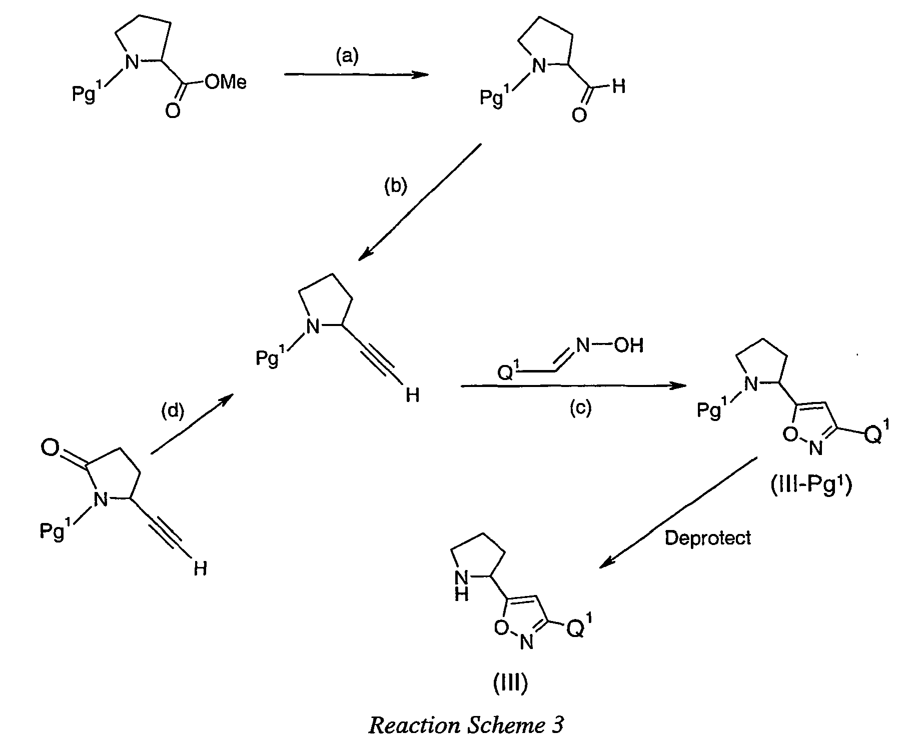

In *Reaction Scheme 3*, Pg¹ is a suitable protecting group as described above. The group Q¹ is as previously defined.

In *Reaction Scheme 3,* step (a) may conveniently be effected by a suitable reducing agent, such as diisobutylaluminium hydride. Step (a) may conveniently be carried out in the presence of a suitable inert solvent or diluent, for example an ether or aromatic hydrocarbon such as toluene or a chlorinated hydrocarbon such as dichloromethane, and at a temperature in the range of, for example, from -78°C to 25°C.

Step (b) may conveniently be carried out by reaction with dimethyl (1-diazo-2-oxopropyl) phosphonate in the presence of a suitable inert solvent or diluent for example a chlorinated hydrocarbon such as dichloromethane and at a temperature in the range of, for example, from -20°C to 50°C.

Alternatively, step (b) may be conducted by reaction with carbon tetrabromide, zinc and triphenylphosphine to provide a 2-(dibromoethenyl) intermediate, in the presence of a suitable inert solvent or diluent for example a chlorinated hydrocarbon such as dichloromethane and at a temperature in the range of, for example, -20 to 50°C. The conversion of the 2-(dibromoethenyl) intermediate to the 2-ethynyl intermediate may then be conducted by reaction with n-butyl lithium in the presence of a suitable inert solvent or diluent for example an ether such as tetrahydrofuran and at a temperature in the range of, for example, -70 to 0°C.

Step (c) may conveniently be effected by treatment with a suitable chlorinating agent, such as N-chlorosuccinimide, to give an α-chloroaldyde oxime intermediate and then a suitable base, such as triethylamine, to give a nitrile oxide intermediate which takes part in a 3+2 cycloaddition reaction. Alternatively, the oxime (Q¹-CH=N-OH) may be directly transformed into the nitrile oxide intermediate by treatment with sodium hypochlorite. Such reactions may conveniently be carried out in the presence of a suitable inert solvent or diluent, for example a chlorinated hydrocarbon such as dichloromethane, and at a temperature in the range of, for example, from -20°C to 50°C.

Step (d) may conveniently be effected by a suitable reducing agent, such as borane, diisobutylaluminium hydride or lithium aluminium hydride. Step (d) may conveniently be carried out in the presence of a suitable inert solvent or diluent, for example an ether or aromatic hydrocarbon such as toluene or a chlorinated hydrocarbon such as dichloromethane, and at a temperature in the range of, for example, from -50°C to 100°C.

In each of *Reaction Schemes 1, 2 and 3*, the protecting group may be removed by any convenient method as described in the literature or known to the skilled chemist as appropriate for the removal of the particular protecting group used.

### Process (b)

### Reaction Conditions for Process (b)

A suitable displaceable group L² in a compound of formula (IV) is, for example, halogeno or a sulfonyloxy group, for example fluoro, chloro, methanesulfonyloxy or toluene-4-sulfonyloxy.

Process (b) is conveniently carried out in the presence of a suitable acid. A suitable acid is, for example, an inorganic acid such as anhydrous hydrogen chloride.

Process (b) may conveniently be carried out in the presence of a suitable inert solvent or diluent for example a ketone such as acetone or an alcohol such as ethanol, butanol or n-hexanol or an aromatic hydrocarbon such as toluene or *N*-methyl pyrrolid-2-one and at a temperature in the range from 0°C to reflux, particularly reflux.

Process (b) may alternatively conveniently be carried out under standard Buchwald conditions as discussed above for process (a).

### Starting Materials for Process (b)

A compound of formula (IV) may be prepared using conventional methods, for example as discussed above.

Pyrazoles of formula (V) are commercially available compounds or they are known in the literature, or they can be prepared by standard processes known in the art.

### Process (c)

### Reaction Conditions for Process (c)

Process (c) is conveniently carried out in a suitable inert solvent or diluent such as N-methylpyrrolidinone or butanol at a temperature in the range from 100 to 200°C, in particular in the range from 150 to 170°C. The reaction is preferably conducted in the presence of a suitable base such as, for example, sodium methoxide or potassium carbonate.

### Starting Materials for Process (c)

Compounds of the formulae (VI) and (VII) are commercially available compounds or they are known in the literature, or they can be prepared by standard processes known in the art.

### Process (d)

### Reaction Conditions for Process (d)

Process (d) is conveniently carried out in a suitable inert solvent or diluent, for example, an alcohol such as ethanol or butanol at a temperature in the range from 50 to 120°C, in particular in the range from 70 to 100°C.

### Starting Materials for Process (d)

A compound of formula (VIII) may be prepared using conventional methods, for example as discussed above.

Hydrazine is a commercially available compound.

### Process (e)

### Reaction Conditions for Process (e)

A suitable displaceable group L³ in a compound of formula (IX) is, for example, halogeno or a sulfonyloxy group, for example fluoro, chloro, methanesulfonyloxy or toluene-4-sulfonyloxy.

Process (e) is conveniently carried out in the presence of a suitable base. A suitable base is, for example, sodium hydride or an organic amine base such as diisopropylethylamine. Another suitable base is an alkali metal alkoxide, for example sodium methoxide or sodium ethoxide.

Process (e) is conveniently carried out in the presence of a suitable inert solvent or diluent, for example a ketone such as acetone, an alcohol such as methanol, ethanol, butanol or n-hexanol, an ether such as tetrahydrofuran or an aromatic hydrocarbon such as toluene or N-methyl pyrrolid-2-one.

Process (e) is conveniently carried out at a temperature in the range from 0°C to reflux, particularly reflux. Conveniently, process (e) may also be performed by heating the reactants in a sealed vessel using a suitable heating apparatus such as a microwave heater.

### Starting Materials for Process (e)

A compound of formula (IX) may be prepared using conventional methods, for example as discussed above.

Compounds of the formula H-Xa are commercially available compounds or they are known in the literature, or they can be prepared by standard processes known in the art.

### Process (f)

### Reaction Conditions for Process (f)

The reaction of process (f) is conveniently carried out using analogous conditions to those described above for process (e).

### Starting Materials for Process (f)

A compound of formula (IX) may be prepared using conventional methods, for example as discussed above.

Compounds of the formula Xb are commercially available compounds or they are known in the literature, or they can be prepared by standard processes known in the art.

### Process

### Reaction Conditions for Process (g)

Process (g) is conveniently carried out in the presence of a suitable base. A suitable base is, for example, an organic amine base, such as for example triethylamine or diisopropylethylamine.

Process (g) is conveniently carried out in the presence of a suitable catalyst. A suitable catalyst is, for example, copper iodide / palladium (II) chloride-bis(triphenyl)phosphine.

Process (g) is conveniently carried out in the presence of a suitable inert solvent or diluent for example acetonitrile, THF or dioxane and at a temperature in the range from 0°C to reflux, particularly reflux. Conveniently, process (g) may also be performed by heating the reactants in a sealed vessel using a suitable heating apparatus such as a microwave heater. Starting Materials for Process (g)

A compound of formula (IX) may be prepared using conventional methods, for example as discussed above.

Compounds of the formula Xc and Xc' are commercially available compounds or they are known in the literature, or they can be prepared by standard processes known in the art.

### Process (h)

### Reaction Conditions for Process (h)

Process (h) is conveniently carried out in the presence of a suitable catalyst. A suitable catalyst is, for example, a palladium (0) catalyst, such as for example tetrakis(triphenyl)phosphine palladium(0). As a person skilled in the art would appreciate, the palladium (0) catalyst may be prepared *in situ.*

Process (h) is conveniently carried out in the presence of a suitable inert solvent or diluent for example THF or dioxane and at a temperature in the range from 0°C to reflux, particularly reflux.

### Starting Materials for Process (h)

A compound of formula (IX) may be prepared using conventional methods, for example as discussed above.

Compounds of the formula M-R³ are commercially available compounds or they are known in the literature, or they can be prepared by standard processes known in the art.

### Process (i)

### Reaction Conditions for Process (i)

Process (i) is conveniently carried out in the presence of a suitable acid. A suitable acid is, for example, concentrated sulfuric acid.

Process (i) is conveniently carried out in the absence of an inert solvent or diluent and at a temperature in the range from room temperature to reflux, particularly reflux.

### Startling Materials for Process (i)

A compound of formula (X) may be prepared using conventional methods, for example as discussed above.

Compounds of the formula H-O-(C1-C6)alkyl are commercially available compounds or they are known in the literature, or they can be prepared by standard processes known in the art.

### Process

### Reaction Conditions for Process (j)

A suitable displaceable group L⁴ in a compound of formula (XI) is, for example, halogeno or a sulfonyloxy group, for example fluoro, chloro, methanesulfonyloxy or toluene-4-sulfonyloxy.

The reaction of process (k) is conveniently carried out using analogous conditions to those described above for process (e).

### Starting Materials for Process (j)

A compound of formula (XI) may be prepared using conventional methods, for example as discussed above.

Compounds of the formula H-Xa, (Xb), (Xc), (Xc') or M-R³ are commercially available compounds or they are known in the literature, or they can be prepared by standard processes known in the art.

### Process k

### Reaction Conditions for Process (k)

Process (k) may conveniently be carried out in a suitable inert solvent or diluent, for example, an ether such as tetrahydrofuran and at a temperature in the range of, for example, from 50 to 150°C, in particular in the range of from 70 to 140°C.

### Starting Materials for Process (k)

A compound of formula (XII) may be prepared using conventional methods, for example as discussed above.

As stated above, compounds of formulae (II), (III), (IV), (V), (VI), (VII), (VIII), HXa, (Xb), (Xc), (Xc') and M-R³ are either commercially available, are known in the literature or may be prepared using known techniques. For example, these compounds may be prepared by analogous processes to those described in WO 03/048133. Examples of preparation methods for certain of these compounds are given hereinafter in the examples.

It will be appreciated that compounds of formula (I) can be converted into further compounds of formula (I) using standard procedures conventional in the art, for example by means of conventional substitution reactions or of conventional functional group modifications either prior to or immediately following the processes mentioned above, and such procedures are included in the process aspect of the invention.

Examples of the types of conversion reactions that may be used include introduction of a substituent by means of an aromatic substitution reaction or of a nucleophilic substitution reaction, reduction of substituents, alkylation of substituents and oxidation of substituents. The reagents and reaction conditions for such procedures are well known in the chemical art.

Particular examples of aromatic substitution reactions include the introduction of a nitro group using concentrated nitric acid; the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group. Particular examples of nucleophilic substitution reactions include the introduction of an alkoxy group or of an alkylamino group, a dialkyamino group or a N-containing heterocycle using standard conditions. Particular examples of reduction reactions include the reduction of a carbonyl group to a hydroxy group with sodium borohydride or of a nitro group to an amino group by catalytic hydrogenation with a nickel catalyst or by treatment with iron in the presence of hydrochloric acid with heating; and particular examples of oxidation reactions include oxidation of alkylthio to alkylsulfinyl or alkylsulfonyl. Other conversion reactions that may be used include the acid catalysed esterification of carboxylic acids with alcohols.

An example of a suitable conversion reaction is the conversion of a compound of formula (I) wherein R³ is a (C1-C6)alkenyl group to a compound of formula (I) wherein R³ is a (C1-C6)alkyl group substituted by a di-[(C1-C6)alkyl]amino group or by a saturated monocyclic 4-,5-,6- or 7-membered ring, which ring comprises nitrogen and one or more heteroatoms independently selected from nitrogen, oxygen and sulfur. Such a conversion may be achieved using standard procedures, for example by conversion of the alkenyl group to a dihydroxyalkyl group with osmium tetroxide, oxidation to the corresponding ketone with a suitable oxidising agent (for example sodium periodate) and conversion of the ketone group to the desired substituent as defined above by reaction with the appropriate amine in the presence of a suitable reducing agent (for example sodium cyanoborohydride).

Another example of a suitable conversion reaction is the conversion of a compound of formula (I) wherein R³ is an optionally substituted (C1-C6)alkoxycarbonyl group to a compound of formula (I) wherein R³ is an optionally substituted carbamoyl, (C1-C6)alkylcarbamoyl or di-[(C1-C6)alkyl]carbamoyl group or an optionally substituted -C(O)R^{3b} group, wherein R^{3b} is as defined above. Such a conversion may be achieved using standard procedures, for example by reaction of the compound of formula (I) wherein R³ is an optionally substituted (C1-C6)alkoxycarbonyl group with ammonia, with an optionally substituted primary, secondary or tertiary amine or with an optionally substituted H-R^{3b} group. As the skilled person would appreciate, this conversion could be conducted starting from the carboxylic acid and preparing an activated ester, for example using 4-(4,6-dimethoxy[1,3,5]triazin-2-yl)-4-methyl-morpholinium chloride, which may then be reacted with the necessary amine.

Another example of a suitable conversion reaction is the conversion of a compound of formula (I) wherein R³ is a (C1-C6)alkoxycarbonyl group to a compound of formula (I) wherein R³ is a hydroxy-(C1-C6)alkyl group. Such a conversion may be achieved using standard procedures, for example by reduction using lithium borohydride or lithium aluminium hydride.

It will be appreciated that the preparation of compounds of formula (I) may involve, at various stages, the addition and removal of one or more protecting groups. The protecting groups used in the processes above may in general be chosen from any of the groups described in the literature or known to the skilled chemist as appropriate for the protection of the group in question and may be introduced by conventional methods. Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower", as in, for example, lower alkyl, signifies that the group to which it is applied preferably has 1 to 4 carbon atoms. It will be understood that these examples are not exhaustive. Where specific examples of methods for the removal of protecting groups are given below these are similarly not exhaustive. The use of protecting groups and methods of deprotection not specifically mentioned are, of course, within the scope of the invention.

A carboxy protecting group may be the residue of an ester-forming aliphatic or arylaliphatic alcohol or of an ester-forming silanol (the said alcohol or silanol preferably containing 1 to 20 carbon atoms). Examples of carboxy protecting groups include straight or branched chain (1 to 12C)alkyl groups (for example isopropyl, and tert-butyl); lower alkoxy- lower alkyl groups (for example methoxymethyl, ethoxymethyl and isobutoxymethyl); lower acyloxy-lower alkyl groups, (for example acetoxymethyl, propionyloxymethyl, butyryloxymethyl and pivaloyloxymethyl); lower alkoxycarbonyloxy-lower alkyl groups (for example 1-methoxycarbonyloxyethyl and 1-ethoxycarbonyloxyethyl); aryl-lower alkyl groups (for example benzyl, 4-methoxybenzyl, 2-nitrobenzyl, 4-nitrobenzyl, benzhydryl and phthalidyl); tri(lower alkyl)silyl groups (for example trimethylsilyl and tert-butyldimethylsilyl); tri(lower alkyl)silyl-lower alkyl groups (for example trimethylsilylethyl); and (2-6C)alkenyl groups (for example allyl). Methods particularly appropriate for the removal of carboxy protecting groups include for example acid-, base-, metal- or enzymically-catalysed cleavage.

Examples of hydroxy protecting groups include lower alkyl groups (for example tert-butyl), lower alkenyl groups (for example allyl); lower alkanoyl groups (for example acetyl); lower alkoxycarbonyl groups (for example tert-butoxycarbonyl); lower alkenyloxycarbonyl groups (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); tri(lower alkyl)silyl (for example trimethylsilyl and tert-butyldimethylsilyl) and aryl-lower alkyl (for example benzyl) groups.

Examples of amino protecting groups include formyl, aryl-lower alkyl groups (for example benzyl and substituted benzyl, 4-methoxybenzyl, 2-nitrobenzyl and 2,4-dimethoxybenzyl, and triphenylmethyl); di-4-anisylmethyl and furylmethyl groups; lower alkoxycarbonyl (for example tert-butoxycarbonyl); lower alkenyloxycarbonyl (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); lower alkanoyloxyalkyl groups (for example pivaloyloxymethyl); trialkylsilyl (for example trimethylsilyl and tert-butyldimethylsilyl); alkylidene (for example methylidene) and benzylidene and substituted benzylidene groups.

Methods appropriate for removal of hydroxy and amino protecting groups include, for example, acid-, base-, metal- or enzymically-catalysed hydrolysis for groups such as 2-nitrobenzyloxycarbonyl, hydrogenation for groups such as benzyl and photolytically for groups such as 2-nitrobenzyloxycarbonyl. For example a tert butoxycarbonyl protecting group may be removed from an amino group by an acid catalysed hydrolysis using trifluoroacetic acid.

The reader is referred to Advanced Organic Chemistry, 4th Edition, by J. March, published by John Wiley & Sons 1992, for general guidance on reaction conditions and reagents and to Protective Groups in Organic Synthesis, 2nd Edition, by T. Green et al., also published by John Wiley & Son, for general guidance on protecting groups.

When a pharmaceutically-acceptable salt of a compound of formula (I) is required, for example an acid-addition salt, it may be obtained by, for example, reaction of said compound with a suitable acid using a conventional procedure. When it is desired to obtain the free base from a salt of the compound of formula (I), a solution of the salt may be treated with a suitable base, for example, an alkali or alkaline earth metal carbonate or hydroxide, for example sodium carbonate, potassium carbonate, calcium carbonate, sodium hydroxide or potassium hydroxide.

As mentioned hereinbefore some of the compounds according to the present invention may contain one or more chiral centers and may therefore exist as stereoisomers. Stereoisomers may be separated using conventional techniques, e.g. chromatography or fractional crystallisation. The enantiomers may be isolated by separation of a racemate for example by fractional crystallisation, resolution or HPLC. The diastereoisomers may be isolated by separation by virtue of the different physical properties of the diastereoisomers, for example, by fractional crystallisation, HPLC or flash chromatography. Alternatively Particular stereoisomers may be made by chiral synthesis from chiral starting materials under conditions which will not cause racemisation or epimerisation, or by derivatisation, with a chiral reagent. When a specific stereoisomer is isolated it is suitably isolated substantially free for other stereoisomers, for example containing less than 20%, particularly less than 10% and more particularly less than 5% by weight of other stereoisomers.

In the section above relating to the preparation of the compounds of formula (I), the expression "inert solvent" refers to a solvent which does not react with the starting materials, reagents, intermediates or products in a manner which adversely affects the yield of the desired product.

Persons skilled in the art will appreciate that, in order to obtain compounds of the invention in an alternative and in some occasions, more convenient manner, the individual process steps mentioned hereinbefore may be performed in different order, and/or the individual reactions may be performed at different stage in the overall route (i.e. chemical transformations may be performed upon different intermediates to those associated hereinbefore with a particular reaction).

Certain intermediates used in the processes described above are novel and form a further feature of the present invention. Accordingly there is provided a compound selected from a compound the formulae (III) and (111-Pg¹) as hereinbefore defined, or a salt thereof. The intermediate may be in the form of a salt of the intermediate. Such salts need not be a pharmaceutically-acceptable salt. For example it may be useful to prepare an intermediate in the form of a pharmaceutically non-acceptable salt if, for example, such salts are useful in the manufacture of a compound of formula (I).

In one aspect, particular intermediate compounds of the invention include, for example, one or more intermediate compounds of the formula (III) selected from:
2-[3-(2-cyanopyrid-3-yl)isoxazol-5-yl]pyrrolidine;
2-[3-(2-chloropyrid-3-yl)isoxazol-5-yl]pyrrolidine;
2-[3-(2-methylaminogyrid-3-yl)isoxazol-5-yl]pyrrolidine;
2-[3-(2-methylpyrid-3-yl)isoxazol-5-yl]pyrrolidine;
2-[3-(3-ethylpyrazin-2-yl)isoxazol-5-yl]pyrrolidine;
2-[3-(3-methylpyrazin-2-yl)isoxazol-5-yl]pyrrolidine;
2-[3-(3-ethoxypyrazin-2-yl)isoxazol-5-yl]pyrrolidine;
2-[3-(3-ethylaminopyrazin-2-yl)isoxazol-5-yl]pyrrolidine;
2-[3-(3-chloropyrazin-2-yl)isoxazol-5-yl]pyrrolidine; and
2-[2-{3-(1-oxopyrid-3-yl)isoxazol-5-yl}pyrrolidine;
and salts thereof.

In another aspect, particular intermediate compounds of the invention include, for example, one or more intermediate compounds of the formula (III) selected from:
S-2-[3-(2-cyanopyrid-3-yl)isoxazol-5-yl]pyrrolidine;
S-2-[3-(2-chloropyrid-3-yl)isoxazol-5-yl]pyrrolidine;
S-2-[3-(2-methylaminopyrid-3-yl)isoxazol-5-yl]pyrrolidine;
S-2-[3-(2-methylpyrid-3-yl)isoxazol-5-yl]pyrrolidine;
S-2-[3-(3-ethylpyrazin-2-yl)isoxazol-5-yl]pyrrolidine;
S-2-[3-(3-methylpyrazin-2-yl)isoxazol-5-yl]pyrrolidine;
S-2-[3-(3-ethoxypyrazin-2-yl)isoxazol-5-yl]pyrrolidine;
S-2-[3-(3-ethylaminopyrazin-2-yl)isoxazol-5-yl]pyrrolidine;
S-2-[3-(3-chloropyrazin-2-yl)isoxazol-5-yl]pyrrolidine; and
S-2-[2-{3-(1-oxopyrid-3-yl)isoxazol-5-yl }pyrrolidine;
and salts thereof.

In another aspect, particular intermediate compounds of the invention include, for example, one or more intermediate compounds of the formula (III-Pg¹) selected from:
N-(tert-butoxycarbonyl)-2-[3-(2-chloropyrid-3-yl)isoxazol-5-yl]pyrrolidine;
N-(tert-butoxycarbonyl)-2-[3-(3-ethylpyrazin-2-yl)isoxazol-5-yl]pyrrolidine;
N-(tert-butoxycarbonyl)-2-[3-(3-methylpyrazin-2-yl)isoxazol-5-yl]pyrrolidine;
N-(tert-butoxycarbonyl)-2-[3-(3-chloropyrazin-2-yl)isoxazol-5-yl]pyrrolidine;
N-(tert-butoxycarbonyl)-2-[3-(3-ethoxypyrazin-2-yl)isoxazol-5-yl]pyrrolidine;
N-(tert-butoxycarbonyl)-2-[3-(3-ethylaminopyrazin-2-yl)isoxazol-5-yl]pyrrolidine;
N-(tert-butoxycarbonyl)-2-[3-(2-methylpyrid-3-yl)isoxazol-5-yl]pyrrolidine;
N-(tert-butoxycarbonyl)-2-[3-(2-cyanopyrid-3-yl)isoxazol-5-yl]pyrrolidine;
N-(tert-butoxycarbonyl)-2-[3-(2-methylaminopyrid-3-yl)isoxazol-5-yl]pyrrolidine; and
N-(tert-butoxycarbonyl)-S-2-[2-{3-(1-oxopyrid-3-yl)isoxazol-5-yl}pyrrolidine;
and salts thereof.

In another aspect, particular intermediate compounds of the invention include, for example, one or more intermediate compounds of the formula (III-Pg¹) selected from:
S-N-(tert-butoxycarbonyl)-2-[3-(2-chloropyrid-3-yl)isoxazol-5-yl]pyrrolidine;
S-N-(tert-butoxycarbonyl)-2-[3-(3-ethylpyrazin-2-yl)isoxazol-5-yl]pyrrolidine;
S-N-(tert-butoxycarbonyl)-2-[3-(3-methylpyrazin-2-yl)isoxazol-5-yl]pyrrolidine;
S-N-(tert-butoxycarbonyl)-2-[3-(3-chloropyrazin-2-yl)isoxazol-5-yl]pyrrolidine;
S-N-(tert-butoxycarbonyl)-2-[3-(3-ethoxypyrazin-2-yl)isoxazol-5-yl]pyrrolidine;
S-N-(tert-butoxycarbonyl)-2-[3-(3-ethylaminopyrazin-2-yl)isoxazol-5-yl]pyrrolidine;
S-N-(tert-butoxycarbonyl)-2-[3-(2-methylpyrid-3-yl)isoxazol-5-yl]pyrrolidine;
S-N-(tert-butoxycarbonyl)-2-[3-(2-cyanopyrid-3-yl)isoxazol-5-yl]pyrrolidine;
S-N-(tert-butoxycarbonyl)-2-[3-(2-methylaminopyrid-3-yl)isoxazol-5-yl]pyrrolidine; and
S-N-(tert-butoxycarbonyl)-S-2-[2-{3-(1-oxopyrid-3-yl)isoxazol-5}pyrrolidine;
and salts thereof.

The activity and selectivity of compounds according to the invention may be determined using an appropriate assay as described, for example, in WO 03/048133, and detailed below.

### Biological Assays

### IGF-1R Kinase Assay

### a) Protein cloning, expression and purification

A DNA molecule encoding a fusion protein containing glutathione-S-transferase (GST), thrombin cleavage site and IGF-1R intracellular domain (amino-acids 930-1367) and subsequently referred to as GST-IGFR, was constructed and cloned into pFastBac1 (Life Technologies Ltd, UK) using standard molecular biology techniques (Molecular Cloning - A Laboratory Manual, Second Edition 1989; Sambrook, Fritsch and Maniatis; Cold Spring Harbour Laboratory Press).

Production of recombinant virus was performed following the manufacturer's protocol.

Briefly, the pFastBac-1 vector containing GST-IGFR was transformed into *E*. *coli* DH10Bac cells containing the baculovirus genome (bacmid DNA) and via a transposition event in the cells, a region of the pFastBac vector containing gentamycin resistance gene and the GST-IGFR expression cassette including the baculovirus polyhedrin promoter was transposed directly into the bacmid DNA. By selection on gentamycin, kanamycin, tetracycline and X-gal, resultant white colonies should contain recombinant bacmid DNA encoding GST-IGFR. Bacmid DNA was extracted from a small scale culture of several BH10Bac white colonies and transfected into Spodoptera frugiperda Sf21 cells grown in TC100 medium (Life Technologies Ltd, UK) containing 10% serum using CellFECTIN reagent (Life Technologies Ltd, UK) following the manufacturer's instructions. Virus particles were harvested by collecting cell culture medium 72 hrs post transfection. 0.5 ml of medium was used to infect 100 ml suspension culture of Sf21s containing 1 x 10⁷ cells/ml. Cell culture medium was harvested 48 hrs post infection and virus titre determined using a standard plaque assay procedure. Virus stocks were used to infect Sf9 and "High 5" cells at a multiplicity of infection (MOI) of 3 to ascertain expression of recombinant GST-IGFR .

The GST-IGFR protein was purified by affinity chromatography on Glutathione-Sepharose followed by elution with glutathione. Briefly, cells were lysed in 50mM HEPES pH 7.5 (Sigma, H3375), 200mM NaCl (Sigma, S7653), Complete Protease Inhibitor cocktail (Roche, 1 873 580) and 1mM DTT (Sigma, D9779), hereinafter referred to as lysis buffer. Clarified lysate supernatant was loaded through a chromatography column packed with Glutathione Sepharose (Amersham Pharmacia Biotech UK Ltd.). Contaminants were washed from the matrix with lysis buffer until the UV absorbance at 280nm returned to the baseline. Elution was carried out with lysis buffer containing 20mM reduced glutathione (Sigma, D2804) and fractions containing the GST fusion protein were pooled and dialysed into a glycerol-containing buffer comprising 50 mM HEPES, pH 7.5, 200 mM NaCl 10% glycerol (v/v), 3 mM reduced glutathione and 1 mM DTT.

### b) Kinase activity assay

The activity of the purified enzyme was measured by phosphorylation of a synthetic poly GluAlaTyr (EAY) 6:3:1 peptide (Sigma-Aldrich Company Ltd, UK, P3899) using an ELISA detection system in a 96-well format.

### b.i) Reagents used

### Stock solutions

| | | | |
|---|---|---|---|
| 200mM | HEPES, pH 7.4 | stored at 4°C | (Sigma, H3375) |
| 1M | DTT | stored at-20°C | (Sigma, D9779) |
| 100mM | Na₃VO₄ | stored at 4°C | (Sigma, 56508) |
| 1M | MnCl₂ | stored at 4°C | (Sigma, M3634) |
| 1mM | ATP | stored at -20°C | (Sigma, A3377) |
| Neat | Triton X-100 | stored at room temperature | (Sigma, T9284) |
| 10mg/ml | BSA | stored at 4°C | (Sigma, A7888) |

### Enzyme solution

GST-IGF-1R fusion protein at 75ng/ml in 100mM HEPES, pH 7.4, 5mM DTT, 0.25mM Na₃VO₄, 0.25% Triton X-100, 0.25mg/ml BSA, freshly prepared.

### Co-factor solution

100mM HEPES, pH 7.4,60mM MnCl₂, 5mM ATP.

### Poly EAY substrate

Sigma substrate poly (Glu, Ala, Tyr) 6:3:1 (P3899). Made up to 1 mg/ml in PBS and stored at -20°C.

### Assay plates

Nunc Maxisorp 96 well immunoplates (Life Technologies Ltd, UK).

### Antibodies

Anti-phosphotyrosine antibody, monoclonal from Upstate Biotechnology Inc., NY, USA (UBI 05-321). Dilute 3µl in 11ml PBS/T + 0.5% BSA per assay plate. Sheep- anti-mouse IgG HRP-conjugated secondary antibody from Amersham Pharmacia Biotech UK Ltd. (NXA931). Dilute 20µl of stock into 11ml PBS/T + 0.5% BSA per assay plate.

### TMB solution

Dissolve 1mg TMB tablet (Sigma T5525) into 1ml DMSO (Sigma, D8779) in the dark for 1 hour at room temperature. Add this solution to 9ml of freshly prepared 50mM phosphate-citrate buffer pH 5.0 + 0.03% sodium perborate [1 buffer capsule (Sigma P4922) per 100ml distilled water].

Stop solution is 1M H₂SO₄ (Fisher Scientific UK. Cat. No. S/9200/PB08).

### Test compound

Dissolve in DMSO to 10mM then dilutions in distilled water to give a range from 200 to 0.0026µM in 1-2% DMSO final concentration in assay well.

### b.ii) Assay protocol

The poly EAY substrate was diluted to 1µg/ml in PBS and then dispensed in an amount of 100µl per well into a 96-well plate. The plate was sealed and incubated overnight at 4°C. Excess poly EAY solution was discarded and the plate was washed (2x PBS/T; 250µl PBS per well), blotting dry between washes. The plate was then washed again (1x 50mM HEPES, pH 7.4; 250µl per well) and blotted dry (this is important in order to remove background phosphate levels). 10µl test compound solution was added with 40µl of kinase solution to each well. Then 50µl of co-factor solution were added to each well and the plate was incubated for 60 minutes at room temperature.

The plate was emptied (i.e. the contents were discarded) and was washed twice with PBS/T (250µl per well), blotting dry between each wash. 100µl of diluted anti-phosphotyrosine antibody were added per well and the plate was incubated for 60 minutes at room temperature.

The plate was again emptied and washed twice with PBS/T (250µl per well), blotting dry between each wash. 100µl of diluted sheep- anti-mouse IgG antibody were added per well and the plate was left for 60 minutes at room temperature. The contents were discarded and the plate washed twice with PBS/T (250µl per well), blotting dry between each wash. 100µl of TMB solution were added per well and the plate was incubated for 5-10 minutes at room temperature (solution turns blue in the presence horse radish peroxidase).

Reaction was stopped with 50µl of H₂SO₄ per well (turns the blue solution yellow) and the plate was read at 450nm in Versamax plate reader (Molecular Devices Corporation, CA, USA) or similar.

The compounds of the Examples were found to have an IC₅₀ in the above test of less than 100µM.

### c) Inhibition of IGF-stimulated cell proliferation

The construction of murine fibroblasts (NIH3T3) over-expressing human IGF-1 receptor has been described by Lammers et al (EMBO J, 8, 1369-1375, 1989). These cells show a proliferative response to IGF-I which can be measured by BrdU incorporation into newly synthesised DNA. Compound potency was determined as causing inhibition of the IGF-stimulated proliferation in the following assay:

### c.i) Reagents used:

Cell Proliferation ELISA, BrdU (colorimetric) [Boehringer Mannheim (Diagnostics and Biochemicals) Ltd, UK. Cat no. 1 647 229].
DMEM, FCS, Glutamine, HBSS (all from Life Technologies Ltd., UK).
Charcoal/Dextran Stripped FBS (HyClone SH30068.02, Perbio Science UK Ltd).
BSA (Sigma, A7888).

Human recombinant IGF-1 Animal/media grade (GroPep Limited ABN 78 008 176 298, Australia. Cat No. IU 100).

### Preparation and Storage of IGF

100µg of lyophilised IGF was reconstituted in 100µl of 10mM HCl.
Add 400µl of 1mg/ml BSA in PBS
25µl aliquots @ 200µg/ml IGF-1
Stored at -20°C.

### For Assay:

10µl of stock IGF + 12.5ml growth medium to give 8X stock of 160ng/ml.

### Complete growth medium

DMEM, 10% FCS, 2mM glutamine.

### Starvation medium

DMEM, 1% charcoal/dextran stripped FCS, 2mM glutamine.

### Test Compound

Compounds are initially dissolved in DMSO to 10mM, followed by dilutions in DMEM + 1% FCS + glutamine to give a range from 100 to 0.0.45µM in 1- 0.00045% DMSO final concentration in assay well.

### c.ii) Assay protocol

### Day 1

Exponentially growing NIH3T3/IGFR cells were harvested and seeded in complete growth medium into a flat-bottomed 96 well tissue culture grade plate (Costar 3525) at 1.2x10⁴ cells per well in a volume of 100µl.

### Day 2

Growth medium was carefully removed from each well using a multi-channel pipette. Wells were carefully rinsed three times with 200µl with HBSS. 100µl of starvation medium was added to each well and the plate was re-incubated for 24 hours.

### Day 3

50µl of a 4X concentrate of test compound was added to appropriate wells. Cells were incubated for 30 minutes with compound alone before the addition of IGF. For cells treated with IGF, an appropriate volume (i.e. 25µl) of starvation medium was added to make a final volume per well up to 200µl followed by 25µl of IGF-1 at 160ng/ml (to give a final concentration of 20ng/ml). Control cells unstimulated with IGF also had an appropriate volume (i.e. 50µl) of starvation medium added to make final volume per well up to 200µl. The plate was re-incubated for 20 hours.

### Day 4

The incorporation of BrdU in the cells (after a 4h incorporation period) was assessed using the BrdU Cell Proliferation Elisa according to the manufacturer's protocol.

The compounds of the Examples were found to have an IC₅₀ in the above test of less than 50M.

### d) Mechanism of Action Assay

Inhibition of IGF-IR mediated signal transduction was determined by measuring changes in phosphorylation of IGF-IR, Akt and MAPK (ERK1 and 2) in response to IGF-I stimulation of MCF-7 cells (ATCC No. Hub-22). A measure of selectivity was provided by the effect on MAPK phosphorylation in response to EGF in the same cell line.

### d.i) Reagents used:

RPMI 1640 medium, RPMI 1640 medium without Phenol Red, FCS, Glutamine (all from Life Technologies Ltd., UK).
Charcoal/Dextran Stripped FBS (HyClone SH30068.02, Perbio Science UK Ltd).
SDS (Sigma, L4390).
2-mercaptoethanol (Sigma, M6250).
Bromophenol blue (Sigma, B5525).
Ponceau S (Sigma, P3504).
Tris base (TRIZMA^{™} base, Sigma, T1503).
Glycine (Sigma, G7403).
Methanol (Fisher Scientific UK. Cat. No. M/3950/21).
Dried milk powder (Marvel^{™}, Premier Brands UK Ltd.).
Human recombinant IGF-1 Animal/media grade (GroPep Limited ABN 78 008 176 298, Australia. Cat No. IU 100).
Human recombinant EGF (Promega Corporation, WI, USA. Cat. No. G5021).

### Complete growth medium

RPMI 1640,10% FCS, 2mM glutamine

### Starvation medium

RPMI1640 medium without Phenol Red, 1% charcoal/dextran stripped FCS, 2mM glutamine.

### Test Compound

Compounds were initially dissolved in DMSO to 10mM, followed by dilutions in RPMI 1640 medium without Phenol Red + 1 % FCS + 2mM glutamine to give a range from 100 to 0.0.45µM in 1- 0.00045% DMSO final concentration in assay well.

### Western transfer buffer

50mM Tris base, 40mM glycine, 0.04% SDS, 20% methanol.

### Laemmli buffer x2:

100mM Tris-HCl pH6.8, 20% glycerol, 4% SDS.

### Sample buffer x4:

200mM 2-mercaptoethanol, 0.2% bromophenol blue in distilled water.

### Primary Antibodies

Rabbit anti-human IGF-1Rβ (Santa Cruz Biotechnology Inc., USA, Cat. No *sc-*713) Rabbit anti-insulin/IGF-1R [pYpY^{1162/1163}] Dual Phosphospecific (BioSource International Inc, CA, USA. Cat No. 44-8041).

Mouse anti-PKBa/Akt (Transduction Laboratories, KY, USA. Cat. No. P67220) Rabbit anti-Phospho-Akt (Ser473) (Cell Signalling Technology Inc, MA, USA. Cat. No.#9271).

Rabbit anti-p44/p42 MAP kinase (Cell Signalling Technology Inc, MA, USA. Cat. No.#9102).

Rabbit anti-Phospho p44/p42 MAP kinase (Cell Signalling Technology Inc, MA, USA. Cat. No.#9101).

Mouse anti-actin clone AC-40 (Sigma-Aldrich Company Ltd, UK, A4700).

**Antibody dilutions**

| **Antibody** | **Dilution in PBST** | **Secondary antibody in PBST** |
|---|---|---|
| IGFR | 1:200 with 5% milk | Anti-rabbit with 5% milk |
| Phospho-IGFR | 1:1000 with 5% milk | Anti-rabbit with 5% milk |
| Akt | 1:1000 with 5% milk | Anti-mouse with 5% milk |
| PhosphoAkt | 1:1000 with 5% milk | Anti-rabbit with 5% milk |
| MAPK | 1:1000 with 5% milk | Anti-rabbit with 5% milk |
| Phospho-MAPK | 1:1000 with 5% milk | Anti-rabbit with 5% milk |
| Actin | 1:1000 with 5% milk | Anti-mouse with 5% milk |

### Secondary antibodies

Goat anti-rabbit, HRP linked (Cell Signalling Technology Inc, MA, USA. Cat. No.#7074).

Sheep- anti-mouse IgG HRP-conjugated (Amersham Pharmacia Biotech UK Ltd. Cat. No. NXA931).

Dilute anti-rabbit to 1:2000 in PBST + 5% milk.

Dilute anti-mouse to 1:5000 in PBST + 5% milk.

### d.ii) Assay Protocol

### Cell treatment

MCF-7 cells were plated out in a 24 well plate at 1x10⁵ cells/well in 1ml complete growth medium. The plate was incubated for 24 hours to allow the cells to settle. The medium was removed and the plate was washed gently 3 times with PBS 2ml/well. 1ml of starvation medium was added to each well and the plate was incubated for 24 hours to serum starve the cells.

Then 25µl of each compound dilution was added and the cells and compound were incubated for 30 minutes at 37°C. After 30 minutes incubation of the compound, 25µl of IGF (for 20ng/ml final concentration) or EGF (for 0.1ng/ml final concentration) was added to each well as appropriate and the cells incubated with the IGF or EGF for 5 minutes at 37°C. The medium was removed (by pipetting) and then 100µl of 2x Laemmli buffer was added. The plates were stored at 4°C until the cells were harvested. (Harvesting should occur within 2 hours following addition of Laemmli buffer to the cells.)

To harvest the cells, a pipette was used to repeatedly draw up and expel the Laemmli buffer/cell mix and transfer into a 1.5ml Eppendorf tube. The harvested cell lysates were kept at -20°C until required. The protein concentration of each lysate could be determined using the DC protein assay kit (Bio-Rad Laboratories, USA, according to manufacturer's instructions).

### Western blot technique

Cell samples were made up with 4x sample buffer, syringed with a 21 gauge needle and boiled for 5 minutes. Samples were loaded at equal volumes and a molecular weight ladder on 4-12% Bis-Tris gels (Invitrogen BV, The Netherlands) and the gels were run in an Xcell *SureLock*^{™} Mini-Cell apparatus (Invitrogen) with the solutions provided and according to the manufacturer's instructions. The gels were blotted onto Hybond C Extra^{™} membrane (Amersham Pharmacia Biotech UK Ltd.) for 1 hour at 30 volts in the Xcell *SureLock*^{™} Mini-Cell apparatus, using Western transfer buffer. The blotted membranes were stained with 0.1% Ponceau S to visualise transferred proteins and then cut into strips horizontally for multiple antibody incubations according to the molecular weight standards. Separate strips were used for detection of IGF-1R, Akt, MAPK and actin control.

The membranes were blocked for 1 hour at room temperature in PBST + 5% milk solution. The membranes were then placed into 3ml primary antibody solution in 4 well plates and the plates were incubated overnight at 4°C. The membranes were washed in 5ml PBST, 3 times for 5 minutes each wash. The HRP-conjugated secondary antibody solution was prepared and 5ml was added per membrane. The membranes were incubated for 1 hour at room temperature with agitation. The membranes were washed in 5ml PBST, 3 times for 5 minutes each wash. The ECL solution (SuperSignal ECL, Pierce, Perbio Science UK Ltd) was prepared and incubated with the membranes for 1 minute (according to manufacturer's instructions), followed by exposure to light sensitive film and development.

The compounds of the Examples were found to have an IC₅₀ in the above test of less than 20µM.

By way of example, the following Table illustrates the activity of representative compounds according to the invention. Column 2 of the Table shows IC₅₀ data from Test (c) described above for the inhibition of IGF-stimulated proliferation in murine fibroblasts (NIH3T3) over-expressing human IGF-1 receptor

| Example Number | IC₅₀ (µM) - Test (c) |
|---|---|
| 1 | 0.040 |
| 3 | 0.006 |
| 7 | 0.004 |

We have found that the compounds of the present invention possess anti-proliferative properties such as anti-cancer properties that are believed to arise from their IGF-1R tyrosine kinase inhibitory activity. Furthermore, certain of the compounds according to the present invention possess substantially better potency against the IGF-1R tyrosine kinase than against other tyrosine kinases enzymes. Such compounds possess sufficient potency against the IGF-1R tyrosine kinase that they may be used in an amount sufficient to inhibit IGF-1R tyrosine kinase whilst demonstrating little, or significantly lower, activity against other tyrosine kinases. Such compounds are likely to be useful for the effective treatment of, for example, IGF-1R driven tumours.

Accordingly, the compounds of the present invention are expected to be useful in the treatment of diseases or medical conditions mediated alone or in part by IGF-1R tyrosine kinase, i.e. the compounds may be used to produce an IGF-1R tyrosine kinase modulatory or inhibitory effect in a warm-blooded animal in need of such treatment. Thus the compounds of the present invention provide a method for the treatment of malignant cells characterised by modulation or inhibition of the IGF-1R tyrosine kinase. Particularly the compounds of the invention may be used to produce an anti-proliferative and/or pro-apoptotic and/or anti-invasive effect mediated alone or in part by the modulation or inhibition of IGF-1R tyrosine kinase. Particularly, the compounds of the present invention are expected to be useful in the prevention or treatment of those tumours that are sensitive to modulation or inhibition of IGF-1R tyrosine kinase that is involved in the signal transduction steps which drive proliferation and survival of these tumour cells. Accordingly the compounds of the present invention are expected to be useful in the treatment and/or prevention of a number of proliferative and hyperproliferative diseases/conditions, examples of which include the following cancers:
(1) carcinoma, including that of the bladder, brain, breast, colon, kidney, liver, lung, ovary, pancreas, prostate, stomach, cervix, colon, thyroid and skin;
(2) hematopoietic tumours of lymphoid lineage, including acute lymphocytic leukaemia, B-cell lymphoma and Burketts lymphoma;
(3) hematopoietic tumours of myeloid lineage, including acute and chronic myelogenous leukaemias, promyelocytic leukaemia and multiple mycloma;
(4) tumours of mesenchymal origin, including fibrosarcoma and rhabdomyosarcoma; and
(5) other tumours, including melanoma, seminoma, tetratocarcinoma, neuroblastoma and glioma.

The compounds of the invention are expected to be especially useful in the treatment of tumours of the breast, colon and prostate and in the treatment of multiple myeloma.

According to this aspect of the invention there is provided a compound of formula (I), or a pharmaceutically-acceptable salt thereof, for use as a medicament.

Thus, according to this aspect of the invention there is provided the use of a compound of formula (I), or a pharmaceutically-acceptable salt thereof, as defined hereinbefore in the manufacture of a medicament for use in the production of an anti-proliferative effect in a warm-blooded animal such as man.

A method for producing an anti-proliferative effect in a warm-blooded animal, such as man, in need of such treatment may comprise administering to said animal an effective amount of a compound of formula (I), or a pharmaceutically-acceptable salt thereof, as hereinbefore defined.

According to a further aspect of the invention there is provided a compound of formula (I), or a pharmaceutically-acceptable salt thereof, for use in the production of an anti-proliferative effect in a warm-blooded animal such as man.

According to a further aspect of the invention there is provided the use of a compound of formula (I), or a pharmaceutically-acceptable salt thereof, as defined hereinbefore in the manufacture of a medicament for use in the production of an anti-proliferative effect which effect is produced alone or in part by inhibiting IGF-1R tyrosine kinase in a warm-blooded animal such as man.

A method for producing an anti-proliferative effect which effect is produced alone or in part by inhibiting IGF-1R, tyrosine kinase in a warm-blooded animal, such as man, in need of such treatment may comprise administering to said animal an effective amount of a compound of formula (I), or a pharmaceutically-acceptable salt thereof, as hereinbefore defined.

According to a further aspect of the invention there is provided a compound of formula (I), or a pharmaceutically-acceptable salt thereof, for use in the production of an anti-proliferative effect which effect is produced alone or in part by inhibiting IGF-1R tyrosine kinase in a warm-blooded animal such as man.

According to a further aspect of the present invention there is provided the use of a compound of formula (I), or a pharmaceutically-acceptable salt thereof, as defined hereinbefore in the manufacture of a medicament for use in the treatment of a disease or medical condition (for example a cancer as mentioned herein) mediated alone or in part by IGF-IR tyrosine kinase.

A method for treating a disease or medical condition (for example a cancer as mentioned herein) mediated alone or in part by IGF-1R tyrosine kinase in a warm-blooded, animal, such as man, in need of such treatment, may comprise administering to said animal an effective amount of a compound of formula (I), or a pharmaceutically-acceptable salt thereof, as defined hereinbefore.

According to a further aspect of the invention there is provided a compound of formula (I), or a pharmaceutically-acceptable salt thereof, for use in the treatment of a disease or medical condition (for example a cancer as mentioned herein) mediated alone or in part by IGF-1R tyrosine kinase.

According to a further aspect of the invention there is provided the use of a compound of formula (I), or a pharmaceutically-acceptable salt thereof, as defined hereinbefore in the manufacture of a medicament for use in the prevention or treatment of those tumours which are sensitive to inhibition of IGF-1R tyrosine kinase involved in the signal transduction steps which lead to the proliferation of tumour cells.

A method for the prevention or treatment of those tumours which are sensitive to inhibition of IGF-1R tyrosine kinase, involved in the signal transduction steps which lead to the proliferation and/or survival of tumour cells in a warm-blooded animal, such as man, in need of such treatment, may comprise administering to said animal an effective amount of a compound of formula (I), or a pharmaceutically-acceptable salt thereof, as defined hereinbefore.

According to a further aspect of the invention there is provided a compound of formula (I), or a pharmaceutically-acceptable salt thereof, for use in the prevention or treatment of those tumours which are sensitive to inhibition of IGF-1R tyrosine kinase, involved in the signal transduction steps which lead to the proliferation and/or survival of tumour cells.

According to a further aspect of the invention there is provided the use of a compound of formula (I), or a pharmaceutically-acceptable salt thereof, as defined hereinbefore in the manufacture of a medicament for use in providing an IGF-1R tyrosine kinase inhibitory effect.

A method for providing an IGF-1R tyrosine kinase inhibitory effect in a warm-blooded animal, such as man, in need of such treatment, may comprise administering to said animal an effective amount of a compound of formula (I), or a pharmaceutically-acceptable salt thereof, as defined hereinbefore.

According to a further aspect of the invention there is provided a compound of formula (I), or a phannaceutically-acceptable salt thereof, for use in providing an IGF-1R tyrosine kinase inhibitory effect.

According to a further aspect of the present invention there is provided the use of a compound of formula (I), or a pharmaceutically-acceptable salt thereof, as defined hereinbefore in the manufacture of a medicament for use in the treatment of a cancer, for example a cancer selected from leukaemia, multiple myeloma lymphoma, bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval cancer.

A method for treating a cancer, for example a cancer selected from selected from leukaemia, multiple myeloma, lymphoma, bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval cancer in a warm-blooded animal, such as man, in need of such treatment, may comprise administering to said animal an effective amount of a compound of formula (I), or a pharmaceutically-acceptable salt thereof, as defined hereinbefore.

According to a further aspect of the invention there is provided a compound of formula (I), or a pharmaceutically-acceptable salt thereof, for use in the treatment of a cancer, for example a cancer selected from leukaemia, multiple myeloma, lymphoma, bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval cancer.

As mentioned above the size of the dose required for the therapeutic or prophlyactic treatment of a particular disease will necessarily be varied depending upon, amongst other things, the host treated, the route of administration and the severity of the illness being treated.

The compounds of the invention may be administered in the form of a pro-drug, by which we mean a compound that is broken down in a warm-blooded animal, such as man, to release a compound of the invention. A pro-drug may be used to alter the physical properties and/or the pharmacokinetic properties of a compound of the invention. A pro-drug can be formed when the compound of the invention contains a suitable group or substituent to which a property-modifying group can be attached. Examples of pro-drugs include in vivo cleavable ester derivatives that may be formed at a carboxylic acid or a hydroxy group in a compound of formula (I).

Accordingly, the present invention includes those compounds of formula (I) as defined hereinbefore when made available by organic synthesis and when made available within the human or animal body by way of cleavage of a pro-drug thereof. Accordingly, the present invention includes those compounds of formula (I) that are produced by organic synthetic means and also such compounds that are produced in the human or animal body by way of metabolism of a precursor compound, that is a compound of formula (I) may be a synthetically-produced compound or a metabolically-produced compound.

A suitable pharmaceutically-acceptable pro-drug of a compound of formula (I) is one that is based on reasonable medical judgement as being suitable for administration to the human or animal body without undesirable pharmacological activities and without undue toxicity.

Various forms of pro-drug have been described, for example in the following documents:
a) Methods in Enzymology, Vol.42 p. 309 to 396, edited by K. Widder, et al. (Academic Press, 1985);
b) Design of Pro-drugs, edited by H. Bundgaard, (Elsevier, 1985);
c) A Textbook of Drug Design and Development, edited by Krogsgaard-Larsen and H. Bundgaard, Chapter 5 "Design and Application of Pro-drugs", edited by H. Bundgaard, p. 113 to 191 1991);
d) H. Bundgaard, Advanced Drug Delivery Review 8, 1 to 38 (1992); and
e) H. Bundgaard, et al., Journal of Pharmaceutical Sciences, 77, 285 (1988).

The compounds of formula (I), and pharmaceutically-acceptable salts thereof, may be used on their own but will generally be administered in the form of a pharmaceutical composition in which the formula (I) compound/salt (active ingredient) is in association with a pharmaceutically-acceptable adjuvant, diluent or carrier.

Thus, the present invention also provides a pharmaceutical composition comprising a compound of formula (I), or a pharmaceutically-acceptable salt thereof, as hereinbefore defined, in association with a pharmaceutically-acceptable adjuvant, diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

The invention further provides a process for the preparation of a pharmaceutical composition of the invention which comprises mixing a compound of formula (I), or a pharmaceutically-acceptable salt thereof, as hereinbefore defined, with a pharmaceutically-acceptable adjuvant, diluent or carrier.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a compound of formula (I) will naturally vary according to the nature and severity of the conditions, the age and sex of the animal or patient and the route of administration, according to well known principles of medicine.

In using a compound of formula (I) for therapeutic or prophylactic purposes it will generally be administered so that a daily dose in the range, for example, 0.1 mg/kg to 75 mg/kg body weight is received, given if required in divided doses. In general lower doses will be administered when a parenteral route is employed. Thus, for example, for intravenous administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration is however preferred, particularly in tablet form. Typically, unit dosage forms will contain about 0.5 mg to 0.5 g of a compound of this invention.

The anti-proliferative treatment defined hereinbefore may be applied as a sole therapy or may involve, in addition to the compounds of the invention, conventional surgery or radiotherapy or chemotherapy. Such chemotherapy may include one or more of the following categories of anti-tumour agents:-
(i) other antiproliferative/antineoplastic drugs and combinations thereof, as used in medical oncology, such as alkylating agents (for example cis-platin, oxaliplatin, carboplatin, cyclophosphamide, nitrogen mustard, melphalan, chlorambucil, busulphan, temozolamide and nitrosoureas); antimetabolites (for example gemcitabine and antifolates such as fluoropyrimidines like 5-fluorouracil and tegafur, raltitrexed, methotrexate, cytosine arabinoside, and hydroxyurea); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere and polokinase inhibitors); and topoisomerase inhibitors (for example epipodophyllotoxins like etoposide and teniposide, amsacrine, topotecan and camptothecin);
(ii) cytostatic agents such as antioestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) anti-invasion agents (for example c-Src kinase family inhibitors like 4-(6-chloro-2,3-methylenedioxyanilino)-7-[2-(4-methylpiperazin-1-yl)ethoxy]-5-tetrahydropyran-4-yloxyquinazoline (AZD0530; International Patent Application WO 01/94341) and N-(2-chloro-6-methylphenyl)-2-{6-[4-(2-hydroxyethyl)piperazin-1-yl]-2-methlpyrium-4-ylamino}thiazole-5-carboxamide(dasatinib,BMS-354825; J. Med. Chem, 2004, 47, 6658-6661), and metalloproteinase inhibitors like marimastat, inhibitors of urokinase plasminogen activator receptor function or antibodies to Heparanase);
(iv) inhibitors of growth factor function: for example such inhibitors include growth factor antibodies and growth factor receptor antibodies (for example the anti-erbB2 antibody trastuzumab [Herceptin^{™}] and the anti-erbB1 antibody cetuximab [Erbitux, C225]); such inhibitors also include tyrosine kinase inhibitors, for example inhibitors of the epidermal growth factor family (for example EGFR family tyrosine kinase inhibitors such as *N*-(3-chloro-4-fluorophenyl)-7-methoxy-6-(3-morpholinopropoxy)quinazolin-4-amine (gefitinib, ZD1839), N-(3-ethynylphenyl)-6,7-bis(2-methoxyethoxy)quinazolin-4-amine (erlotinib, OSI-774) and 6-acrylamido-*N*-(3-chloro-4-fluorophenyl)-7-(3-morpholinopropoxy)-quinazolin-4-amine (CI 1033), erbB2 tyrosine kinase inhibitors such as lapatinib, inhibitors of the hepatocyte growth factor family, inhibitors of the platelet-derived growth factor family such as imatinib, inhibitors of serine/threonine kinases (for example . Ras/Raf signalling inhibitors such as farnesyl transferase inhibitors, for example sorafenib (BAY 43-9006)), inhibitors of cell signalling through MEK and/or AKT kinases, inhibitors of the hepatocyte growth factor family, c-kit inhibitors, abl kinase inhibitors, IGF receptor (insulin-like growth factor) kinase inhibitors; aurora kinase inhibitors (for example AZD1152, PH739358, VX-680, MLN8054, R763, MP235, MP529, VX-528 AND AX39459) and cyclin dependent kinase inhibitors such as CDK2 and/or CDK4 inhibitors;
(v) antiangiogenic agents such as those which inhibit the effects of vascular endothelial growth factor, [for example the anti-vascular endothelial cell growth factor antibody bevacizumab (Avastin^{™}) and VEGF receptor tyrosine kinase inhibitors such as 4-(4-bromo-2-fluoroanilino)-6-methoxy-7-(1-methylpiperidin-4-ylmethoxy)quinazoline (ZD6474; Example 2 within WO 01/32651), 4-(4-fluoro-2-methylindol-5-yloxy)-6-methoxy-7-(3-pyrrolidin-1-ylpropoxy)quinazoline (AZD2171; Example 240 within WO 00/47212), vatalariib (PTK787; WO 98/35985) and SU11248 (sunitinib; WO 01/60814), compounds such as those disclosed in International Patent Applications WO97/22596, WO 97/30035, WO 97/32856 and WO 98/13354 and compounds that work by other mechanisms (for example linomide, inhibitors of integrin αvβ3 function and angiostatin)];
(vi) vascular damaging agents such as Combretastatin A4 and compounds disclosed in International Patent Applications WO 99/02166, WO 00/40529, WO 00/41669, WO 01/92224, WO 02/04434 and WO 02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

According to this aspect of the invention there is provided a pharmaceutical product comprising a compound of formula (I), or a pharmaceutically-acceptable salt thereof, as defined hereinbefore and an additional anti-tumour agent as defined hereinbefore for the conjoint treatment of cancer.

Although the compounds of formula (I) are primarily of value as therapeutic agents for use in warm-blooded animals (including man), they are also useful whenever it is required to inhibit the effects of IGF-1R tyrosine kinases. Thus, they are useful as pharmacological standards for use in the development of new biological tests and in the search for new pharmacological agents.

### Examples

The invention will now be further described with reference to the following illustrative examples: in which, unless stated otherwise:
(i) temperatures are given in degrees Celsius (°C); operations were carried out at room or ambient temperature, that is, at a temperature in the range of 18 to 25°C;
(ii) organic solutions were dried over anhydrous magnesium sulfate; evaporation of solvent was carried out using a rotary evaporator under reduced pressure (600-4000 Pascals; 4.5-30mmHg) with a bath temperature of up to 60°C;
(iii) chromatography means flash chromatography on silica gel; thin layer chromatography (TLC) was carried out on silica gel plates;
(iv) in general, the course of reactions was followed by TLC and reaction times are given for illustration only;
(v) final products had satisfactory proton nuclear magnetic resonance (NMR) spectra and/or mass spectral data;
(vi) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 300 MHz, in DMSO-d₆ unless otherwise indicated. The following abbreviations have been used: s, singlet; d, doublet; t, triplet; q, quartet; m, multiplet; br, broad. Where NMR spectra are broad (due to hindered rotation or slow proton exchange), NMR spectra were run at 100°C;
(viii) chemical symbols have their usual meanings; SI units and symbols are used;
(ix) solvent ratios are given in volume:volume (v/v) terms; and
(x) mass spectra were run with an electron energy of 70 electron volts in the chemical ionization (CI) mode using a direct exposure probe; where indicated ionization was effected by electron impact (EI), fast atom bombardment (FAB) or electrospray (ESP); values for m/z are given; generally, only ions which indicate the parent mass are reported; and unless otherwise stated, the mass ion quoted is (MH)⁺;
(xi) the following abbreviations have been used:
   - THF: tetrahydrofuran;
   - EtOAc: ethyl acetate;
   - DCM: dichloromethane;
   - DMSO: dimethylsulfoxide;
   - DIPEA: diisopropylethylamine;
   - NMP: N-methylpyrrolid-2-one;
   - tBuOH: tert-butyl alcohol;
   - TFA: trifluoroacetic acid;
   - DMF: N,N-dimethylformamide; and
   - DMA: N,N-dimethylacetamide.

### Example 1

### S-2-[2-{3-(3-Ethoxypyrazin-2-yl)isoxazol-5-yl}pyrrolidin-1-yl]-6-methyl-4-(5-methyl-1H-pyrazol-3-ylamino)pyrimidine

A mixture of 2-chloro-6-methyl-4-(5-methyl-1*H*-pyrazol-3-ylamino)pyrimidine (66mg, 0.29mmol), S-2-[3-(3-ethoxypyrazin-2-yl)isoxazol-5-yl]pyrrolidine (92mg, 0.35mmol) and N,N-diisopropylethylamine (101µl, 0.58mmol) in dioxane (4ml) was heated at 150°C for one hour under microwave irradiation. The mixture was allowed to cool, the volatiles removed by evaporation and the residue purified by column chromatography on silica gel eluting with 2M methanolic ammonia / DCM (0:100 increasing in polarity to 10:90). The purified product was triturated with diethyl ether to give the title compound (20mg, 15%) as a cream solid; NMR Spectrum (DMSO-*d₆* + *d₄*-acetic acid at 100°C) 1.30 (t, 3H), 2.05 (m, 2H), 2.12 (s, 3H), 2.15 (s, 3H), 2.30-2.40 (m, 2H), 2.65-2.80 (m, 2H), 4.45 (q, 2H), 5.45 (dd, 1H), 6.00 (s, 1H), 6.18 (s, 1H), 6.62 (s, 1H), 8.25 (m, 2H); Mass Spectrum 448 [MH]+.

The 2-chloro-6-methyl-4-(5-methyl-1H-pyrazol-3-ylamino)pyrimidine starting material was prepared as follows:

Solid sodium carbonate (1.2g, 11.3mmol) was added to a solution of 2,4-dichloro-6-methylpyrimidine (1.7g, 10.3mmol) and 5-amino-3-methyl-1*H*-pyrazole (1.0g, 10.3mmol) in dry ethanol (50ml) and the mixture heated and stirred at 42°C for 3 days. The mixture was allowed to cool, the insoluble material was removed by filtration and the filter pad washed with ethanol (10ml). The volatiles were removed from the filtrate by evaporation, keeping the bath temperature below 40°C. The residue was immediately purified by chromatography on silica gel eluting with methanol / DCM (5:95 increasing in polarity to 20:80) to give 2-chloro-6-methyl-4-(5-methyl-1*H-*pyrazol-3-ylamino)pyrimidine (758mg, 33%) as a white solid;
NMR Spectrum (CDCl₃) 2.17 (s, 3H), 2.11 (s, 3H), 5.88 (br s, 1H), 7.85 (br s, 1H), 8.80 (br s, 1H); Mass Spectrum 224 [MH]+.

The S-2-[3-(3-ethoxypyrazin-2-yl)isoxazol-5-yl]pyrrolidine starting material was prepared as follows:

2,2,6,6-Tetramethylpiperidine (19.7ml, 117mmol) was added to a solution of n-butyl lithium (69.3ml of a 1.6M solution in hexane, 111mmol) in anhydrous THF (200ml) at -76°C under an atmosphere of nitrogen, keeping the reaction temperature below -70°C. The reaction mixture was stirred at -70°C for 15 minutes then allowed to warm to 0°C and stirred for a further 30 minutes before being cooled to -76°C. 2-Chloropyrazine (10g, 87.3mmol) was added dropwise such that the reaction temperature was kept below -70°C. The reaction mixture was then stirred at -70°C for 30 minutes. Ethyl formate (7.5ml, 98mmol) was then .. added such that the reaction temperature was kept below -70°C. The reaction mixture was then stirred at -70°C for 1.5 hours. Glacial acetic acid (13ml, 218mmol) was added at -70°C and the mixture then allowed to warm to ambient temperature and the volatiles were removed by evaporation. The residue dissolved in ethanol (100ml) and hydroxylamine (6.83g, 105mmol) and triethylamine (24.2ml, 175mmol) were added. The mixture was heated at 50°C for 18 hours and the volatiles were then removed by evaporation. The residue was dissolved in diethylether and any remaining insoluble material was removed by filtration. The filtrate was washed with water, the solvent removed from the organic layer by evaporation and the residue purified by chromatography on silica gel eluting with DCM, then with diethylether / DCM (1:4) and finally with EtOAc to give 3-chloropyrazine-2-carboxaldehyde oxime (5.21g, 37.9%) as a solid; NMR Spectrum 8.37 (s, 1H), 8.50 (d, 1H), 8.70 (d, 1H), 12.25 (s, 1H).

A mixture of 3-chloropyrazine-2-carboxaldehyde oxime (4g, 25.4mmol) and S-N-tert-butoxycarbonyl-2-ethynylpyrrolidine (prepared as described in Bull. Soc. Chim. Fr. 1997, 134, 141-144 and J. Med. Chem. 1994, 37, 4455-4463) (5.94g, 30.48mmol) in DCM (200ml) was stirred at 0°C and sodium hypochlorite (28.9ml of a 13% aqueous solution, 50.8mmol) was added dropwise over one hour, the mixture was then allowed to warm to ambient temperature. The organic phase was separated and dried (MgSO₄) and the solvent removed by evaporation. The residue was purified by chromatography on silica gel eluting with hexanes / EtOAc (100:0 increasing in polarity to 50:50). The partially purified product was dissolved in DCM (50ml) and TFA (20ml) added. The mixture stirred at ambient temperature for 1 hour. Water (100ml) was added and the mixture was washed with DCM (2 x 50ml).
The aqueous portion was basified to pH9 by addition of a saturated aqueous solution of sodium hydrogen carbonate and the aqueous mixture extracted with DCM (4 x 100ml). The organic extracts were combined, dried (MgSO₄) and the solvent removed by evaporation to give S-2-[3-(3-chloropyrazin-2-yl)isoxazol-5-yl]pyrrolidine (1.83g, 29%) as a brown oil; NMR Spectrum (CDCl₃) 2.00-2.20 (m, 3H), 2.40 (m, 1H), 3.20 (m, 2H), 4.60 (dd, 1H), 6.95 (s, 1H), 8.55 (d, 1H), 8.85 (d, 1H); Mass Spectrum 251 [MH]+.

Sodium hydrogen carbonate (1.22g, 14.6mmol) was added to a solution of S-2-[3-(3-chloropyrazin-2-yl)isoxazol-5-yl]pyrrolidine (1.83g, 7.3mmol) and di-tert-butyl dicarbonate (1.91g, 8.7mmol) in DCM (150ml) and the mixture stirred at ambient temperature for 18 hours. The mixture was washed with water (100ml), the organic phase separated, dried (MgSO₄) and the volatiles removed by evaporation. The residue was purified by chromatography on silica gel eluting with hexanes / EtOAc (100:0 increasing in polarity to 0:100) to give S-N-tert-butoxycarbonyl-2-[3-(3-chloropyrazin-2-yl)isoxazol-5-yl]pyrrolidine (1.08g, 42%) as a clear oil; NMR Spectrum (CDCl₃) 1.30-1.50 (m, 9H), 2.00 (m, 2H), 2.20 (m, 1H), 2.30 (m, 1H), 3.40-3.70 (m, 2H), 5.10 (m, 1H), 6.70 (m, 1H), 8.45 (br s, 1H), (8.60 (br s, 1H); Mass Spectrum 295 [MH-C₄H₉]+.

S-N-tert-Butoxycarbonyl-2-[3-(3-chloropyrazin-2-yl)isoxazol-5-yl]pyrrolidine (150mg, 0.42mmol) was added to sodium ethoxide (4ml of a 21% solution in ethanol) and the mixture heated to 70°C for 30 minutes under microwave irradiation. The volatiles were removed by evaporation, water (50ml) was added to the residue and the mixture extracted with EtOAc (3 x 50ml). The organic extracts were combined, dried (MgSO₄) and the solvent removed by evaporation. The residue was dissolved in DCM (10ml) and TFA (5ml) was added. The mixture was stirred at ambient temperature for one hour. The mixture was concentrated by evaporation and passed down an SCX-2 column eluting with methanol to elute neutral impurities and then with 2M methanolic ammonia to elute the product. The solvent was removed by evaporation to give S-2-[3-(3-ethoxypyrazin-2-yl)isoxazol-5-yl]pyrrolidine (92mg, 84%) as a brown oil; NMR Spectrum (CDCl₃) 1.55 (t, 3H), 2.00-2.25 (m, 3H), 2.45 (m, 1H), 3.30 (m, 2H), 4.60 (q, 2H), 4.70 (t, 1H), 6.98 (s, 1H), 8.25 (d, 1H), 8.35 (d, 1H); Mass Spectrum 261 [MH]+.

### Example 2

### S-2-[2-{3-(3-Ethylaminopyrazin-2-yl)isoxazol-5-yl}pyrrolidin-1-yl]-6-methyl-4-(5-methyl-1H-pyrazol-3-ylamino)pyrimidine

A mixture of 2-chloro-6-methyl-4-(5-methyl-1*H*-pyrazol-3-ylamino)pyrimidine (66mg, 0.28mmol), S-2-[3-(3-ethylaminopyrazin-2-yl)isoxazol-5-yl]pyrrolidine (88mg, 0.34mmol) and N,N-diisopropylethylamine (0.097ml, 0.56mmol) in dioxane (4ml) was heated at 70°C under microwave irradiation for one hour. The volatiles were removed by evaporation and the residue purified by chromatography on silica gel eluting with 2M methanolic ammonia / DCM (0:100 increasing in polarity to 10:90) the purified material was then re-purified by reverse phase HPLC using a C 18 column eluting with water / acetonitrile / TFA (95:5:0.2 decreasing in polarity to 0:100:0.2). The fractions containing product were combined and passed through an isolute SCX-2 ion exchange column. The column was eluted with methanol to elute any neutrals, followed by 2M methanolic ammonia to elute the product. The solvent was removed by evaporation to give the title compound (12mg, 10%) as a cream solid; NMR Spectrum (DMSO-*d₆* + *d₄*-acetic acid at 100°C) 1.30 (t, 3H), 2.00-2.15 (m, 3H), 2.20 (s, 3H), 2.25 (s, 3H), 2.40 (m, 1H), 3.55 (q, 2H), 3.70 (m, 1H), 3.85 (m, 1H), 5.50 (m, 1H), 6.80 (s, 1H), 7.10 (br s, 1H), 7.45 (br s, 1H), 7.55 (br s, 1H), 7.85 (d, 1H), 8.10 (d, 1H); Mass Spectrum 447 [MH]+.

The S-2-[3-(3-ethylaminopyrazin-2-yl)isoxazol-5-yl]pyrrolidine starting material was prepared as follows:

S-N-tert-Butoxycarbonyl-2-[3-(3-chloropyrazin-2-yl)isoxazol-5-yl]pyrrolidine (150mg, 0.42mmol) was added to ethylamine (4ml of a 2M solution in THF) and the mixture heated at 70°C for 90 minutes under microwave irradiation. The volatiles were removed by evaporation, the residue dissolved in DCM (10ml) and TFA (3ml) added and the mixture stirred at ambient temperature for 2 hours. The mixture was concentrated by evaporation and passed down a SCX-2 column eluting with methanol to remove neutral impurities and then with 2M methanolic ammonia to elute the product. The solvent was removed by evaporation to give S-2-[3-(3-ethylaminopyrazin-2-yl)isoxazol-5-yl]pyrrolidine (88mg, 80%) as a brown oil; NMR Spectrum (CDCl₃) 1.40 (t, 3H), 2.00-2.20 (m, 3H), 2.45 (m, 1H), 3.20-3.40 (m, 2H), 3.70 (m, 2H), 4.65 (dd, 1H), 7.05 (s, 1H), 7.50 (br s, 1H), 7.95 (d, 1H), 8.20 (d, 1H); Mass Spectrum 260 [MH]+.

### Example 3

### S-2-[2-{3-(2-Methylpyrid-3-yl)isoxazol-5-yl}pyrrolidin-1-yl]-6-methyl-4-(5-methyl-1H-pyrazol-3-ylamino)pyrimidine

A mixture of 2-chloro-6-methyl-4-(5-methyl-1H-pyrazol-3-ylamino)pyrimidine (165mg, 0.74mmol), S-2-[3-(2-methylpyrid-3-yl)isoxazol-5-yl]pyrrolidine (256mg, 1.1mmol), N,N-diisopropylamine (0.345ml, 2.0mmol) in hexanol (10ml) was heated at 130°C for 18 hours. The mixture was allowed to cool and volatiles removed by evaporation. The residue was purified by chromatography on silica gel eluting with EtOAc / methanol (100:0 increasing polarity 92:8). The purified product was triturated with ether / hexanes and collected by filtration to give the title compound (122mg, 31%); NMR Spectrum 2.03-2.1 (m, 3H), 2.12 (s, 3H), 2.19 (s, 3H), 2.32-2.43 (m, 1H), 2.59 (s, 3H), 3.68-3.78 (m, 2H), 5.47 (d, 1H), 6.04 (s, 1H), 6.18 (s, 1H), 6.55 (s, 1H), 7.28 (dd, 1H), 7.85 (s, 1H), 8.50 (d, 1H), 8.72 (s, 1H), 11.50 (s, 1H); Mass Spectrum 417 [MH]+.

The S-2-[3-(2-methylpyrid-3-yl)isoxazol-5-yl]pyrrolidine starting material was prepared as follows:-

A mixture of the 3-hydroxymethyl-2-methylpyridine (9.0g, 73.1mmol) and manganese (IV) dioxide (28.1g, 322mmol) in DCM (100ml) was heated at reflux for two days. The insolubles were removed by filtration through diatomaceous earth and the filter pad was washed with methanol /DCM. The solvent was removed from the filtrate by evaporation to give 2-methylpyridine-3-carboxaldehyde (7.5g, 85%) as an oil; NMR Spectrum 2.78 (s, 3H), 7.43 (dd, 1H), 8.15 (dd, 1H), 8.66 (dd, 1H).

A solution of hydroxylamine hydrochloride (3.16g, 45.1mmol) in water (15ml) was added to a cooled solution of sodium hydroxide (2.46g, 61.5mmol) in water (15ml). The resulting aqueous solution was added to a mixture of 2-methylpyridine-3-carboxaldehyde (5.0g, 40.9mmol), water (44ml), ethanol (44ml) and ice (70g). The mixture was stirred at ambient temperature for 18 hours and adjusted to pH=7 by the addition of 2M hydrochloric acid. The resulting precipitate was collected by filtration washed with water and dried to give 2-methylpyridine-3-carboxaldehyde oxime (3.5g, 62%); NMR Spectrum 2.55 (s, 3H), 7.22 (dd, 1H), 7.94 (dd, 1H), 8.39 (s, 1H), 8.42 (dd, 1H).

A stirred suspension of 2-methylpyridine-3-carboxaldehyde oxime (3.23g, 0.24mol) and S-N-tert-butoxycarbonyl-2-ethynylpyrrolidine (prepared as described in Bull. Soc. Chim. Fr. 1997,134,141-144 and J. Med. Chem. 1994, 37, 4455-4463) (9.26g, 0.47mol) in DCM (150ml) was cooled in an ice bath. Sodium hypochlorite (27.13ml of a 13% aqueous solution) was added slowly, keeping the temperature below 10°C. The mixture was then allowed to warm to ambient temperature and stirred for 18 hours. The organic layer was separated, dried (MgSO₄₎ and the solvent removed by evaporation. The residue was purified by chromatography on silica gel eluting with 15% EtOAc / hexane (15:85 increasing in polarity to 100:0) to give S-N-tert-butoxycarbonyl-2-[3-(2-methylpyrid-3-yl)isoxazol-5-yl]pyrrolidine (5.09g, 65%); NMR_Spectrum 1.42-1.23 (m, 9H), 1.92-2.0 (m, 3H), 2.26-2.38 (m, 1H), 2.62 (s, 3H), 3.32-3.42 (m, 1H), 3.42-3.55(m, 1H), 4.95-5.05 (m, 1H), 6.75 (s, 1H), 7.35 (dd, 1H), 7.90 (d, 1H), 8.55 (dd, 1H); Mass Spectrum 330 [MH]+.

2M Hydrochloric acid (140ml) was added to a solution of the S-N-tert-butoxycarbonyl-2-[3-(2-methylpyrid-3-yl)isoxazol-5-yl]pyrrolidine (4.45g, 13.5mmol) in methanol (140ml) and the mixture stirred at ambient temperature for 18 hours and then heated at reflux for 8 hours. The mixture was concentrated by evaporation and adjusted to pH=10-11 by the addition of 40% aqueous sodium hydroxide solution. The mixture was extracted with DCM, dried (MgSO₄) and the volatiles removed by evaporation. The residue was purified by chromatography on silica gel eluting with methanol / DCM (3:97 increasing in polarity to 5:95) to give S-2-[3-(2-methylpyrid-3-yl)isoxazol-5-yl]pyrrolidine (1.2g, 40%); NMR Spectrum 1.70-1.85 (m, 3H), 2.09-2.18 (m, 1H), 2.60 (s, 3H), 2.88-2.92 (m, 2H), 4.37 (dd, 1H), 2.70 (s, 1H), 7.34 (dd, 1H), 7.90 (dd, 1H), 8.55 (dd, 1H).

### Example 4

### S-6-Chloro-2-[2-{3-(2-methylpyrid-3-yl)isoxazol-5-yl}pyrrolidin-1-yl]-4-(5-methyl-1H-pyrazol-3-ylamino)pyrimidine

A mixture of 2,6-dichloro-4-(5-methyl-1*H*-pyrazol-3-ylamino)pyrimidine (947mg, 3.9mmol), S-2-[3-(2-methylpyrid-3-yl)isoxazol-5-yl]pyrrolidine (983mg, 4.28mmol) and N,N-diisopropylethylamine (1.64ml, 9.5mmol) in xylene (20ml) was heated at 80°C for 18 hours. The mixture was allowed to cool and the volatiles removed by evaporation. The residue was triturated with EtOAc / methanol / water, the product collected by filtration to give the title compound (170mg, 20%); NMR Spectrum 2.04-2.15 (m, 3H), 2.19 (s, 3H), 2.34-2.42 (m, 1H), 2.58 (s, 3H), 3.64-3.71 (m, 1H), 3.70-3.78 (m, 1H), 5.44 (d, 1H), 6.01 (s, 1H), 6.38 (s, 1H), 6.60 (s, 1H), 7.29 (dd, 1H), 7.86 (d, 1H), 8.52 (d, 1H), 9.24 (s, 1H), 11.62 (s, 1H); Mass Spectrum 437[MH]+.

The 2,6-dichloro-4-(5-methyl-1*H*-pyrazol-3-ylamino)pyrimidine starting material was prepared as follows:

A mixture of 2,4,6-trichloropyrimidine (1.0g, 5.4mmol), 3-amino-5-methyl-1*H* pyrrazole (0.53g, 5.4mmol), and sodium carbonate (0.57g, 5.4mmol) in ethanol (25ml) was stirred at ambient temperature for 18 hours. Water was added and the resulting precipitate was collected by filtration washed with water and a small amount of methanol, and dried to give 2,6-dichloro-4-(5-methyl-1*H*-pyrazol-3-ylamino)pyrimidine (1.15g, 88%) as a colourless crystalline solid; NMR Spectrum 2.23 (s, 3H), 6.01 (s, 1H), 7.24 (s, 1H), 10.25 (br s, 1H), 11.9 (br s, 1H); Mass Spectrum 244 [MH]+.

### Example 5

### S-2-[2-{3-(2-Chloropyrid-3-yl)isoxazol-5-yl}pyrrolidin-1-yl]-6-methyl-4-(5-methyl-1H-pyrazol-3-ylamino)pyrimidine

A mixture of 2-chloro-6-methyl-4-(5-methyl-1*H*-pyrazol-3-ylamino)pyrimidine (400mg, 1.78mol), S-2-[3-(2-chloropyrid-3-yl)isoxazol-5-yl]pyrrolidine (630mg, 2.5mol) and N,N-diisopropylethylamine (0.69ml, 4.0mmol) in xylene (10ml) was heated at 130°C under nitrogen, for 18 hours. The mixture was allowed to cool and the volatiles removed by evaporation. The residue was dissolved in ethyl acetate, washed with water, dried (MgSO₄) and the solvent removed by evaporation. The residue was purified by chromatography on silica gel eluting with EtOAc to give the title compound (286mg, 37%); NMR Spectrum 2.05-2.1 (m, 3H), 2.15 (s, 3H), 2.19 (s, 3H), 2.32-2.41 (m, 1H), 3.68-3.78 (m, 2H), 5.48 (d, 1H), 6.05 (s, 1H), 6.20 (s, 1H), 6.66 (s,1H), 7.52 (dd, 1H), 8.09 (d, 1H), 8.50 (d, 1H), 8.72 (s, 1H), 11.52 (s,1H); Mass Spectrum 437 [MH]+.

The S-2-[3-(2-chloropyrid-3-yl)isoxazol-5-yl]pyrrolidine starting material was prepared as follows:

A solution of hydroxylamine hydrochloride (533mg, 7.6mmol) in water (1.8ml) was added dropwise to sodium hydroxide (708mg, 17mmol) in water (2ml). The resulting solution was then added to a solution of 2-chloropyrid-3-ylcarboxaldehyde (1g, 7mmol) in ethanol (7ml), water (7ml) and ice (15g). The mixture was stirred at ambient temperature for 18 hours. The mixture was neutralised to pH7 with 6M hydrochloric acid. The solid product was collected by filtration, washed with water and dried to give 2-chloropyrid-3-ylcarboxaldehyde oxime (800mg, 73%); NMR Spectrum 7.45 (dd, 1H), 8.18 (dd, 1H), 8.32 (s, 1H), 8.42 (dd, 1H); Mass Spectrum 157 [MH]+.

Sodium hypochlorite (5.3ml of a 13% aqueous solution) was added dropwise to a vigorously stirred suspension of 2-chloropyrid-3-ylcarboxaldehyde oxime (800mg, 5.1mmol) and S-N-tert-butoxycarbonyl-2-ethynylpyrrolidine (prepared as described in Bull. Soc. Chim. Fr. 1997, 134, 141-144 and J. Med. Chem. 1994, 37, 4455-4463) (1.99g, 10.2mmol) in DCM (20ml) at about 0 to 5°C. The mixture was allowed to warm and stirred at ambient temperature for 18 hours. The volatiles were removed by evaporation and the residue purified by chromatography on silica gel eluting with EtOAc / hexane (20:80) to give S-N-(tert-butoxycarbonyl)-2-[3-(2-chloropyrid-3-yl)isoxazol-5-yl]pyrrolidine (955mg, 54%); NMR Spectrum 1.22-1.42 (m, 9H), 1.95-2.0 (m, 3H), 2.22-2.38 (m, 1H), 3.30-3.40 (m, 1H), 3.43-3.55 (m, 1H), 5.0 (s, 1H), 6.78 (s, 1H), 7.58 (s, 1H), 8.12 (d, 1H), 8.55 (dd, 1H); Mass Spectrum 350 [MH]+.

TFA (4ml) was added to a solution of S-N-(tert-butoxycarbonyl)-2-[3-(2-chloropyrid-3-yl)isoxazol-5-yl]pyrrolidine (1.5g, 4.3mmol) in DCM (10ml) and the mixture stirred for 3 days at ambient temperature. The volatiles were removed by evaporation and the residue dissolved in water. The aqueous mixture was adjusted to pH10-11 with 40% aqueous sodium hydroxide solution and extracted with DCM. The extracts were combined, dried (Na₂SO₄) and the solvent removed by evaporation to give S-2-[3-(2-chloropyrid-3-yl)isoxazol-5-yl]pyrrolidine (930mg, 87%); Mass Spectrum 350 [MH]+.

### Example 6

### S-6-Methoxy-2-[2-{3-(2-methylpyrid-3-yl)isoxazol-5-yl}pyrrolidin-1-yl]-4-(5-methyl-1H-pyrazol-3-ylamino)pyrimidine

Sodium methoxide (1.4g of a 25% solution in methanol, 6.4mmol) was added to a mixture of S-6-chloro-2-[2-{3-(2-methylpyrid-3-yl)isoxazol-5-yl}pyrrolidin-1-yl]-4-(5-methyl-1*H*-pyrazol-3-ylamino)pyrimidine (140mg, 3.2mmol) in methanol (3ml) and the mixture was heated at 130°C under microwave irradiation for 105 minutes. The mixture was allowed to cool and the solvent removed by evaporation the residue was partitioned between EtOAc and water. The organic layer was separated dried (MgSO₄) and the solvent removed by evaporation. The residue was purified by reverse phase HPLC using a C18 column eluting with water / acetonitrile / TFA (95:5:0.2 decreasing in polarity to 0:100:0.2). The fractions containing product were combined, concentrated by evaporation and basified with saturated aqueous sodium hydrogen carbonate solution. The resulting precipitated product was collected by filtration, washed with water and dried under vacuum to give the title compound (70mg, 50%); NMR Spectrum 2.04-2.18(m, 3H), 2.18 (s, 3H), 2.37-2.44 (m, 1H), 2.60 (s, 3H), 3.70-3.78 (m, 5H), 5.43 (d, 1H), 5.76 (s, 1H), 5.96 (s, 1H), 6.55 (s, 1H), 7.28 (dd, 1H), 7.89 (d, 1H), 8.52 (d, 1H); Mass Spectrum 433 [MH]+.

### Example 7

### S-2-[2-{3-(2-Cyanonyrid-3-yl)isoxazol-5yl}pyrrolidin-1-yl]-6-methyl-4-(5-methyl-1H-pyrazol-3-ylamino)pyrimidine

A mixture of 2-chloro-6-methyl-4-(5-methyl-1*H*-pyrazol-3-ylamino)pyrimidine (140mg, 0.62mmol), S-2-[3-(2-cyanopyrid-3-yl)isoxazol-5-yl]pyrrolidine (250mg, 1.04mmol), and N,N-diisopropylethylamine (0.242ml, 1.4mmol) in xylene (10ml), was heated at 80°C for 18 hours. The mixture was allowed to cool and the volatiles removed by evaporation. The residue was purified by reverse phase HPLC using a C18 column eluting with water / acetonitrile / TFA (95:5:0.2 decreasing in polarity to 0:100:0.2). The fractions containing product were combined, concentrated by evaporation and basified with saturated aqueous sodium hydrogen carbonate solution. The resulting precipitated product was collected by filtration, washed with water and dried under vacuum to give the title compound (130mg, 49%); NMR Spectrum 2.02-2.12 (m, 3H), 2.12 (s, 3H), 2.18 (s, 3H), 2.35-2.45 (m, 1H), 3.69-3.78 (m, 2H), 5.50 (dd, 1H), 5.95 (s, 1H), 6.20 (s, 1H), 6.81 (s, 1H), 7.80 (dd, 1H), 8.31 (d, 1H), 8.81 (d, 1H); Mass Spectrum 428 [MH]+.

The S-2-[3-(2-cyanopyrid-3-yl)isoxazol-5-yl]pyrrolidine starting material was prepared as follows:

A mixture of S-N-(tert-butoxycarbonyl)-2-[3-(2-chloropyrid-3-yl)isoxazol-5-yl]pyrrolidine (1.28g, 3.67mmol), copper(I)cyanide (1.31g, 14.7mmol), bis-palladium(0) tris-dibenzylideneacetone (134mg, 0.15mmol), 1,1'-bis(diphenylphosphino)ferrocene (479mg, 0.59mmol) and tetraethylammonium cyanide (574mg, 3.68mmol) in dry dioxane (20ml) was thoroughly degassed by repeated evacuation and refilling with nitrogen and then the mixture was heated at reflux under nitrogen for 2 days. The mixture was allowed to cool and was diluted with EtOAc / methanol and insoluble matter was removed by filtration. The filtrate was washed with water and the organic layer separated, dried (MgSO₄) and the solvent removed by evaporation. The residue was purified by chromatography on silica gel eluting with EtOAc / hexane (30:70) to give S-N-(tert-butoxycarbonyl)-2-[3-(2-cyanopyrid-3-yl)isoxazol-5-yl]pyrrolidine (578mg, 47%); NMR Spectrum 1.22-1.42 (m, 9H), 1.89-2.0 (m, 3H), 2.23-2.39 (m, 1H), 3.37-3.43 (m, 1H), 3.43-3.54 (m, 1H), 5.02-5.12 (m, 1H), 6.96 (s, 1H), 7.89 (dd, 1H), 8.38 (d, 1H), 8.86 (dd,1H).

TFA (1ml) was added to a solution of S-N-(tert-butoxycarbonyl)-2-[3-(2-cyanopyrid-3-yl)isoxazol-5-yl]pyrrolidine (570mg, 1.67mmol) in DCM (5ml) and the mixture stirred for 3 days at ambient temperature. The volatiles were removed by evaporation and the residue dissolved in water. The aqueous mixture was adjusted to pH=10-11 with 40% aqueous sodium hydroxide solution and extracted with DCM. The extracts were combined, dried (Na₂SO₄) and the solvent removed by evaporation. The residue was purified by chromatography on silica gel eluting with methanol / DCM (1:39) to give S-2-[3-(2-cyanopyrid-3-yl)isoxazol-5-yl]pyrrolidine (250mg, 63%); Mass Spectrum 241 [MH]+.

### Example 8

### S-2-[2-{3-(2-Methylpyrid-3-yl)isoxazol-5-yl}pyrrolidin-1-yl]-4-(5-methyl-1H-pyrazol-3-ylamino)-6-morpholinopyrimidine

A mixture of S-6-chloro-2-[2-{3-(2-methylpyrid-3-yl)isoxazol-5-yl}pyrrolidin-1-yl]-4-(5-methyl-1H-pyrazol-3-ylamino)pyrimidine (prepared as described in Example 4) (190mg, 0.436mmol) and morpholine (4ml) was heated at 120°C under microwave irradiation for 2 hours. The mixture was allowed to cool, diluted with water, extracted with EtOAc. The combined extracts were washed with water, dried (Na₂SO₄) and the solvent removed by evaporation and the product dried under vacuum to give the title compound (127mg, 60%); NMR Spectrum 2.02-2.15 (m, 3H), 2.18 (s, 3H), 2.57 (s, 3H), 2.65-2.70 (m, 2H), 3.32-3.40 (m, 4H), 3.50-3.52 (m, 1H), 3.55-3.70 (m, 4H), 3.68-3.79 (m, 2H), 5.38 (dd, 1H), 5.78 (s, 1H), 5.99 (s, 1H), 6.55 (s, 1H), 7.28 (dd, 1H), 7.81 (dd, 1H), 8.25 (s, 1H), 8.52 (d, 1H), 11.40 (s, 1H); Mass Spectrum 486 [MH]+.

### Example 9

### S-4-(5-cyclopropyl-1H-pyrazol-3-ylamino)-2-[2-{3-(2-methoxpyrid-3-yl)isoxazol-5-yl}prrolidin-1-yl]-6-morpholinopyrimidine

A mixture of S-6-chloro-4-(5-cyclopropyl-1*H*-pyrazol-3-ylamino)-2-[2-{3-(2-methoxypyrid-3-yl)isoxazol-5-yl}pyrrolidin-1-yl]pyrimidine (Example 269 of WO2005/040159) (200mg, 0.45mmol) and morpholine (3ml) were treated as described in Example 8 to give the title compound (175mg, 74%); NMR Spectrum 0.65 (s, 2H), 0.86 (s, 2H), 1.78-1.85 (m, 1H), 2.00-2.12 (m, 3H), 2.31-2.40 (m, 1H), 3.31-3.41 (m, 4H), 3.54-3.61 (m, 4H), 3.68-3.75 (m, 2H), 3.96 (s, 3H), 5.40 (d, 1H), 5.78 (s, 1H), 5.94 (s, 1H), 6.60 (s, 1H), 7.08 (dd, 1H), 8.10 (d, 1H), 8.29 (d, 1H), 11.45 (s, 1H); Mass Spectrum 530 [MH]+.

### Example 10

### S-2-[2-{3-(2-methoxypyrid-3-yl)isoxazol-5-yl}pyrrolidin-1-yl]-4-(5-methyl-1H-pyrazol-3-ylamino-6-morpholinopyrimidine

A mixture of S-6-chloro-2-[2-{3-(2-methoxypyrid-3-yl)isoxazol-5-yl}pyrrolidin-1-yl]-4-(5-methyl-1*H-*pyrazol-3-ylamino)pyrimidine (190mg, 0.43mmol) and morpholine (4ml) was heated at 120°C under microwave irradiation for 2 hours. The mixture was allowed to cool, diluted with water, extracted with EtOAc. The combined extracts were washed with water, dried (Na₂SO₄) and the solvent removed by evaporation and the product dried under vacuum to give the title compound (163mg, 77%); NMR Spectrum 2.02-2.12 (m, 3H), 2.18 (s, 3H), 2.31-2.40 (m, 1H), 3.31-3.41 (m, 4H), 3.55-3.63 (m, 4H), 3.66-3.77 (m, 2H), 3.99 (s, 3H), 5.40 (d, 1H), 5.80 (s, 1H), 5.99 (s, 1H), 6.62 (s, 1H), 7.11 (dd, 1H), 8.15 (d, 1H), 8.28 (d, 1H), 8.32 (s, 1H),11.43 (s, 1H); Mass Spectrum 504 [MH]+.

The S-6-chloro-2-[2-{3-(2-methoxypyrid-3-yl)isoxazol-5-yl}pyrrolidin-1-yl]-4-(5-methyl-1H-pyrazol-3-ylamino)pyrimidine starting material was prepared as follows:-

A mixture of 2,6-dichloro-4-(5-methyl-1*H*-pyrazol-3-ylamino)pyrimidine (prepared as described in Method 29 of WO2005/040159) (414mg, 1.7mmol), S-2-[3-(2-methoxypyrid-3-yl)isoxazol-5-yl]pyrrolidine (prepared as described in Method 64 of WO2005/040159) (460mg, 1.9mmol) and DIPEA (0.787ml, 4.5mmol) in 1-hexanol (10ml) was heated at 80°C for 18 hours. The mixture was allowed to cool and the volatiles removed by evaporation. The residue was purified by chromatography on silica gel eluting with EtOAc/hexanes (0:100 increasing in polarity to 45:55) to give the title compound (375mg, 49%); NMR Spectrum 2.04-2.15 (m, 3H), 2.20 (s, 3H), 2.34-2.41 (m, 1H), 3.62-3.79 (m, 2H), 3.92 (s, 3H), 5.45 (dd, 1H), 6.04 (s, 1H), 6.40 (s, 1H), 6.65 (s, 1H), 7.07 (dd, 1H), 8.10 (d, 1H), 8.25 (d, 1H), 9.18 (s, 1H), 11.60 (s, 1H).); Mass Spectrum 453 [MH]+.

### Example 11

### S-6-Chloro-2-[2-{3-(2-cyanopyrid-3-yl)isoxazol-5-yl}pyrrolidin-1-yl]-4-(5-methyl-1H-pyrazol-3-ylamino)pyrimidine

A mixture of 2,6-dichloro-4-(5-methyl-1*H*-pyrazol-3-ylamino)pyrimidine (prepared as described in Method 29 of WO2005/040159) (574mg, 2.36mmol), S-2-[3-(2-cyanopyrid-3-yl)isoxazol-5-yl]pyrrolidine (prepared as described in Example 7) (627mg, 2.6mmol) and DIPEA (0.994ml, 5.7mmol) in 1-hexanol (10ml) was heated at 80°C for 18 hours. The mixture was allowed to cool and the volatiles removed by evaporation. The residue was dissolved in DCM/methanol washed with water, dried (Na₂SO₄) and the solvent removed by evaporation. The residue was purified by chromatography on silica gel eluting with EtOAc/hexanes (45:55 increasing in polarity to 50:50) to give the title compound (380mg, 36%); NMR Spectrum 2.06-2.18 (m, 3H), 2.18 (s, 3H), 2.39-2.47 (m, 1H), 3.62-3.75 (m, 2H), 5.49 (d, 1H), 6.00 (s, 1H), 6.41 (s, 1H), 6.84 (s, 1H), 7.81 (dd, 1H), 8.29 (dd, 1H), 8.81 (s, 1H), 9.17 (s, 1H), 11.60 (s, 1H); Mass Spectrum 448 [MH]+.

### Example 12

### S-2-[2-{3-(2-Cyanopyrid-3-yl)isoxazol-5-yl)pyrrolidin-1-yl]-4-(5-methyl-1H-pyrazol-3-ylamino)-6-morpholinopyrimidine

A mixture of S-6-chloro-2-[2-{3-(2-cyanopyrid-3-yl)isoxazol-5-yl}pyrrolidin-1-yl]-4-(5-methyl-1H-pyrazol-3-ylamino)pyrimidine (180mg, 0.4mmol) and morpholine (4ml) was heated at 120°C under microwave irradiation for 2 hours. The mixture was allowed to cool, diluted with water, extracted with EtOAc. The combined extracts were washed with water, dried (Na₂SO₄) and the solvent removed by evaporation. The residue was purified by reverse phase HPLC using a C18 column eluting with water / acetonitrile / TFA (70:30:0.2 decreasing in polarity to 30:70:0.2). Fractions containing product were concentrated by evaporation, basified with sodium hydrogen carbonate solution. The precipitated product was collected by filtration and dried under vacuum to give the title compound (60mg, 30%); NMR Spectrum 2.06-2.14 (m, 3H), 2.17 (s, 3H), 2.38-2.46 (m, 1H), 3.32-3.41 (m, 4H), 3.55-3.62 (m, 4H), 3.69-3.83 (m, 2H), 5.44 (d, 1H), 5.78 (s, 1H), 5.98 (s, 1H), 6.85 (s, 1H), 7.87 (dd, 1H), 8.33 (dd, 1H), 8.52 (s, 1H), 8.84 (d, 1H); Mass Spectrum 499 [MH]+.

### Example 13

### S-2-[2-{3-(2-Chloropyrid-3-yl)isoxazol-5-yl}pyrrolidin-1-yl]-4-(5-methyl-1H-pyrazol-3-ylamino)pyrimidine

A mixture of 2-chloro-4-(5-methyl-1*H*-pyrazol-3-ylamino)pyrimidine (prepared as described in Method 26 of WO2005/040159) (196mg, 0.93mmol), S-2-[3-(2-chloropyrid-3-yl)isoxazol-5-yl]pyrrolidine (prepared as described in Example 5) (350mg, 1.4mmol) and DIPEA (0.43ml, 2.3mmol) in 1-hexanol (10ml) was heated at 130°C for 18 hours. The mixture was allowed to cool and the volatiles removed by evaporation. The residue was purified by chromatography on silica gel eluting with EtOAc/methanol (100:0 increasing in polarity to 97:3). The purified product was triturated with ether and collected by filtration to give the title compound (133mg, 34%); NMR Spectrum 2.02-2.14 (m, 3H), 2.18 (s, 3H), 2.34-2.40 (m, 1H), 3.65-3.79 (m, 2H), 5.45 (d, 1H), 6.06 (s, 1H), 6.29 (s, 1H), 6.60 (s, 1H), 7.52 (dd, 1H), 7.82 (d, 1H), 8.08 (d, 1H), 8.50 d, 1H), 8.84 (s, 1H), 11.50 (s, 1H); Mass Spectrum 423 [MH]+.

### Example 14

### S-2-[2-{3-(2-Methylaminopyrid-3-yl)isoxazol-5-yl}pyrrolidin-1-yl]-4-(5-methyl-1H-pyrazol-3-ylamino)pyrimidine

A mixture of 2-chloro-4-(5-methyl-1*H*-pyrazol-3-ylamino)pyrimidine (prepared as described in Method 26 of WO2005/040159) (200mg, 0.96mmol), S-2-[3-(2-methylaminopyrid-3-yl)isoxazol-5-yl]pyrrolidine (320mg, 1.3mmol) and DIPEA (0.42ml, 2.4mmol) in 1-hexanol (10ml) was heated at 130°C for 18 hours. The mixture was allowed to cool and poured onto a 50g isolute SCX-2 ion exchange column. The column was eluted with methanol to elute any neutrals, followed by 7M methanolic ammonia to elute the product. The volatiles were removed by evaporation and the residue purified by chromatography on silica gel eluting with EtOAc/methanol (100:0 increasing in polarity to 97:3) to give the title compound (150mg, 38%); NMR Spectrum 2.02-2.15 (m, 3H), 2.20 (s, 3H), 2.32-2.41 (m, 1H), 3.04 (s, 3H), 3.65-3.72 (m, 1H), 3.75-3.80 (m, 1H), 5.44 (dd, 1H), 6.06 (s, 1H), 6.80 (s, 1H), 6.62 (dd, 1H), 6.78 (s, 1H), 7.20 (s, 1H), 7.89 (dd, 2H), 8.17 (dd, 1H), 8.90 (s, 1H), 11.55 (s, 1H); Mass spectrum 416 [M-H]-.

The S-2-[3-(2-methylaminopyrid-3-yl)isoxazol-5-yl]pyrrolidine starting material was prepared as follows:-

A mixture of S-*N*-(tert-butoxycarbonyl)-2-[3-(2-chloropyrid-3-yl)isoxazol-5-yl]pyrrolidine (prepared as described in Example 5) (4.0g, 11.5mmol) and methylamine (16ml of a 2M solution in THF) was heated at 145°C in a sealed vessel, under microwave irradiation for 10 hours. The mixture was allowed to cool and the volatiles removed by evaporation. The residue was dissolved in DCM, washed with water, dried (Na₂SO₄) and the solvent removed by evaporation to give S-*N*-(tert-butoxycarbonyl)-2-[3-(2-methylaminopyrid-3-yl)isoxazol-5-yl]pyrrolidine (3.0g, 76%) as an oil; NMR Spectrum 1.20-1.42 (m, 9H), 1.88-2.01 (m, 3H), 2.22-2.35 (m, 1H), 3.00 (d, 3H), 3.32-3.42 (m, 1H), 3.44-3.53 (m, 1H), 4.93-5.05 (m, 1H), 6.67 (dd, 1H), 6.94-7.01 (m, 1H), 7.30 (d, 1H), 8.00 (d, 1H), 8.18 (d, 1H); Mass Spectrum 345 [MH]+.

TFA (10ml) was added to a solution of S-*N*-(tert-butoxycarbonyl)-2-[3-(2-methylaminopyrid-3-yl)isoxazol-5-yl]pyrrolidine (3.0g, 8.7mmol) in DCM (50ml) and the mixture stirred for 18 hours at ambient temperature. The volatiles were removed by evaporation and the residue dissolved in water. The aqueous mixture was adjusted to pH10-11 with 40% aqueous sodium hydroxide solution and extracted with DCM. The extracts were combined, dried (Na₂SO₄) and the solvent removed by evaporation to give S-2-[3-(2-methylaminopyrid-3-yl)isoxazol-5-yl]pyrrolidine (1.61g, 76%); NMR Spectrum 1.70-1.84 (m, 3H), 2.08-2.18 (m, 1H), 2.87-2.95 (m, 2H), 2.98 (d, 3H), 4.31 (dd, 1H), 6.65 (dd, 1H), 6.94 (s, 1H), 7.28-7.35 (m, 1H), 7.98 (dd, 1H), 8.18 (dd, 1H).

### Example 15

### S-6-Methyl-2-[2-{3-(2-methylaminopyrid-3-yl)isoxazol-5-yl}pyrrolidin-1-yl]-4-(5-methyl-1H-pyrazol-3-ylamino)pyrimidine

A mixture of 2-chloro-6-methyl-4-(5-methyl-1*H*-pyrazol-3-ylamino)pyrimidine (200mg, 0.896mmol), S-2-[3-(2-methylaminopyrid-3-yl)isoxazol-5-yl]pyrrolidine (prepared as described in Example 14) (306mg, 1.25mmol) and DIPEA (345mg, 2.6mmol) in xylene (10ml) was heated at 130°C for 18 hours. The mixture was allowed to cool and the volatiles removed by evaporation. The residue was purified by chromatography on silica gel eluting with EtOAc/hexanes (25:75 increasing in polarity to 100:0) to give the title compound (195mg, 51%); NMR Spectrum 2.05-2.10 (m, 3H), 2.10 (s, 3H), 2.19 (s, 3H), 2.32-2.40 (m, 1H), 3.04 (d, 3H), 3.65-3.71 (m, 1H), 3.71-3.80 (m, 1H), 5.45 (d, 1H), 6.0 (s, 1H), 6.19 (s, 1H), 6.62 (dd, 1H), 6.78 (s, 1H), 7.20 (s, 1H), 7.88 (d, 1H), 8.17 (dd, 1H), 8.70 (s, 1H), 11.5 (s, 1H); Mass Spectrum 430 [M-H]-.

### Example 16

### S-2-[2-{3-(2-Methylprid-3-yl)isoxazol-5-yl}pyrrolidin-1-yl]-4-(5-methyl-1H-pyrazol-3-ylamino)pyrimidine

A mixture of 2-chloro-4-(5-methyl-1*H*-pyrazol-3-ylamino)pyrimidine (prepared as described in Method 26 of WO2005/040159) (200mg, 0.96mmol), S-2-[3-(2-methylpyrid-3-yl)isoxazol-5-yl]pyrrolidine (prepared as described in Example 3) (328mg, 1.4mmol) and DIPEA (0.42ml, 2.4mmol) in 1-hexanol (10ml) was heated at 150°C for 18 hours. The mixture was allowed to cool and poured onto a 50g isolute SCX-2 ion exchange column. The column was eluted with methanol to elute any neutrals, followed by 7M methanolic ammonia to elute the product. The volatiles were removed by evaporation and the residue purified by chromatography on silica gel eluting with EtOAc/methanol (100:0 increasing in polarity to 95:5). The purified product was triturated with ether and collected by filtration to give the title compound (250mg, 65%); NMR Spectrum 2.05-2.14 (m, 3H), 2.18 (s, 3H), 2.32-2.45 (m, 1H), 2.58 (s, 3H), 3.65-3.72 (m, 1H), 3.73-3.80 (m, 1H), 5.45 (d, 1H), 6.06 (s, 1H), 6.30 (s, 1H), 6.55 (s, 1H), 7.80 (dd, 1H), 7.87 (dd, 2H), 8.50 (dd, 1H), 8.89 (s, 1H), 11.53 (s, 1H); Mass Spectrum 401 [M-H].

### Example 17

### S-6-(2-Methoxyethoxy)-2-[2-{3-(2-methylpyrid-3-yl)isoxazol-5-yl}pyrrolidin-1-yl]-4-(5-methyl-1H-pyrazol-3-ylamino)pyrimidine

Sodium hydride (110mg, 2.3mmol) was added to 2-methoxyethanol (20ml) and the mixture was stirred for 10 minutes while being purged with nitrogen. S-6-Chloro-2-[2-{3-(2-methylpyrid-3-yl)isoxazol-5-yl}pyrrolidin-1-yl]-4-(5-methyl-1*H*-pyrazol-3-ylamino)pyrimidine (prepared as described in Example 4) (200mg, 0.46mmol) was added and the mixture heated at 120°C in a sealed vessel under microwave irradiation for 8.5 hours. The mixture was allowed to cool, was diluted with water and extracted with EtOAc. The extracts were combined, dried (Na₂SO₄) and the solvent removed by evaporation. The residue was purified by chromatography on silica gel eluting with EtOAc/hexanes (60:40 increasing in polarity to 90:10) to give the title compound (85mg, 39%); NMR Spectrum 2.04-2.18 (m, 3H), 2.18 (s, 3H), 2.37-2.46 (m, 1H), 2.58 (s, 3H), 3.25 (s, 3H), 3.55 (t, 2H), 3.67-3.80 (m, 2H), 4.28 (t, 2H), 5.40 (d, 1H), 5.78 (s, 1H), 5.99 (s, 1H), 6.58 (s, 1H), 7.29 (dd, 1H), 7.87 (d, 1H), 8.50 (d, 1H), 8.61 (s, 1H), 11.49 (s, 1H); Mass Spectrum 477 [MH]+.

### Example 18

### S-6-Chloro-2-[2-[3-{3-ethylpyrazin-2-yl)isoxazol-5-yl}pyrrolidin-1-yl]-4-(5-methyl-1H-pyrazol-3-ylamino)pyrimidine

A mixture of 2,6-dichloro-4-(5-methyl-1*H*-pyrazol-3-ylamino)pyrimidine (prepared as described in Method 29 of WO2005/040159) (721mg, 2.95mmol), S-2-[3-(3-ethylpyrazin-2-yl)isoxazol-5-yl]pyrrolidine (790mg, 3.25mmol) and zinc (II)acetate (540mg, 2.9mmol) in isopropanol (20ml) was heated at reflux for 18 hours. The mixture was allowed to cool and the volatiles removed by evaporation. Water was added to the residue and the mixture basified with sodium hydrogen carbonate solution. The mixture was extracted with EtOAc, the extracts combined, dried (Na₂SO₄) and the solvent removed by evaporation. The residue was purified by chromatography on silica gel eluting with EtOAc/hexane (60:40) and the purified product triturated with hexane and collected by filtration to give the title compound (583mg, 44%); NMR Spectrum 1.16-1.24 (m, 3H), 2.04-2.23 (m, 3H), 2.32-2.41 (m, 1H), 3.07-3.17 (m, 2H), 3.50-3.67 (m, 1H), 3.72-3.84 (m, 1H), 5.40 (d, 1H), 5.88 (s, 1H), 6.73 (s, 1H), 8.58 (s, 1H), 8.67 (s, 1H), 9.70 (s, 1H), 11.81-11.97 (m, 1H); Mass Spectrum 452 [MH]+.

The S-2-[3-(3-ethylpyrazin-2-yl)isoxazol-5-yl]pyrrolidine starting material was prepared as follows:

A mixture of 2-methyl-3-ethylpyrazine (22.35g, 183mmol), selenium dioxide (30g, 270mmol), and diatomaceous earth (30g) in dioxane (250ml) was heated at reflux for 18 hours. The mixture was allowed to cool and the solid material removed by filtration through diatomaceous earth. The filter pad was washed with methanol several times and the volatiles removed from the combined filtrate by evaporation. The residue was purified by chromatography on silica gel eluting eluting with EtOAC/hexanes (0:100 increasing in polarity to15:85) to give 3-ethylpyrazine-2-carboxaldehyde (3.0g, 12%); NMR Spectrum 1.22 (t, 3H), 3.19 (q, 2H), 8.78 (s, 1H), 8.80 (s, 1H), 10.10 (s, 1H).

A solution of sodium hydroxide (4.52g, 0.113mol) in water (15ml) was added to a solution of hydroxylamine hydrochloride (3.48g, 50mmol) in water (15ml). The mixture was cooled in an ice bath and then added carefully to a solution of 3-ethylpyrazine-2-carboxaldehyde (6.15g, 45mmol) in a mixture of ethanol (85ml), water (85ml), and ice (80g). The mixture was stirred at ambient temperature for 18 hours and then neutralised to pH7 with 6M hydrochloric acid. The mixture was concentrated by evaporation and the resulting precipitated product collected by filtration. The product was washed with water and dried under vacuum to give 3-ethylpyrazine-2-carboxaldehyde oxime (2.3g, 34%); NMR Spectrum 1.20 (t, 3H), 3.05 (q, 2H), 8.25 (s, 1H), 8.52 (s, 2H), 11.89 (s, 1H); Mass Spectrum 152 [MH]+.

Sodium hypochlorite (6.87ml of a 13% solution of in water, 12mmol) was added to solution of S-*N*-tertbutoxycarbonyl-2-ethynylpyrrolidine (prepared as described in Bull. Soc. Chim. Fr. 1997, 134, 141-144 and J. Med. Chem. 1994, 37, 4455-4463) (1.94g, 10mmol) and 3-ethylpyrazine-2-carboxaldehyde oxime (1g, 6.6mmol) in DCM (50ml) at 0°C. The mixture was allowed to warm to ambient temperature and stirred for 18 hours. The DCM layer was separated, dried (Na₂SO₄), and the solvent removed by evaporation. The residue was purified by chromatography on silica gel eluting with EtOAc/hexanes (0:100 increasing to 20:80) to give S-*N*-(tert-butoxycarbonyl)-2-[3-(3-ethylpyrazin-2-yl)isoxazol-5-yl]pyrrolidine (1.3g, 57%); NMR Spectrum 1.20-1.45 (m, 12H), 1.90-2.02 (m, 3H), 2.25-2.38 (m, 1H), 3.15 (q, 2H), 3.33-3.41 (m, 1H), 3.47-3.55 (m, 1H), 5.05 (m, 1H), 6.77 (m, 1H), 8.64 (d, 1H), 8.69 (d, 1H); Mass Spectrum 343 [M-H]-.

A solution of 4M hydrogen chloride in dioxane (15ml) was added to a solution of S-N-(tert-butoxycarbonyl)-2-[3-(3-ethylpyrazin-2-yl)isoxazol-5-yl]pyrrolidine (1.3g, 3.8mmol) in methanol (50ml) and the mixture stirred at ambient temperature for 18 hours. The volatiles were removed by evaporation and the residue dissolved in water, basified with aqueous ammonia and extracted with DCM. The extracts were combined, dried (Na₂SO₄) and the solvent removed by evaporation to give S-2-[3-(3-ethylpyrazin-2-yl)isoxazol-5-yl]pyrrolidine (700mg, 76%).

### Example 19

### S-6-(2-Methoxyethoxy)-2-[2-{3-(3-ethylpyrazin-2-yl)isoxazol-5-yl}pyrrolidin-1-yl]-4-(5-methyl-1H-pyrazol-3-ylamino)pyrimidine

Sodium hydride (107mg, 2.2mmol) was added to 2-methoxyethanol (20ml) and the mixture was stirred for 10 minutes while being purged with nitrogen. S-6-chloro-2-{2-[3-(3-ethylpyrazin-2-yl)isoxazol-5-yl]pyrrolidin-1-yl}-4-(5-methyl-1*H-*pyrazol-3-ylamino)pyrimidine (200mg, 0.44mmol) was added and the mixture heated at 120°C in a sealed vessel under microwave irradiation for 5.5 hours. The mixture was allowed to cool, was diluted with water and extracted with EtOAc. The extracts were combined washed with water, dried (Na₂SO₄) and the solvent removed by evaporation. The residue was purified by chromatography on silica gel eluting with EtOAc/hexanes (40:60 increasing to 60:40) to give the title compound (80mg, 37%); NMR Spectrum 1.22 (t.3H), 2.03-2.16 (m, 3H), 2.19 (s, 3H), 2.35-2.48 (m, 1H), 3.14 (q, 2H), 3.24 (s, 3H), 3.55 (t, 2H), 3.31-3.67 (m, 2H), 4.28 (t, . 2H), 5.43 (d, 1H), 5.80 (s, 1H), 6.00 (s, 1H), 6.68 (s, 1H), 8.50 (s, 1H), 8.62 (s, 2H), 11.48 (s, 1H); Mass Spectrum 492 [M]+.

### Example 20

### S-6-Methoxy-2-[2-{3-(3-ethylpyrazin-2-yl)isoxazol-5-yl}pyrrolidin-1-yl]-4-(5-methyl-1H-pyrazol-3-ylamino)pyrimidine

A mixture of S-6-chloro-2-{2-[3-(3-ethylpyrazin-2-yl)isoxazol-5-yl]pyrrolidin-1-yl}-4-(5-methyl-1*H*-pyrazol-3-ylamino)pyrimidine (200mg, 0.44mmol) and sodium methoxide (500mg of a 25% solution in methanol, 2.2mmol) in dry methanol (20ml) was heated in a sealed vessel at 120°C under microwave radiation for 6.5 hours. The mixture was allowed to cool and the volatiles removed by evaporation. The residue was dissolved in EtOAc, washed with water, dried (Na₂SO₄) and the solvent removed by evaporation. The residue was purified by chromatography on silica gel eluting with EtOAc/hexane (50:50 increasing to 60:40) to give the title compound (70mg, 35%); NMR Spectrum 1.22 (t, 3H), 2.04-2.14 (m, 3H), 2.19 (s, 3H), 2.39-2.46 (m, 1H), 3.14 (q, 2H), 3.72 (s, 3H), 3.72-3.80 (m, 2H), 5.45 (dd, 1H), 5.80 (s, 1H), 5.99 (s, 1H), 6.68 (s, 1H), 8.57 (d, 1H), 8.62 (d, 1H), 11.48 (s, 1H).; Mass Spectrum 452 [MH]+.

### Example 21

### S-6-(2-Hydroxyethoxy)-2-[2-{3-(2-methylpyrid-3-yl)isoxazol-5-yl}pyrrolidin-1-yl]4-(5-methyl-1H-pyrazol-3-ylamino)pyrimidine

Sodium hydride (110mg, 2.3mmol) was added to ethylene glycol (20ml) and the mixture was stirred for 10 minutes while being purged with nitrogen. S-6-Chloro-2-[2-{3-(2-methylpyrid-3-yl)isoxazol-5-yl}pyrrolidin-1-yl]-4-(5-methyl-1*H*-pyrazol-3-ylamino)pyrimidine (prepared as described in Example 4) (200mg, 0.46mmol) was added and the mixture heated at 120°C in a sealed vessel under microwave irradiation for 14.5 hours. The mixture was allowed to cool, was diluted with water and extracted with EtOAc. The extracts were combined washed with water, dried (Na₂SO₄) and the solvent removed by evaporation. The residue was purified by chromatography on silica gel eluting with methanol/EtOAc/hexanes (0:75:25 increasing to 2:98:0). The product was further purified by reverse phase HPLC using a C18 column eluting with water / acetonitrile / TFA (95:5:0.2 decreasing in polarity to 0:100:0.2) the fractions containing product were combined and passed through a 50g isolute SCX-2 ion exchange column. The column was eluted with methanol to elute any neutral impurities, followed by 7M methanolic ammonia to elute the product. The solvent was evaporated to give the title compound (16mg, 8%); NMR Spectrum 2.04-2.15 (m, 3H), 2.17 (s, 3H), 2.37-2.44 (m, 1H), 2.58 (s, 3H), 3.62 (t, 2H), 3.68-3.80 (m, 2H), 4.11-4.22 (m, 2H), 4.35 (s, 1H), 5.40 (d, 1H), 5.79 (s, 1H), 5.98 (s, 1H), 6.58 (s, 1H), 7.29 (dd, 1H), 7.87 (d, 1H), 8.51 (d, 1H), 8.64 (s, 1H), 11.5 (s, 1H); Mass Spectrum 463 [MH]+.

### Example 22

### S-6-(3-Hydroxypropoxy)-2-[2-{3-(2-methylpyrid-3-yl)isoxazol-5-yl}pyrrolidin-1-yl]-4-(5-methyl-1H-pyrazol-3-ylamino)pyrimidine

Sodium hydride (155mg, 3.2mmol) was added to 1, 3-propanediol (20ml) and the mixture was stirred for 10 minutes while being purged with nitrogen. S-6-Chloro-2-[2-{3-(2-methylpyrid-3-yl)isoxazol-5-yl}pyrrolidin-1-yl]-4-(5-methyl-1*H*-pyrazol-3-ylamino)pyrimidine (prepared as described in Example 4) (200mg, 0.46mmol) was added and the mixture heated at 120°C in a sealed vessel under microwave irradiation for 12 hours. The mixture was allowed to cool, was diluted with water and extracted with EtOAc. The extracts were combined washed with water, dried (Na₂SO₄) and the solvent removed by evaporation. The residue was purified by chromatography on silica gel eluting with methanol/EtOAc (0:100 increasing to 7:93) to give the title compound (100mg, 46%); NMR Spectrum 1.78 (t, 2H), 2.04-2.16 (m, 3H), 2.18 (s, 3H), 2.35-2.44 (m, 1H), 2.59 (s, 3H), 3.49 (q, 2H), 3.67-3.80 (m, 2H), 4.06 (t, 1H), 4.18-4.28 (m, 2H), 5.40 (d, 1H), 5.77 (s, 1H), 5.98 (s, 1H), 6.58 (s, 1H), 7.29 (dd, 1H), 7.86 (d, 1H), 8.51 (dd, 1H), 8.60 (s, 1H), 11.49 (s, 1H); Mass Spectrum 475 [M-H]-.

### Example 23

### 6-((2R)-2-Hydroxpropoxy)-2-[(2S)-2-{3-(2-methylpyrid-3-yl)isoxazol-5-yl}-pyrrolidin-1-yl]-4-(5-methyl-1H-pyrazol-3-ylamino)pyrimidine

Sodium hydride (110mg, 2.3mmol) was added to (R)-(-)-1, 2 propanediol (3g, 39mmol) in THF (20ml) and the mixture was stirred for 10 minutes while being purged with nitrogen. S-6-Chloro-2-[2-{3-(2-methylpyrid-3-yl)isoxazol-5-yl}pyrrolidin-1-yl]-4-(5-methyl-1*H*-pyrazol-3-ylamino)pyrimidine (Example 4) (200mg, 0.46mmol) was added and the mixture heated at 120°C in a sealed vessel under microwave irradiation for 17 hours. The mixture was allowed to cool, was diluted with water and extracted with EtOAc. The extracts were combined washed with water, dried (Na₂SO₄) and the solvent removed by evaporation. The residue was purified by chromatography on silica gel eluting with methanol/EtOAc (0:100 increasing in polarity to 5:95) to give the title compound (85mg, 39%); NMR Spectrum 1.08 (s, 3H), 2.04-2.16 (m, 3H), 2.18 (s, 3H), 2.35-2.45 (m, 1H), 2.59 (s, 3H), 3.67-3.80 (m, 2H), 3.90 (q, 1H), 3.97-4.06 (m, 2H), 4.32 (d, 1H), 5.40 (d, 1H), 5.80 (s, 1H), 5.98 (s, 1H), 6.58 (s, 1H), 7.30 (dd, 1H), 7.87 (dd, 1H), 8.52 (dd, 1H), 8.61 (s, 1H), 11.48 (s, 1H); Mass Spectrum 477 [MH]+.

### Example 24

### S-6-chloro-2-[2-{3-(1-oxopyrid-3-yl)isoxazol-5-yl}pyrrolidin-1-yl]-4-(5-methyl-1H-pyrazol-3-ylamino)pyrimidine

A mixture of 2,6-dichloro-4-(5-methyl-1*H*-pyrazol-3-ylamino)pyrimidine (prepared as described in Method 29 of WO2005/040159) (240mg, 0.98mmol), S-2-[2-{3-(1-oxopyrid-3-yl)isoxazol-5-yl}pyrrolidine (240mg, 1.03mmol) and zinc acetate (180mg, 0.98mmol) in isopropanol (20ml) was heated at reflux for 18 hours. The mixture was allowed to cool and the volatiles removed by evaporation. Water was added to the residue and the mixture basified with sodium hydrogen carbonate solution. The mixture was extracted with EtOAc, the extracts combined, dried (Na₂SO₄) and the solvent removed by evaporation. The residue was purified by chromatography on silica gel eluting with EtOAc/hexanes (0:100 increasing in polarity to 5:95) and the purified product triturated with ether/hexane and collected by filtration to give S-6-chloro-2-[2-{3-(1-oxopyrid-3-yl)isoxazol-5-yl}pyrrolidin-1-yl]-4-(5-methyl-1*H*-pyrazol-3-ylamino)pyrimidine (227mg, 53%); Mass Spectrum 439 [MH]+.

The S-2-[2-{3-(1-oxopyrid-3-yl)isoxazol-5-yl}pyrrolidine starting material was prepared as follows:

Sodium hypochlorite (26ml of a 13% solution of in water, 45mmol) was added to solution of S-*N*-tertbutoxycarbonyl-2-ethynylpyrrolidine (prepared as described in Bull. Soc. Chim. Fr. 1997, 134, 141-144 and J. Med. Chem. 1994, 37, 4455-4463) (7.19g, 36.9mmol) and pyridine-3-carboxaldehyde oxime (3.0g, 24.5mmol) in DCM (100ml) at 0°C. The mixture was allowed to warm to ambient temperature and stirred for 18 hours. The DCM layer was separated, dried (Na₂SO₄ and the solvent removed by evaporation. The residue was purified by chromatography on silica gel eluting with EtOAc/hexanes (40:60 increasing in polarity to 55:45) to give S-*N*-(tert-butoxycarbonyl)-2-[2-{3-(pyrid-3-yl)isoxazol-5-yl}pyrrolidine (1.72g, 22%); NMR Spectrum 1.24-1.45 (m, 9H), 1.9-2.0 (m, 3H), 2.22-2.35 (m, 1H), 3.38-3.44 (m, 1H), 3.48-3.56 (m, 1H), 4.96-5.07 (m, 1H), 6.94-7.04 (m, 1H), 7.55 (dd, 1H), 8.26 (dd, 1H), 8.70 (d, 1H), 9.07 (s, 1H); Mass Spectrum 315 [MH]+.

A mixture of meta-chloroperbenzoic acid (mCPBA) (1.33g, 7.7mmol) and S-N-(tert-butoxycarbonyl)-2-[2-{3-(pyrid-3-yl)isoxazol-5-yl}pyrrolidine (1.62g, 5.1mmol) in DCM (50ml) was stirred at ambient temperature for 18 hours. The mixture was diluted with DCM, washed with water, dried (Na₂SO₄) and the solvent removed by evaporation. The residue was purified by chromatography on silica gel eluting with methanol/EtOAc (0:100 increasing to 5:95) to give S-*N*-(tert-butoxycarbonyl)-2-[2-{3-(1-oxopyrid-3-yl)isoxazol-5-yl}pyrrolidine (1.3g, 76%); Mass Spectrum 332 [MH]+.

A solution of 4M hydrogen chloride in dioxane (20ml) was added to a solution of S-N-(tert-butoxycarbonyl)-2-[2-{3-(1-oxopyrid-3-yl)isoxazol-5-yl}pyrrolidine (3.1g, 9.3mmol) in methanol (50ml) and the mixture heated at 60°C for 1 hour. The volatiles were removed by evaporation and the residue dissolved in methanol and applied to an isolute SCX2 ion exchange column. The column was eluted with DCM / methanol (4:1) to remove neutrals and then with 7M methanolic ammonia to elute the product. The solvent was removed by evaporation to give S-2-[2-{3-(1-oxopyrid-3-yl)isoxazol-5-yl}pyrrolidine (1.94g, 90%); Mass Spectrum 232 [M13]+.

### Example 25

### S-6-(2-methoxyethoxy)-2-[2-{3-(1-oxopyrid-3-yl)isoxazol-5-yl}pyrrolidin-1-yl]-4-(5-methyl-1H-pyrazol-3-ylamino)pyrimidine

Sodium hydride (168mg, 3.5mmol) was added to 2-methoxyethanol (20ml) and the mixture was stirred for 10 minutes while being purged with nitrogen. S-6-chloro-2-[2-{3-(1-oxopyrid-3-yl)isoxazol-5-yl }pyrrolidin-1-yl]-4-(5-methyl-1H-pyrazol-3-ylamino)pyrimidine (200mg, 0.5mmol) was added and the mixture heated at 120°C in a sealed vessel under microwave irradiation for 10 hours. The mixture was allowed to cool, was diluted with water and extracted with EtOAc. The extracts were combined washed with water, dried (Na₂SO₄) and the solvent removed by evaporation. The residue was purified by chromatography on silica gel eluting with EtOAc/hexanes (0:100 increasing in polarity to 7:93) to give S-6-(2-methoxyethoxy)-2-[2-{3-(1-oxopyrid-3-yl)isoxazol-5-yl}pyrrolidin-1-yl]-4-(5-methyl-1*H-*pyrazol-3-ylamino)pyrimidine (115mg, 48%); NMR Spectrum 2.02-2.11 (m, 3H), 2.19 (s, 3H), 2.35-2.43 (m, 1H), 3.25 (s, 3H), 3.55 (t, 2H), 3.67-3.80 (m, 2H), 4.28 (t, 2H), 5.36 (d, 1H), 5.79 (s, 1H), 5.96 (s, 1H), 7.47 (dd, 1H), 7.70 (d, 1H), 8.24 (d, 1H), 8.60 (s, 1H), 8.63 (s, 1H), 11.48 (s, 1H); Mass Spectrum 479 [MH]+.

### Example 26

### S-6-(2-Methoxyethoxy)-2-[2-{3-(2-cyanopyrid-3-yl)isoxazol-5-yl}pyrrolidin-1-yl]-4-(5-methyl-1H-pyrazol-3-ylamino)pyrimidine

A mixture of S-6-(2-methoxyethoxy)-2-[2-{3-(1-oxopyrid-3-yl)isoxazol-5-yl}pyrrolidin-1-yl]-4-(5-methyl-1*H*-pyrazol-3-ylamino)pyrimidine (280mg, 0.59mmol), trimethylsilylcyanide (0.56ml, 4.2mmol), 1,8-diazobicyclo[5.4.0]undec-7-ene (DBU) (0.56ml, 3.8mmol) in THF (4ml) was heated at 140°C in a sealed vessel under microwave irradiation for 2 hours. The mixture was allowed to cool and the volatiles removed by evaporation. The residue was purified by chromatography on silica gel eluting with EtOAc/hexanes (50:50 increasing in polarity to 70:30) to give the title compound (80mg, 28%); NMR Spectrum 2.04-2.16 (m, 3H), 2.19 (s, 3H), 3.24 (s, 3H), 3.55 (t, 2H), 3.68-3.80 (m, 2H), 4.28 (t, 2H), 5.45 (d, 1H), 5.80 (s, 1H), 5.98 (s, 1H), 6.87 (s, 1H), 7.82 (dd, 1H), 8.81 (d, 1H), 8.62 (s, 1H), 8.81 (d, 1H), 11.5 (s, 1H); Mass Spectrum 488 [MH]+.

### Example 27

### S-6-(1-Cyanocyclopropyl)methoxy-2-[2-{3-(2-methylpyrid-3-yl)isoxazol-5-yl}pyrrolidin-1-yl]-4-(5-methyl-1H-pyrazol-3-ylamino)pyrimidine

Sodium hydride (176mg, 3.6mmol) was added to (1-cyanocyclopropyl)methanol (500mg, 5.2mmol) in THF (20ml) and the mixture was stirred for 10 minutes while being purged with nitrogen. S-6-Chloro-2-[2-{3-(2-methylpyrid-3-yl)isoxazol-5-yl}pyrrolidin-1-yl]-4-(5-methyl-1*H-*pyrazol-3-ylamino)pyrimidine (prepared as described in Example 4) (200mg, 0.46mmol) was added and the mixture heated at 120°C in a sealed vessel under microwave irradiation for 11 hours. The mixture was allowed to cool, was diluted with water and extracted with EtOAc. The extracts were combined washed with water, dried (Na₂SO₄) and the solvent removed by evaporation. The residue was purified by chromatography on silica gel eluting with methanol/EtOAc (0:100 increasing in polarity to 5:95) to give the title compound (50mg, 22%); NMR Spectrum 1.05-1.13 (m, 2H), 1.22-1.30 (m, 2H), 2.04-2.17 (m, 3H), 2.19 (s, 3H), 2.38-2.46 (m, 1H), 2.60 (s, 3H), 3.68-3.80 (m, 2H), 4.18 (d, 1H), 4.29 (d, 1H), 5.4 (d, 1H), 5.36 (s, 1H), 6.00 (s, 1H), 6.58 (s, 1H), 7.30 (dd, 1H), 7.86 (d, 1H), 8.52 (d, 1H), 8.72 (s, 1H), 11.52 (s, 1H); Mass Spectrum 498 [MH]+.

### Example 28

### S-6-(3-Cyanopropoxy)-2-[2-{3-(2-methylpyrid-3-yl)isoxazol-5-yl}pyrrolidin-1-yl]-4-(5-methyl-1H-pyrazol-3-ylamino)pyrimidine

Sodium hydride (175mg, 3.6mmol) was added to 3-cyanopropanol (500mg, 6mmol) in THF (20ml) and the mixture was stirred for 10 minutes while being purged with nitrogen. S-6-Chloro-2-[2-{3-(2-methylpyrid-3-yl)isoxazol-5-yl}pyrrolidin-1-yl]-4-(5-methyl-1*H-*pyrazol-3-ylamino)pyrimidine (prepared as described in Example 4) (200mg, 0.46mmol) was added and the mixture heated at 120°C in a sealed vessel under microwave irradiation for 10.5 hours. The mixture was allowed to cool, was diluted with water and extracted with EtOAc. The extracts were combined washed with water, dried (Na₂SO₄) and the solvent removed by evaporation. The residue was purified by chromatography on silica gel eluting with methanol/EtOAc (0:100 increasing to 3:97) to give the title compound (50mg, 23%); NMR Spectrum 1.90-1.97 (m, 2H), 2.04-2.15 (m, 3H), 2.19 (s, 3H), 2.38-2.47 (m, 1H), 2.50-2.55 (m, 2H), 2.59 (s, 3H), 3.69-3.80 (m, 2H), 4.20-4.30 (m, 2H), 5.41 (d, 1H), 5.80 (s, 1H), 5.96 (s, 1H), 6.58 (s, 1H), 7.29 (dd, 1H), 7.86 (d, 1H), 8.51 (dd, 1H), 8.68 (dd, 1H), 11.49 (s, 1H); Mass Spectrum 486 [MH]+.

### Example 29

### S-6-methoxy-2-[2-{3-(1-oxopyrid-3-yl)isoxazol-5-yl}pyrrolidin-1-yl]-4-(5-methyl-1H-pyrazol-3-ylamino) pyrimidine

A mixture of S-6-chloro-2-[2-{3-(1-oxopyrid-3-yl)isoxazol-5-yl}pyrrolidin-1-yl]-4-(5-methyl-1*H*-pyrazol-3-ylamino)pyrimidine (600mg, 1.4mmol) and sodium methoxide (1.5g of a 25% solution in methanol, 6.6mmol) in dry methanol (20ml) was heated in a sealed vessel at 120°C under microwave radiation for 10 hours. The mixture was allowed to cool and the volatiles removed by evaporation. The residue was dissolved in EtOAc, washed with water, dried (Na₂SO₄) and the solvent removed by evaporation. The residue was purified by chromatography on silica, gel eluting with methanol/EtOAc (0:100 increasing in polarity to 5:95) to give S-6-methoxy-2-[2-{3-(1-oxopyrid-3-yl)isoxazol-5-yl}pyrrolidin-1-yl]-4-(5-methyl-1*H*-pyrazol-3-ylamino)pyrimidine (320mg, 54%); NMR Spectrum 2.00-2.09 (m, 3H), 2.10-2.20 (m, 3H), 2.28-2.40 (m, 1H), 3.29 (s, 3H), 3.55-3.66 (m, 1H), 3.72-3.80 (m, 1H), 5.85 (d, 1H), 5.78 (s, 1H), 6.18 (s, 1H), 7.04 (s, 1H), 7.51 (dd, 1H), 7.78 (d, 1H), 8.28 (d, 1H), 8.69 (s, 1H), 9.10 (s, 1H), 11.72 (s, 1H); Mass Spectrum 435 [MH]+.

### Example 30

### S-6-Methoxy-2-[2-{3-(2-cyanopyrid-3-yl)isoxazol-5-yl}pyrrolidin-1-yl]-4-(5-methyl-1H-pyrazol-3-ylamino)pyrimidine

A mixture of S-6-methoxy-2-[2-{3-(1-oxopyrid-3-yl)isoxazol-5-yl}pyrrolidin-1-yl]-4-(5-methyl-1*H*-pyrazol-3-ylamino)pyrimidine (320mg, 0.74mmol), trimethylsilylcyanide (0.79ml, 6mmol) and 1,8-diazobicyclo[5.4.0]undec-7-ene (DBU) (0.88ml, 6mmol) in THF (20ml) was heated at 140°C in a sealed vessel under microwave irradiation for 2 hours. The mixture was allowed to cool and the volatiles removed by evaporation. The residue was purified by chromatography on silica gel eluting with EtOAc/hexanes (50:50 increasing in polarity to 60:40) to give the title compound (99mg, 32%); NMR Spectrum 2.05-2.19 (m, 6H), 2.35-2.44 (m, 1H), 3.58-3.70 (m, 2H), 3.72-3.84 (m, 3H), 5.42 (d, 1H), 5.83 (s, 1H), 6.17 (s, 1H), 6.95 (s, 1H), 7.87 (dd, 1H), 8.38 (d, 1H), 8.84 (d, 1H), 9.11 (s, 1H), 11.74 (s, 1H); Mass Spectrum 444 [MH]+.

### Example 31

### S-6-Chloro-2-[2-{3-(3-methylpyrazin-2-yl)isoxazol-5-yl}pyrrolidin-1-yl]-4-(5-methyl-1H-pyrazol-3-ylamino)pyrimidine

A mixture of 2,6-dichloro-4-(5-methyl-1*H*-pyrazol-3-ylamino)pyrimidine (prepared as described in Method 29 of WO2005/040159) (486mg, 2mmol), S-2-[3-(3-methylpyrazin-2-yl)isoxazol-5-yl]pyrrolidine (506mg, 2.2mmol) and DIPEA (0.77ml, 4.4mmol) in 1-hexanol (20ml) was heated at 75°C for 18 hours. The mixture was allowed to cool and applied to an isolute SCX2 ion exchange column. The column was eluted with DCM / methanol (4:1) to remove neutrals and then with 7M methanolic ammonia to elute the product. The solvent was removed by evaporation and the residue was recrystallised from DCM/ether to give the title compound (671mg, 77%); NMR Spectrum 2.04-2.10 (2H, m), 2.12-2.19 (m, 1H), 2.17 (s, 3H), 2.36-2.44 (m, 1H), 2.75 (s, 3H), 3.63-3.70 (m, 1H), 3.73-3.79 (m, 1H), 5.45-5.50 (m, 1H), 6.02 (s, 1H), 6.40 (s, 1H), 6.62 (s, 1H), 8.53-8.61 (m, 2H), 9.25 (s, 1H), 11.64 (s, 1H); Mass Spectrum 437 [MH]+.

The S-2-[3-(3-methylpyrazin-2-yl)isoxazol-5-yl]pyrrolidine starting material was prepared as follows:

A mixture of 2,3-dimethylpyrazine (20g, 18.5mmol), selenium dioxide (41.06g, 37mmol) and diatomeous earth (20g) in EtOAc (500ml) was stirred and heated at 70°C for 2 hours. The mixture was allowed to cool and the insoluble matter was removed by filtration through diatomeous earth. The filtrate was washed with saturated aqueous sodium hydrogen carbonate solution and then saturated aqueous sodium chloride solution, dried (MgSO₄) and the solvent removed by evaporation. The residue was suspended in water (100ml) and hydroxylamine (45ml of a 50% aqueous solution) was added. The mixture was stirred at ambient temperature for 18 hours and the mixture then extracted with EtOAc. The extracts were combined, washed with saturated aqueous sodium chloride solution, dried (MgSO₄) and the solvent removed by evaporation. The residue was triturated with isohexane to give 3-methylpyrazine-2-carboxaldehyde oxime (9.65g, 38%); NMR Spectrum 2.67 (s, 3H), 8.23 (s, 1H), 8.45-8.49 (m, 2H), 11.87 (s, 1H).

Sodium hypochlorite (18ml of a 13% aqueous solution, 25.9mmol) was added dropwise to a stirred suspension of 3-methylpyrazine-2-carboxaldehyde oxime (2.74g, 20mmol) and S-*N-*tertbutoxycarbonyl-2-ethynylpyrrolidine (prepared as described in Bull. Soc. Chim. Fr. 1997, 134, 141-144 and J. Med. Chem. 1994, 37, 4455-4463) (5.85g, 30mmol) in DCM (50ml) at 0°C. The mixture was stirred for 1 hour at 0°C then allowed to warm to ambient temperature and stirred for 18 hours. The mixture was diluted with water and extracted with DCM. The extracts were combined, dried (MgSO₄) and the solvent removed by evaporation. The residue was purified by chromatography on silica gel eluting first with DCM and then with EtOAc/hexanes (25:75) to give S-*N-*(tert-butoxycarbonyl)-2-[3-(3-methylpyrazin-2-yl)isoxazol-5-yl]pyrrolidine (2.62g, 48%); Mass Spectrum 275 [M-C₄H₉]+.

TFA (20ml) was added to a solution of S-*N*-(tert-butoxycarbonyl)-2-[3-(3-methylpyrazin-2-yl)isoxazol-5-yl]pyrrolidine (2.6g, 7.9mmol) in DCM (100ml) and the mixture stirred for 18 hours at ambient temperature. The volatiles were removed by evaporation and the residue dissolved in water. The aqueous mixture was adjusted to pH10-11 with 40% aqueous sodium hydroxide solution and extracted with DCM. The extracts were combined, dried (MgSO₄) and the solvent removed by evaporation to give S-2-[3-(3-methylpyrazin-2-yl)isoxazol-5-yl]pyrrolidine (1.69g, 93%) as an oil; NMR Spectrum (CDCl₃) 1.81-2.04 (m, 3H), 2.17 (s, 1H), 2.19-2.32 (m, 1H), 2.91 (s, 3H), 3.03-3.19 (m, 2H), 4.41-4.50 (m, 1H), 6.77 (s, 1H), 8.51 (s, 2H); Mass Spectrum 231 [MH]+.

### Example 32

### S-6-Methoxy-2-[2-{3-(3-methylpyrazin-2-yl)isoxazol-5-yl}pyrrolidin-1-yl]-4-(5-methyl-1H-pyrazol-3-ylamino)pyrimidine

A mixture of S-6-chloro-2-[2-{3-(3-methylpyrazin-2-yl)isoxazol-5-yl}pyrrolidin-1-yl]-4-(5-methyl-1*H-*pyrazol-3-ylamino)pyrimidine (218mg, 0.5mmol) and sodium methoxide (1.0ml of a 25% solution in methanol, 2.5mmol) in dry methanol (4ml) was heated in a sealed vessel at 100°C under microwave radiation for 2 hours. The mixture was allowed to cool and the volatiles removed by evaporation. The residue was dissolved in EtOAc, washed with water, dried (Na₂SO₄) and the solvent removed by evaporation. The residue was purified by chromatography on silica gel eluting with methanol/DCM (3:97). The purified product was triturated with DCM/hexane to give the title compound (85mg, 39%) 87% ee; NMR Spectrum 2.04-2.11 (m, 2H), 2.13-2.20 (m, 1H), 2.16 (s, 3H), 2.36-2.45 (m, 1H), 2.77 (s, 3H), 3.66-3.82 (m, 2H), 3.73 (s, 3H), 5.42-5.49 (m, 1H), 5.80 (s, 1H), 5.99 (s, 1H), 6.69 (s, 1H), 8.55-8.59 (m, 2H), 8.62 (s, 1H), 11.49 (s, 1H): Mass Spectrum 433 [MH]+.

### Example 33

### S-6-Ethyl-2-[2-{3-(3-methylpyrazin-2-yl)isoxazol-5-yl}pyrrolidin-1-yl]-4-(5-methyl-1H-pyrazol-3-ylamino)pyrimidine

A mixture of 2-chloro-6-ethyl-4-(5-methyl-1*H*-pyrazol-3-ylamino)pyrimidine (200mg, 0.84mmol), S-2-[3-(3-methylpyrazin-2-yl)isoxazol-5-yl]pyrrolidine (213 mg, 0.93mmol) and DIPEA (0.35ml, 2.6mmol) in 1-hexanol (4ml) was heated under microwave irradiation at 130°C for 3 hours and then at 150°C for 1 hour. The mixture was allowed to cool and the solid product collected by filtration, washed with ether and dried to give the title compound (115mg, 32%); NMR Spectrum 1.12 (t, 3H), 2.02-2.08 (m, 2H), 2.09-2.21 (m, 1H), 2.16 (s, 3H), 2.34-2.43 (m, 3H), 2.75 (s, 3H), 3.66-3.81 (m, 2H), 5.44-5.51 (m, 1H), 6.06 (s, 1H), 6.21 (s, 1H), 6.55 (s, 1H), 8.52-8.60 (m, 2H), 8.71 (s, 1H), 11.49 (s, 1H); Mass Spectrum 431 [MH+].

The 2-chloro-6-ethyl-4-(5-methyl-1*H*-pyrazol-3-ylamino)pyrimidine starting material was prepared as follows:

A mixture of 2,4-dichloro-6-ethylpyrimidine (4.0g, 22.6mmol), 3-amino-5-methylpyrazole (2.19g, 22.6mmol) and sodium carbonate (2.88g, 27.1mmol) in ethanol (100ml) was heated at 40°C for 4 days. Insoluble material was removed from the hot mixture by filtration and the solvent removed from the resulting filtrate. The residue was purified by chromatography on silica gel eluting with hexane/EtOAc (50:50 in creasing in polarity to 0:100). The purified product was triturated with ether to give 2-chloro-6-ethyl-4-(5-methyl-1*H*-pyrazol-3-ylamino)pyrimidine (667mg, 12%) as a white solid; NMR Spectrum 1.18 (t, 3H), 2.21 (s, 3H), 2.55 (q, 2H), 6.03 (s, 1H), 6.95 (s, 1H); Mass Spectrum 238 [MH]+.

### Example 34

### S-6-Ethyl-2-[2-{3-(2-cyanopyrid-3-yl)isoxazol-5-yl}pyrrolidin-1-yl]-4-(5-methyl-1H-pyrazol-3-ylamino)pyrimidine

A mixture of 2-chloro-6-ethyl-4-(5-methyl-1*H*-pyrazol-3-ylamino)pyrimidine (200mg, 0.84mmol), S-2-[3-(2-cyanopyrid-3-yl)isoxazol-5-yl]pyrrolidine (223 mg, 0.93mmol) and DIPEA (0.35ml, 2.6mmol) in 1-hexanol (4ml) was heated under microwave irradiation at 150°C for 3 hours. The mixture was allowed to cool and the volatiles removed by evaporation. The residue was purified by chromatography on silica gel eluting with EtOAc/methanol (95:5) to give the title compound (68mg, 18%); NMR Spectrum 1.12 (t, 3H), 2.04-2.22 (m, 3H), 2.17 (s, 3H), 2.32-2.44 (m, 3H), 3.70-3.83 (m, 2H), 5.46-5.51 (m, 1H), 6.04 (s, 1H), 6.23 (s, 1H), 6.82 (s, 1H), 7.79-7.87 (m, 1H), 8.312 (d, 1H), 8.72 (s, 1H), 8.81-8.87 (m, 1H) 11.47 (s, 1H); Mass Spectrum 442 [MH]+.

### Example 35

### S-6-(2-Methoxyethoxy)-2-[2-{3-(3-methylpyrazin-2-yl)isoxazol-5-yl}pyrrolidin-1-yl]-4-(5-methyl-1H-pyrazol-3-ylamino)pyrimidine

Sodium hydride (140mg of a 60% suspension in oil, 3.43mmol) was added to 2-methoxyethanol (18ml) and the mixture was stirred for 10 minutes while being purged with nitrogen. S-6-chloro-2-{2-[3-(3-methylpyrazin-2-yl)isoxazol-5-yl]pyrrolidin-1-yl}-4-(5-methyl-1*H*-pyrazol-3-ylamino)pyrimidine (300mg, 0.69mmol) was added and the mixture heated at 120°C in a sealed vessel under microwave irradiation for 3 hours. The mixture was allowed to cool, diluted with water and extracted with EtOAc. The extracts were combined washed with water and then saturated aqueous sodium chloride solution, dried (Na₂SO₄) and the solvent removed by evaporation. The residue was purified by chromatography on silica gel eluting with EtOAc/hexanes/methanol (50:50:0 increasing in polarity to 95:0:5). The purified product was triturated with DCM/hexane, collected by filtration and dried to give the title compound (8mg, 2.4%); NMR Spectrum 2.02-2.11 (m, 2H), 2.12-2.20 (m, 1H), 2.16 (s, 3H), 2.34-2.49 (m, 1H), 2.76 (s, 3H), 3.23 (s, 3H), 3.48-3.57 (m, 2H), 3.64-3.82 (m, 2H), 4.26 (t, 2H), 5.39-5.45 (m, 1H), 5.78 (s, 1H), 5.98 (s, 1H), 6.69 (s, 1H), 8.51-8.56 (m, 2H), 8.65 (s, 1H), 11.50 (s, 1H); Mass Spectrum 477 [MH]+.

### Example 36

### S-6-(2-Ethoxy)-2-[2-{3-(3-methylpyrazin-2-yl)isoxazol-5-yl}pyrrolidin-1-yl]-4-(5-methyl-1H-pyrazol-3-ylamino)pyrimidine

A mixture of S-6-chloro-2-{2-[3-(3-methylpyrazin-2-yl)isoxazol-5-yl]pyrrolidin-1-yl}-4-(5-methyl-1*H*-pyrazol-3-ylamino)pyrimidine (200mg, 0.46mmol) and sodium ethoxide (155 mg, 2.29mmol) in anhydrous ethanol (18ml) was heated at 120°C in a sealed vessel under microwave irradiation for 3 hours. The mixture was allowed to cool, diluted with water and extracted with EtOAc. The extracts were combined washed with water and then saturated aqueous sodium chloride solution, dried (Na₂SO₄ and the solvent removed by evaporation. The residue was purified by chromatography on silica gel eluting with EtOAc/hexanes/methanol (50:50:0 increasing in polarity to 95:0:5). The purified product was triturated with ether/hexane, collected by filtration and dried to give the title compound (22mg, 11%); NMR Spectrum 1.19 (t, 3H), 2.03-2.11 (m, 2H), 2.12-2.21 (m, 1H), 2.16 (s, 3H), 2.31-2.46 (m, 1H, m), 2.76 (s, 3H), 3.65-3.80 (m, 2H), 4.19 (q, 2H), 5.40-5.56 (m, 1H), 5.78 (s, 1H), 5.96 (s, 1H), 6.69 (s, 1H, ), 8.51-8.60 (m, 3H), 11.49 (s, 1H); Mass Spectrum 477 [MH]+.

### Example 37

### S-6-(3-Methoxypropoxy)-2-[2-{3-(3-methylpyrazin-2-yl)isoxazol-5-yl}pyrrolidin-1-yl]-4-(5-methyl-1H-pyrazol-3-ylamino)pyrimidine

Sodium hydride (92mg of a 60% suspension in oil, 2.29mmol) was added to 3-methoxypropanol (18ml) and the mixture was stirred for 10 minutes while being purged with nitrogen. S-6-chloro-2-{2-[3-(3-methylpyrazin-2-yl)isoxazol-5-yl]pyrrolidin-1-yl}-4-(5-methyl-1*H*-pyrazol-3-ylamino)pyrimidine (200mg, 0.46mmol) was added and the mixture heated at 120°C in a sealed vessel under microwave irradiation for 3 hours. The mixture was allowed to cool, diluted with water and extracted with EtOAc. The extracts were combined washed with water and then saturated aqueous sodium chloride solution, dried (Na₂SO₄) and the solvent removed by evaporation. The residue was purified by chromatography on silica gel eluting with EtOAc/hexanes/methanol (50:50:0 increasing in polarity to 98:0:2) to give the title compound (33mg, 15%); NMR Spectrum 1.79-1.86 (m, 2H), 2.03-2.11 (m, 2H), 2.12-2.19 (m, 1H), 2.16 (s, 3H), 2.36-2.45 (m, 1H), 2.76 (s, 3H), 3.19 (s, 3H), 3.37 (t, 2H), 3.66-3.81 (m, 2H), 4.18 (t, 2H), 5.40-5.46 (m, 1H), 5.79 (s, 1H), 5.99 (s, 1H), 6.70 (s, 1H), 8.53-8.65 (m, 3H), 11.49 (s, 1H); Mass Spectrum 492 [MH]+.

### Example 38

### S-6-(2-Ethoxyethoxy)-2-[2-{3-(3-methylpyrazin-2-yl)isoxazol-5-yl}pyrrolidin-1-yl]-4-(5-methyl-1H-pyrazol-3-ylamino)pyrimidine

Sodium hydride (92mg of a 60% suspension in oil, 2.29mmol) was added to 2-ethoxyethanol (18ml) and the mixture was stirred for 10 minutes while being purged with nitrogen. S-6-chloro-2-{2-[3-(3-methylpyrazin-2-yl)isoxazol-5-yl]pyrrolidin-1-yl}-4-(5-methyl-1*H*-pyrazol-3-ylamino)pyrimidine (200mg, 0.46mmol) was added and the mixture heated at 120°C in a sealed vessel under microwave irradiation for 3 hours. The mixture was allowed to cool, diluted with water and extracted with EtOAc. The extracts were combined washed with water and then saturated aqueous sodium chloride solution, dried (Na₂SO₄) and the solvent removed by evaporation. The residue was purified by chromatography on silica gel eluting with EtOAc/hexanes/methanol (50:50:0 increasing in polarity to 98:0:2). The purified product was triturated with ether/hexane, collected by filtration and dried to give the title compound (62mg, 27%); NMR Spectrum 1.06 (t, 3H), 2.04-2.11 (m, 2H), 2.12-2.20 (m, 1H), 2.16 (s, 3H), 2.34-2.44 (m, 1H), 2.75 (s, 3H), 3.41 (q, 2H), 3.51-3.61 (m, 2H), 3.66-3.79. (m, 2H), 4.24 (t, 2H), 5.40-5.44 (m, 1H), 5.80 (s, 1H), 5.98 (s, 1H), 6.69 (s, 1H), 8.51-8.55 (m, 2H), 8.60 (s, 1H), 11.48 (s, 1H); Mass Spectrum 492 [MH]+.

### Example 39

### S-6-[(1-cyanocyclopropyl)methoxyl-2-[2-{3-(3-methylpyrazin-2-yl)isoxazol-5-yl}pyrrolidin-1-yl]-4-(5-methyl-1H-pyrazol-3-ylamino)pyrimidine

Sodium hydride (100mg of a 60% suspension in oil, 2.5mmol) was added to 1-cyanocyclopropyl)methanol (prepared as described in Example 22 of US-5, 859,014) (245mg, 2.5mmol) in dry THF (18ml) and the mixture was stirred for 10 minutes while being purged with nitrogen. S-6-chloro-2-{2-[3-(3-methylpyrazin-2-yl)isoxazol-5-yl]pyrrolidin-1-yl}-4-(5-methyl-1*H*-pyrazol-3-ylamino)pyrimidine (218mg, 0.5mmol) was added and the mixture heated at 120°C in a sealed vessel under microwave irradiation for 3 hours. The mixture was allowed to cool, diluted with water and extracted with EtOAc. The extracts were combined washed with water and then saturated aqueous sodium chloride solution, dried (Na₂SO₄) and the solvent removed by evaporation. The residue was purified by chromatography on silica gel eluting with EtOAc/hexanes/methanol (50:50:0 increasing in polarity to 98:0:2). The purified product was triturated with DCM/hexane, collected by filtration and dried to give the title compound (15mg, 6%); NMR Spectrum 1.01-1.13 (m, 2H), 1.19-1.38 (m, 2H), 2.04-2.11 (m, 2H), 2.12-2.20 (m, 1H), 2.16 (s, 3H), 2.34-2.44 (m, 1H), 2.76 (s, 3H), 3.68-3.80 (m, 2H), 4.16 (d, 1H), 4.28 (d, 1H), 5.37-5.42 (m, 1H), 5.81 (s, 1H), 5.98 (s, 1H), 6.69 (s, 1H), 8.51-8.56 (m, 2H), 8.69 (s, 1H), 11.48 (s, 1H); Mass Spectrum 499 [MH]+.

### Example 40

### S-6-(3-Cyanopropoxy)-2-[2-{3-(3-methylpyrazin-2-yl)isoxazol-5-yl)pyrrolidin-1-yl]-4-(5-methyl-1H-pyrazol-3-ylamino)pyrimidine

Sodium hydride (100mg of a 60% suspension in oil, 2.5mmol) was added to 3-cyanopropanol (prepared as described in Chem. Abs. 1950, 44, 4460b) (212mg, 2.5mmol) in dry THF (18ml) and the mixture was stirred for 10 minutes while being purged with nitrogen. S-6-chloro-2-{2-[3-(3-methylpyrazin-2-yl)isoxazol-5-yl]pyrrolidin-1-yl}-4-(5-methyl-1*H-*pyrazol-3-ylamino)pyrimidine (218mg, 0.5mmol) was added and the mixture heated at 120°C in a sealed vessel under microwave irradiation for 3 hours. The mixture was allowed to cool, diluted with water and extracted with EtOAc. The extracts were combined washed with water and then saturated aqueous sodium chloride solution, dried (Na₂SO₄) and the solvent removed by evaporation. The residue was purified by chromatography on silica gel eluting with EtOAc/hexanes/methanol (50:50:0 increasing in polarity to 98:0:2). The purified product was triturated with DCM/hexane, collected by filtration and dried to give the title compound (8mg, 3%) 67%ee; NMR Spectrum 1.88-1.96 (m, 2H), 2.04-2.11 (m, 2H), 2.12-2.20 (m, 1H), 2.16 (s, 3H), 2.36-2.44 (m, 1H), 2.47-2.55 (m, 2H), 2.76 (s, 3H), 3.67-3.81 (m, 2H), 4.23 (t, 2H), 5.40-5.45 (m, 1H), 5.78 (s, 1H), 5.98 (s, 1H), 6.70 (s, 1H), 8.52-8.58 (m, 2H), 8.66 (s, 1H), 11.49 (s, 1H); Mass Spectrum 487 [MH]+.

### Example 41

### S-6-Methoxy-2-[2-{3-(3-methoxypyrazin-2-yl)isoxazol-5-yl}pyrrolidin-1-yl]-4-(5-methyl-1H-pyrazol-3-ylamino)pyrimidine

A mixture of S-6-chloro-2-[2-{3-(3-methoxypyrazin-2-yl)isoxazol-5-yl}pyrrolidin-1-yl]-4-(5-methyl-1*H*-pyrazol-3-ylamino)pyrimidine (prepared as described in Example 162 of WO2005/040159) (200mg, 0.45mmol) and sodium methoxide (1.0ml of a 25% solution in methanol, 2.5mmol) in dry methanol (10ml) was heated in a sealed vessel at 120°C under microwave radiation for 3 hours. More sodium methoxide (0.5ml of a 25% solution in methanol, 1.25mmol) was added and the mixture heated under microwave radiation for a further 4 hours at 120°C. The mixture was allowed to cool and the volatiles removed by evaporation. The residue was dissolved in EtOAc, washed with water and then brine, dried (Na₂SO₄) and the solvent removed by evaporation. The residue was purified by chromatography on silica gel eluting with EtOAc/hexanes (50:50 increasing in polarity to 100:0). The purified product was triturated with DCM/hexane collected by filtration and dried to give the title compound (19mg, 9%); NMR Spectrum 2.00-2.20 (m, 3H), 2.18 (s, 3H), 2.30-2.50 (m, 1H), 3.65-3.80 (m, 1H), 3.70 (s, 3H), 4.00 (s, 3H), 5.40-5.45 (t, 1H), 5.70-5.85 (br s, 1H), 5.85-6.00 (br s, 1H), 6.65 (s, 1H), 8.30 (t, 2H), 8.50-8.70 (br s, 1H), 11.45-11.60 (br s, 1H); Mass Spectrum 450 [MH]+.

### Example 42

### S-6-(2-Methoxyethoxy)-2-[2-{3-(3-(2-methoxyethoxy)pyrazin-2-yl)isoxazol-5-yl}pyrrolidin-1-yl]-4-(5-methyl-1H-pyrazol-3-ylamino)pyrimidine

Sodium hydride (150mg of a 40% suspension in oil, 3.75mmol) was added to 2-methoxyethanol (10ml) and the mixture was stirred for 15 minutes while being purged with nitrogen. S-6-chloro-2-{2-[3-(3-methoxypyrazin-2-yl)isoxazol-5-yl]pyrrolidin-1-yl}-4-(5-methyl-1*H*-pyrazol-3-ylamino)pyrimidine (prepared as described in Example 162 of WO2005/040159) (200mg, 0.44mmol) was added and the mixture heated at 120°C in a sealed vessel under microwave irradiation for 5 hours. The mixture was allowed to cool and poured onto a 20g isolute SCX ion exchange column. The column was eluted with methanol to elute any neutrals, followed by 2M methanolic ammonia to elute the product. The solvent was removed by evaporation and the residue was purified by chromatography on silica gel eluting with EtOAc/hexanes/methanol (50:50:0 increasing in polarity to 95:0:5). The purified product was triturated with DCM/hexane, collected by filtration and dried to give the title compound (21mg, 9%); NMR Spectrum 2.00-2.20, (m, 3H), 2.18 (s, 3H), 2.30-2.50 (m, 1H), 3.20 (s, 3H), 3.30 (s, 3H), 3.48-3.60 (m, 2H), 3.60-3.80 (m, 4H), 4.20-4.30 (t, 2H), 4.50-4.60 (t, 2H), 5.40-5.45 (d, 1H), 5.75 (s, 1H), 5.95 (s, 1H), 6.70 (s, 1H), 8.25-8.35 (dd, 2H), 8.50-8.70 (brs, 1H), 11.40-11.60 (br s, 1H); Mass Spectrum 538 [MH]+.

### Example 43

### S-6-(2-Methoxyethoxy)-2-[2-{3-(3-hydroxypyrazin-2-yl)isoxazol-5-yl}pyrrolidin-1-yl]-4-(5-methyl-1H-pyrazol-3-ylamino)pyrimidine

The title compound was isolated as the second product from the preparation of Example 39 (12mg, 6%); NMR Spectrum 2.00-2.20, (m, 3H), 2.18 (s, 3H), 2.30-2.50 (m, 1H), 3.20 (s, 3H), 3.48-3.60 (m, 2H), 3.60-3.80 (m, 2H), 4.20-4.30 (t, 2H), 5.35-5.45 (d, 1H), 5.70-5.85 (br s, 1H), 5.85-6.00 (br s, 1H), 6.72 (s, 1H), 7.45-7.55 (m, 2H), 8.55-8.65 (br s, 1H), 11.60-12.00 (br s, 1H); Mass Spectrum 480 [MH]+.

### Example 44

### S-6-(2-Methoxyethoxy)-2-[2-{3-(4-cyanopyrid-3-yl)isoxazol-5-yl}pyrrolidin-1-yl]-4-(5-methyl-1H-pyrazol-3-ylamino)pyrimidine

The title compound was isolated as the second product from the preparation of Example 26 (5mg, 2%); NMR Spectrum 2.03-2.15 (m, 3H), 2.19 (s, 3H), 2.38-2.47 (m, 1H), 3.25 (s, 3H), 3.53 (t, 2H), 3.67-3.81 (m, 2H), 4.27 (t, 2H), 5.41 (d, 1H), 5.79 (s, 1H), 5.97 (s, 1H), 6.97 (s, 1H), 8.09 (d, 1H), 8.44 (d, 1H), 8.62 (s, 1H), 9.17 (s, 1H), 11.5 (s,1H); Mass Spectrum 488 [MH]+.

### Example 45

### S-6-Methoxy-2-[2-{3-(2-cyanopyrid-3-yl)isoxazol-5-yl}pyrrolidin-1-yl]-4-(5-methyl-1H-pyrazol-3-ylamino)pyrimidine

The title compound was isolated as the second product from the preparation of Example 30 (8mg, 2%); NMR Spectrum 2.01-2.10 (m, 3H), 2.09-2.20 (m, 3H), 2.32-2.40 (m, 1H), 3.52-3.68 (m, 2H), 3.74-3.82 (m, 3H), 5.39 (d, 1H), 5.79 (s, 1H), 6.17 (s, 1H), 7.10 (s, 1H), 8.15 (d, 1H), 8.48 (dd, 1H), 9.10 (s, 1H), 9.20 (s, 1H), 11.74 (s, 1H); Mass Spectrum 444 [MH]+.

## Claims

1. A compound of formula (I): 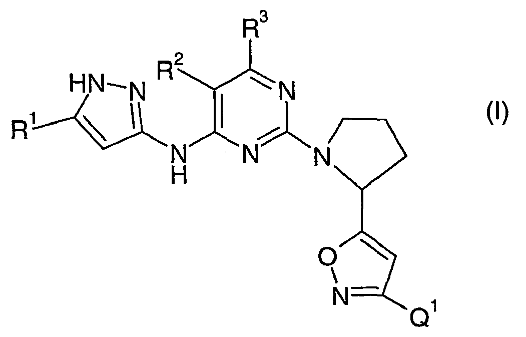 wherein:
**R¹** is selected from methyl, ethyl, isopropyl and cyclopropyl;
**R²** is selected from hydrogen and halogeno;
**R³** is selected from hydrogen, hydroxy and halogeno, or from a (C1-C6)alkyl, (C2-C6)alkenyl, (C2-C6)alkynyl, (C1-C6)alkoxy, (C1-C6)alkylcarbonyl, (C1-C6)alkoxycarbonyl, amino, (C1-C6)alkylamino, di-[(C1-C6)alkyl]amino, (C3-C8)cycloalkylamino, carbamoyl, (C1-C6)alkylcarbamoyl, di-[(C1-C6)alkyl]carbamoyl, -C(O)R^{3b}, -NHR^{3b}, -SR^{3a} or - N(R^{3c})C(O)R^{3a} group, wherein R^{3a} is selected from a (C1-C6)alkyl or (C1-C6)alkoxy group, R^{3b} is a saturated monocyclic 4-, 5- or 6-membered heterocyclic ring comprising at least one ring heteroatom selected from nitrogen, oxygen and sulfur and R^{3c} is selected from hydrogen and (C1-C6)alkyl,
or R³ is a saturated monocyclic 5- or 6-membered heterocyclic ring comprising at least one ring heteroatom selected from nitrogen, oxygen and sulfur,
each of which groups or rings within R³ may be optionally substituted by one or more substituents independently selected from (C1-C6)alkyl, (C1-C6)alkoxy, (C1-C6)alkoxy(C1-C6)alkyl, (C1-C6)alkoxy(C1-C6)alkoxy, halogeno, hydroxy, trifluoromethyl, tri-[(C1-C4)alkyl]silyl, cyano, amino, (C1-C6)alkylamino, di-[(C1-C6)alkyl]amino, amino(C1-C6)alkyl, (C1-C6)alkylamino(C1-C6)alkyl, di-[(C1-C6)alkyl]amino(C1-C6)alkyl, (C1-C6)alkoxycarbonyl, carbamoyl, (C1-C6)alkylcarbamoyl, di-[(C1-C6)alkyl]carbamoyl, (C1-C6)alkylthio, (C1-C6)alkylsulfonyl, (C1-C6)alkylsulfinyl, (C1-C6)alkanoyl, an alkanoylamino group -N(R^{3d})C(O)R^{3e} wherein R^{3d} is selected from hydrogen and (C1-C6)alkyl and R^{3e} is selected from a (C1-C6)alkyl or (C1-C6)alkoxy group, or a saturated monocyclic 3-, 4-, 5-, 6- or 7-membered ring, which ring may optionally comprise one or more heteroatoms selected from nitrogen, oxygen and sulfur, any of which substituents may be optionally substituted by one or more (C1-C4)alkyl, hydroxy or cyano groups;
**Q¹** is a 5- or 6-membered heteroaromatic ring comprising at least one ring heteroatom selected from nitrogen, oxygen and sulfur,
and wherein Q¹ is substituted by one or more substituents independently selected from (C1-C6)alkyl and (C1-C6)alkoxy (either of which (C1-C6)alkyl and (C1-C6)alkoxy groups may be optionally substituted by one or more substituents independently selected from (C1-C4)alkoxy, halogeno, amino, hydroxy and trifluoromethyl), oxo, halogeno, nitro, cyano, -NR⁴R⁵, carboxy, hydroxy, (C2-C6)alkenyl, (C3-C8)cycloalkyl, (C1-C6)alkoxycarbonyl, (C1-C6)alkylcarbonyl, (C2-C6)alkanoylamino, phenylcarbonyl, -S(O)ₙ(C1-C6)alkyl, -C(O)NR⁶R⁷ and -SO₂NR⁸R⁹, wherein R⁴, R⁵, R⁶, R⁷, R⁸ and R⁹ are each independently selected from hydrogen and (C1-C6)alkyl, or R⁴ and R⁵, or R⁶ and R⁷ or R⁸ and R⁹, when taken together with the nitrogen atom to which they are attached, may each independently form a saturated heterocyclic ring and n is 0, 1 or 2,
and wherein any saturated monocyclic ring optionally bears 1 or 2 oxo or thioxo substituents;
or a pharmaceutically-acceptable salt thereof,
provided that the compound of formula (I) is not:
5-chloro-2-{2-[3-(2-methoxypyrid-3-yl)isoxazol-5-yl]pyrrolidin-1-yl}-4-(5-methyl-1*H-*pyrazol-3-ylamino)pyrimidine;
S-5-chloro-2-{2-[3-(2-methoxypyrid-3-yl)isoxazol-5-yl]pyrrolidin-1-yl}-4-(5-methyl-1*H-*pyrazol-3-ylamino)pyrimidine;
S-4-(5-ethyl-1*H*-pyrazol-3-ylamino)-2-[2-[3-(2-methoxypyrid-3-yl)isoxazol-5-yl]pyrrolidin-1-yl]pyrimidine;
S-4-(5-methyl-1H-pyrazol-3-ylamino)-2-[2-[3-(2-methoxypyrid-3-yl)isoxazol-5-yl]pyrrolidin-1-yl]pyrimidine;
S-4-(5-cyclopropyl-1H-pyrazol-3-ylamino)-2-[2-[3-(2-methoxypyrid-3-yl)isoxazol-5-yl]pyrrolidin-1-yl]pyrimidine;
S-6-chloro-4-(5-methyl-1*H-*pyrazol-3-ylamino)-2-[2-{3-(3-methoxypyrazin-2-yl)isoxazol-5-yl }pyrrolidin-1-yl]pyrimidine;
S-6-morpholino-4-(5-methyl-1*H*-pyrazol-3-ylamino)-2-[2-{3-(3-methoxypyrazin-2-yl)isoxazol-5-yl}pyrrolidin-1-yl]pyrimidine;
S-6-morpholino-4-(5-methyl-1*H*-pyrazol-3-ylamino)-2-[2-{3-(3-hydroxypyrazin-2-yl)isoxazol-5-yl}pyrrolidin-1-yl]pyrimidine;
S-6-methyl-4-(5-methyl-1*H*-pyrazol-3-ylamino)-2-[2-{3-(3-methoxypyrazin-2-yl)isoxazol-5-yl}pyrrolidin-1-yl]pyrimidine;
S-6-methyl-4-(5-ethyl-1*H*-pyrazol-3-ylamino)-2-[2-{3-(3-methoxypyrazin-2-yl)isoxazol-5-yl }pyrrolidin-1-yl]pyrimidine;
S-6-chloro-4-(5-ethyl-1*H*-pyrazol-3-ylamino)-2-[2-{3-(3-methoxypyrazin-2-yl)isoxazol-5-yl}pyrrolidin-1-yl]pyrimidine;
S-6-methyl-4-(5-methyl-1*H*-pyrazol-3-ylamino)-2-[2-[3-(2-methoxypyrid-3-yl)isoxazol-5-yl]pyrrolidin-1-yl]pyrimidine;
S-5-fluoro-4-(5-cyclopropyl-1*H*-pyrazol-3-ylamino)-2-[2-[3-(2-methoxypyrid-3-yl)isoxazol-5-yl]pyrrolidin-1-yl]pyrimidine;
S-5-fluoro-4-(5-ethyl-1*H*-pyrazol-3-ylamino)-2-[2-[3-(2-methoxypyrid-3-yl)isoxazol-5-yl]pyrrolidin-1-yl]pyrimidine;
S-6-(2-hydroxyethoxy)-4-(5-methyl-1*H*-pyrazol-3-ylamino)-2-[2-[3-(2-methoxypyrid-3-yl)isoxazol-5-yl]pyrrolidin-1-yl]pyrimidine;
S-6-chloro-4-(5-cyclopropyl-1*H*-pyrazol-3-ylamino)-2-[2-[3-(2-methoxypyrid-3-yl)isoxazol-5-yl]pyrrolidin-1-yl]pyrimidine;
S-6-chloro-4-(5-ethyl-1*H*-pyrazol-3-ylamino)-2-[2-[3-(2-methoxypyrid-3-yl)isoxazol-5-yl]pyrrolidin-1-yl]pyrimidine;
S-6-(2-hydroxyethoxy)-4-(5-cyclopropyl-1*H*-pyrazol-3-ylamino)-2-[2-[3-(2-methoxypyrid-3-yl)isoxazol-5-yl]pyrrolidin-1-yl]pyrimidine;
S-5-fluoro-4-(5-methyl-1*H*-pyrazol-3-ylamino)-2-[2-[3-(2-methoxypyrid-3-yl)isoxazol-5-yl]pyrrolidin-1-yl]pyrimidine;
S-6-(2-hydroxyethoxy)-4-(5-ethyl-1*H*-pyrazol-3-ylamino)-2-[2-[3-(2-methoxypyrid-3-yl)isoxazol-5-yl]pyrrolidin-1-yl]pyrimidine;
S-6-methyl-4-(5-cyclopropyl-1*H*-pyrazol-3-ylamino)-2-[2-{3-(3-methoxypyrazin-2-yl)isoxazol-5-yl}pyrrolidin-1-yl]pyrimidine;
S-6-morpholino-4-(5-ethyl-1H-pyrazol-3-ylamino)-2-[2-{3-(3-methoxypyrazin-2-yl)isoxazol-5-yl }pyrrolidin-1-yl]pyrimidine;
S-6-chloro-4-(5-methyl-1H-pyrazol-3-ylamino)-2-[2-[3-(2-methoxypyrid-3-yl)isoxazol-5-yl]pyrrolidin-1-yl]pyrimidine; or
S-6-morpholino-4-(5-ethyl-1H-pyrazol-3-ylamino)-2-[2-{3-(3-hydroxypyrazin-2-yl)isoxazol-5-yl }pyrrolidin-1-yl]pyrimidine.

2. A compound of formula (I) according to claim 1, wherein:
R¹ is selected from methyl, ethyl, isopropyl and cyclopropyl;
R² is selected from hydrogen and halogeno;
R³ is selected from hydrogen, hydroxy and halogeno, or from a (C1-C6)alkyl, (C2-C6)alkenyl, (C2-C6)alkynyl, (C1-C6)alkoxy, (C1-C6)alkylcarbonyl, (C1-C6)alkoxycarbonyl, amino, (C1-C6)alkylamino, di-[(C1-C6)alkyl]amino, (C3-C8)cycloalkylamino, carbamoyl, (C1-C6)alkylcarbamoyl, di-[(C1-C6)alkyl]carbamoyl, -C(O)R^{3b}, -NHR^{3b}, -SR^{3a} or - N(R^{3c})C(O)R^{3a} group, wherein R^{3a} is selected from a (C1-C6)alkyl or (C1-C6)alkoxy group, R^{3b} is a saturated monocyclic 4-, 5- or 6-membered heterocyclic ring comprising at least one ring heteroatom selected from nitrogen, oxygen and sulfur and R^{3c} is selected from hydrogen and (C1-C6)alkyl,
or R³ is a saturated monocyclic 5- or 6-membered heterocyclic ring comprising at least one ring heteroatom selected from nitrogen, oxygen and sulfur,
each of which groups or rings within R³ may be optionally substituted by one or more substituents independently selected from (C1-C6)alkyl, (C1-C6)alkoxy, (C1-C6)alkoxy(C1-C6)alkyl, (C1-C6)alkoxy(C1-C6)alkoxy, halogeno, hydroxy, trifluoromethyl, tri-[(C1-C4)alkyl]silyl, cyano, amino, (C1-C6)alkylamino, di-[(C1-C6)alkyl]amino, amino(C1-C6)alkyl, (C1-C6)alkylamino(C1-C6)alkyl, di-[(C1-C6)alkyl]amino(C1-C6)alkyl, (C1-C6)alkoxycarbonyl, carbamoyl, (C1-C6)alkylcarbamoyl, di-[(C1-C6)alkyl]carbamoyl, (C1-C6)alkylthio, (C1-C6)alkylsulfonyl, (C1-C6)alkylsulfinyl, (C1-C6)alkanoyl, an alkanoylamino group -N(R^{3d})C(O)R^{3e} wherein R^{3d} is selected from hydrogen and (C1-C6)alkyl and R^{3e} is selected from a (C1-C6)alkyl or (C1-C6)alkoxy group, or a saturated monocyclic 3-, 4-, 5-, 6- or 7-membered ring, which ring may optionally comprise one or more heteroatoms selected from nitrogen, oxygen and sulfur, any of which substituents may be optionally substituted by one or more (C1-C4)alkyl, hydroxy or cyano groups;
Q¹ is a 5- or 6-membered heteroaromatic ring comprising at least one ring heteroatom selected from nitrogen, oxygen and sulfur,
and wherein Q¹ is substituted by one or more substituents independently selected from (C1-C6)alkyl and (C1-C6)alkoxy (either of which (C1-C6)alkyl and (C1-C6)alkoxy groups may be optionally substituted by one or more substituents independently selected from halogeno, amino, hydroxy and trifluoromethyl), halogeno, nitro, cyano, -NR⁴R⁵, carboxy, hydroxy, (C2-C6)alkenyl, (C3-C8)cycloalkyl, (C1-C6)alkoxycarbonyl, (C1-C6)alkylcarbonyl, (C2-C6)alkanoylamino, phenylcarbonyl, -S(O)ₙ(C1-C6)alkyl, -C(O)NR⁶R⁷ and-SO₂NR⁸R⁹, wherein R⁴, R⁵, R⁶, R⁷, R⁸ and R⁹ are each independently selected from hydrogen and (C1-C6)alkyl, or R⁴ and R⁵, or R⁶ and R⁷, or R⁸ and R⁹, when taken together with the nitrogen atom to which they are attached, may each independently form a saturated heterocyclic ring and n is 0, 1 or 2,
and wherein any saturated monocyclic ring optionally bears 1 or 2 oxo or thioxo substituents;
or a pharmaceutically-acceptable salt thereof.

3. A compound of formula (I) according to claim 1 or 2, wherein R¹ is methyl or cyclopropyl.

4. A compound of formula (I) according to claim 3, wherein R¹ is methyl.

5. A compound of formula (I) according to any one or more of claims 1 to 4, wherein R² is hydrogen.

6. A compound of formula (I) according to any one or more of claims 1 to 5, wherein R³ is selected from hydrogen and halogeno, or from a (C1-C4)alkyl or (C1-C3)alkoxy group,
or R³ is a saturated monocyclic 5- or 6-membered heterocyclic ring comprising at least one ring heteroatom selected from nitrogen and oxygen,
each of which groups or rings within R³ may be optionally substituted by one or more substituents independently selected from (C1-C6)alkyl, (C1-C6)alkoxy, (C1-C6)alkoxy(C1-C6)alkyl, (C1-C6)alkoxy(C1-C6)alkoxy, halogeno, hydroxy, trifluoromethyl, tri-[(C1-C4)alkyl]silyl, cyano, amino, (C1-C6)alkylamino, di-[(C1-C6)alkyl]amino, amino(C1-C6)alkyl, (C1-C6)alkylamino(C1-C6)alkyl, di-[(C1-C6)alkyl]amino(C1-C6)alkyl, (C1-C6)alkoxycarbonyl, carbamoyl, (C1-C6)alkylcarbamoyl, di-[(C1-C6)alkyl]carbamoyl, (C1-C6)alkylthio, (C1-C6)alkylsulfonyl, (C1-C6)alkylsulfinyl, (C1-C6)alkanoyl, an alkanoylamino group -N(R^{3d})C(O)R^{3e} wherein R^{3d} is selected from hydrogen and (C1-C6)alkyl and R^{3e} is selected from a (C1-C6)alkyl or (C1-C6)alkoxy group, or a saturated monocyclic 3-, 4-, 5-, 6- or 7-membered ring, which ring may optionally comprise one or more heteroatoms selected from nitrogen, oxygen and sulfur, any of which substituents may be optionally substituted by one or more (C1-C4)alkyl, hydroxy or cyano groups;
and wherein any saturated monocyclic ring within R³ optionally bears 1 or 2 oxo or thioxo substituents.

7. A compound of formula (I) according to claim 6, wherein R³ is selected from hydrogen and halogeno, or from a (C1-C3)alkyl or (C1-C3)alkoxy group,
or R³ is a saturated monocyclic 6-membered heterocyclic ring comprising at least one ring heteroatom selected from nitrogen and oxygen,
each of which groups or rings within R³ may be optionally substituted by one or more substituents independently selected from (C1-C6)alkyl, (C1-C6)alkoxy, (C1-C6)alkoxy(C1-C6)alkyl, (C1-C6)alkoxy(C1-C6)alkoxy, halogeno, hydroxy, trifluoromethyl, tri-[(C1-C4)alkyl]silyl, cyano, amino, (C1-C6)alkylamino, di-[(C1-C6)alkyl]amino, amino(C1-C6)alkyl, (C1-C6)alkylamino(C1-C6)alkyl, di-[(C1-C6)alkyl]amino(C1-C6)alkyl, (C1-C6)alkoxycarbonyl, carbamoyl, (C1-C6)alkylcarbamoyl, di-[(C1-C6)alkyl]carbamoyl, (C1-C6)alkylthio, (C1-C6)alkylsulfonyl, (C1-C6)alkylsulfinyl, (C1-C6)alkanoyl, an alkanoylamino group -N(R^{3d})C(O)R^{3e} wherein R^{3d} is selected from hydrogen and (C1-C6)alkyl and R^{3e} is selected from a (C1-C6)alkyl or (C1-C6)alkoxy group, or a saturated monocyclic 3-, 4-, 5-, 6- or 7-membered ring, which ring may optionally comprise one or more heteroatoms selected from nitrogen, oxygen and sulfur, any of which substituents may be optionally substituted by one or more (C1-C4)alkyl, hydroxy or cyano groups;
and wherein any saturated monocyclic ring within R³ optionally bears 1 or 2 oxo or thioxo substituents.

8. A compound of formula (I) according to claim 7, wherein R³ is selected from hydrogen and halogeno, or from a (C1-C3)alkyl or (C1-C3)alkoxy group,
or R³ is a saturated monocyclic 6-membered heterocyclic ring comprising at least one ring heteroatom selected from nitrogen and oxygen,
each of which groups or rings within R³ may be optionally substituted by one or more substituents independently selected from cyano, hydroxy, cyclopropyl and (C1-C3)alkoxy, any of which substituents may be optionally substituted by one or more cyano groups.

9. A compound of formula (I) according to claim 8, wherein R³ is selected from hydrogen, chloro, methyl, ethyl, methoxy, ethoxy, 2-hydroxyethoxy, 2-methoxyethoxy, 2-hydroxypropoxy, (1-cyanocyclopropyl)methoxy, 3-cyanopropoxy, 3-hydroxypropoxy, 3-methoxypropoxy, 2-ethoxyethoxy and morpholino.

10. A compound of formula (I) according to any one or more of claims 1 to 9, wherein Q¹ is selected from pyridyl, imidazolyl, isoxazolyl, pyrazolyl, furyl, pyrazinyl, pyridazinyl, pyrimidinyl, pyrrolyl, thiazolyl, oxazolyl, isothiazolyl, triazolyl, tetrahydrofuranyl and thienyl,
and wherein Q¹ is substituted by one or more substituents independently selected from (C1-C6)alkyl and (C1-C6)alkoxy (either of which (C1-C6)alkyl and (C1-C6)alkoxy groups may be optionally substituted by at least one substituent independently selected from (C1-C3)alkoxy, halogeno, amino, hydroxy and trifluoromethyl), oxo, halogeno, nitro, cyano, -NR⁴R⁵, carboxy, hydroxy, (C2-C6)alkenyl, (C3-C8)cycloalkyl, (C1-C6)alkoxycarbonyl, (C1-C6)alkylcarbonyl, (C2-C6)alkanoylamino, phenylcarbonyl, -S(O)ₙ(C1-C6)alkyl, -C(O)NR⁶R⁷ and -SO₂NR⁸R⁹, wherein R⁴, R⁵, R⁶, R⁷, R⁸ and R⁹ are each independently selected from hydrogen and (C1-C6)alkyl, or R⁴ and R⁵, or R⁶ and R⁷, or R⁸ and R⁹, when taken together with the nitrogen atom to which they are attached, may each independently form a saturated heterocyclic ring and n is 0, 1 or 2,
and wherein any saturated monocyclic ring optionally bears 1 or 2 oxo or thioxo substituents.

11. A compound of formula (I) according to claim 10, wherein Q¹ is selected from pyridyl, imidazolyl, isoxazolyl, pyrazolyl, furyl, pyrazinyl, pyridazinyl, pyrimidinyl, pyrrolyl, thiazolyl, oxazolyl, isothiazolyl, triazolyl, tetrahydrofuranyl and thienyl,
and wherein Q¹ is substituted by at least one substituent independently selected from oxo, halogeno, hydroxy, cyano, (C1-C4)alkyl, (C1-C4)alkoxy, which (C1-C4)alkoxy may be optionally substituted by (C1-C3)alkoxy and -NR⁴R⁵, wherein R⁴ and R⁵ are each independently selected from hydrogen and (C1-C6)alkyl, or R⁴ and R⁵, when taken together with the nitrogen atom to which they are attached, may each independently form a saturated heterocyclic ring.

12. A compound of formula (I) according to claim 10 or 11, wherein Q¹ is selected from pyridyl and pyrazinyl.

13. A compound of formula (I) selected from one or more of:
S-2-[2-{3-(3-ethoxypyrazin-2-yl)isoxazol-5-yl}pyrrolidin-1-yl]-6-methyl-4-(5-methyl-1*H-*pyrazol-3-ylamino)pyrimidine;
S-2-[2-{3-(3-ethylaminopyrazin-2-yl)isoxazol-5-yl}pyrrolidin-1-yl]-6-methyl-4-(5-methyl-1*H*-pyrazol-3-ylamino)pyrimidine;
S-2-[2-{3-(2-methylpyrid-3-yl)isoxazol-5-yl}pyrrolidin-1-yl]-6-methyl-4-(5-methyl-1*H-*pyrazol-3-ylamino)pyrimidine;
S-6-chloro-2-[2-{3-(2-methylpyrid-3-yl)isoxazol-5-yl}pyrrolidin-1-yl]-4-(5-methyl-1*H-*pyrazol-3-ylamino)pyrimidine;
S-2-[2-{3-(2-chloropyrid-3-yl)isoxazol-5-yl}pyrrolidin-1-yl]-6-methyl-4-(5-methyl-1*H-*pyrazol-3-ylamino)pyrimidine;
S-6-methoxy-2-[2-{3-(2-methylpyrid-3-yl)isoxazol-5-yl}pyrrolidin-1-yl]-4-(5-methyl-1*H-*pyrazol-3-ylamino)pyrimidine;
S-2-[2-{3-(2-cyanopyrid-3-yl)isoxazol-5-yl}pyrrolidin-1-yl]-6-methyl-4-(5-methyl-1*H-*pyrazol-3-ylamino)pyrimidine;
S-2-[2-{3-(2-methylpyrid-3-yl)isoxazol-5-yl)pyrrolidin-1-yl]-4-(5-methyl-1*H*-pyrazol-3-ylamino)-6-morpholinopyrimidine;
S-4-(5-cyclopropyl-1*H*-pyrazol-3-ylamino)-2-[2-{3-(2-methoxypyrid-3-yl)isoxazol-5-yl }pyrrolidin-1-yl]-6-morpholinopyrimidine;
S-2-[2-{3-(2-methoxypyrid-3-yl)isoxazol-5-yl}pyrrolidin-1-yl]-4-(5-methyl-1*H*-pyrazol-3-ylamino-6-morpholinopyrimidine;
S-6-chloro-2-[2-{3-(2-cyanopyrid-3-yl)isoxazol-5-yl}pyrrolidin-1-yl]-4-(5-methyl-1*H-*pyrazol-3-ylamino)pyrimidine;
S-2-[2-{3-(2-cyanopyrid-3-yl)isoxazol-5-yl}pyrrolidin-1-yl]-4-(5-methyl-1*H*-pyrazol-3-ylamino)-6-morpholinopyrimidine;
S-2-[2-{3-(2-chloropyrid-3-yl)isoxazol-5-yl}pyrrolidin-1-yl]-4-(5-methyl-1*H*-pyrazol-3-ylamino)pyrimidine;
S-2-[2-{3-(2-methylaminopyrid-3-yl)isoxazol-5-yl}pyrrolidin-1-yl]-4-(5-methyl-1*H*-pyrazol-3-ylamino)pyrimidine;
S-6-methyl-2-[2-{3-(2-methylaminopyrid-3-yl)isoxazol-5-yl}pyrrolidin-1-yl]-4-(5-methyl-1*H*-pyrazol-3-ylamino)pyrimidine;
S-2-[2-{3-(2-methylpyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(2-methylpyrid-3-yl)isoxazol-5-yl}pyrrolidin-1-yl]-4-(5-methyl-1*H*-pyrazol-3-ylamino)pyrimidine;
S-6-chloro-2-[2-{3-(3-ethylpyrazin-2-yl)isoxazol-5-yl}pyrrolidin-1-yl]-4-(5-methyl-1*H-*pyrazol-3-ylamino)pyrimidine;
S-6-(2-methoxyethoxy)-2-[2-{3-(3-ethylpyrazin-2-yl)isoxazol-5-yl}pyrrolidin-1-yl]-4-(5-methyl-1*H*-pyrazol-3-ylamino)pyrimidine;
S-6-methoxy-2-[2-{3-(3-ethylpyrazin-2-yl)isoxazol-5-yl}pyrrolidin-1-yl]-4-(5-methyl-1*H-*pyrazol-3-ylamino)pyrimidine;
S-6-(2-hydroxyethoxy)-2-[2-{3-(2-methylpyrid-3-yl)isoxazol-5-yl}pyrrolidin-1-yl]-4-(5-methyl-1*H*-pyrazol-3-ylamino)pyrimidine;
S-6-(3-hydroxypropoxy)-2-[2-{3-(2-methylpyrid-3-yl)isoxazol-5-yl}pyrrolidin-1-yl]-4-(5-methyl-1*H*-pyrazol-3-ylamino)pyrimidine;
6-((2R)-2-hydroxypropoxy)-2-[(2S)-2-{3-(2-methylpyrid-3-yl)isoxazol-5-yl}pyrrolidin-1-yl]-4-(5-methyl-1*H*-pyrazol-3-ylamino)pyrimidine;
S-6-chloro-2-[2-{3-(1-oxopyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(1-oxopyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(2-cyanopyrid-3-yl)isoxazol-5-yl}pyrrolidin-1-yl]-4-(5-methyl-1*H*-pyrazol-3-ylamino)pyrimidine;
S-6-(1-cyanocyclopropyl)methoxy-2-[2-{3-(2-methylpyrid-3-yl)isoxazol-5-yl}pyrrolidin-1-yl]-4-(5-methyl-1*H*-pyrazol-3-ylamino)pyrimidine;
S-6-(3-cyanopropoxy)-2-[2-{3-(2-methylpyrid-3-yl)isoxazol-5-yl}pyrrolidin-1-yl]-4-(5-methyl-1*H*-pyrazol-3-ylamino)pyrimidine;
S-6-methoxy-2-[2-{3-(1-oxopyrid-3-yl)isoxazol-5-yl}pyrrolidin-1-yl]-4-(5-methyl-1*H-*pyrazol-3-ylamino)pyrimidine;
S-6-methoxy-2-[2-{3-(2-cyanopyrid-3-yl)isoxazol-5-yl}pyrrolidin-1-yl]-4-(5-methyl-1H-pyrazol-3-ylamino)pyrimidine;
S-6-chloro-2-[2-{3-(3-methylpyrazin-2-yl)isoxazol-5-yl)}pyrrolidin-1-yl]-4-(5-methyl-1*H-*pyrazol-3-ylamino)pyrimidine;
S-6-methoxy-2-[2-{3-(3-methylpyrazin-2-yl)isoxazol-5-yl}pyrrolidin-1-yl]-4-(5-methyl-1*H-*pyrazol-3-ylamino)pyrimidine;
S-6-ethyl-2-[2-{3-(3-methylpyrazin-2-yl)isoxazol-5-yl}pyrrolidin-1-yl]-4-(5-methyl-1*H-*pyrazol-3-ylamino)pyrimidine;
S-6-ethyl-2-[2-{3-(2-cyanopyrid-3-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(2-ethoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(3-methoxypropoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(2-ethoxyethoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-[(1-cyanocyclopropyl)methoxy]-2-[2-{3-(3-methylpyrazin-2-yl)isoxazol-5-yl}pyrrolidin-1-yl]-4-(5-methyl-1*H*-pyrazol-3-ylamino)pyrimidine;
S-6-(3-cyanopropoxy)-2-[2-{3-(3-methylpyrazin-2-yl)isoxazol-5-yl}pyrrolidin-1-yl]-4-(5-methyl-1*H*-pyrazol-3-ylamino)pyrimidine;
S-6-methoxy-2-[2-{3-(3-methoxypyrazin-2-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(3-(2-methoxyethoxy)pyrazin-2-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(3-hydroxypyrazin-2-yl)isoxazol-5-yl}pyrrolidin-1-yl]-4-(5-methyl-1*H*-pyrazol-3-ylamino)pyrimidine;
S-6-(2-methoxyethoxy)-2-[2-{3-(4-cyanopyrid-3-yl)isoxazol-5-yl}pyrrolidin-1-yl]-4-(5-methyl-1*H*-pyrazol-3-ylamino)pyrimidine; and
S-6-methoxy-2-[2-{3-(2-cyanopyrid-3-yl)isoxazol-5-yl}pyrrolidin-1-yl]-4-(5-methyl-1H-pyrazol-3-ylamino)pyrimidine;
and pharmaceutically-acceptable salts thereof.

14. A pharmaceutical composition which comprises a compound of formula (I), or a pharmaceutically-acceptable salt thereof, according to any one or more of claims 1 to 13 in association with a pharmaceutically-acceptable adjuvant, diluent or carrier.

15. A pharmaceutical product which comprises a compound of formula (I), or a pharmaceutically-acceptable salt thereof, according to any one or more of claims 1 to 13 and an additional anti-tumour agent for the conjoint treatment of cancer.

16. A compound of formula (I), or a pharmaceutically-acceptable salt thereof, according to any one or more of claims 1 to 13 for use as a medicament.

17. Use of a compound of formula (I), or a pharmaceutically-acceptable salt thereof, According to any one or more of claims 1 to 13 in the manufacture of a medicament for use in the production of an anti-proliferative effect in a warm-blooded animal.

18. Use of a compound of Formula (I), or a pharmaceutically-acceptable salt thereof according to any one or more of claims 1 to 13 in the manufacture of a medicament for use in the treatment of a disease or medical condition mediated alone or in part by IGF-1R tyrosine kinase in a warm-blooded animal.

19. Use of a compound of formula (I), or a pharmaceutically-acceptable salt thereof, according to any one or more of claims 1 to 13 in the manufacture of a medicament for use in the prevention or treatment of those tumours which are sensitive to inhibition of IGF-1R tyrosine kinase involved in the signal transduction steps which lead to the proliferation of tumour cells in a warm-blooded animal.

20. Use of a compound of formula (I), or a pharmaceutically-acceptable salt thereof, according to any one or more of claims 1 to 13 in the manufacture of a medicament for the treatment of cancer in a warm-blooded animal.

21. A process for the preparation of a compound of formula (I), or a pharmaceutically-acceptable salt thereof, according to claim 1 which comprises:
(a) the reaction, conveniently in the presence of a suitable base, of a compound of formula (II): 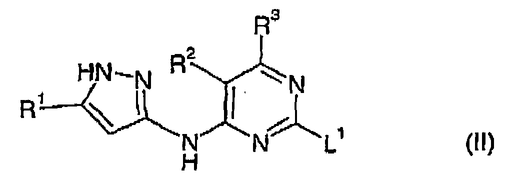 wherein L¹ represents a suitable displaceable group and R¹, R² and R³ are as defined in claim 1 except that any functional group is protected if necessary, with a compound of formula (III): 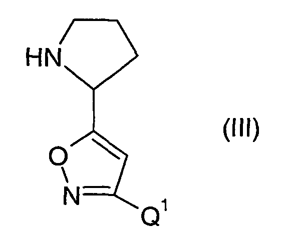 wherein Q¹ is as defined in claim 1 except that any functional group is protected if necessary; or
(b) the reaction, conveniently in the presence of a suitable acid, of a compound of formula (IV): 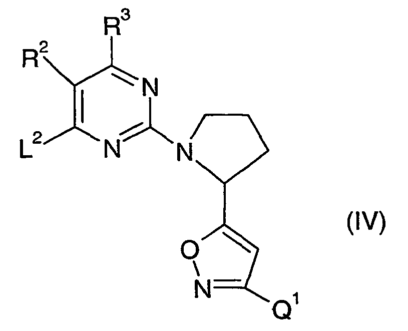 wherein L² is a suitable displaceable group and R², R³ and Q¹ are as defined in claim 1 except that any functional group is protected if necessary, with a pyrazole of formula (V): 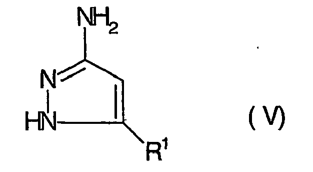 wherein R¹ is as defined in claim 1 except that any functional group is protected if necessary; or
(c) the reaction, conveniently in the presence of a suitable base, of a compound of formula (VI): 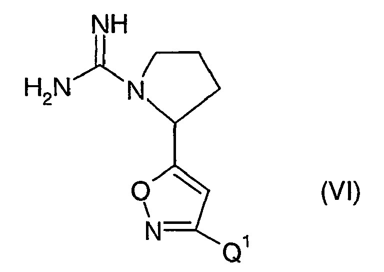 wherein Q¹ is as defined in claim 1 except that any functional group is protected if necessary, with a compound of formula (VII): 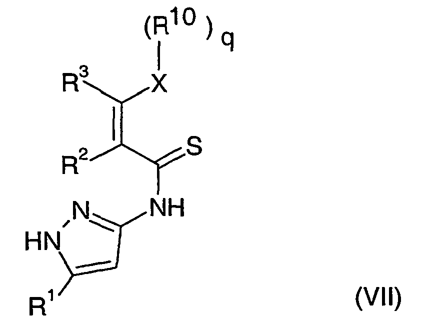 wherein X represents an oxygen atom and q is 1 or X represents a nitrogen atom and q is 2, R¹⁰ is a (C1-C6)alkyl group and R¹ R² and R³ are as defined in claim 1 except that any functional group is protected if necessary; or
(d) the reaction of a compound of formula (VIII): 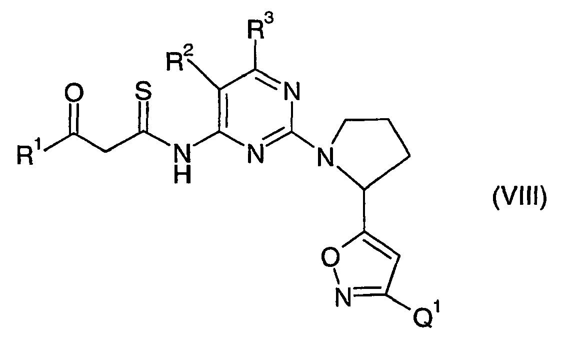 wherein R¹, R², R³ and Q¹ are as defined in claim 1 except that any functional group is protected if necessary, with hydrazine; or
(e) for compounds of formula (I) wherein R³ is a (C1-C6)alkoxy, amino, (C1-C6)alkylamino, di-[(C1-C6)alkyl]amino, -NHR^{3b} or -SR^{3a} group wherein R^{3a} and R^{3b} are as defined in claim 1 (and the group R³ is optionally substituted by at least one group as defined in claim 1), the reaction, conveniently in the presence of a suitable base, of a compound of formula (IX): 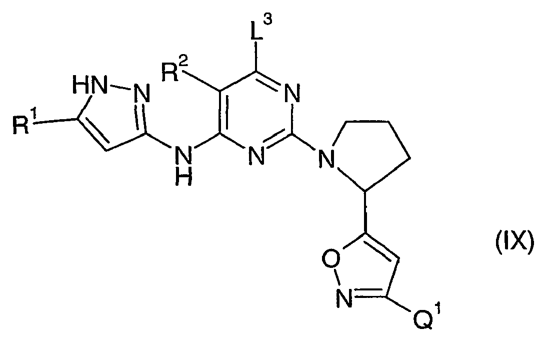 wherein L³ is a suitable displaceable group and R¹, R² and Q¹ are as defined in claim 1 except that any functional group is protected if necessary, with a compound of formula:
H-Xa
wherein Xa represents OR¹¹, NH₂, NHR¹¹, N(R¹¹)₂, NHR^{3b} or SR^{3a}, wherein R¹¹ is an, optionally substituted, (C1-C6)alkyl group and R^{3a} and R^{3b} are each as defined in claim 1 except that any functional group is protected if necessary; or
(f) for compounds of formula (I) wherein R³ is an, optionally substituted, saturated monocyclic 5- or 6-membered heterocyclic ring comprising at least one ring nitrogen and, optionally, one or more additional heteroatoms selected from nitrogen, oxygen and sulfur, the reaction, conveniently in the presence of a suitable base, of a compound of formula (IX): 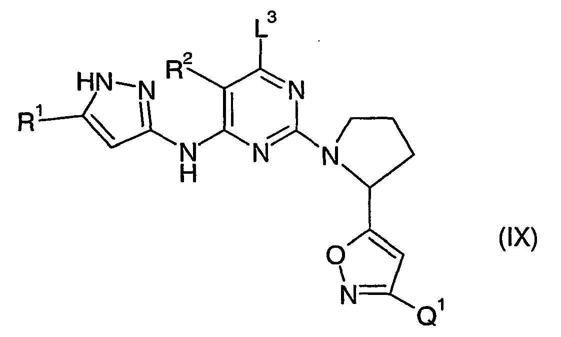 wherein L³ is a suitable displaceable group and R¹, R²and Q¹ are as defined in claim 1 except that any functional group is protected if necessary, with a compound of formula (Xb): 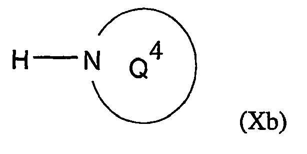 wherein Q⁴ is a saturated monocyclic 5- or 6-membered heterocyclic ring optionally comprising one or more heteroatoms selected from nitrogen, oxygen and sulfur in addition to the nitrogen atom shown above, which ring is optionally substituted by at least one group as defined in claim 1; or
(g) for compounds of formula (I) wherein R³ is a (C2-C6)alkenyl or (C2-C6)alkynyl group (and the group R³ is optionally substituted by at least one group as defined in claim 1), the reaction, conveniently in the presence of a suitable base and a suitable catalyst, of a compound of formula (IX): 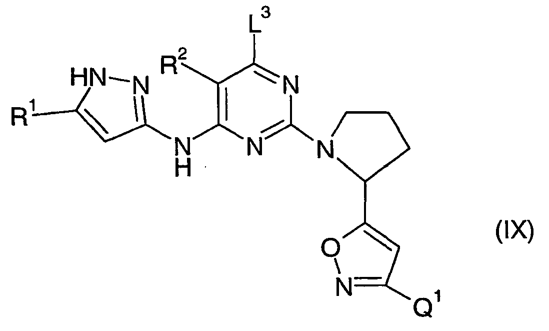 wherein L³ is a suitable displaceable group and R¹, R² and Q¹ are as defined in claim 1 except that any functional group is protected if necessary, with a compound of formula (Xc) or of formula (Xc'):
H- C≡ C- R¹² (Xc) 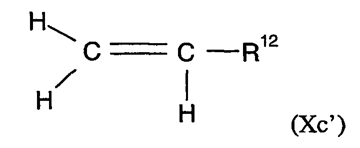
wherein R¹² is selected from hydrogen and an optionally substituted (1-4C)alkyl or (C1-C4)alkoxycarbonyl group; or
(h) for compounds of formula (I) wherein R³ is attached to the pyrimidine ring through a carbon atom, the reaction, conveniently in the presence of a suitable catalyst, of a compound of formula (IX): 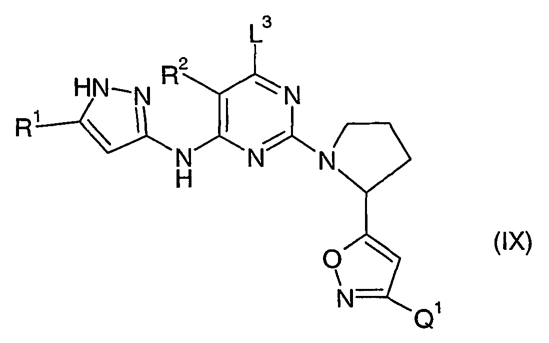 wherein L³ is a suitable displaceable group and R¹, R² and Q¹ are as defined in claim 1 except that any functional group is protected if necessary, with a compound of the formula M-R ³ wherein R³ is appropriately selected from the R³ groups as defined in claim 1 and M is a metallic group; or
(i) for compounds of formula (I) wherein R³ is a (C1-C6)alkoxycarbonyl group (and the group R³ is optionally substituted by at least one group as defined in claim 1), the reaction, conveniently in the presence of a suitable acid, of a compound of formula (X): 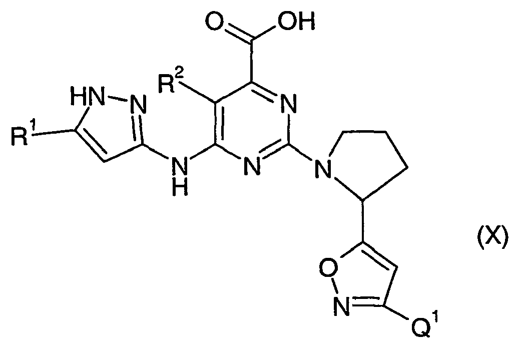 wherein R¹, R² and Q¹ are as defined in claim 1 except that any functional group is protected if necessary, with a compound of formula:
H-O-(C1-C6)alkyl
wherein the (C1-C6)alkyl group is optionally substituted by at least one group as defined in claim 1 as a substituent for R³ and any functional group is protected if necessary; 5 or
(j) for compounds of formula (I) wherein R³ is a (C1-C6)alkyl, (C3-C6)alkenyl, (C3-C6)alkynyl or (C1-C6)alkoxy group substituted by at least one group as defined in claim 1, reacting a compound of formula (XI): 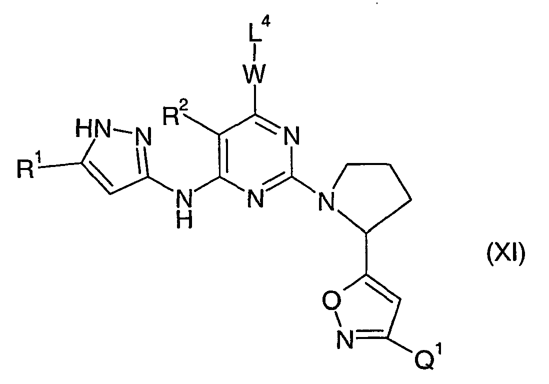 wherein L⁴ is a suitable displaceable group, W is an optionally substituted (C1-C6)alkyl, (C3-C6)alkenyl, (C3-C6)alkynyl or (C1-C6)alkoxy group and R¹, R² and Q¹ are as defined in claim 1 except that any functional group is protected if necessary, with a compound of formula H-Xa, (Xb), (Xc), (Xc') or M-R³; or
(k) for compounds of formula (I) wherein Q¹ is a pyridyl group containing a substituent in the ortho or the para position in the ring relative to the pyridine nitrogen (and optionally containing one or more additional substituents as defined in claim 1), reacting a compound of formula (XII): 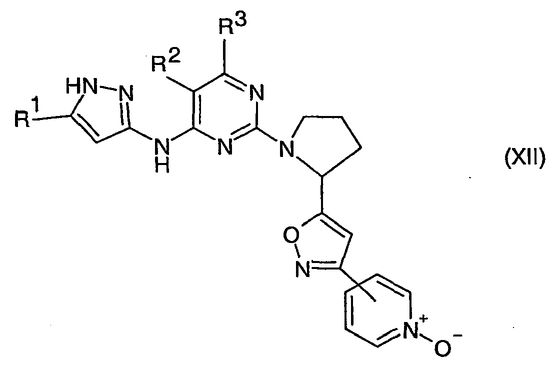
wherein R¹, R² and R³ are as defined in claim 1 except that any functional group is protected if necessary, with a reagent or reagents that combine an acylating, phosphorylating, sulfonating, sulfenylating or silylating agent with a nucleophile.
and optionally after process (a), (b), (c), (d) (e), (f), (g), (h), (i), (j) or (k) carrying out one or more of the following:
• converting the compound obtained to a further compound of the invention
• forming a pharmaceutically-acceptable salt of the compound.

## Patentansprüche

1. Verbindung der Formel (I): 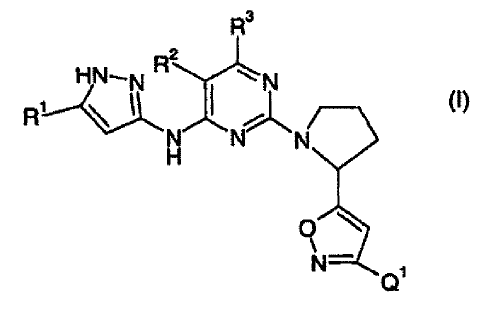 wobei:
**R¹** ausgewählt ist aus Methyl, Ethyl, Isopropyl und Cyclopropyl;
**R²** ausgewählt ist aus Wasserstoff und Halogen;
**R³** ausgewählt ist aus Wasserstoff, Hydroxy und Halogen, oder aus einer (C1-C6)-Alkyl-, (C2-C6)-Alkenyl-, (C2-C6)-Alkinyl-, (C1-C6)-Alkoxy-, (C1-C6)-Alkylcarbonyl-, (C1-C6)-Alkoxycarbonyl-, Amino-, (C1-C6)-Alkylamino-, Di-[(C1-C6)-alkyl]-amino-, (C3-C8)-Cycloalkylamino-, Carbamoyl-, (C1-C6-Alkylcarbamoyl-, Di-[(C1-C6)-alkyl]-carbamoyl-, -C(O)R^{3b}, -NHR^{3b}-, -SR3^{a-} oder -N(R^{3c})C(O)R^{3a}-Gruppe, wobei R^{3a} ausgewählt ist aus einer (C1-C6)-Alkyl- oder (C1-C6)-Alkoxygruppe, R^{3b} für einen gesättigten monocyclischen 4-, 5- oder 6-gliedrigen heterocyclischen Ring mit wenigstens einem Ringheteroatom, ausgewählt aus Stickstoff, Sauerstoff und Schwefel, steht und R^{3c} ausgewählt ist aus Wasserstoff und (C1-C6)-Alkyl,
oder R³ für einen gesättigten monocyclischen 5- oder 6-gliedrigen heterocyclischen Ring mit wenigstens einem Ringheteroatom, ausgewählt aus Stickstoff, Sauerstoff und Schwefel, steht,
die Gruppen oder Ringe in R³ jeweils gegebenenfalls mit einem oder mehreren Substituenten, unabhängig ausgewählt aus (C1-C6) -Alkyl, (C1-C6) -Alkoxy, (C1-C6) -Alkoxy- (C1-C6) -alkyl, (C1-C6) -Alkoxy-(C1-C6)-alkoxy, Halogen, Hydroxy, Trifluormethyl, Tri-[(C1-C4)-alkyl]silyl, Cyano, Amino, (C1-C6)-Alkylamino, Di-[(C1-C6)-alkyl]amino, Amino-(C1-C6) -alkyl, (C1-C6)-Alkylamino-(C1-C6)-alkyl, Di-[(C1-C6)alkyl]amino-(C1-C6)-alkyl, (C1-C6) - Alkoxycarbonyl, Carbamoyl, (C1-C6)-Alkylcarbamoyl, Di-[(C1-C6)-alkyl]carbamoyl, (C1-C6)-Alkylthio, (C1-C6) -Alkylsulfonyl, (C1-C6) -Alkylsulfinyl, (C1-C6)-Alkanoyl, einer Alkanoylaminogruppe -N(R^{3d})C(O)R^{3e}, wobei R^{3d} ausgewählt ist aus Wasserstoff und (C1-C6)-Alkyl und R^{3e} ausgewählt ist aus einer (C1-C6)-Alkyl- oder (C1-C6)-Alkoxygruppe, oder einem gesättigten monocyclischen 3-, 4-, 5-, 6- oder 7-gliedrigen Ring, der gegebenenfalls ein oder mehrere Heteroatome, ausgewählt aus Stickstoff, Sauerstoff und Schwefel, enthalten kann, substituiert sein können, wobei die Substituenten jeweils gegebenenfalls mit einer oder mehreren (C1-C4)-Alkyl-, Hydroxy- oder Cyanogruppen substituiert sein können;
**Q¹** für einen 5- oder 6-gliedrigen heteroaromatischen Ring mit wenigstens einem Ringheteroatom, ausgewählt aus Stickstoff, Sauerstoff und Schwefel, steht,
und wobei Q¹ mit einem oder mehreren Substituenten, unabhängig ausgewählt aus (C1-C6)-Alkyl und (C1-C6) -Alkoxy (wobei die (C1-C6) -Alkyl- und (C1-C6)-Alkoxygruppe jeweils gegebenenfalls mit einem oder mehreren Substituenten, unabhängig ausgewählt aus (C1-C4)-Alkoxy, Halogen, Amino, Hydroxy und Trifluormethyl, substituiert sein können), Oxo, Halogen, Nitro, Cyano, -NR⁴R⁵, Carboxy, Hydroxy, (C2-C6)-Alkenyl, (C3-C8)-Cycloalkyl, (C1-C6)-Alkoxycarbonyl, (C1-C6)-Alkylcarbonyl, (C2-C6)-Alkanoylamino, Phenylcarbonyl, -S(O)ₙ(C1-C6) -Alkyl, -C(O)NR⁶R⁷ und
-SO₂NR⁸R⁹, wobei R⁴, R⁵, R⁶, R⁷, R⁸ und R⁹ jeweils unabhängig ausgewählt sind aus Wasserstoff und (C1-C6) -Alkyl, oder R⁴ und R⁵, bzw. R⁶ und R⁷, bzw. R⁸ und R⁹ jeweils zusammengenommen mit dem Stickstoffatom, an das sie gebunden sind, unabhängig voneinander einen gesättigten heterocyclischen Ring bilden können und n gleich 0, 1 oder 2 ist, substituiert ist,
und wobei ein gesättigter monocyclischer Ring jeweils gegebenenfalls 1 oder 2 Oxo- oder Thioxo-Substituenten trägt;
oder ein pharmazeutisch annehmbares Salz davon,
vorausgesetzt, daß es sich bei der Verbindung der Formel (I) nicht um:
5-Chlor-2-{2-[3-(2-methoxypyrid-3-yl)isoxazol-5-yl]pyrrolidin-1-yl}-4-(5-methyl-1*H*-pyrazol-3-ylamino)pyrimidin;
S-5-Chlor-2-{2-[3-(2-methoxypyrid-3-yl)isoxazol-5-yl]pyrrolidin-1-yl}-4-(5-methyl-1H-pyrazol-3-ylamino)pyrimidin;
S-4-(5-Ethyl-1*H*-pyrazol-3-ylamino)-2-[2-[3-(2-methoxypyrid-3-yl)isoxazol-5-yl]pyrrolidin-1-yl]-pyrimidin;
S-4-(5-Methyl-1*H*-pyrazol-3-ylamino)-2-[2-[3-(2-methoxypyrid-3-yl)isoxazol-5-yl]pyrrolidin-l-yl]-pyrimidin;
S-4-(5-Cyclopropyl-1*H*-pyrazol-3-ylamino)-2-[2-[3-(2-methoxypyrid-3-yl)isoxazol-5-yl]pyrrolidin-1-yl]pyrimidin;
S-6-Chlor-4-(5-methyl-1*H*-pyrazol-3-ylamino)-2-[2-{3-(3-methoxypyrazin-2-yl)isoxazol-5-yl}-pyrrolidin-1-yl}pyrimidin;
S-6-Morpholino-4-(5-methyl-1*H*-pyrazol-3-ylamino)-2-[2-{3-(3-methoxypyrazin-2-yl)isoxazol-5-yl}pyrrolidin-l-yl]pyrimidin;
S-6-Morpholino-4-(5-methyl-1*H*-pyrazol-3-ylamino)-2-[2-{3-(3-hydroxypyrazin-2-yl)isoxazol-5-yl}pyrrolidin-1-yl]pyrimidin;
S-6-Methyl-4-(5-methyl-1*H*-pyrazol-3-ylamino)-2-[2-{3-(3-methoxypyrazin-2-yl)isoxazol-5-yl}-pyrrolidin-1-yl]pyrimidin;
S-6-Methyl-4-(5-ethyl-1*H*-pyrazol-3-ylamino)-2-[2-{3-(3-methoxypyrazin-2-yl)isoxazol-5-yl}-pyrrolidin-1-yl]pyrimidin;
S-6-Chlor-4-(5-ethyl-1*H*-pyrazol-3-ylamino)-2-[2-{3-(3-methoxypyrazin-2-yl)isoxazol-5-yl}-pyrrolidin-1-yl]pyrimidin;
S-6-Methyl-4-(5-methyl-1*H*-pyrazol-3-ylamino)-2-[2-[3-(2-methoxypyrid-3-yl)isoxazol-5-yl]pyrrolidin-1-yl]pyrimidin;
S-5-Fluor-4-(5-CyClopropyl-1*H*-pyrazol-3-ylamino)-2-[2-[3-(2-methoxypyrid-3-yl)isoxazol-5-yl]pyrrolidin-1-yl]pyrimidin;
S-5-Fluor-4-(5-ethyl-1*H*-pyrazol-3-ylamino)-2-[2-[3-(2-methoxypyrid-3-yl)isoxazol-5-yl]pyrrolidin-1-yl]pyrimidin;
S-6-(2-Hydroxyethoxy)-4-(5-methyl-1*H*-pyrazol-3-ylamino)-2-[2-[3-(2-methoxypyrid-3-yl)isoxazol-5-yl]pyrrolidin-1-yl]pyrimidin;
S-6-Chlor-4-(5-CyClopropyl-1*H-*pyrazol-3-ylamino)-2-[2-[3-(2-methoxypyrid-3-yl)isoxazol-5-yl]pyrrolidin-1-yl]pyrimidin;
S-6-Chlor-4-(5-ethyl-1*H*-pyrazol-3-ylamino)-2-[2-[3-(2-methoxypyrid-3-yl)isoxazol-5-yl]pyrrolidin-1-yl]pyrimidin;
S-6-(2-Hydroxyethoxy)-4-(5-CyClopropyl-1*H*-pyrazol-3-ylamino)-2-[2-[3-(2-methoxypyrid-3-yl)isoxazol-5-yl]pyrrolidin-1-yl]pyrimidin;
S-5-Fluor-4-(5-methyl-1*H*-pyrazol-3-ylamino)-2-[2-[3-(2-methoxypyrid-3-yl)isoxazol-5-yl]pyrrolidin-1-yl]pyrimidin;
S-6-(2-Hydroxyethoxy)-4-(5-ethyl-1*H-*pyrazol-3-ylamino)-2-[2-[3-(2-methoxypyrid-3-yl)isoxazol-5-yl]pyrrolidin-1-yl]pyrimidin;
S-6-Methyl-4-(5-CyClopropyl-1H-pyrazol-3-ylamino)-2-[2-{3-(3-methoxypyrazin-2-yl)isoxazol-5-yl}pyrrolidin-1-yl]pyrimidin;
S-6-Morpholino-4-(5-ethyl-1H-pyrazol-3-ylamino)-2-[2-{3-(3-methoxypyrazin-2-yl)isoxazol-5-yl}pyrrolidin-1-yl]pyrimidin;
S-6-Chlor-4-(5-methyl-1H-pyrazol-3-ylamino)-2-[2-[3-(2-methoxypyrid-3-yl)isoxazol-5-yl]pyrrolidin-1-yl]pyrimidin; oder
S-6-Morpholino-4-(5-ethyl-1H-pyrazol-3-ylamino)-2-[2-{3-(3-hydroxypyrazin-2-yl)isoxazol-5-yl}pyrrolidin-1-yl]pyrimidin handelt.

2. Verbindung der Formel (I) nach Anspruch 1, wobei:
R¹ ausgewählt ist aus Methyl, Ethyl, Isopropyl und Cyclopropyl;
R² ausgewählt ist aus Wasserstoff und Halogen;
R³ ausgewählt ist aus Wasserstoff, Hydroxy und Halogen, oder aus einer (C1-C6)-Alkyl-, (C2-C6)-Alkenyl-, (C2-C6)-Alkinyl-, (C1-C6)-Alkoxy-, (C1-C6)-Alkylcarbonyl-, (C1-C6)-Alkoxycarbonyl-, Amino-, (C1-C6)-Alkylamino-, Di-[(C1-C6)-alkyl]-amino-, (C3-C8)-Cycloalkylamino-, Carbamoyl-, (C1-C6-Alkylcarbamoyl-, Di-[(C1-C6)-alkyl]-carbamoyl-, -C(O)R^{3b}, -NHR^{3b}-, -SR3^{a-} oder -N(R^{3c})C(O)R^{3a}-Gruppe, wobei R^{3a} ausgewählt ist aus einer (C1-C6)-Alkyl- oder (C1-C6)-Alkoxygruppe, R^{3b} für einen gesättigten monocyclischen 4-, 5-oder 6-gliedrigen heterocyclischen Ring mit wenigstens einem Ringheteroatom, ausgewählt aus Stickstoff, Sauerstoff und Schwefel, steht und R³' ausgewählt ist aus Wasserstoff und (C1-C6)-Alkyl,
oder R³ für einen gesättigten monocyclischen 5-oder 6-gliedrigen heterocyclischen Ring mit wenigstens einem Ringheteroatom, ausgewählt aus Stickstoff, Sauerstoff und Schwefel, steht,
die Gruppen oder Ringe in R³ jeweils gegebenenfalls mit einem oder mehreren Substituenten, unabhängig ausgewählt aus (C1-C6) -Alkyl, (C1-C6) -Alkoxy, (C1-C6)-Alkoxy-(C1-C6)-alkyl, (C1-C6) -Alkoxy-(C1-C6)-alkoxy, Halogen, Hydroxy, Trifluormethyl, Tri-[(C1-C4)-alkyl]silyl, Cyano, Amino, (C1-C6)-Alkylamino, Di-[(C1-C6)-alkyl]amino, Amino-(C1-C6) -alkyl, (C1-C6) -Alkylamino- (C1-C6) -alkyl, Di-[(C1-C6)alkyl]amino-(C1-C6)-alkyl, (C1-C6)-Alkoxycarbonyl, Carbamoyl, (C1-C6)-Alkylcarbamoyl, Di-[(C1-C6)-alkyl]carbamoyl, (C1-C6)-Alkylthio, (C1-C6) -Alkylsulfonyl, (C1-C6) -Alkylsulfinyl, (C1-C6)-Alkanoyl, einer Alkanoylaminogruppe -N(R^{3d})C(O)R^{3e}, wobei R^{3d} ausgewählt ist aus Wasserstoff und (C1-C6)-Alkyl und R^{3e} ausgewählt ist aus einer (C1-C6)-Alkyl- oder (C1-C6)-Alkoxygruppe, oder einem gesättigten monocyclischen 3-, 4-, 5-, 6- oder 7-gliedrigen Ring, der gegebenenfalls ein oder mehrere Heteroatome, ausgewählt aus Stickstoff, Sauerstoff und Schwefel, enthalten kann, substituiert sein können, wobei die Substituenten jeweils gegebenenfalls mit einer oder mehreren (C1-C4)-Alkyl-, Hydroxy- oder Cyanogruppen substituiert sein können;
Q¹ für einen 5- oder 6-gliedrigen heteroaromatischen Ring mit wenigstens einem Ringheteroatom, ausgewählt aus Stickstoff, Sauerstoff und Schwefel, steht,
und wobei Q¹ mit einem oder mehreren Substituenten, unabhängig ausgewählt aus (C1-C6)-Alkyl und (C1-C6) -Alkoxy (wobei die (C1-C6) -Alkyl- und (C1-C6)-Alkoxygruppe jeweils gegebenenfalls mit einem oder mehreren Substituenten, unabhängig ausgewählt aus Halogen, Amino, Hydroxy und Trifluormethyl, substituiert sein können), Halogen, Nitro, Cyano, -NR⁴R⁵, Carboxy, Hydroxy, (C2-C6)-Alkenyl, (C3-C8)Cycloalkyl, (C1-C6)-Alkoxycarbonyl, (C1-C6)-Alkylcarbonyl, (C2-C6)-Alkanoylamino, Phenylcarbonyl, -S(O)ₙ(C1-C6)-Alkyl, -C(O)NR⁶R⁷ und -SO₂NR⁸R⁹, wobei R⁴, R⁵, R⁶, R⁷, R⁸ und R⁹ jeweils unabhängig ausgewählt sind aus Wasserstoff und (C1-C6) -Alkyl, oder R⁴ und R⁵, bzw. R⁶ und R⁷, bzw. R⁸ und R⁹ jeweils zusammengenommen mit dem Stickstoffatom, an das sie gebunden sind, unabhängig voneinander einen gesättigten heterocyclischen Ring bilden können und n gleich 0, 1 oder 2 ist, substituiert ist,
und wobei ein gesättigter monocyclischer Ring jeweils gegebenenfalls 1 oder 2 Oxo- oder Thioxo-Substituenten trägt;
oder ein pharmazeutisch annehmbares Salz davon.

3. Verbindung der Formel (I) nach Anspruch 1 oder 2, wobei R¹ für Methyl oder Cyclopropyl steht.

4. Verbindung der Formel (I) nach Anspruch 3, wobei R¹ für Methyl steht.

5. Verbindung der Formel (I) nach einem oder mehreren der Anprüche 1 bis 4, wobei R² für Wasserstoff steht.

6. Verbindung der Formel (I) nach einem oder mehreren der Ansprüche 1 bis 5, wobei R³ ausgewählt ist aus Wasserstoff und Halogen, oder aus einer(C1-C4)-Alkyl- oder (C1-C3)-Alkoxygruppe,
oder R³ für einen gesättigten monocyclischen 5- oder 6-gliedrigen heterocyclischen Ring mit wenigstens einem Ringheteroatom, ausgewählt aus Stickstoff und Sauerstoff, steht,
wobei die Gruppen oder Ringe in R³ jeweils gegebenenfalls mit einem oder mehreren Substituenten, unabhängig ausgewählt aus (C1-C6)-Alkyl, (C1-C6) -Alkoxy, (C1-C6) -Alkoxy- (C1-C6) - alkyl, (C1-C6)-Alkoxy-(C1-C6)-alkoxy, Halogen, Hydroxy, Trifluormethyl, Tri-[(C1-C4)-alkyl]silyl, Cyano, Amino, (C1-C6)-Alkylamino, Di-[(C1-C6)-alkyl] amino, Amino- (C1-C6) -alkyl, (C1-C6)-Alkylamino-(C1-C6)-alkyl, Di-[(C1-C6)alkyl]amino-(C1-C6)-alkyl, (C1-C6)-Alkoxycarbonyl, Carbamoyl, (C1-C6) -Alkylcarbamoyl, Di-[(C1-C6)-alkyl]carbamoyl, (C1-C6)-Alkylthio, (C1-C6)-Alkylsulfonyl, (C1-C6) -Alkylsulfinyl, (C1-C6)-Alkanoyl, einer Alkanoylaminogruppe -N(R^{3d})C(O)R^{3e}, wobei R^{3d} ausgewählt ist aus Wasserstoff und (C1-C6) -Alkyl und R^{3e} ausgewählt ist aus einer (C1-C6)-Alkyl- oder (C1-C6)-Alkoxygruppe, oder einem gesättigten monocyclischen 3-, 4-, 5-, 6-oder 7-gliedrigen Ring, der gegebenenfalls ein oder mehrere Heteroatome, ausgewählt aus Stickstoff, Sauerstoff und Schwefel, enthalten kann, substituiert sein können, wobei die Substituenten jeweils gegebenenfalls mit einer oder mehreren (C1-C4)-Alkyl-, Hydroxy- oder Cyanogruppen substituiert sein können;
und wobei ein in R³ vorhandener gesättigter monocyclischer Ring gegebenenfalls 1 oder 2 Oxo- oder Thioxo-Substituenten trägt.

7. Verbindung der Formel (I) nach Anspruch 6, wobei R³ ausgewählt ist aus Wasserstoff und Halogen oder aus einer (C1-C3)-Alkyl- oder (C1-C3)-Alkoxygruppe,
oder R³ für einen gesättigten monocyclischen 6-gliedrigen heterocyclischen Ring mit wenigstens einem Ringheteroatom, ausgewählt aus Stickstoff und Sauerstoff, steht,
wobei die Gruppen oder Ringe in R³ jeweils gegebenenfalls mit einem oder mehreren Substituenten, unabhängig ausgewählt aus (C1-C6)-Alkyl, (C1-C6) -Alkoxy, (C1-C6) -Alkoxy- (C1-C6) - alkyl, (C1-C6)-Alkoxy-(C1-C6)-alkoxy, Halogen, Hydroxy, Trifluormethyl, Tri-[(C1-C4)-alkyl]silyl, Cyano, Amino, (C1-C6)-Alkylamino, Di-[(C1-C6)-alkyl] amino, Amino- (C1-C6) -alkyl, (C1-C6)-Alkylamino-(C1-C6)-alkyl, Di-[(C1-C6)alkyl]amino-(C1-C6)-alkyl, (C1-C6)-Alkoxycarbonyl, Carbamoyl, (C1-C6) -Alkylcarbamoyl, Di-[(C1-C6) - alkyl]carbamoyl, (C1-C6)-Alkylthio, (C1-C6)-Alkylsulfonyl, (C1-C6)-Alkylsulfinyl, (C1-C6)-Alkanoyl, einer Alkanoylaminogruppe -N(R^{3d})C(O)R^{3e}, wobei R^{3d} ausgewählt ist aus Wasserstoff und (C1-C6) -Alkyl und R^{3e} ausgewählt ist aus einer (C1-C6)-Alkyl- oder (C1-C6)-Alkoxygruppe, oder einem gesättigten monocyclischen 3-, 4-, 5-, 6-oder 7-gliedrigen Ring, der gegebenenfalls ein oder mehrere Heteroatome, ausgewählt aus Stickstoff, Sauerstoff und Schwefel, enthalten kann, substituiert sein können, wobei die Substituenten jeweils gegebenenfalls mit einer oder mehreren (C1-C4)-Alkyl-, Hydroxy- oder Cyanogruppen substituiert sein können;
und wobei ein in R³ vorhandener gesättigter monocyclischer Ring gegebenenfalls 1 oder 2 Oxo- oder Thioxo-Substituenten trägt.

8. Verbindung der Formel (I) nach Anspruch 7, wobei R³ ausgewählt ist aus Wasserstoff und Halogen oder aus einer (C1-C3)-Alkyl- oder (C1-C3)-Alkoxygruppe,
oder R³ für einen gesättigten monocyclischen 6-gliedrigen heterocyclischen Ring mit wenigstens einem Ringheteroatom, ausgewählt aus Stickstoff und Sauerstoff, steht
wobei die Gruppen oder Ringe in R³ jeweils gegebenenfalls mit einem oder mehreren Substituenten, unabhängig ausgewählt aus Cyano, Hydroxy, Cyclopropyl und (C1-C3)-Alkoxy, substituiert sein können, wobei die Substituenten jeweils gegebenenfalls mit einer oder mehreren Cyanogruppen substituiert sein können.

9. Verbindung der Formel (I) nach Anspruch 8, wobei R³ ausgewählt ist aus Wasserstoff, Chlor, Methyl, Ethyl, Methoxy, Ethoxy, 2-Hydroxyethoxy, 2-Methoxyethoxy, 2-Hydroxypropoxy, (1-Cyanocyclopropyl)methoxy, 3-Cyanopropoxy, 3-Hydroxypropoxy, 3-Methoxypropoxy, 2-Ethoxyethoxy und Morpholino.

10. Verbindung der Formel (I) nach einem oder mehreren der Ansprüche 1 bis 9, wobei Q¹ ausgewählt ist aus Pyridyl, Imidazolyl, Isoxazolyl, Pyrazolyl, Furyl, Pyrazinyl, Pyridazinyl, Pyrimidinyl, Pyrrolyl, Thiazolyl, Oxazolyl, Isothiazolyl, Triazolyl, Tetrahydrofuranyl und Thienyl,
und wobei Q¹ mit einem oder mehreren Substituenten, unabhängig ausgewählt aus (C1-C6)-Alkyl und (C1-C6) -Alkoxy (wobei die (C1-C6) -Alkyl- und (C1-C6)-Alkoxygruppe jeweils gegebenenfalls mit einem oder mehreren Substituenten, unabhängig ausgewählt aus (C1-C3)-Alkoxy, Halogen, Amino, Hydroxy und Trifluormethyl, substituiert sein können), Oxo, Halogen, Nitro, Cyano, -NR⁴R⁵, Carboxy, Hydroxy, (C2-C6)-Alkenyl, (C3-C8)-Cycloalkyl, (C1-C6)-Alkoxycarbonyl, (C1-C6)-Alkylcarbonyl, (C2-C6)-Alkanoylamino, Phenylcarbonyl, -S(O)ₙ(C1-C6) -Alkyl, -C(O)NR⁶R⁷ und -SO₂NR⁸R⁹, wobei R⁴, R⁵, R⁶, R⁷, R⁸ und R⁹ jeweils unabhängig ausgewählt sind aus Wasserstoff und (C1-C6)-Alkyl, oder R⁴ und R⁵, bzw. R⁶ und R⁷, bzw. R⁸ und R⁹ jeweils zusammengenommen mit dem Stickstoffatom, an das sie gebunden sind, unabhängig voneinander einen gesättigten heterocyclischen Ring bilden können und n gleich 0, 1 oder 2 ist, substituiert ist,
und wobei ein gesättigter monocyclischer Ring jeweils gegebenenfalls 1 oder 2 Oxo- oder Thioxo-Substituenten trägt.

11. Verbindung der Formel (I) nach Anspruch 10, wobei Q¹ ausgewählt ist aus Pyridyl, Imidazolyl, Isoxazolyl, Pyrazolyl, Furyl, Pyrazinyl, Pyridazinyl, Pyrimidinyl, Pyrrolyl, Thiazolyl, Oxazolyl, Isothiazolyl, Triazolyl, Tetrahydrofuranyl und Thienyl,
und wobei Q¹ mit wenigstens einem Substituenten, unabhängig ausgewählt aus Oxo, Halogen, Hydroxy, Cyano, (C1-C4)-Alkyl, (C1-C4)-Alkoxy, wobei das (C1-C4)-Alkoxy gegebenenfalls mit (C1-C3)-Alkoxy und -NR⁴R⁵ substituiert sein kann, wobei R⁴ und R⁵ jeweils unabhängig ausgewählt sind aus Wasserstoff und (C1-C6)Alkyl, oder R⁴ und R⁵ zusammengenommen mit dem Stickstoffatom, an das sie gebunden sind, jeweils unabhängig einen gesättigten heterocyclischen Ring bilden können, substituiert ist.

12. Verbindung der Formel (I) nach Anspruch 10 oder 11, wobei Q¹ ausgewählt ist aus Pyridyl und Pyrazinyl.

13. Verbindung der Formel (I), ausgewählt aus einer oder mehreren der folgenden Verbindungen:
S-2-[2-{3-(3-Ethoxypyrazin-2-yl)isoxazol-5-yl}pyrrolidin-1-yl]-6-methyl-4-(5-methyl-1H-pyrazol-3-ylamino)pyrimidin;
S-2-[2-{3-(3-Ethylaminopyrazin-2-yl)isoxazol-5-yl}pyrrolidin-1-yl]-6-methyl-4-(5-methyl-1H-pyrazol-3-ylamino)pyrimidin;
S-2-[2-{3-(2-Methylpyrid-3-yl)isoxazol-5-yl}-pyrrolidin-1-yl]-6-methyl-4-(5-methyl-1*H*-pyrazol-3-ylamino)pyrimidin;
S-6-Chlor-2-[2-{3-(2-methylpyrid-3-yl)isoxazol-5-yl}pyrrolidin-1-yl]-4-(5-methyl-1*H*-pyrazol-3-ylamino)pyrimidin;
S-2-[2-{3-(2-Chlorpyrid-3-yl)isoxazol-5-yl}-pyrrolidin-1-yl]-6-methyl-4-(5-methyl-1*H*-pyrazol-3-ylamino)pyrimidin;
S-6-Methoxy-2-[2-{3-(2-methylpyrid-3-yl)isoxazol-5-yl}pyrrolidin-1-yl]-4-(5-methyl-1*H*-pyrazol-3-ylamino)pyrimidin;
S-2-[2-{3-(2-Cyanopyrid-3-yl)isoxazol-5-yl}-pyrrolidin-1-yl]-6-methyl-4-(5-methyl-1*H*-pyrazol-3-ylamino)pyrimidin;
S-2-[2-{3-(2-Methylpyrid-3-yl)isoxazol-5-yl}-pyrrolidin-1-yl]-4-(5-methyl-1*H*-pyrazol-3-ylamino)-6-morpholinopyrimidin;
S-4-(5-Cyclopropyl-1*H*-pyrazol-3-ylamino)-2-[2-{3-(2-methoxypyrid-3-yl)isoxazol-5-yl}pyrrolidin-l-yl]-6-morpholinopyrimidin;
S-2-[2-{3-(2-Methoxypyrid-3-yl)isoxazol-5-yl}-pyrrolidin-1-yl]-4-(5-methyl-1*H*-pyrazol-3-ylamino-6-morpholinopyrimidin;
S-6-Chlor-2-[2-{3-(2-cyanopyrid-3-yl)isoxazol-5-yl}pyrrolidin-1-yl]-4-(5-methyl-1*H*-pyrazol-3-ylamino)pyrimidin;
S-2-[2-{3-(2-Cyanopyrid-3-yl)isoxazol-5-yl}-pyrrolidin-1-yl]-4-(5-methyl-1*H*-pyrazol-3-ylamino)-6-morpholinopyrimidin;
S-2-[2-{3-(2-Chlorpyrid-3-yl)isoxazol-5-yl}-pyrrolidin-1-yl]-4-(5-methyl-1*H*-pyrazol-3-ylamino)pyrimidin;
S-2-[2-{3-(2-Methylaminopyrid-3-yl)isoxazol-5-yl}pyrrolidin-1-yl]-4-(5-methyl-1*H*-pyrazol-3-ylamino)pyrimidin;
S-6-Methyl-2-[2-{3-(2-methylaminopyrid-3-yl)-isoxazol-5-yl}pyrrolidin-1-yl]-4-(5-methyl-1*H-*pyrazol-3-ylamino)pyrimidin;
S-2-[2-{3-(2-Methylpyrid-3-yl)isoxazol-5-yl}-pyrrolidin-1-yl]-4-(5-methyl-1*H*-pyrazol-3-ylamino)pyrimidin;
S-6-(2-Methoxyethoxy)-2-[2-{3-(2-methylpyrid-3-yl)isoxazol-5-yl}pyrrolidin-1-yl]-4-(5-methyl-1*H-*pyrazol-3-ylamino)pyrimidin;
S-6-Chlor-2-[2-{3-(3-ethylpyrazin-2-yl)isoxazol-5-yl}pyrrolidin-1-yl]-4-(5-methyl-1*H*-pyrazol-3-ylamino)pyrimidin;
S-6-(2-Methoxyethoxy)-2-[2-{3-(3-ethylpyrazin-2-yl)isoxazol-5-yl}pyrrolidin-1-yl]-4-(5-methyl-1*H-*pyrazol-3-ylamino)pyrimidin;
S-6-Methoxy-2-[2-{3-(3-ethylpyrazin-2-yl)isoxazol-5-yl}pyrrolidin-1-yl]-4-(5-methyl-1*H*-pyrazol-3-ylamino)pyrimidin;
S-6-(2-Hydroxyethoxy)-2-[2-{3-(2-methylpyrid-3-yl)isoxazol-5-yl}pyrrolidin-1-yl]-4-(5-methyl-1*H-*pyrazol-3-ylamino)pyrimidin;
S-6-(3-Hydroxypropoxy)-2-[2-{3-(2-methylpyrid-3-yl)isoxazol-5-yl}pyrrolidin-1-yl]-4-(5-methyl-1*H-*pyrazol-3-ylamino)pyrimidin;
6-((2R)-2-Hydroxypropoxy)-2-[(2S)-2-{3-(2-methylpyrid-3-yl)isoxazol-5-yl}pyrrolidin-1-yl]-4-(5-methyl-1*H-*pyrazol-3-ylamino)pyrimidin;
S-6-Chlor-2-[2-{3-(1-oxopyrid-3-yl)isoxazol-5-yl}pyrrolidin-1-yl]-4-(5-methyl-1*H*-pyrazol-3-ylamino)pyrimidin;
S-6-(2-Methoxyethoxy)-2-[2-{3-(1-oxopyrid-3-yl)isoxazol-5-yl}pyrrolidin-1-yl]-4-(5-methyl-1*H-*pyrazol-3-ylamino)pyrimidin;
S-6-(2-Methoxyethoxy)-2-[2-{3-(2-cyanopyrid-3-yl)isoxazol-5-yl}pyrrolidin-1-yl]-4-(5-methyl-1*H-*pyrazol-3-ylamino)pyrimidin;
S-6-(1-Cyanocyclopropyl)methoxy-2-[2-{3-(2-methylpyrid-3-yl)isoxazol-5-yl}pyrrolidin-1-yl]-4-(5-methyl-1*H*-pyrazol-3-ylamino)pyrimidin;
S-6-(3-Cyanopropoxy)-2-[2-{3-(2-methylpyrid-3-yl)isoxazol-5-yl}pyrrolidin-1-yl]-4-(5-methyl-1*H-*pyrazol-3-ylamino)pyrimidin;
S-6-Methoxy-2-[2-{3-(1-oxopyrid-3-yl)isoxazol-5-yl}pyrrolidin-1-yl]-4-(5-methyl-1*H*-pyrazol-3-ylamino)pyrimidin;
S-6-Methoxy-2-[2-{3-(2-cyanopyrid-3-yl)isoxazol-5-yl}pyrrolidin-1-yl]-4-(5-methyl-1*H*-pyrazol-3-ylamino)pyrimidin;
S-6-Chlor-2-[2-{3-(3-methylpyrazin-2-yl)isoxazol-5-yl}pyrrolidin-1-yl]-4-(5-methyl-1*H*-pyrazol-3-ylamino)pyrimidin;
S-6-Methoxy-2-[2-{3-(3-methylpyrazin-2-yl)-isoxazol-5-yl}pyrrolidin-1-yl]-4-(5-methyl-1*H-*pyrazol-3-ylamino)pyrimidin;
S-6-Ethyl-2-[2-{3-(3-methylpyrazin-2-yl)isoxazol-5-yl}pyrrolidin-1-yl]-4-(5-methyl-1*H*-pyrazol-3-ylamino)pyrimidin;
S-6-Ethyl-2-[2-{3-(2-cyanopyrid-3-yl)isoxazol-5-yl}pyrrolidin-1-yl]-4-(5-methyl-1*H*-pyrazol-3-ylamino)pyrimidin;
S-6-(2-Methoxyethoxy)-2-[2-{3-(3-methylpyrazin-2-yl)isoxazol-5-yl}pyrrolidin-1-yl]-4-(5-methyl-1*H-*pyrazol-3-ylamino)pyrimidin;
S-6-(2-Ethoxy)-2-[2-{3-(3-methylpyrazin-2-yl)-isoxazol-5-yl}pyrrolidin-1-yl]-4-(5-methyl-1*H-*pyrazol-3-ylamino)pyrimidin;
S-6-(3-Methoxypropoxy)-2-[2-{3-(3-methylpyrazin-2-yl)isoxazol-5-yl}pyrrolidin-1-yl]-4-(5-methyl-1*H-*pyrazol-3-ylamino)pyrimidin;
S-6-(2-Ethoxyethoxy)-2-[2-{3-(3-methylpyrazin-2-yl)isoxazol-5-yl}pyrrolidin-1-yl]-4-(5-methyl-1*H-*pyrazol-3-ylamino)pyrimidin;
S-6-[(1-Cyanocyclopropyl)methoxy]-2-[2-{3-(3-methylpyrazin-2-yl)isoxazol-5-yl}pyrrolidin-1-yl]-4-(5-methyl-1H-pyrazol-3-ylamino)pyrimidin;
S-6-(3-Cyanopropoxy)-2-[2-{3-(3-methylpyrazin-2-yl)isoxazol-5-yl}pyrrolidin-1-yl]-4-(5-methyl-1*H-*pyrazol-3-ylamino)pyrimidin;
S-6-Methoxy-2-[2-{3-(3-methoxypyrazin-2-yl)-isoxazol-5-yl}pyrrolidin-1-yl]-4-(5-methyl-1*H-*pyrazol-3-ylamino)pyrimidin;
S-6-(2-Methoxyethoxy)-2-[2-{3-(3-(2-methoxyethoxy)pyrazin-2-yl)isoxazol-5-yl}pyrrolidin-1-yl]-4-(5-methyl-1*H*-pyrazol-3-ylamino)pyrimidin;
S-6-(2-Methoxyethoxy)-2-[2-{3-(3-hydroxypyrazin-2-yl)isoxazol-5-yl}pyrrolidin-1-yl]-4-(5-methyl-1*H-*pyrazol-3-ylamino)pyrimidin;
S-6-(2-Methoxyethoxy)-2-[2-{3-(4-cyanopyrid-3-yl)isoxazol-5-yl}pyrrolidin-1-yl]-4-(5-methyl-1*H-*pyrazol-3-ylamino)pyrimidin; und
S-6-Methoxy-2-[2-{3-(2-cyanopyrid-3-yl)isoxazol-5-yl}pyrrolidin-1-yl]-4-(5-methyl-1*H*-pyrazol-3-ylamino)pyrimidin;
und pharmazeutisch annehmbaren Salzen davon.

14. Pharmazeutische Zusammensetzung, die eine Verbindung der Formel (I) oder ein pharmazeutisch annehmbares Salz davon nach einem oder mehreren der Ansprüche 1 bis 13 in Assoziation mit einem pharmazeutisch annehmbaren Adjuvans, Verdünnungsmittel oder Trägerstoff umfaßt.

15. Pharmazeutisches Produkt, das eine Verbindung der Formel (I) oder ein pharmazeutisch annehmbares Salz davon nach einem oder mehreren der Ansprüche 1 bis 13 sowie ein zusätzliches Antitumormittel zur gemeinsamen Behandlung von Krebs umfaßt.

16. Verbindung der Formel (I) oder ein pharmazeutisch annehmbares Salz davon nach einem oder mehreren der Ansprüche 1 bis 13 zur Verwendung als Arzneimittel.

17. Verwendung einer Verbindung der Formel (I) oder eines pharmazeutisch annehmbaren Salzes davon nach einem oder mehreren der Ansprüche 1 bis 13 bei der Herstellung eines Arzneimittels zur Verwendung bei der Erzeugung eines antiproliferativen Effekts in einem Warmblüter.

18. Verwendung einer Verbindung der Formel (I) oder eines pharmazeutisch annehmbaren Salzes davon nach einem oder mehreren der Ansprüche 1 bis 13 bei der Herstellung eines Arzneimittels zur Verwendung bei der Behandlung einer Krankheit oder eines medizinischen Leidens, die bzw. das allein oder zum Teil durch IGF-1R-Tyrosinkinase vermittelt wird, bei einem Warmblüter.

19. Verwendung einer Verbindung der Formel (I) oder eines pharmazeutisch annehmbaren Salzes davon nach einem oder mehreren der Ansprüche 1 bis 13 bei der Herstellung eines Arzneimittels zur Verwendung bei der Vorbeugung oder Behandlung solcher Tumoren, die auf die Hemmung der an den zur Proliferation von Tumorzellen führenden Signaltransduktionsschritten beteiligten IGF-1R-Tyrosinkinase empfindlich reagieren, bei einem Warmblüter.

20. Verwendung einer Verbindung der Formel (I) oder eines pharmazeutisch annehmbaren Salzes davon nach einem oder mehreren der Ansprüche 1 bis 13 bei der Herstellung eines Arzneimittels zur Behandlung von Krebs bei einem Warmblüter.

21. Verfahren zur Herstellung einer Verbindung der Formel (I) oder eines pharmazeutisch annehmbaren Salzes davon nach Anspruch 1, wobei das Verfahren folgendes umfaßt:
(a) die Umsetzung, zweckmäßigerweise in Gegenwart einer geeigneten Base, einer Verbindung der Formel (II) : 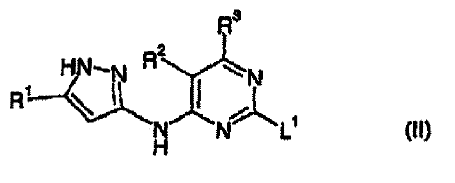 wobei L¹ eine geeignete verdrängbare Gruppe repräsentiert und R¹, R² und R³ die in Anspruch 1 angegebene Bedeutung besitzen, mit der Ausnahme daß, falls notwendig, eine funktionelle Gruppe jeweils mit einer Verbindung der Formel (III): 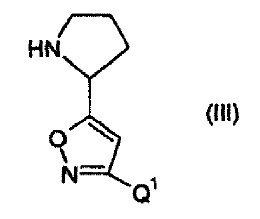 wobei Q¹ die in Anspruch 1 angegebene Bedeutung besitzt, mit der Ausnahme, daß, falls notwendig, eine funktionelle Gruppe jeweils geschützt ist, geschützt ist, oder
(b) die Umsetzung, zweckmäßigerweise in Gegenwart einer geeigneten Säure, einer Verbindung der Formel (IV): 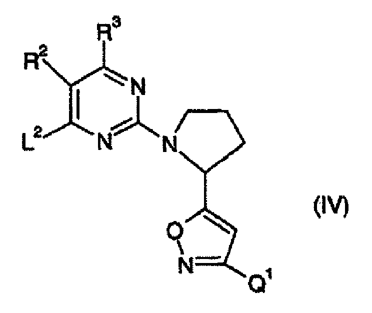 wobei L² für eine geeignete verdrängbare Gruppe steht und R², R³ und Q¹ die in Anspruch 1 angegebene Bedeutung besitzen, mit der Ausnahme, daß, falls notwendig, eine funktionelle Gruppe jeweils mit einem Pyrazol der Formel (V): 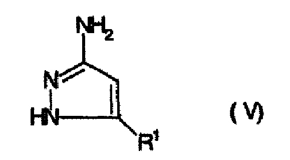 wobei R¹ die in Anspruch 1 angegebene Bedeutung besitzt, mit der Ausnahme, daß, falls notwendig, eine funktionelle Gruppe jeweils geschützt ist, geschützt ist, oder
(c) die Umsetzung, zweckmäßigerweise in Gegenwart einer geeigneten Base, einer Verbindung der Formel (VI): 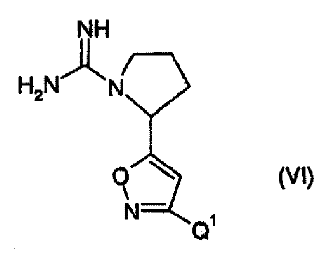 wobei Q¹ die in Anspruch 1 angegebene Bedeutung besitzt, mit der Ausnahme, daß, falls notwendig, eine funktionelle Gruppe jeweils mit einer Verbindung der Formel (VII) : 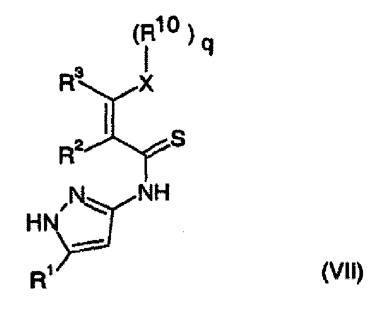 wobei X ein Sauerstoffatom repräsentiert und q gleich 1 ist oder X ein Stickstoffatom repräsentiert und q gleich 2 ist, R¹⁰ für eine (C1-C6) -Alkylgruppe steht und R¹, R² und R³ die in Anspruch 1 angegebene Bedeutung besitzen, mit der Ausnahme, daß, falls notwendig, eine funktionelle Gruppe jeweils geschützt ist, geschützt ist, oder
(d) die Umsetzung einer Verbindung der Formel (VIII): 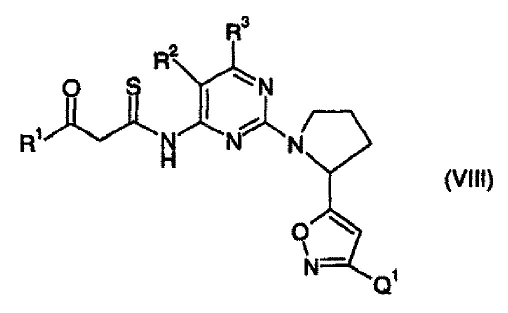 wobei R¹, R², R³ und Q¹ die in Anspruch 1 angegebene Bedeutung besitzen, mit der Ausnahme, daß, falls notwendig, eine funktionelle Gruppe jeweils mit Hydrazin geschützt ist, oder
(e) bei Verbindungen der Formel (I), wobei R³ für eine (C1-C6)-Alkoxy-, Amino-, (C1-C6)-Alkylamino-, Di- [(C1-C6) -alkyl] amino-, -NHR^{3b}- oder -SR^{3a}-Gruppe, wobei R^{3a} und R^{3b} die in Anspruch 1 angegebene Bedeutung besitzen (und die Gruppe R³ gegebenenfalls mit wenigstens einer Gruppe mit der in Anspruch 1 angegebenen Bedeutung substituiert ist), steht, die Umsetzung, zweckmäßigerweise in Gegenwart einer geeigneten Base, einer Verbindung der Formel (IX): 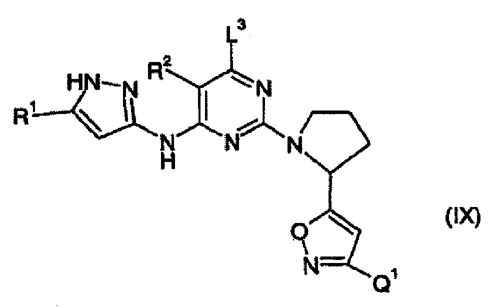 wobei L³ für eine geeignete verdrängbare Gruppe steht und R¹, R² und Q¹ die in Anspruch 1 angegebene Bedeutung besitzen, mit der Ausnahme, daß, falls notwendig, eine funktionelle Gruppe jeweils mit einer Verbindung der Formel:
H-Xa,
wobei Xa OR¹¹, NH₂, NHR¹¹, N(R¹¹)₂, NHR^{3b} oder SR^{3a} repräsentiert, wobei R¹¹ für eine gegebenenfalls substituierte (C1-C6)-Alkylgruppe steht und R^{3a} und R^{3b} jeweils die in Anspruch 1 angegebene Bedeutung besitzen, mit der Ausnahme, daß, falls notwendig, eine funktionelle Gruppe geschützt ist, geschützt ist, oder
(f) bei Verbindungen der Formel (I), wobei R³ für einen gegebenenfalls substituierten, gesättigten monocyclischen 5- oder 6-gliedrigen heterocyclischen Ring mit wenigstens einem Ringstickstoff und gegebenenfalls einem oder mehreren zusätzlichen Heteroatomen, ausgewählt aus Stickstoff, Sauerstoff und Schwefel, steht, die Umsetzung zweckmäßigerweise in Gegenwart einer geeigneten Base, einer Verbindung der Formel (IX): 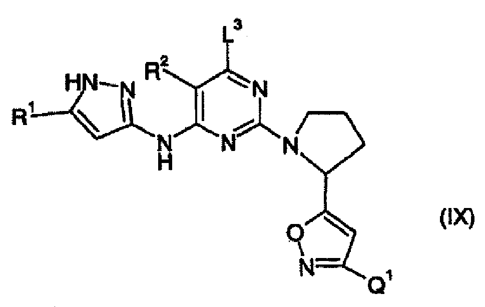 wobei L³ für eine geeignete verdrängbare Gruppe steht und R¹, R² und Q¹ die in Anspruch 1 angegebene Bedeutung besitzen, mit Ausnahme, daß, falls notwendig, eine funktionelle Gruppe jeweils mit einer Verbindung der Formel (Xb): 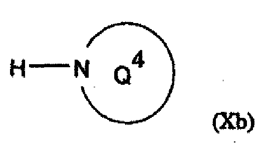 wobei Q⁴ für einen gesättigten monocyclischen 5- oder 6-gliedrigen heterocyclischen Ring mit gegebenenfalls einem oder mehreren Heteroatomen, ausgewählt aus Stickstoff, Sauerstoff und Schwefel, zusätzlich zum oben dargestellten Stickstoffatom, wobei der Ring gegebenenfalls mit wenigstens einer Gruppe mit der in Anspruch 1 angegebenen Bedeutung substituiert ist, geschützt ist, oder
(g) bei Verbindungen der Formel (I), wobei R³ für eine (C2-C6)-Alkenyl- oder (C2-C6)-Alkinylgruppe steht (und die Gruppe R³ gegebenenfalls mit wenigstens einer Gruppe mit der in Anspruch 1 angegebenen Bedeutung substituiert ist), die Umsetzung, zweckmäßigerweise in Gegenwart einer geeigneten Base und eines geeigneten Katalysators, einer Verbindung der Formel (IX): 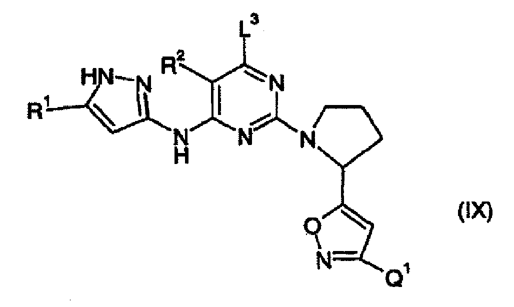 wobei L³ für eine geeignete verdrängbare Gruppe steht und R¹, R² und Q¹ die in Anspruch 1 angegebene Bedeutung besitzen, mit der Ausnahme, daß, falls notwendig, eine funktionelle Gruppe jeweils mit einer Verbindung der Formel (Xc) oder der Formel (Xc'):
H-C≡C-R¹² (Xc) 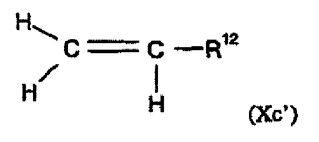
wobei R¹² ausgewählt ist aus Wasserstoff und einer gegebenenfalls substituierten (1-4C)-Alkyl- oder (C1-C4)-Alkoxycarbonylgruppe, geschützt ist, oder
(h) bei Verbindungen der Formel (I), wobei R³ an den Pyrimidinring über ein Kohlenstoffatom gebunden ist, die Umsetzung, zweckmäßigerweise in Gegenwart eines geeigneten Katalysators, einer Verbindung der Formel (IX) : 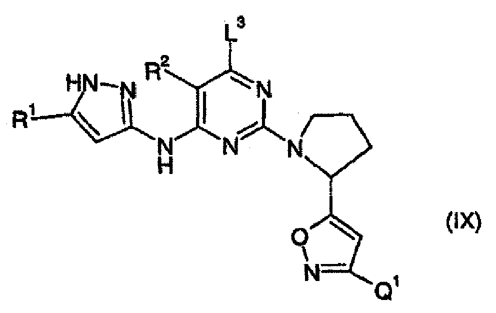 wobei L³ für eine geeignete verdrängbare Gruppe steht und R¹, R² und Q¹ die in Anspruch 1 angegebene Bedeutung besitzen, mit der Ausnahme, daß, falls notwendig, eine funktionelle Gruppe jeweils mit einer Verbindung der Formel M-R³ wobei R³ entsprechenderweise aus den Gruppen R³ mit der in Anspruch 1 angegebenen Bedeutung ausgewählt ist und M für eine Metallgruppe steht, geschützt ist, oder
(i) bei Verbindungen der Formel (I), wobei R³ für eine (C1-C6)-Alkoxycarbonylgruppe steht (und die Gruppe R³ gegebenenfalls mit wenigstens einer Gruppe mit der in Anspruch 1 angegebenen Bedeutung substituiert ist), die Umsetzung, zweckmäßigerweise in Gegenwart einer geeigneten Säure, einer Verbindung der Formel (X): 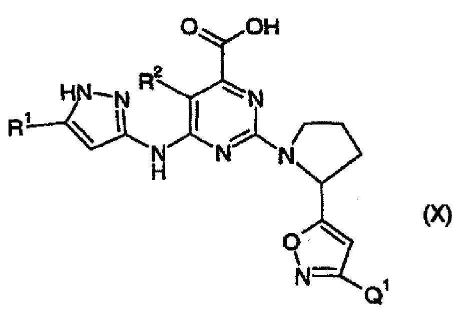 wobei R¹, R² und Q¹ die in Anspruch 1 angegebene Bedeutung besitzen, mit der Ausnahme, daß, falls notwendig eine funktionelle Gruppe jeweils mit einer Verbindung der Formel:
H-O-(C1-C6)-Alkyl,
wobei die (Cl-C6)-Alkylgruppe gegebenenfalls mit wenigstens einer Gruppe mit der in Anspruch 1 angegebenen Bedeutung als Substituent für R³ substituiert ist und eine funktionelle Gruppe jeweils, falls notwendig, geschützt ist, geschützt ist, oder
(j) bei Verbindungen der Formel (I), wobei R³ für eine mit wenigstens einer Gruppe mit der in Anspruch 1 angegebenen Bedeutung substituierte (C1-C6)-Alkyl-, (C3-C6)-Alkenyl-, (C3-C6)-Alkinyl- oder (C1-C6)-Alkoxygruppe steht, Umsetzen einer Verbindung der Formel (XI): 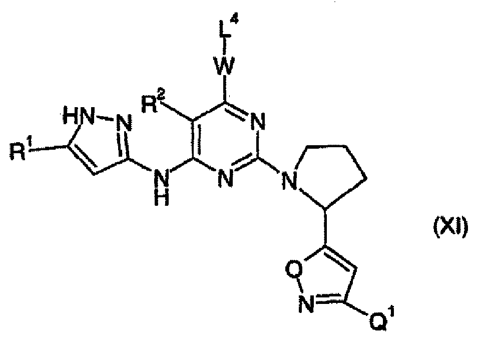 wobei L⁴ für eine geeignete verdrängbare Gruppe, W für eine gegebenenfalls substituierte (C1-C6)-Alkyl-, (C3-C6)-Alkenyl-, (C3-C6)-Alkinyl- oder (C1-C6)-Alkoxygruppe steht und R¹, R² und Q¹ die in Anspruch angegebene Bedeutung besitzen, mit Ausnahme, daß, falls notwendig, eine funktionelle Gruppe mit einer Verbindung der Formel H-Xa, (Xb), (Xc), (Xc') oder M-R³ geschützt ist, oder
(k) bei Verbindungen der Formel (I), wobei Q¹ für eine Pyridylgruppe mit einem Substituenten in ortho- oder para-Stellung im Ring relativ zum Pyridinstickstoff (und gegebenenfalls mit einem oder mehreren zusätzlichen Substituenten der in Anspruch 1 angegebenen Bedeutung) steht, Umsetzen einer Verbindung der Formel (XII): 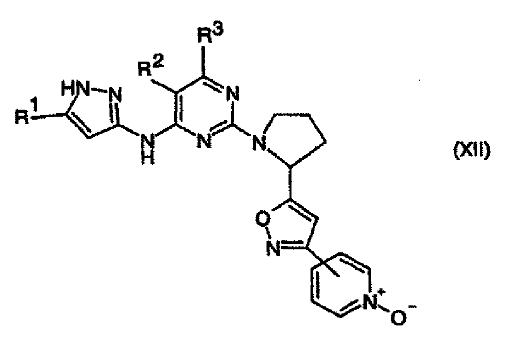
wobei R¹, R² und R³ die in Anspruch 1 angegebene Bedeutung besitzen, mit Ausnahme, daß, falls notwendig, eine funktionelle Gruppe geschützt ist, mit einem Reagens oder Reagentien, bei dem bzw. bei denen ein Acylierungs-, Phosphorylierungs-, Sulfonierungs-, Sulfenylierungs- oder Silylierungsmittel mit einem Nukleophil kombiniert ist,
und gegebenenfalls nach Verfahren (a), (b), (c), (d), (e), (f), (g), (h), (i), (j) oder (k) Durchführen eines oder mehrerer der folgenden Punkte:
• Umwandeln der erhaltenen Verbindung in eine weitere erfindungsgemäße Verbindung,
• Bilden eines pharmazeutisch annehmbaren Salzes der Verbindung.

## Revendications

1. Composé de formule (1) : 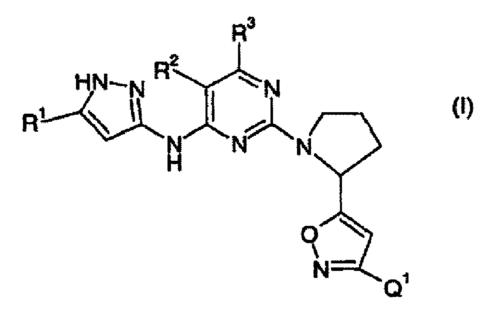 dans laquelle :
**R¹** est choisi parmi méthyle, éthyle, isopropyle et cyclopropyle ;
**R²** est choisi parmi hydrogène et halogéno ;
**R³** est choisi parmi hydrogène, hydroxy et halogéno, ou parmi un groupement (C1-C6) alkyle, (C2-C6)alcényle, (C2-C6)-alcynyle, (C1-C6) alcoxy, (C1-C6) - alkylcarbonyle, (C1-C6)alcoxycarbonyle, amino, (C1-C6)alkylamino, di-[(C1-C6)alkyl]-amino, (C3-C8)cycloalkylamino, carbamoyle, (C1-C6)alkylcarbamoyle, di-[(C1-C6)alkyl]-carbamoyle, -C(O)R^{3b}, -NHR^{3b}, -SR^{3a} ou -N(R^{3c})C(O)R^{3a}, où R^{3a} est choisi parmi un groupement (C1-C6) alkyle ou (C1-C6)alcoxy,
R^{3b} est un cycle hétérocyclique monocyclique saturé à 4, 5 ou 6 chaînons, comprenant au moins un hétéroatome du cycle choisi parmi azote, oxygène et soufre, et R^{3c} est choisi parmi hydrogène et (C1-C6)alkyle,
ou R³ est un cycle hétérocyclique monocyclique saturé à 5 ou 6 chaînons, comprenant au moins un hétéroatome du cycle choisi parmi azote, oxygène et soufre,
chacun desdits groupements ou cycles dans R³ pouvant éventuellement être substitué par un ou plusieurs substituants choisis indépendamment parmi (C1-C6)alkyle, (C1-C6)-alcoxy, (C1-C6)alcoxy(C1-C6) alkyle, (C1-C6) - alcoxy(C1-C6)alcoxy, halogéno, hydroxy, trifluorométhyle, tri-[(C1-C4)alkyl]silyle, cyano, amino, (C1-C6)alkylamino, di-[(C1-C6)alkyl]amino, amino (C1-C6) alkyle, (C1-C6) alkylamino (C1-C6) alkyle, di-[(C1-C6)-alkyl]amino(C1-C6)alkyle, (C1-C6)alcoxycarbonyle, carbamoyle, (C1-C6)alkylcarbamoyle, di-[(C1-C6)alkyl]carbamoyle, (C1-C6)alkylthio, (C1-C6)alkylsulfonyle, (C1-C6)alkylsulfinyle, (C1-C6)alcanoyle, un groupement alcanoylamino -N(R^{3d})C(O)R^{3e} où R^{3d} est choisi parmi hydrogène et (C1-C6) alkyle et R^{3e} est choisi parmi un groupement (C1-C6)alkyle ou (C1-C6) alcoxy, ou un cycle monocyclique saturé à 3, 4, 5, 6 ou 7 chaînons, ledit cycle pouvant éventuellement comprendre un ou plusieurs hétéroatomes choisis parmi azote, oxygène et soufre, l'un quelconque desdits substituants pouvant éventuellement être substitué par un
ou plusieurs groupements (C1-C4)alkyle, hydroxy ou cyano ;
**Q¹** est un cycle hétéroaromatique à 5 ou 6 chaînons, comprenant au moins un hétéroatome du cycle choisi parmi azote, oxygène et soufre,
et où Q¹ est substitué par un ou plusieurs substituants choisis indépendamment parmi (C1-C6) alkyle et (C1-C6)alcoxy (l'un ou l'autre desdits groupements (C1-C6)alkyle et (C1-C6)alcoxy pouvant éventuellement être substitué par un ou plusieurs substituants choisis indépendamment parmi (C1-C4)alcoxy, halogéno, amino, hydroxy et trifluorométhyle), oxo, halogéno, nitro, cyano, -NR⁴R⁵, carboxy, hydroxy, (C2-C6)alcényle, (C3-C8)-cycloalkyle, (C1-C6)alcoxycarbonyle, (C1-C6)-alkylcarbonyle, (C2-C6)alcanoylamino, phénylcarbonyle, -S(O)ₙ(C1-C6)alkyle, -C(O)NR⁶R⁷ et -SO₂NR⁸R⁹, où R⁴, R⁵, R⁶, R⁷, R⁸ et R⁹ sont choisis chacun indépendamment parmi hydrogène et (C1-C6) alkyle, ou R⁴ et R⁵, ou R⁶ et R⁷, ou R⁸ et R⁹, lorsqu'ils sont pris ensemble avec l'atome d'azote auquel ils sont liés, peuvent former chacun indépendamment un cycle hétérocyclique saturé, et n est 0, 1 ou 2,
et où un cycle monocyclique saturé quelconque porte éventuellement 1 ou 2 substituants oxo ou thioxo ;
ou un sel pharmaceutiquement acceptable de celui-ci,
à condition que le composé de formule (I) ne soit pas :
la 5-chloro-2-{2-[3-(2-méthoxypyrid-3-yl)isoxazol-5-yl]pyrrolidin-1-yl}-4-(5-méthyl-1*H*-pyrazol-3-ylamino)pyrimidine ;
la S-5-chloro-2-{2-[3-(2-méthoxypyrid-3-yl)-isoxazol-5-yl]pyrrolidin-1-yl}-4-(5-méthyl-1*H-*pyrazol-3-ylamino)pyrimidine ;
la S-4-(5-éthyl-1*H*-pyrazol-3-ylamino)-2-[2-[3-(2-méthoxypyrid-3-yl)isoxazol-5-yl]pyrrolidin-1-yl]-pyrimidine ;
la S-4-(5-méthyl-1H-pyrazol-3-ylamino)-2-[2-[3-(2-méthoxypyrid-3-yl)isoxazol-5-yl]pyrrolidin-1-yl]-pyrimidine ;
la S-4-(5-Cylopropyl-1H-pyrazol-3-ylamino)-2-[2-[3-(2-méthoxypyrid-3-yl)isoxazol-5-yl]pyrrolidin-1-yl]pyrimidine ;
la S-6-chloro-4-(5-méthyl-1*H*-pyrazol-3-ylamino)-2-[2-{3-(3-méthoxypyrazin-2-yl)isoxazol-5-yl}-pyrrolidin-1-yl]pyrimidine ;
la S-6-morpholino-4-(5-méthyl-1*H*-pyrazol-3-ylamino)-2-[2-{3-(3-méthoxypyrazin-2-yl)isoxazol-5-yl}pyrrolidin-1-yl]pyrimidine ;
la S-6-morpholino-4-(5-méthyl-1*H*-pyrazol-3-ylamino)-2-[2-{3-(3-hydroxypyrazin-2-yl)isoxazol-5-yl}pyrrolidin-l-yl]pyrimidine ;
la S-6-méthyl-4-(5-méthyl-1*H*-pyrazol-3-ylamino)-2-[2-{3-(3-méthoxypyrazin-2-yl)isoxazol-5-yl}-pyrrolidin-1-yl]pyrimidine ;
la S-6-méthyl-4-(5-éthyl-1*H*-pyrazol-3-ylamino)-2-[2-{3-(3-méthoxypyrazin-2-yl)isoxazol-5-yl}-pyrrolidin-1-yl]pyrimidine ;
la S-6-chloro-4-(5-éthyl-1*H*-pyrazol-3-ylamino)-2-[2-{3-(3-méthoxypyrazin-2-yl)isoxazol-5-yl}-pyrrolidin-1-yl]pyrimidine ;
la S-6-méthyl-4-(5-méthyl-1*H*-pyrazol-3-ylamino)-2-[2-[3-(2-méthoxypyrid-3-yl)isoxazol-5-yl]-pyrrolidin-1-yl]pyrimidine ;
la S-5-fluoro-4-(5-Cyclopropyl-1*H*-pyrazol-3-ylamino)-2-[2-[3-(2-méthoxypyrid-3-yl)isoxazol-5-yl]pyrrolidin-l-yl]pyrimidine ;
la S-5-fluoro-4-(5-éthyl-1*H*-pyrazol-3-ylamino)-2-[2-[3-(2-méthoxypyrid-3-yl)isoxazol-5-yl]-pyrrolidin-1-yl]pyrimidine ;
la S-6-(2-hydroxyéthoxy)-4-(5-méthyl-1*H*-pyrazol-3-ylamino)-2-[2-[3-(2-méthoxypyrid-3-yl)isoxazol-5-yl]pyrrolidin-1-yl]pyrimidine ;
la S-6-chloro-4-(5-Cyclopropyl-1*H*-pyrazol-3-yl-amino)-2-[2-[3-(2-méthoxypyrid-3-yl)isoxazol-5-yl]pyrrolidin-1-yl]pyrimidine ;
la S-6-chloro-4-(5-éthyl-1*H*-pyrazol-3-ylamino)-2-[2-[3-(2-méthoxypyrid-3-yl)isoxazol-5-yl]-pyrrolidin-1-yl]pyrimidine ;
la S-6-(2-hydroxyéthoxy)-4-(5-cyclopropyl-1*H-*pyrazol-3-ylamino)-2-[2-[3-(2-méthoxypyrid-3-yl)isoxazol-5-yl]pyrrolidin-1-yl]pyrimidine ;
la S-5-fluoro-4-(5-méthyl-1*H*-pyrazol-3-ylamino)-2-[2-[3-(2-méthoxypyrid-3-yl)isoxazol-5-yl]-pyrrolidin-1-yl]pyrimidine ;
la S-6-(2-hydroxyéthoxy)-4-(5-éthyl-1*H*-pyrazol-3-ylamino)-2-[2-[3-(2-méthoxypyrid-3-yl)isoxazol-5-yl]pyrrolidin-1-yl]pyrimidine ;
la S-6-méthyl-4-(5-cyclopropyl-1H-pyrazol-3-yl-amino)-2-[2-{3-(3-méthoxypyrazin-2-yl)isoxazol-5-yl}pyrrolidin-1-yl]pyrimidine ;
la S-6-morpholino-4-(5-éthyl-1H-pyrazol-3-ylamino)-2-[2-{3-(3-méthoxypyrazin-2-yl)isoxazol-5-yl}pyrrolidin-1-yl]pyrimidine ;
la S-6-chloro-4-(5-méthyl-1H-pyrazol-3-ylamino)-2-[2-[3-(2-méthoxypyrid-3-yl)isoxazol-5-yl]-pyrrolidin-1-yl]pyrimidine ; ou
la S-6-morpholino-4-(5-éthyl-1H-pyrazol-3-ylamino)-2-[2-{3-(3-hydroxypyrazin-2-yl)isoxazol-5-yl}pyrrolidin-1-yl]pyrimidine.

2. Composé de formule (I) selon la revendication 1,
**caractérisé en ce que** :
R¹ est choisi parmi méthyle, éthyle, isopropyle et cyclopropyle ;
R² est choisi parmi hydrogène et halogéno ;
R³ est choisi parmi hydrogène, hydroxy et halogéno,
ou parmi un groupement (C1-C6)alkyle, (C2-C6)-alcényle, (C2-C6)alcynyle, (C1-C6)alcoxy, (C1-C6)-alkylcarbonyle, (C1-C6)alcoxycarbonyle, amino, (C1-C6)alkylamino, di-[(C1-C6)alkyl]amino, (C3-C8)-cycloalkylamino, carbamoyle, (C1-C6)alkylcarbamoyle, di-[(C1-C6)alkyl]carbamoyle, -C(O)R^{3b} -NHR^{3b}, -SR^{3a} ou -N(R^{3c})C(O)R^{3a}, où R^{3a} est choisi parmi un groupement (C1-C6)alkyle ou (C1-C6)-alcoxy, R^{3b} est un cycle hétérocyclique monocyclique saturé à 4, 5 ou 6 chaînons, comprenant au moins un hétéroatome du cycle choisi parmi azote, oxygène et soufre, et R^{3c} est choisi parmi hydrogène et (C1-C6)alkyle,
ou R³ est un cycle hétérocyclique monocyclique saturé à 5 ou 6 chaînons, comprenant au moins un hétéroatome du cycle choisi parmi azote, oxygène et soufre,
chacun desdits groupements ou cycles dans R³ pouvant éventuellement être substitué par un ou plusieurs substituants choisis indépendamment parmi (C1-C6)alkyle, (C1-C6) alcoxy, (C1-C6) - alcoxy (C1-C6) alkyle, (C1-C6) alcoxy (C1-C6) alcoxy, halogéno, hydroxy, trifluorométhyle, tri-[(C1-C4)alkyl]silyle, cyano, amino, (C1-C6)alkylamino, di-[(C1-C6)alkyl]amino, amino (C1-C6) alkyle, (C1-C6)alkylamino(C1-C6) alkyle, di-[(C1-C6)alkyl]-amino(C1-C6)alkyle, (C1-C6)alcoxycarbonyle, carbamoyle, (C1-C6)alkylcarbamoyle, di-[(C1-C6)-alkyl]carbamoyle, (C1-C6)alkylthio, (C1-C6)alkyl-sulfonyle, (C1-C6)alkylsulfinyle, (C1-C6)-alcanoyle, un groupement alcanoylamino -N(R^{3d})C(O)R^{3e} où R^{3d} est choisi parmi hydrogène et (C1-C6)alkyle et R^{3e} est choisi parmi un groupement (C1-C6) alkyle ou (C1-C6) alcoxy, ou un cycle monocyclique saturé à 3, 4, 5, 6 ou 7 chaînons, ledit cycle pouvant éventuellement comprendre un ou plusieurs hétéroatomes choisis parmi azote, oxygène et soufre, l'un quelconque desdits substituants pouvant éventuellement être substitué par un ou plusieurs groupements (C1-C4)alkyle, hydroxy ou cyano ;
Q¹ est un cycle hétéroaromatique à 5 ou 6 chaînons, comprenant au moins un hétéroatome du cycle choisi parmi azote, oxygène et soufre,
et où Q¹ est substitué par un ou plusieurs substituants choisis indépendamment parmi (C1-C6)-alkyle et (C1-C6)alcoxy (l'un ou l'autre desdits groupements (C1-C6) alkyle et (C1-C6)alcoxy pouvant éventuellement être substitué par un ou plusieurs substituants choisis indépendamment parmi halogéno, amino, hydroxy et trifluorométhyle), halogéno, nitro, cyano, -NR⁴R⁵, carboxy, hydroxy, (C2-C6)alcényle, (C3-C8)cycloalkyle, (C1-C6)-alcoxycarbonyle, (C1-C6)alkylcarbonyle, (C2-C6)-alcanoylamino, phénylcarbonyle, -S(O)ₙ(C1-C6)-alkyle, -C(O)NR⁶R⁷ et -SO₂NR⁸R⁹, où R⁴, R⁵, R⁶, R⁷, R⁸ et R⁹ sont choisis chacun indépendamment parmi hydrogène et (C1-C6) alkyle, ou R⁴ et R⁵, ou R⁶ et R⁷, ou R⁸ et R⁹, lorsqu'ils sont pris ensemble avec l'atome d'azote auquel ils sont liés, peuvent former chacun indépendamment un cycle hétérocyclique saturé, et n est 0, 1 ou 2,
et où un cycle monocyclique saturé quelconque porte éventuellement 1 ou 2 substituants oxo ou thioxo ;
ou un sel pharmaceutiquement acceptable de celui-ci.

3. Composé de formule (I) selon la revendication 1 ou 2, **caractérisé en ce que** R¹ est méthyle ou cyclopropyle.

4. Composé de formule (I) selon la revendication 3, **caractérisé en ce que** R¹ est méthyle.

5. Composé de formule (I) selon une ou plusieurs des revendications 1 à 4, **caractérisé en ce que** R² est hydrogène.

6. Composé de formule (I) selon une ou plusieurs des revendications 1 à 5, **caractérisé en ce que** R³ est choisi parmi hydrogène et halogéno, ou parmi un groupement (C1-C4)alkyle ou (C1-C3)alcoxy,
ou R³ est un cycle hétérocyclique monocyclique saturé à 5 ou 6 chaînons, comprenant au moins un hétéroatome du cycle choisi parmi azote et oxygène,
chacun desdits groupements ou cycles dans R³ pouvant éventuellement être substitué par un ou plusieurs substituants choisis indépendamment parmi (C1-C6)alkyle, (C1-C6)alcoxy, (C1-C6)alcoxy-(C1-C6)alkyle, (C1-C6)alcoxy(C1-C6)alcoxy, halogéno, hydroxy, trifluorométhyle, tri-[(C1-C4)-alkyl] silyle, cyano, amino, (C1-C6)alkylamino, di-[(C1-C6) alkyl] amino, amino (C1-C6) alkyle, (C1-C6)-alkylamino (C1-C6) alkyle, di-[(C1-C6)alkyl]amino-(C1-C6)alkyle, (C1-C6)alcoxycarbonyle, carbamoyle, (C1-C6)alkylcarbamoyle, di-[(C1-C6)alkyl] carbamoyle, (C1-C6) alkylthio, (C1-C6) alkylsulfonyle, (C1-C6)-alkylsulfinyle, (C1-C6)alcanoyle, un groupement alcanoylamino -N(R^{3d}) C(O) R^{3e} où R^{3d} est choisi parmi hydrogène et (C1-C6) alkyle et R^{3e} est choisi parmi un groupement (C1-C6)alkyle ou (C1-C6)alcoxy, ou un cycle monocyclique saturé à 3, 4, 5, 6 ou 7 chaînons, ledit cycle pouvant éventuellement comprendre un ou plusieurs hétéroatomes choisis parmi azote, oxygène et soufre, l'un quelconque desdits substituants pouvant éventuellement être substitué par un ou plusieurs groupements (C1-C4)alkyle, hydroxy ou cyano ;
et où un cycle monocyclique saturé quelconque dans R³ porte éventuellement 1 ou 2 substituants oxo ou thioxo.

7. Composé de formule (I) selon la revendication 6, **caractérisé en ce que** R³ est choisi parmi hydrogène et halogéno, ou parmi un groupement (C1-C3) alkyle ou (C1-C3) alcoxy,
ou R³ est un cycle hétérocyclique monocyclique saturé à 6 chaînons, comprenant au moins un hétéroatome du cycle choisi parmi azote et oxygène,
chacun desdits groupements ou cycles dans R³ pouvant éventuellement être substitué par un ou plusieurs substituants choisis indépendamment parmi (C1-C6) alkyle, (C1-C6) alcoxy, (C1-C6)-alcoxy (C1-C6) alkyle, (C1-C6) alcoxy (C1-C6) alcoxy, halogéno, hydroxy, trifluorométhyle, tri-[(C1-C4)alkyl]silyle, cyano, amino, (C1-C6)-alkylamino, di-[(C1-C6)alkyl]amino, amino (C1-C6)-alkyle, (C1-C6)alkylamino(C1-C6)alkyle, di-[(C1-C6) alkyl] amino (C1-C6) alkyle, (C1-C6)alcoxy-carbonyle, carbamoyle, (C1-C6) alkylcarbamoyle, di-[(C1-C6)alkyl]carbamoyle, (C1-C6)alkylthio, (C1-C6) alkylsulfonyle, (C1-C6) alkylsulfinyle, (C1-C6)alcanoyle, un groupement alcanoylamino -N (R^{3d}) C (O) R^{3e} où R^{3d} est choisi parmi hydrogène et (C1-C6)alkyle et R^{3e} est choisi parmi un groupement (C1-C6) alkyle ou (C1-C6) alcoxy, ou un cycle monocyclique saturé à 3, 4, 5, 6 ou 7 chaînons, ledit cycle pouvant éventuellement comprendre un ou plusieurs hétéroatomes choisis parmi azote, oxygène et soufre, l'un quelconque desdits substituants pouvant éventuellement être substitué par un ou plusieurs groupements (C1-C4)alkyle, hydroxy ou cyano ;
et où un cycle monocyclique saturé quelconque dans R³ porte éventuellement 1 ou 2 substituants oxo ou thioxo.

8. Composé de formule (I) selon la revendication 7, **caractérisé en ce que** R³ est choisi parmi hydrogène et halogéno, ou parmi un groupement (C1-C3) alkyle ou (C1-C3) alcoxy,
ou R³ est un cycle hétérocyclique monocyclique saturé à 6 chaînons, comprenant au moins un hétéroatome du cycle choisi parmi azote et oxygène,
chacun desdits groupements ou cycles dans R³ pouvant éventuellement être substitué par un ou plusieurs substituants choisis indépendamment parmi cyano, hydroxy, cyclopropyle et (C1-C3)-alcoxy, l'un quelconque desdits substituants pouvant éventuellement être substitué par un ou plusieurs groupements cyano.

9. Composé de formule (I) selon la revendication 8, **caractérisé en ce que** R³ est choisi parmi hydrogène, chloro, méthyle, éthyle, méthoxy, éthoxy, 2-hydroxyéthoxy, 2-méthoxyéthoxy, 2-hydroxypropoxy, (1-cyanocyclopropyl)méthoxy, 3-cyanopropoxy, 3-hydroxypropoxy, 3-méthoxypropoxy, 2-éthoxyéthoxy et morpholino.

10. Composé de formule (I) selon une ou plusieurs des revendications 1 à 9, **caractérisé en ce que** Q¹ est choisi parmi pyridyle, imidazolyle, isoxazolyle, pyrazolyle, furyle, pyrazinyle, pyridazinyle, pyrimidinyle, pyrrolyle, thiazolyle, oxazolyle, isothiazolyle, triazolyle, tétrahydrofuranyle et thiényle,
et où Q¹ est substitué par un ou plusieurs substituants choisis indépendamment parmi (C1-C6) alkyle et (C1-C6) alcoxy (l'un ou l'autre desdits groupements (C1-C6)alkyle et (C1-C6)alcoxy pouvant éventuellement être substitué par au moins un substituant choisi indépendamment parmi (C1-C3)alcoxy, halogéno, amino, hydroxy et trifluorométhyle), oxo, halogéno, nitro, cyano, -NR⁴R⁵, carboxy, hydroxy, (C2-C6)alcényle, (C3-C8)cycloalkyle, (C1-C6)alcoxycarbonyle, (C1-C6)alkylcarbonyle, (C2-C6)alcanoylamino, phénylcarbonyle, -S(O)ₙ(C1-C6)alkyle, -C(O)NR⁶R⁷ et -SO₂NR⁸R⁹, où R⁴, R⁵, R⁶, R⁷, R⁸ et R⁹ sont choisis chacun indépendamment parmi hydrogène et (C1-C6)alkyle, ou R⁴ et R⁵, ou R⁶ et R⁷, ou R⁸ et R⁹, lorsqu'ils sont pris ensemble avec l'atome d'azote auquel ils sont liés, peuvent former chacun indépendamment un cycle hétérocyclique saturé, et n est 0, 1 ou 2,
et où un cycle monocyclique saturé quelconque porte éventuellement 1 ou 2 substituants oxo ou thioxo.

11. Composé de formule (I) selon la revendication 10, **caractérisé en ce que** Q¹ est choisi parmi pyridyle, imidazolyle, isoxazolyle, pyrazolyle, furyle, pyrazinyle, pyridazinyle, pyrimidinyle, pyrrolyle, thiazolyle, oxazolyle, isothiazolyle, triazolyle, tétrahydrofuranyle et thiényle,
et où Q¹ est substitué par au moins un substituant choisi indépendamment parmi oxo, halogéno, hydroxy, cyano, (C1-C4)alkyle, (C1-C4)alcoxy, ledit (C1-C4)alcoxy pouvant être éventuellement substitué par (C1-C3) alcoxy et -NR⁴R⁵, où R⁴ et R⁵ sont choisis chacun indépendamment parmi hydrogène et (C1-C6)alkyle, ou R⁴ et R⁵, lorsqu'ils sont pris ensemble avec l'atome d'azote auquel ils sont liés, peuvent former chacun indépendamment un cycle hétérocyclique saturé.

12. Composé de formule (I) selon la revendication 10 ou 11, **caractérisé en ce que** Q¹ est choisi parmi pyridyle et pyrazinyle.

13. Composé de formule (I), **caractérisé en ce qu'**il est choisi parmi un ou plusieurs parmi :
la S-2-[2-{3-(3-éthoxypyrazin-2-yl)isoxazol-5-yl}pyrrolidin-1-yl]-6-méthyl-4-(5-méthyl-1*H-*pyrazol-3-ylamino)pyrimidine ;
la S-2-[2-{3-(3-éthylaminopyrazin-2-yl)isoxazol-5-yl}pyrrolidin-1-yl]-6-méthyl-4-(5-méthyl-1*H-*pyrazol-3-ylamino)pyrimidine ;
la S-2-[2-{3-(2-méthylpyrid-3-yl)isoxazol-5-yl}-pyrrolidin-1-yl]-6-méthyl-4-(5-méthyl-1*H*-pyrazol-3-ylamino)pyrimidine ; la S-6-chloro-2-[2-{3-(2-méthylpyrid-3-yl)-isoxazol-5-yl}pyrrolidin-1-yl]-4-(5-méthyl-1*H-*pyrazol-3-ylamino)pyrimidine;
la S-2-[2-{3-(2-chloropyrid-3-yl)isoxazol-5-yl}-pyrrolidin-1-yl]-6-méthyl-4-(5-méthyl-1*H*-pyrazol-3-ylamino)pyrimidine ;
la S-6-méthoxy-2-[2-{3-(2-méthylpyrid-3-yl)-isoxazol-5-yl}pyrrolidin-1-yl]-4-(5-méthyl-1*H-*pyrazol-3-ylamino)pyrimidine ;
la S-2-[2-{3-(2-cyanopyrid-3-yl)isoxazol-5-yl}-pyrrolidin-1-yl]-6-méthyl-4-(5-méthyl-1*H*-pyrazol-3-ylamino)pyrimidine ;
la S-2-[2-{3-(2-méthylpyrid-3-yl)isoxazol-5-yl}-pyrrolidin-1-yl]-4-(5-méthyl-1*H*-pyrazol-3-ylamino)-6-morpholinopyrimidine ;
la S-4-(5-cyclopropyl-1*H*-pyrazol-3-ylamino)-2-[2-{3-(2-méthoxypyrid-3-yl)isoxazol-5-yl}pyrrolidin-1-yl]-6-morpholinopyrimidine ;
la S-2-[2-{3-(2-méthoxypyrid-3-yl)isoxazol-5-yl}-pyrrolidin-1-yl]-4-(5-méthyl-1*H*-pyrazol-3-ylamino-6-morpholinopyrimidine ;
la S-6-chloro-2-[2-{3-(2-cyanopyrid-3-yl)isoxazol-5-yl}pyrrolidin-1-yl]-4-(5-méthyl-1*H*-pyrazol-3-ylamino)pyrimidine;
la S-2-[2-{3-(2-cyanopyrid-3-yl)isoxazol-5-yl}-pyrrolidin-1-yl]-4-(5-méthyl-1*H*-pyrazol-3-ylamino)-6-morpholinopyrimidine;
la S-2-[2-{3-(2-chloropyrid-3-yl)isoxazol-5-yl}-pyrrolidin-1-yl]-4-(5-méthyl-1*H*-pyrazol-3-ylamino)pyrimidine ;
la S-2-[2-{3-(2-méthylaminopyrid-3-yl)isoxazol-5-yl}pyrrolidin-1-yl]-4-(5-méthyl-1*H*-pyrazol-3-yl-amino)pyrimidine ;
la S-6-méthyl-2-[2-{3-(2-méthylaminopyrid-3-yl)-isoxazol-5-yl}pyrrolidin-1-yl]-4-(5-méthyl-1*H-*pyrazol-3-ylamino)pyrimidine ;
la S-2-[2-{3-(2-méthylpyrid-3-yl)isoxazol-5-yl}-pyrrolidin-1-yl]-4-(5-méthyl-1*H*-pyrazol-3-yl-amino) pyrimidine ;
la S-6-(2-méthoxyéthoxy)-2-[2-{3-(2-méthylpyrid-3-yl)isoxazol-5-yl}pyrrolidin-1-yl]-4-(5-méthyl-1*H-*pyrazol-3-ylamino)pyrimidine ;
la S-6-chloro-2-[2-{3-(3-éthylpyrazin-2-yl)-isoxazol-5-yl}pyrrolidin-1-yl]-4-(5-méthyl-1*H-*pyrazol-3-ylamino)pyrimidine ;
la S-6-(2-méthoxyéthoxy)-2-[2-{3-(3-éthylpyrazin-2-yl)isoxazol-5-yl}pyrrolidin-1-yl]-4-(5-méthyl-1*H*-pyrazol-3-ylamino)pyrimidine ;
la S-6-méthoxy-2-[2-{3-(3-éthylpyrazin-2-yl)-isoxazol-5-yl}-pyrrolidin-1-yl]-4-(5-méthyl-1*H-*pyrazol-3-ylamino)pyrimidine ;
la S-6-(2-hydroxyéthoxy)-2-[2-{3-(2-méthylpyrid-3-yl)isoxazol-5-yl}pyrrolidin-1-yl]-4-(5-méthyl-1*H-*pyrazol-3-ylamino)pyrimidine ;
la S-6-(3-hydroxypropoxy)-2-[2-{3-(2-méthylpyrid-3-yl)isoxazol-5-yl}pyrrolidin-1-yl]-4-(5-méthyl-1*H*-pyrazol-3-ylamino)pyrimidine ;
la 6-((2R)-2-hydroxypropoxy)-2-[(2S)-2-{3-(2-méthylpyrid-3-yl)isoxazol-5-yl}pyrrolidin-1-yl]-4-(5-méthyl-1*H*-pyrazol-3-ylamino)pyrimidine ;
la S-6-chloro-2-[2-{3-(1-oxopyrid-3-yl)isoxazol-5-yl}pyrrolidin-1-yl]-4-(5-méthyl-1*H*-pyrazol-3-ylamino)pyrimidine ;
la S-6-(2-méthoxyéthoxy)-2-[2-{3-(1-oxopyrid-3-yl)isoxazol-5-yl}pyrrolidin-1-yl]-4-(5-méthyl-1*H-*pyrazol-3-ylamino)pyrimidine ;
la S-6-(2-méthoxyéthoxy)-2-[2-{3-(2-cyanopyrid-3-yl)isoxazol-5-yl}pyrrolidin-1-yl]-4-(5-méthyl-1*H-*pyrazol-3-ylamino)pyrimidine ;
la S-6-(1-cyanocyclopropyl)méthoxy-2-[2-{3-(2-méthylpyrid-3-yl)isoxazol-5-yl}pyrrolidin-1-yl]-4-(5-méthyl-1*H*-pyrazol-3-ylamino)pyrimidine ;
la S-6-(3-cyanopropoxy)-2-[2-{3-(2-méthylpyrid-3-yl)isoxazol-5-yl}pyrrolidin-1-yl]-4-(5-méthyl-1*H-*pyrazol-3-ylamino)pyrimidine ;
la S-6-méthoxy-2-[2-{3-(1-oxopyrid-3-yl)isoxazol-5-yl}pyrrolidin-1-yl]-4-(5-méthyl-1*H*-pyrazol-3-ylamino) pyrimidine ;
la S-6-méthoxy-2-[2-{3-(2-cyanopyrid-3-yl)-isoxazol-5-yl}pyrrolidin-1-yl]-4-(5-méthyl-1*H-*pyrazol-3-ylamino) pyrimidine ;
la S-6-chloro-2-[2-{3-(3-méthylpyrazin-2-yl)-isoxazol-5-yl}pyrrolidin-1-yl]-4-(5-méthyl-1*H-*pyrazol-3-ylamino) pyrimidine ;
la S-6-méthoxy-2-[2-{3-(3-méthylpyrazin-2-yl)-isoxazol-5-yl}pyrrolidin-1-yl]-4-(5-méthyl-1*H-*pyrazol-3-ylamino)pyrimidine ;
la S-6-éthyl-2-[2-{3-(3-méthylpyrazin-2-yl)-isoxazol-5-yl}pyrrolidin-1-yl]-4-(5-méthyl-1*H-*pyrazol-3-ylamino) pyrimidine ;
la S-6-éthyl-2-[2-{3-(2-cyanopyrid-3-yl) isoxazol-5-yl}pyrrolidin-1-yl]-4-(5-méthyl-1*H*-pyrazol-3-ylamino)pyrimidine ;
la S-6-(2-méthoxyéthoxy)-2-[2-{3-(3-méthylpyrazin-2-yl)isoxazol-5-yl}pyrrolidin-1-yl]-4-(5-méthyl-1*H*-pyrazol-3-ylamino)pyrimidine ;
la S-6-(2-éthoxy)-2-[2-{3-(3-méthylpyrazin-2-yl)-isoxazol-5-yl}pyrrolidin-1-yl]-4-(5-méthyl-1*H-*pyrazol-3-ylamino)pyrimidine ;
la S-6-(3-méthoxypropoxy)-2-[2-{3-(3-méthyl-pyrazin-2-yl)isoxazol-5-yl}pyrrolidin-1-yl]-4-(5-méthyl-1*H*-pyrazol-3-ylamino)pyrimidine ;
la S-6-(2-éthoxyéthoxy)-2-[2-{3-(3-méthylpyrazin-2-yl)isoxazol-5-yl}pyrrolidin-1-yl]-4-(5-méthyl-1*H*-pyrazol-3-ylamino)pyrimidine ;
la S-6-[(1-cyanocyclopropyl)méthoxy]-2-[2-{3-(3-méthylpyrazin-2-yl)isoxazol-5-yl}pyrrolidin-1-yl]-4-(5-méthyl-1*H*-pyrazol-3-ylamino)pyrimidine ;
la S-6-(3-cyanopropoxy)-2-[2-{3-(3-méthylpyrazin-2-yl)isoxazol-5-yl}pyrrolidin-1-yl]-4-(5-méthyl-1*H*-pyrazol-3-ylamino)pyrimidine ;
la S-6-méthoxy-2-[2-{3-(3-méthoxypyrazin-2-yl)-isoxazol-5-yl}pyrrolidin-1-yl]-4-(5-méthyl-1*H-*pyrazol-3-ylamino)pyrimidine ;
la S-6-(2-méthoxyéthoxy)-2-[2-{3-(3-(2-méthoxyéthoxy)pyrazin-2-yl)isoxazol-5-yl}pyrrolidin-1-yl]-4-(5-méthyl-1*H*-pyrazol-3-ylamino)pyrimidine ;
la S-6-(2-méthoxyéthoxy)-2-[2-{3-(3-hydroxy-pyrazin-2-yl)isoxazol-5-yl}pyrrolidin-1-yl]-4-(5-méthyl-1*H*-pyrazol-3-ylamino) pyrimidine ;
la S-6-(2-méthoxyéthoxy)-2-[2-{3-(4-cyanopyrid-3-yl)isoxazol-5-yl}pyrrolidin-1-yl]4-(5-méthyl-1*H-*pyrazol-3-ylamino) pyrimidine ; et
la S-6-méthoxy-2-[2-{3-(2-cyanopyrid-3-yl)-isoxazol-5-yl}pyrrolidin-1-yl]-4-(5-méthyl-1*H-*pyrazol-3-ylamino) pyrimidine ;
et des sels pharmaceutiquement acceptables de celles-ci.

14. Composition pharmaceutique qui comprend un composé de formule (I), ou un sel pharmaceutiquement acceptable de celui-ci, selon une ou plusieurs des revendications 1 à 13, en association avec un adjuvant, un diluant ou un support pharmaceutiquement acceptable.

15. Produit pharmaceutique qui comprend un composé de formule (I), ou un sel pharmaceutiquement acceptable de celui-ci, selon une ou plusieurs des revendications 1 à 13, et un agent antitumoral supplémentaire pour le traitement conjoint du cancer.

16. Composé de formule (I), ou un sel pharmaceutiquement acceptable de celui-ci, selon une ou plusieurs des revendications 1 à 13, destiné à être utilisé comme médicament.

17. Utilisation d'un composé de formule (I), ou d'un sel pharmaceutiquement acceptable de celui-ci, selon une ou plusieurs des revendications 1 à 13, dans la fabrication d'un médicament destiné à être utilisé dans la production d'un effet anti-prolifératif chez un animal à sang chaud.

18. Utilisation d'un composé de formule (I), ou d'un sel pharmaceutiquement acceptable de celui-ci, selon une ou plusieurs des revendications 1 à 13, dans la fabrication d'un médicament destiné à être utilisé dans le traitement d'une maladie ou d'une affection médicale médiée uniquement ou en partie par IGF-1R tyrosine kinase chez un animal à sang chaud.

19. Utilisation d'un composé de formule (I), ou d'un sel pharmaceutiquement acceptable de celui-ci, selon une ou plusieurs des revendications 1 à 13, dans la fabrication d'un médicament destiné à être utilisé dans la prévention ou le traitement des tumeurs qui sont sensibles à l'inhibition de IGF-1R tyrosine kinase impliquée dans les étapes de transduction de signal qui conduisent à la prolifération des cellules tumorales chez un animal à sang chaud.

20. Utilisation d'un composé de formule (I), ou d'un sel pharmaceutiquement acceptable de celui-ci, selon une ou plusieurs des revendications 1 à 13, dans la fabrication d'un médicament destiné au traitement du cancer chez un animal à sang chaud.

21. Procédé de préparation d'un composé de formule (I), ou d'un sel pharmaceutiquement acceptable de celui-ci, selon la revendication 1, qui comprend :
(a) la réaction, commodément en présence d'une base convenable, d'un composé de formule (II) : 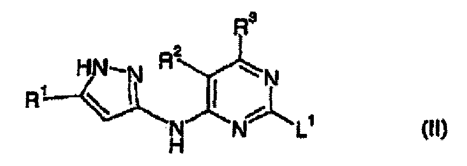 dans laquelle L¹ représente un groupement déplaçable convenable et R¹, R² et R³ sont tels que définis dans la revendication 1, sauf que l'on protège un groupe fonctionnel quelconque, le cas échéant, avec un composé de formule (III) : 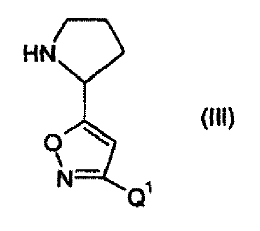 dans laquelle Q¹ est tel que défini dans la revendication 1, sauf que l'on protège un groupe fonctionnel quelconque, le cas échéant ; ou
(b) la réaction, commodément en présence d'un acide convenable, d'un composé de formule (IV) : 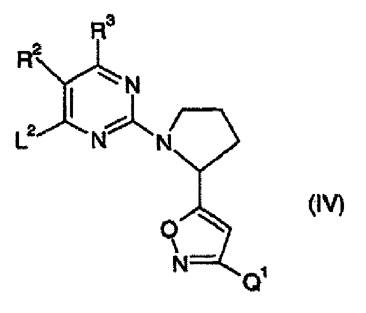 dans laquelle L² est un groupement déplaçable convenable et R², R³ et Q¹ sont tels que définis dans la revendication 1, sauf que l'on protège un groupe fonctionnel quelconque, le cas échéant, avec un pyrazole de formule (V) : 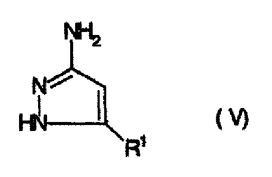 dans laquelle R¹ est tel que défini dans la revendication 1, sauf que l'on protège un groupe fonctionnel quelconque, le cas échéant ; ou
(c) la réaction, commodément en présence d'une base convenable, d'un composé de formule (VI) : 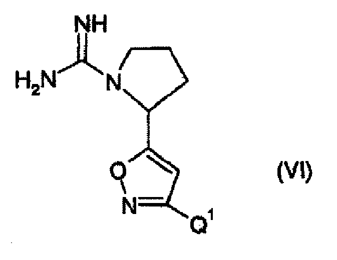 dans laquelle Q¹ est tel que défini dans la revendication 1, sauf que l'on protège un groupe fonctionnel quelconque, le cas échéant, avec un composé de formule (VII) : 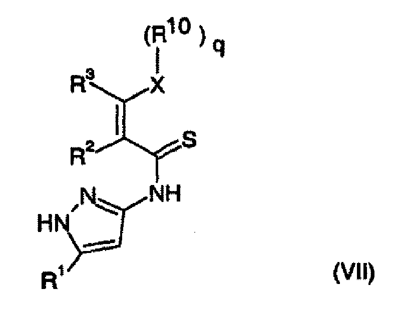 dans laquelle X représente un atome d'oxygène et q est 1 ou X représente un atome d'azote et q est 2, R¹⁰ est un groupement (C1-C6) - alkyle et R¹, R² et R³ sont tels que définis dans la revendication 1, sauf que l'on protège un groupe fonctionnel quelconque, le cas échéant ; ou
(d) la réaction d'un composé de formule (VIII) : 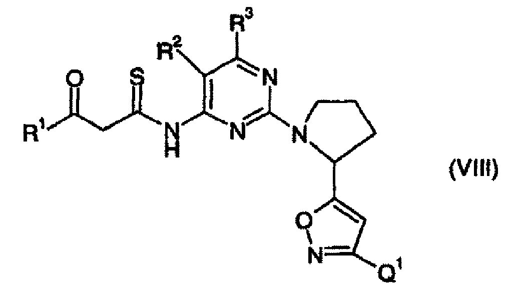 dans laquelle R¹, R², R³ et Q¹ sont tels que définis dans la revendication 1, sauf que l'on protège un groupe fonctionnel quelconque, le cas échéant, avec de l'hydrazine ; ou
(e) pour des composés de formule (I) dans laquelle R³ est un groupement (C1-C6)alcoxy, amino, (C1-C6)alkylamino, di-[(C1-C6)alkyl]-amino, -NHR^{3b} ou -SR^{3a}, où R^{3a} et R^{3b} sont tels que définis dans la revendication 1 (et le groupement R³ est éventuellement substitué par au moins un groupement tel que défini dans la revendication 1), la réaction, commodément en présence d'une base convenable, d'un composé de formule (IX) : 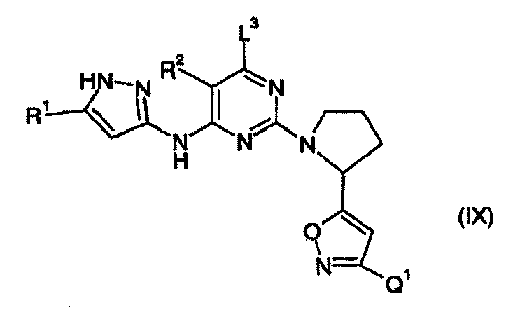 dans laquelle L³ est un groupement déplaçable convenable et R¹, R² et Q¹ sont tels que définis dans la revendication 1, sauf que l'on protège un groupe fonctionnel quelconque, le cas échéant, avec un composé de formule :
H-Xa
dans laquelle Xa représente OR¹¹, NH₂, NHR¹¹, N (R¹¹) ₂, NHR^{3b} ou SR^{3a}, dans laquelle R¹¹ est un groupement (C1-C6)alkyle éventuellement substitué et R^{3a} et R^{3b} sont chacun tels que définis dans la revendication 1, sauf que l'on protège un groupe fonctionnel quelconque, le cas échéant ; ou
(f) pour des composés de formule (I) dans laquelle R³ est un cycle hétérocyclique monocyclique saturé à 5 ou 6 chaînons, éventuellement substitué, comprenant au moins un atome d'azote du cycle et, éventuellement, un ou plusieurs hétéroatomes supplémentaires choisis parmi azote, oxygène et soufre, la réaction, commodément en présence d'une base convenable, d'un composé de formule (IX) : 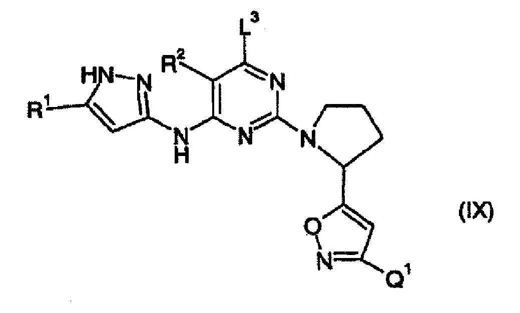 dans laquelle L³ est un groupement déplaçable convenable et R¹, R² et Q¹ sont tels que définis dans la revendication 1, sauf que l'on protège un groupe fonctionnel quelconque, le cas échéant, avec un composé de formule (Xb) : 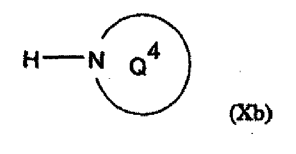 dans laquelle Q⁴ est un cycle hétérocyclique monocyclique saturé à 5 ou 6 chaînons, comprenant éventuellement un ou plusieurs hétéroatomes choisis parmi azote, oxygène et soufre, outre l'atome d'azote montré ci-dessus, ledit cycle étant éventuellement substitué par au moins un groupement tel que défini dans la revendication 1 ; ou
(g) pour des composés de formule (I) dans laquelle R³ est un groupement (C2-C6)alcényle ou (C2-C6)alcynyle (et le groupement R³ est éventuellement substitué par au moins un groupement tel que défini dans la revendication 1), la réaction, commodément en présence d'une base convenable et d'un catalyseur convenable, d'un composé de formule (IX) : 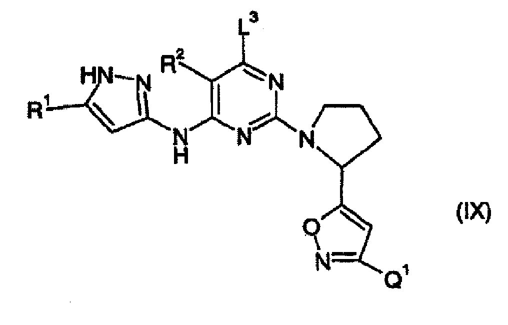 dans laquelle L³ est un groupement déplaçable convenable et R¹, R² et Q¹ sont tels que définis dans la revendication 1, sauf que l'on protège un groupe fonctionnel quelconque, le cas échéant, avec un composé de formule (Xc) ou de formule (Xc') :
H-C≡C-R¹² (Xc) 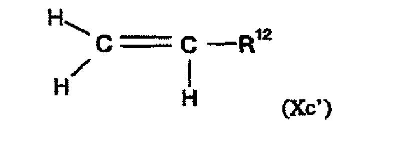
dans lesquelles R¹² est choisi parmi hydrogène et un groupement (C1-C4)alkyle ou (C1-C4)-alcoxycarbonyle éventuellement substitué ; ou
(h) pour des composés de formule (I) dans laquelle R³ est lié au cycle pyrimidine par un atome de carbone, la réaction, commodément en présence d'un catalyseur convenable, d'un composé de formule (IX) : 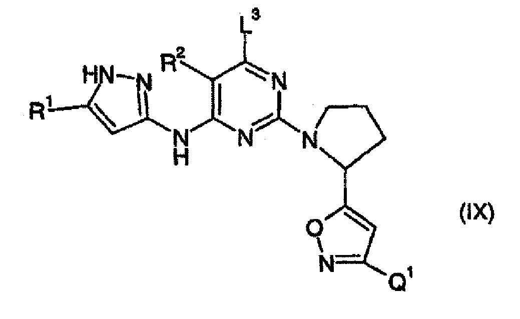 dans laquelle L³ est un groupement déplaçable convenable et R¹, R² et Q¹ sont tels que définis dans la revendication 1, sauf que l'on protège un groupe fonctionnel quelconque, le cas échéant, avec un composé de formule M-R³, dans laquelle R³ est choisi, de manière appropriée, parmi les groupements R³ tels que définis dans la revendication 1 et M est un groupement métallique ; ou
(i) pour des composés de formule (I) dans laquelle R³ est un groupement (C1-C6)alcoxy-carbonyle (et le groupement R³ est éventuellement substitué par au moins un groupement tel que défini dans la revendication 1), la réaction, commodément en présence d'un acide convenable, d'un composé de formule (X) : 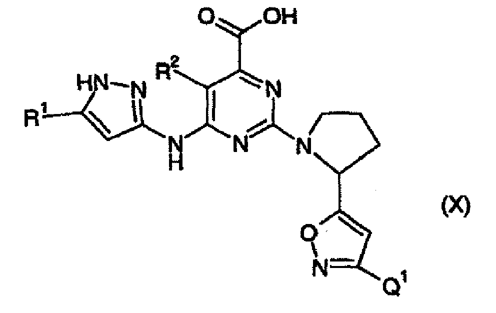 dans laquelle R¹, R² et Q¹ sont tels que définis dans la revendication 1, sauf que l'on protège un groupe fonctionnel quelconque, le cas échéant, avec un composé de formule :
H-O-(C1-C6) alkyle
dans laquelle le groupement (C1-C6)alkyle est éventuellement substitué par au moins un groupement tel que défini dans la revendication 1 en tant que substituant pour R³ et on protège un groupe fonctionnel quelconque, le cas échéant ; ou
(j) pour des composés de formule (I) dans laquelle R³ est un groupement (C1-C6) alkyle, (C3-C6)alcényle, (C3-C6)alcynyle ou (C1-C6) - alcoxy substitué par au moins un groupement tel que défini dans la revendication 1, la réaction d'un composé de formule (XI) : 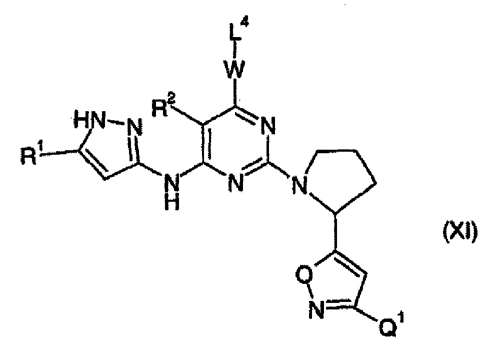 dans laquelle L⁴ est un groupement déplaçable convenable, W est un groupement (C1-C6)-alkyle, (C3-C6)alcényle, (C3-C6)alcynyle ou (C1-C6) alcoxy éventuellement substitué, et R¹, R² et Q¹ sont tels que définis dans la revendication 1, sauf que l'on protège un groupe fonctionnel quelconque, le cas échéant, avec un composé de formule H-Xa, (Xb), (Xc), (Xc') ou M-R³ ; ou
(k) pour des composés de formule (I) dans laquelle Q¹ est un groupement pyridyle contenant un substituant en position ortho ou para du cycle par rapport à l'azote de pyridine (et contenant éventuellement un ou plusieurs substituants supplémentaires tels que définis dans la revendication 1), la réaction d'un composé de formule (XII) : 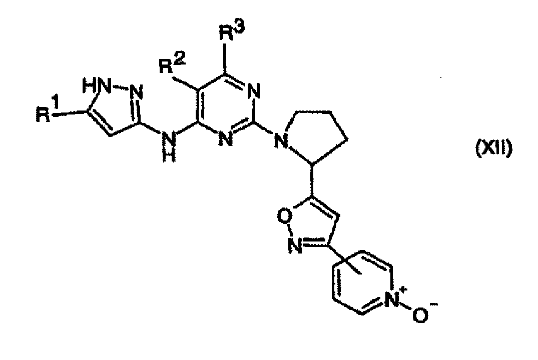
dans laquelle R¹, R² et R³ sont tels que définis dans la revendication 1, sauf que l'on protège un groupe fonctionnel quelconque, le cas échéant, avec un réactif ou des réactifs qui combinent un agent d'acylation, de phosphorylation, de sulfonation, de sulfénylation ou de silylation avec un nucléophile,
et éventuellement, après les procédés (a), (b), (c), (d), (e), (f), (g), (h), (i), (j) ou (k), la mise en oeuvre d'une ou de plusieurs des étapes suivantes:
• la transformation du composé obtenu en un autre composé de l'invention,
• la formation d'un sel pharmaceutiquement acceptable du composé.
